(19) Europäisches Patentamt
European Patent Office
Office européen des brevets

(11) EP 3 138 563 A1

(12) EUROPEAN PATENT APPLICATION

(43) Date of publication:
08.03.2017 Bulletin 2017/10

(21) Application number: 16192470.9

(22) Date of filing: 22.04.2011

(51) Int Cl.:
*A61K 31/4704* (2006.01) *A61K 31/404* (2006.01)
*A61K 31/443* (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 22.04.2010 US 327078 P
22.04.2010 US 327091 P
29.04.2010 US 329510 P

(62) Document number(s) of the earlier application(s) in accordance with Art. 76 EPC:
11718596.7 / 2 560 649

(71) Applicant: Vertex Pharmaceuticals Inc.
Boston, MA 02210 (US)

(72) Inventors:
- VAN GOOR, Fredrick F.
San Diego, CA California 92109 (US)
- ALARGOVA, Rossitza Gueorguieva
Brighton, MA Massachusetts 02135 (US)
- ALCACIO, Tim Edward
San Diego, CA California 92108 (US)
- AREKAR, Sneha G.
Brighton, MA Massachusetts 02135 (US)
- JOHNSTON, Steven C.
Lichtfield, NH New Hampshire 03052 (US)
- KADIYALA, Irina Nikolaevna
Newton, MA Massachusetts 02457 (US)
- KESHAVARZ-SHOKRI, Ali
San Diego, CA California 92130 (US)
- KRAWIEC, Mariusz
Marlborough, MA Massachusetts 01752 (US)
- LEE, Elaine, Chungmin
Cambridge, MA Massachusetts 02142 (US)
- MEDEK, Ales
Winchester, MA Massachusetts 01890 (US)
- MUDUNURI, Praveen
Waltham, MA Massachusetts 02453 (US)
- SULLIVAN, Mark Jeffrey
Framingham, MA Massachusetts 01702 (US)
- ZAMAN, Noreen Tasneem
Acton, MA Massachusetts 01720 (US)
- ZHANG, Beili
San Diego, CA California 92130 (US)
- ZHANG, Yuegang
Wayland, MA Massachusetts 01778 (US)
- ZLOKARNIK, Gregor
La Jolla, CA California 92037 (US)

(74) Representative: Carpmaels & Ransford LLP
One Southampton Row
London WC1B 5HA (GB)

Remarks:
This application was filed on 05-10-2016 as a divisional application to the application mentioned under INID code 62.

# (54) PHARMACEUTICAL COMPOSITIONS AND ADMINISTRATIONS THEREOF

(57) The present invention relates to pharmaceutical compositions comprising a compound of Formula I in combination with one or both of a Compound of Formula II and/or a Compound of Formula III. The invention also relates to solid forms and to pharmaceutical formulations thereof, and to methods of using such compositions in the treatment of CFTR mediated diseases, particularly cystic fibrosis.



Printed by Jouve, 75001 PARIS (FR)

(Cont. next page)

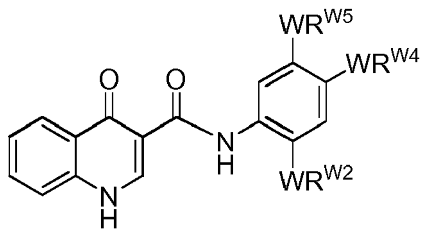

Formula I

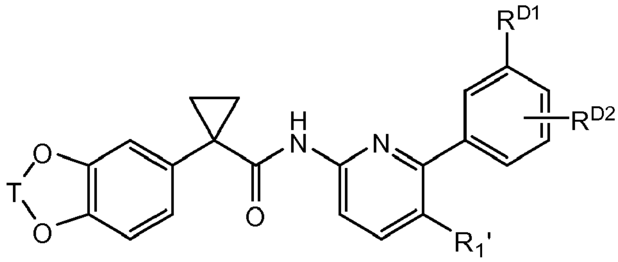

Formula II

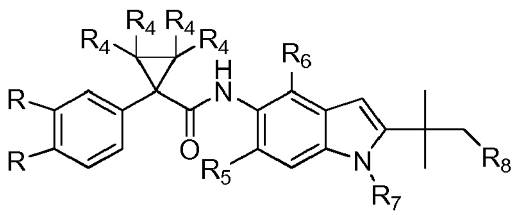

Formula III

## Description

### CLAIM OF PRIORITY

This application claims priority to United States provisional application 61/327,078, filed on April 22, 2010, United States provisional application 61/327,091, filed on April 22, 2010, and United States provisional application 61/329,510, filed on April 29, 2010. The entire contents of the priority applications are incorporated by reference.

### FIELD OF THE INVENTION

The present invention relates to pharmaceutical compositions comprising a compound of Formula I in combination with one or both of a Compound of Formula II and/or a Compound of Formula III. The invention also relates to solid forms and to pharmaceutical formulations thereof, and to methods of using such compositions in the treatment of CFTR mediated diseases, particularly cystic fibrosis.

### BACKGROUND

Cystic fibrosis (CF) is a recessive genetic disease that affects approximately 30,000 children and adults in the United States and approximately 30,000 children and adults in Europe. Despite progress in the treatment of CF, there is no cure.

CF is caused by mutations in the cystic fibrosis transmembrane conductance regulator (CFTR) gene that encodes an epithelial chloride ion channel responsible for aiding in the regulation of salt and water absorption and secretion in various tissues. Small molecule drugs, known as potentiators that increase the probability of CFTR channel opening, represent one potential therapeutic strategy to treat CF. Potentiators of this type are disclosed in WO 2006/002421, which is herein incorporated by reference in its entirety. Another potential therapeutic strategy involves small molecule drugs known as CF correctors that increase the number and function of CFTR channels. Correctors of this type are disclosed in WO 2005/075435, which are herein incorporated by reference in their entirety.

Specifically, CFTR is a cAMP/ATP-mediated anion channel that is expressed in a variety of cells types, including absorptive and secretory epithelia cells, where it regulates anion flux across the membrane, as well as the activity of other ion channels and proteins. In epithelia cells, normal functioning of CFTR is critical for the maintenance of electrolyte transport throughout the body, including respiratory and digestive tissue. CFTR is composed of approximately 1480 amino acids that encode a protein made up of a tandem repeat of transmembrane domains, each containing six transmembrane helices and a nucleotide binding domain. The two transmembrane domains are linked by a large, polar, regulatory (R)-domain with multiple phosphorylation sites that regulate channel activity and cellular trafficking.

The gene encoding CFTR has been identified and sequenced (See Gregory, R. J. et al. (1990) Nature 347:382-386; Rich, D. P. et al. (1990) Nature 347:358-362), (Riordan, J. R. et al. (1989) Science 245:1066-1073). A defect in this gene causes mutations in CFTR resulting in cystic fibrosis ("CF"), the most common fatal genetic disease in humans. Cystic fibrosis affects approximately one in every 2,500 infants in the United States. Within the general United States population, up to 10 million people carry a single copy of the defective gene without apparent ill effects. In contrast, individuals with two copies of the CF associated gene suffer from the debilitating and fatal effects of CF, including chronic lung disease.

In patients with CF, mutations in CFTR endogenously expressed in respiratory epithelia leads to reduced apical anion secretion causing an imbalance in ion and fluid transport. The resulting decrease in anion transport contributes to enhanced mucus accumulation in the lung and the accompanying microbial infections that ultimately cause death in CF patients. In addition to respiratory disease, CF patients typically suffer from gastrointestinal problems and pancreatic insufficiency that, if left untreated, results in death. In addition, the majority of males with cystic fibrosis are infertile and fertility is decreased among females with cystic fibrosis. In contrast to the severe effects of two copies of the CF associated gene, individuals with a single copy of the CF associated gene exhibit increased resistance to cholera and to dehydration resulting from diarrhea - perhaps explaining the relatively high frequency of the CF gene within the population.

Sequence analysis of the CFTR gene of CF chromosomes has revealed a variety of disease causing mutations (Cutting, G. R. et al. (1990) Nature 346:366-369; Dean, M. et al. (1990) Cell 61:863:870; and Kerem, B-S. et al. (1989) Science 245:1073-1080; Kerem, B-S et al. (1990) Proc. Natl. Acad. Sci. USA 87:8447-8451). To date, greater than 1000 disease causing mutations in the CF gene have been identified (http://www.genet.sickkids.on.ca/cftr/app). The most prevalent mutation is a deletion of phenylalanine at position 508 of the CFTR amino acid sequence, and is commonly referred to as ΔF508-CFTR. This mutation occurs in approximately 70% of the cases of cystic fibrosis and is associated with a severe disease.

The deletion of residue 508 in ΔF508-CFTR prevents the nascent protein from folding correctly. This results in the inability of the mutant protein to exit the ER, and traffic to the plasma membrane. As a result, the number of channels

present in the membrane is far less than observed in cells expressing wild-type CFTR. In addition to impaired trafficking, the mutation results in defective channel gating. Together, the reduced number of channels in the membrane and the defective gating lead to reduced anion transport across epithelia leading to defective ion and fluid transport. (Quinton, P. M. (1990), FASEB J. 4: 2709-2727). Studies have shown, however, that the reduced numbers of ΔF508-CFTR in the membrane are functional, albeit less than wild-type CFTR. (Dalemans et al. (1991), Nature Lond. 354: 526-528; Denning et al., supra; Pasyk and Foskett (1995), J. Cell. Biochem. 270: 12347-50). In addition to ΔF508-CFTR, other disease causing mutations in CFTR that result in defective trafficking, synthesis, and/or channel gating could be up- or down-regulated to alter anion secretion and modify disease progression and/or severity.

Although CFTR transports a variety of molecules in addition to anions, it is clear that this role (the transport of anions) represents one element in an important mechanism of transporting ions and water across the epithelium. The other elements include the epithelial $Na^+$ channel, ENaC, $Na^+/2Cl^-/K^+$ co-transporter, $Na^+$-$K^+$-ATPase pump and the basolateral membrane $K^+$ channels, that are responsible for the uptake of chloride into the cell.

These elements work together to achieve directional transport across the epithelium via their selective expression and localization within the cell. Chloride absorption takes place by the coordinated activity of ENaC and CFTR present on the apical membrane and the $Na^+$-$K^+$-ATPase pump and $Cl^-$ ion channels expressed on the basolateral surface of the cell. Secondary active transport of chloride from the luminal side leads to the accumulation of intracellular chloride, which can then passively leave the cell via $Cl^-$ channels, resulting in a vectorial transport. Arrangement of $Na^+/2Cl^-/K^+$ co-transporter, $Na^+$-$K^+$-ATPase pump and the basolateral membrane $K^+$ channels on the basolateral surface and CFTR on the luminal side coordinate the secretion of chloride via CFTR on the luminal side. Because water is probably never actively transported itself, its flow across epithelia depends on tiny transepithelial osmotic gradients generated by the bulk flow of sodium and chloride.

As discussed above, it is believed that the deletion of residue 508 in ΔF508-CFTR prevents the nascent protein from folding correctly, resulting in the inability of this mutant protein to exit the ER, and traffic to the plasma membrane. As a result, insufficient amounts of the mature protein are present at the plasma membrane and chloride transport within epithelial tissues is significantly reduced. In fact, this cellular phenomenon of defective ER processing of ABC transporters by the ER machinery has been shown to be the underlying basis not only for CF disease, but for a wide range of other isolated and inherited diseases.

Compounds which are potentiators of CFTR protein, such as those of Formula I, and compounds which are correctors of CFTR protein, such as those of Formula II or Formula III, have been shown independently to have utility in the treatment of CFTR modulated diseases, such as Cystic Fibrosis.

Accordingly, there is a need for novel treatments of CFTR mediated diseases which involve CFTR corrector and potentiator compounds.

Particularly, there is a need for combination therapies to treat CFTR mediated diseases, such as Cystic Fibrosis, which include CFTR potentiator and corrector compounds.

More particularly, there is a need for combination therapies to treat CFTR mediated diseases, such as Cystic Fibrosis, which include CFTR potentiator compounds, such as compounds of Formula I, in combination with CFTR corrector compounds such as compounds of Formula II and/or Formula III.

Even more particularly, there is a need for combination therapies to treat CFTR mediated diseases, such as Cystic Fibrosis, comprising CFTR potentiator compounds, such as Compound 1, in combination with CFTR corrector compounds, such as Compound 2 and/or Compound 3.

## SUMMARY OF THE INVENTION

These and other needs are met by the present invention which is directed to pharmaceutical compositions comprising:

A Compound of Formula I

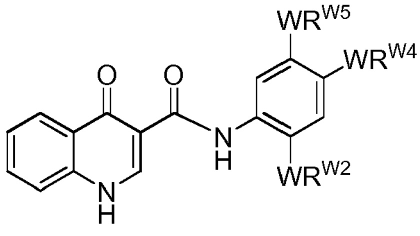

**Formula I**

or pharmaceutically acceptable salts thereof, wherein:

Each of $WR^{W2}$ and $WR^{W4}$ is independently selected from CN, $CF_3$, halo, $C_{2-6}$ straight or branched alkyl, $C_{3-12}$ membered cycloaliphatic, phenyl, a 5-10 membered heteroaryl or 3-7 membered heterocyclic, wherein said heteroaryl or heterocyclic has up to 3 heteroatoms selected from O, S, or N, wherein said $WR^{W2}$ and $WR^{W4}$ is independently and optionally substituted with up to three substituents selected from -OR', $-CF_3$, $-OCF_3$, SR', S(O)R', $SO_2R'$, $-SCF_3$, halo, CN, -COOR', -COR', $-O(CH_2)_2N(R')_2$, $-O(CH_2)N(R')_2$,$-CON(R')_2$, $-(CH_2)_2OR'$, $-(CH_2)OR'$, $-CH_2CN$, optionally substituted phenyl or phenoxy,$-N(R')_2$, -NR'C(O)OR', -NR'C(O)R', $-(CH_2)_2N(R')_2$, or $-(CH_2)N(R')_2$;
$WR^{W5}$ is selected from hydrogen, $-OCF_3$, $-CF_3$, -OH, $-OCH_3$, $-NH_2$, -CN, $-CHF_2$, -NHR', $-N(R')_2$, -NHC(O)R', -NHC(O)OR', $-NHSO_2R'$, $-CH_2OH$, $-CH_2N(R')_2$, -C(O)OR', $-SO_2NHR'$, $-SO_2N(R')_2$, or $-CH_2NHC(O)OR'$; and
Each R' is independently selected from an optionally substituted group selected from a $C_{1-8}$ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;

provided that:

i) $WR^{W2}$ and $WR^{W4}$ are not both -Cl; $WR^{W2}$, $WR^{W4}$ and $WR^{W5}$ are not $-OCH_2CH_2Ph$, $-OCH_2CH_2$(2-trifluoromethyl-phenyl),$-OCH_2CH_2$-(6,7-dimethoxy-1,2,3,4-tetrahydroisoquinolin-2-yl), or substituted 1*H*-pyrazol-3-yl;

in combination with one or both of:
A Compound of Formula II

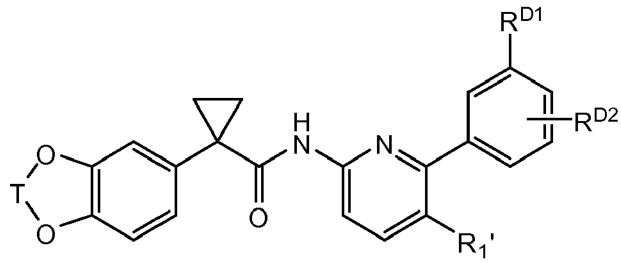

Formula II

or pharmaceutically acceptable salts thereof, wherein:

T is $-CH_2-$, $-CH_2CH_2-$, $-CF_2-$, $-C(CH_3)_2-$, or -C(O)-;
$R_1'$ is H, $C_{1-6}$ aliphatic, halo, $CF_3$, $CHF_2$, $O(C_{1-6}$ aliphatic); and
$R^{D1}$ or $R^{D2}$ is $Z^DR_9$
wherein:

$Z^D$ is a bond, CONH, $SO_2NH$, $SO_2N(C_{1-6}$ alkyl), $CH_2NHSO_2$, $CH_2N(CH_3)SO_2$, $CH_2NHCO$, COO, $SO_2$, or CO; and
$R_9$ is H, $C_{1-6}$ aliphatic, or aryl; and/or

A Compound of Formula III

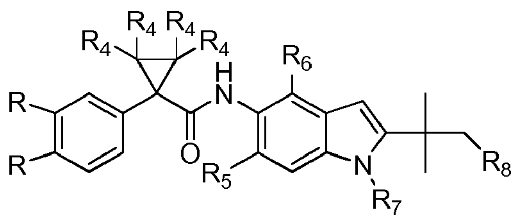

Formula III

or pharmaceutically acceptable salts thereof, wherein:

R is H, OH, $OCH_3$ or two R taken together form $-OCH_2O-$ or $-OCF_2O-$;
$R_4$ is H or alkyl;
$R_5$ is H or F;
$R_6$ is H or CN;
$R_7$ is H, $-CH_2CH(OH)CH_2OH$, $-CH_2CH_2N^+(CH_3)_3$, or $-CH_2CH_2OH$;
$R_8$ is H, OH, $-CH_2CH(OH)CH_2OH$, $-CH_2OH$, or $R_7$ and $R_8$ taken together form a five membered ring.

In another aspect, the pharmaceutical composition comprises Compound 1

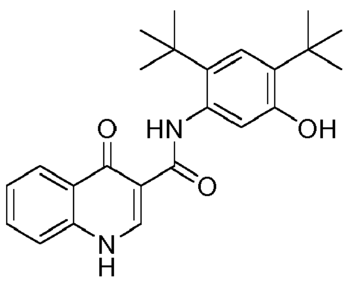

Compound 1

in combination with Compound 2 and/or Compound 3.

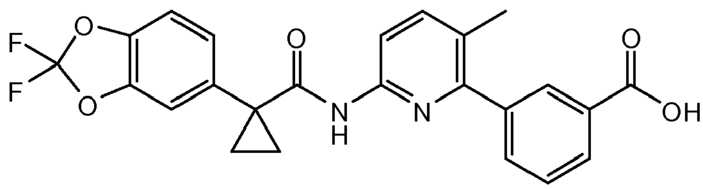

Compound 2

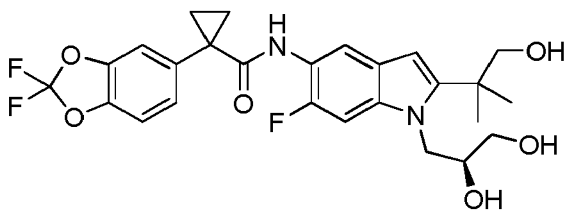

Compound 3

In one aspect, the pharmaceutical composition comprises Compound 1, Compound 2, and Compound 3.

In another aspect, the invention is directed to a pharmaceutical composition comprising at least one component from Column A of Table I, and at least one component from Column B and/or Column C of Table I. These components are described in the corresponding sections of the following pages as embodiments of the invention. For convenience,

Table I recites the section number and corresponding heading title of the embodiments of the compounds, solid forms and formulations. For example, the embodiments of the compounds of Formula I are disclosed in section **II.A.1.** of this specification.

**Table I**

| **Column A Embodiments** | | **Column B Embodiments** | | **Column C Embodiments** | |
|---|---|---|---|---|---|
| **Section** | **Heading** | **Section** | **Heading** | **Section** | **Heading** |
| **II.A.1.** | Compounds of Formula I | **II.B.1.** | Compounds of Formula II | **II.C.1.** | Compounds of Formula III |
| **II.A.2.** | Compound 1 | **II.B.2.** | Compound 2 | **II.C.2.** | Compound 3 |
| **III.A.1.a.** | Compound 1 Form C | **III.B.1.a.** | Compound 2 Form I | **III.C.1.a.** | Compound 3 Form A |
| **IV.A.1.a.** | Compound 1 First Formulation | **III.B.2.a.** | Compound 2 Solvate Form A | **III.C.2.a.** | Compound 3 Amorphous Form |
| **IV.A.2.a.** | Compound 1 Tablet and SDD Formulation | **III.B.3.a.** | Compound 2 HCl Salt Form A | **IV.B.1.a.** | Compound 3 Tablet Formulation |

In one aspect, the invention includes a pharmaceutical composition comprising a component selected from any embodiment described in Column A of Table I in combination with a component selected from any embodiment described in Column B and/or a component selected from any embodiment described in Column C of Table I.

In one embodiment of this aspect, the composition comprises an embodiment described in Column A in combination with an embodiment described in Column B. In another embodiment, the composition comprises an embodiment described in Column A in combination with an embodiment described in Column C. In another embodiment, the composition comprises a combination of an embodiment described in Column A, an embodiment described in Column B, and an embodiment described in Column C.

In one embodiment of this aspect, the Column A component is a compound of Formula I. In another embodiment, the Column A component is Compound 1. In another embodiment, the Column A component is Compound 1 Form C. In another embodiment, the Column A component is Compound 1 First Formulation. In another embodiment, the Column A component is Compound 1 Tablet and SDD Formulation.

In one embodiment of this aspect, the Column B component is a compound of Formula II. In another embodiment, the Column B component is Compound 2. In another embodiment, the Column B component is Compound 2 Form I. In another embodiment, the Column B component is Compound 2 Solvate Form A. In another embodiment, the Column B component is Compound 2 HCl Salt Form A.

In one embodiment of this aspect, the Column C component is a compound of Formula III. In another embodiment, the Column C component is Compound 3. In another embodiment, the Column C component is Compound 3 Form A. In another embodiment, the Column C component is Compound 3 Amorphous Form. In another embodiment, the Column C component is Compound 3 Tablet Formulation.

Various components listed in Table I have been disclosed and can be found in US 2011/0065928 A1, US 2010/0184739, US 2010/0267768, US 2011/0064811, US 2009/0105272, US 2009/0246820, US 2009/0099230, US Pat No. 7,776,905, US Pat. No. 7,645,789, US Pat. No. 7,495,103, US Pat. No. 7,553,855, US 2010/0074949, US 2010/0256184, US Pat. No. 7,741,321, US Pat. No. 7,659,268, US 2008/0306062A1, US 2009/0170905 A1, US 2009/0176839 and US 2010/0087490, the contents of which are incorporated herein by reference.

## LIST OF FIGURES

**Figure 1-1** is an X-Ray powder diffraction pattern of Form C of Compound 1.
**Figure 1-2** is a DSC trace of Compound 1 Form C.
**Figure 1-3** is a TGA trace of Compound 1 Form C.
**Figure 1-4** is a Raman spectrum of Compound 1 Form C.
**Figure 1-5** is an FTIR spectrum of Compound 1 Form C.
**Figure 1-6** is a Solid State NMR Spectrum of Compound 1 Form C.
**Figure 2-1** is an X-ray diffraction pattern calculated from a single crystal structure of Compound 2 Form I.
**Figure 2-2** is an actual X-ray powder diffraction pattern of Compound 2 Form I.
**Figure 2-3** is a conformational picture of Compound 2 Form I based on single crystal X-ray analysis.

**Figure 2-4** is an X-ray powder diffraction pattern of Compound 2 Solvate Form A.

**Figure 2-5** is a Stacked, multi-pattern spectrum of the X-ray diffraction patterns of Compound 2 Solvate Forms selected from:

1) Compound 2, Methanol Solvate Form A;

2) Compound 2, Ethanol Solvate Form A;

3) Compound 2 Acetone Solvate Form A;

4) Compound 2, 2-Propanol Solvate Form A;

5) Compound 2, Acetonitrile Solvate Form A;

6) Compound 2, Tetrahydrofuran Solvate Form A;

7) Compound 2, Methyl Acetate Solvate Form A;

8) Compound 2, 2-Butanone Solvate Form A;

9) Compound 2, Ethyl Formate Solvate Form A; and

10) Compound 2 2-Methyltetrahydrofuran Solvate Form A.

**Figure 2-6** is an X-ray diffraction pattern of Compound 2, Methanol Solvate Form A.

**Figure 2-7** is an X-ray diffraction pattern of Compound 2, Ethanol Solvate Form A.

**Figure 2-8** is an X-ray diffraction pattern of Compound 2 Acetone Solvate Form A.

**Figure 2-9** is an X-ray diffraction pattern of Compound 2, 2-Propanol Solvate Form A.

**Figure 2-10** is an X-ray diffraction pattern of Compound 2, Acetonitrile Solvate Form A.

**Figure 2-11** is an X-ray diffraction pattern of Compound 2, Tetrahydrofuran Solvate Form A.

**Figure 2-12** is an X-ray diffraction pattern of Compound 2, Methyl Acetate Solvate Form A.

**Figure 2-13** is an X-ray diffraction pattern of Compound 2, 2-Butanone Solvate Form A.

**Figure 2-14** is an X-ray diffraction pattern of Compound 2, Ethyl Formate Solvate Form A.

**Figure 2-15** is an X-ray diffraction pattern of Compound 2, 2-Methyltetrahydrofuran Solvate Form A.

**Figure 2-16** is a conformational image of Compound 2 Acetone Solvate Form A based on single crystal X-ray analysis.

**Figure 2-17** is a conformational image of Compound 2 Solvate Form A based on single crystal X-ray analysis as a dimer.

**Figure 2-18** is a conformational image of Compound 2 Solvate Form A showing hydrogen bonding between carboxylic acid groups based on single crystal X-ray analysis.

**Figure 2-19** is a conformational image of Compound 2 Solvate Form A showing acetone as the solvate based on single crystal X-ray analysis.

**Figure 2-20** is a conformational image of the dimer of Compound 2 HCl Salt Form A.

**Figure 2-21** is a packing diagram of Compound 2 HCl Salt Form A.

**Figure 2-22** is an X-ray diffraction pattern of Compound 2 HCl Salt Form A calculated from the crystal structure.

**Figure 2-23** is a $^{13}$C SSNMR Spectrum of Compound 2 Form I.

**Figure 2-24** is a $^{19}$F SSNMR Spectrum of Compound 2 Form I (15.0 kHz Spinning).

**Figure 2-25** is a $^{13}$C SSNMR Spectrum of Compound 2 Acetone Solvate Form A.

**Figure 2-26** is a $^{19}$F SSNMR Spectrum of Compound 2 Acetone Solvate Form A (15.0 kHz Spinning).

**Figure 3-1** is an X-ray powder diffraction pattern calculated from a single crystal of Compound 3 Form A.

**Figure 3-2** is an actual X-ray powder diffraction pattern of Compound 3 Form A prepared by the slurry technique (2 weeks) with DCM as the solvent.

**Figure 3-3** is an actual X-ray powder diffraction pattern of Compound 3 Form A prepared by the fast evaporation method from acetonitrile.

**Figure 3-4** is an actual X-ray powder diffraction pattern of Compound 3 Form A prepared by the anti solvent method using EtOAc and heptane.

**Figure 3-5** is a conformational picture of Compound 3 Form A based on single crystal X-ray analysis.

**Figure 3-6** is a conformational picture showing the stacking order of Compound 3 Form A.

**Figure 3-7** is a $^{13}$C SSNMR spectrum (15.0 kHz spinning) of Compound 3 Form A.

**Figure 3-8** is a $^{19}F$ SSNMR spectrum (12.5 kHz spinning) of Compound 3 Form A.
**Figure 3-9** is an X-ray powder diffraction pattern of Compound 3 amorphous form from the fast evaporation rotary evaporation method.
**Figure 3-10** is an X-ray powder diffraction pattern of Compound 3 amorphous form prepared by spray dried methods.
**Figure 3-11** is a solid state $^{13}C$ NMR spectrum (15.0 kHz spinning) of Compound 3 amorphous form.
**Figure 3-12** is a solid state $^{19}F$ NMR spectrum (12.5 kHz spinning) of Compound 3 amorphous form.

## DETAILED DESCRIPTION

### I. Definitions

As used herein, the following definitions shall apply unless otherwise indicated.
The term "ABC-transporter" as used herein means an ABC-transporter protein or a fragment thereof comprising at least one binding domain, wherein said protein or fragment thereof is present *in vivo* or *in vitro*. The term "binding domain" as used herein means a domain on the ABC-transporter that can bind to a modulator. See, e.g., Hwang, T. C. et al., J. Gen. Physiol. (1998): 111(3), 477-90.
The term "CFTR" as used herein means cystic fibrosis transmembrane conductance regulator or a mutation thereof capable of regulator activity, including, but not limited to, ΔF508 CFTR, R117H CFTR, and G551D CFTR (see, e.g., http://www.genet.sickkids.on.ca/cftr/, for CFTR mutations).
As used herein, the term "active pharmaceutical ingredient" or "API" refers to a biologically active compound. Exemplary APIs include the CF potentiator N-[2,4-bis(1,1-dimethylethyl)-5-hydroxyphenyl]-1,4-dihydro-4-oxoquinoline-3-carboxamide (Compound 1). Exemplary APIs also include the CF correctors 3-(6-(1-(2,2-Difluorobenzo[d][1,3]dioxol-5-yl)cyclopropanecarboxamido)-3-methylpyridin-2-yl)benzoic acid (Compound 2) and (*R*)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide (Compound 3).
The term "modulating" as used herein means increasing or decreasing by a measurable amount.
The term "normal CFTR" or "normal CFTR function" as used herein means wild-type like CFTR without any impairment due to environmental factors such as smoking, pollution, or anything that produces inflammation in the lungs.
The term "reduced CFTR" or "reduced CFTR function" as used herein means less than normal CFTR or less than normal CFTR function.
As used herein, the term "amorphous" refers to a solid material having no long range order in the position of its molecules. Amorphous solids are generally supercooled liquids in which the molecules are arranged in a random manner so that there is no well-defined arrangement, e.g., molecular packing, and no long range order. Amorphous solids are generally isotropic, i.e. exhibit similar properties in all directions and do not have definite melting points. For example, an amorphous material is a solid material having no sharp characteristic crystalline peak(s) in its X-ray power diffraction (XRPD) pattern (i.e., is not crystalline as determined by XRPD). Instead, one or several broad peaks (e.g., halos) appear in its XRPD pattern. Broad peaks are characteristic of an amorphous solid. See, US 2004/0006237 for a comparison of XRPDs of an amorphous material and crystalline material.
As used herein, the term "substantially amorphous" refers to a solid material having little or no long range order in the position of its molecules. For example, substantially amorphous materials have less than about 15% crystallinity (e.g., less than about 10% crystallinity or less than about 5% crystallinity). It is also noted that the term 'substantially amorphous' includes the descriptor, 'amorphous', which refers to materials having no (0%) crystallinity.
As used herein, the term "dispersion" refers to a disperse system in which one substance, the dispersed phase, is distributed, in discrete units, throughout a second substance (the continuous phase or vehicle). The size of the dispersed phase can vary considerably (e.g. single molecules, colloidal particles of nanometer dimension, to multiple microns in size). In general, the dispersed phases can be solids, liquids, or gases. In the case of a solid dispersion, the dispersed and continuous phases are both solids. In pharmaceutical applications, a solid dispersion can include: an amorphous drug in an amorphous polymer; an amorphous drug in crystalline polymer; a crystalline drug in an amorphous polymer; or a crystalline drug in crystalline polymer. In this invention, a solid dispersion can include an amorphous drug in an amorphous polymer or an amorphous drug in crystalline polymer. In some embodiments, a solid dispersion includes the polymer constituting the dispersed phase, and the drug constitutes the continuous phase. Or, a solid dispersion includes the drug constituting the dispersed phase, and the polymer constitutes the continuous phase.
As used herein, the term "solid dispersion" generally refers to a solid dispersion of two or more components, usually one or more drugs (e.g., one drug (e.g., Compound 1)) and polymer, but possibly containing other components such as surfactants or other pharmaceutical excipients, where the drug(s) (e.g., Compound 1) is substantially amorphous (e.g., having about 15% or less (e.g., about 10% or less, or about 5% or less)) of crystalline drug (e.g., N-[2,4-bis(1,1-dimethylethyl)-5-hydroxyphenyl]-1,4-dihydro-4-oxoquinoline-3-carboxamide) or amorphous (i.e., having no crystalline drug), and the physical stability and/or dissolution and/or solubility of the substantially amorphous or amorphous drug

is enhanced by the other components. Solid dispersions typically include a compound dispersed in an appropriate carrier medium, such as a solid state carrier. For example, a carrier comprises a polymer (e.g., a water-soluble polymer or a partially water-soluble polymer) and can include optional excipients such as functional excipients (e.g., one or more surfactants) or nonfunctional excipients (e.g., one or more fillers). Another exemplary solid dispersion is a co-precipitate or a co-melt of N-[2,4-bis(1,1-dimethylethyl)-5-hydroxyphenyl]-1,4-dihydro-4-oxoquinoline-3-carboxamide with at least one polymer.

A "Co-precipitate" is a product after dissolving a drug and a polymer in a solvent or solvent mixture followed by the removal of the solvent or solvent mixture. Sometimes the polymer can be suspended in the solvent or solvent mixture. The solvent or solvent mixture includes organic solvents and supercritical fluids. A "co-melt" is a product after heating a drug and a polymer to melt, optionally in the presence of a solvent or solvent mixture, followed by mixing, removal of at least a portion of the solvent if applicable, and cooling to room temperature at a selected rate.

As used herein "crystalline" refers to compounds or compositions where the structural units are arranged in fixed geometric patterns or lattices, so that crystalline solids have rigid long range order. The structural units that constitute the crystal structure can be atoms, molecules, or ions. Crystalline solids show definite melting points.

As used herein the phrase "substantially crystalline", means a solid material that is arranged in fixed geometric patterns or lattices that have rigid long range order. For example, substantially crystalline materials have more than about 85% crystallinity (e.g., more than about 90% crystallinity or more than about 95% crystallinity). It is also noted that the term 'substantially crystalline' includes the descriptor 'crystalline', which is defined in the previous paragraph.

As used herein, "crystallinity" refers to the degree of structural order in a solid. For example, Compound 1, which is substantially amorphous, has less than about 15% crystallinity, or its solid state structure is less than about 15% crystalline. In another example, Compound 1, which is amorphous, has zero (0%) crystallinity.

As used herein, an "excipient" is an inactive ingredient in a pharmaceutical composition. Examples of excipients include fillers or diluents, surfactants, binders, glidants, lubricants, disintegrants, and the like.

As used herein, a "disintegrant" is an excipient that hydrates a pharmaceutical composition and aids in tablet dispersion. Examples of disintegrants include sodium croscarmellose and/or sodium starch glycolate.

As used herein, a "diluent" or "filler" is an excipient that adds bulkiness to a pharmaceutical composition. Examples of fillers include lactose, sorbitol, celluloses, calcium phosphates, starches, sugars (e.g., mannitol, sucrose, or the like) or any combination thereof.

As used herein, a "surfactant" is an excipient that imparts pharmaceutical compositions with enhanced solubility and/or wetability. Examples of surfactants include sodium lauryl sulfate (SLS), sodium stearyl fumarate (SSF), polyoxyethylene 20 sorbitan mono-oleate (e.g., Tween™), or any combination thereof.

As used herein, a "binder" is an excipient that imparts a pharmaceutical composition with enhanced cohesion or tensile strength (e.g., hardness). Examples of binders include dibasic calcium phosphate, sucrose, corn (maize) starch, microcrystalline cellulose, and modified cellulose (e.g., hydroxymethyl cellulose).

As used herein, a "glidant" is an excipient that imparts a pharmaceutical compositions with enhanced flow properties. Examples of glidants include colloidal silica and/or talc.

As used herein, a "colorant" is an excipient that imparts a pharmaceutical composition with a desired color. Examples of colorants include commercially available pigments such as FD&C Blue # 1 Aluminum Lake, FD&C Blue #2, other FD&C Blue colors, titanium dioxide, iron oxide, and/or combinations thereof.

As used herein, a "lubricant" is an excipient that is added to pharmaceutical compositions that are pressed into tablets. The lubricant aids in compaction of granules into tablets and ejection of a tablet of a pharmaceutical composition from a die press. Examples of lubricants include magnesium stearate, stearic acid (stearin), hydrogenated oil, sodium stearyl fumarate, or any combination thereof.

As used herein, "friability" refers to the property of a tablet to remain intact and withhold its form despite an external force of pressure. Friability can be quantified using the mathematical expression presented in equation 1:

$$\% friabiliy = 100 \times \frac{(W_0 - W_f)}{W_0} \qquad (1)$$

wherein $W_o$ is the original weight of the tablet and $W_f$ is the final weight of the tablet after it is put through the friabilator.

Friability is measured using a standard USP testing apparatus that tumbles experimental tablets for 100 revolutions. Some tablets of the present invention have a friability of less than about 1% (e.g., less than about 0.75%, less than about 0.50%, or less than about 0.30%)

As used herein, "mean particle diameter" is the average particle diameter as measured using techniques such as laser light scattering, image analysis, or sieve analysis.

As used herein, "bulk density" is the mass of particles of material divided by the total volume the particles

occupy. The total volume includes particle volume, inter-particle void volume and internal pore volume. Bulk density is not an intrinsic property of a material; it can change depending on how the material is processed.

The term "aliphatic" or "aliphatic group," as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "carbocycle," "cycloaliphatic" or "cycloalkyl"), that has a single point of attachment to the rest of the molecule. Unless otherwise specified, aliphatic groups contain 1-20 aliphatic carbon atoms. In some embodiments, aliphatic groups contain 1-10 aliphatic carbon atoms. In other embodiments, aliphatic groups contain 1-8 aliphatic carbon atoms. In still other embodiments, aliphatic groups contain 1-6 aliphatic carbon atoms, and in yet other embodiments aliphatic groups contain 1-4 aliphatic carbon atoms. In some embodiments, "cycloaliphatic" (or "carbocycle" or "cycloalkyl") refers to a monocyclic $C_3$-$C_8$ hydrocarbon or bicyclic or tricyclic $C_8$-$C_{14}$ hydrocarbon that is completely saturated or that contains one or more units of unsaturation, but which is not aromatic, that has a single point of attachment to the rest of the molecule wherein any individual ring in said bicyclic ring system has 3-7 members. Suitable aliphatic groups include, but are not limited to, linear or branched, substituted or unsubstituted alkyl, alkenyl, alkynyl groups and hybrids thereof such as (cycloalkyl)alkyl, (cycloalkenyl)alkyl or (cycloalkyl)alkenyl. Suitable cycloaliphatic groups include cycloalkyl, bicyclic cycloalkyl (e.g., decalin), bridged bicycloalkyl such as norbornyl or [2.2.2]bicyclo-octyl, or bridged tricyclic such as adamantyl.

The term "alkyl" as used herein refers to a saturated aliphatic hydrocarbon group containing 1-15 (including, but not limited to, 1-8, 1-6, 1-4, 2-6, 3-12) carbon atoms. An alkyl group can be straight or branched.

The term "heteroaliphatic," as used herein, means aliphatic groups wherein one or two carbon atoms are independently replaced by one or more of oxygen, sulfur, nitrogen, phosphorus, or silicon. Heteroaliphatic groups may be substituted or unsubstituted, branched or unbranched, cyclic or acyclic, and include "heterocycle," "heterocyclyl," "heterocycloaliphatic," or "heterocyclic" groups.

The term "heterocycle," "heterocyclyl," "heterocycloaliphatic," or "heterocyclic" as used herein means non-aromatic, monocyclic, bicyclic, or tricyclic ring systems in which one or more ring members is an independently selected heteroatom. In some embodiments, the "heterocycle," "heterocyclyl," "heterocycloaliphatic," or "heterocyclic" group has three to fourteen ring members in which one or more ring members is a heteroatom independently selected from oxygen, sulfur, nitrogen, or phosphorus, and each ring in the system contains 3 to 7 ring members.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2*H*-pyrrolyl), NH (as in pyrrolidinyl) or $NR^+$ (as in N-substituted pyrrolidinyl)).

The term "unsaturated," as used herein, means that a moiety has one or more units of unsaturation.

The term "aryl" used alone or as part of a larger moiety as in "aralkyl," "aralkoxy," or "aryloxyalkyl," refers to monocyclic, bicyclic, and tricyclic ring systems having a total of five to fourteen ring members, wherein at least one ring in the system is aromatic and wherein each ring in the system contains 3 to 7 ring members. The term "aryl" may be used interchangeably with the term "aryl ring." The term "aryl" also refers to heteroaryl ring systems as defined herein below.

An aliphatic or heteroaliphatic group, or a non-aromatic heterocyclic ring may contain one or more substituents. Suitable substituents on the saturated carbon of an aliphatic or heteroaliphatic group, or of a non-aromatic heterocyclic ring are selected from those listed above for the unsaturated carbon of an aryl or heteroaryl group and additionally include the following: =O, =S, =NNHR*, $=NN(R^*)_2$, =NNHC(O)R*, $=NNHCO_2$(alkyl), $=NNHSO_2$(alkyl), or =NR*, where each R* is independently selected from hydrogen or an optionally substituted $C_{1-6}$ aliphatic. Optional substituents on the aliphatic group of R* are selected from $NH_2$, NH($C_{1-4}$ aliphatic), N($C_{1-4}$ aliphatic)$_2$, halo, $C_{1-4}$ aliphatic, OH, O($C_{1-4}$ aliphatic), $NO_2$, CN, $CO_2H$, $CO_2$($C_{1-4}$ aliphatic), O(halo $C_{1-4}$ aliphatic), or halo($C_{1-4}$ aliphatic), wherein each of the foregoing $C_{1-4}$aliphatic groups of R* is unsubstituted.

Optional substituents on the nitrogen of a non-aromatic heterocyclic ring are selected from $-R^+$, $-N(R^+)_2$, $-C(O)R^+$, $-CO_2R^+$, $-C(O)C(O)R^+$, $-C(O)CH_2C(O)R^+$,$-SO_2R^+$, $-SO_2N(R^+)_2$, $-C(=S)N(R^+)_2$, $-C(=NH)-N(R^+)2$, or $-NR^+SO_2R^+$; wherein $R^+$ is hydrogen, an optionally substituted $C_{1-6}$ aliphatic, optionally substituted phenyl, optionally substituted -O(Ph), optionally substituted $-CH_2$(Ph), optionally substituted $-(CH_2)_{1-2}$(Ph); optionally substituted -CH=CH(Ph); or an unsubstituted 5-6 membered heteroaryl or heterocyclic ring having one to four heteroatoms independently selected from oxygen, nitrogen, or sulfur, or, notwithstanding the definition above, two independent occurrences of $R^+$, on the same substituent or different substituents, taken together with the atom(s) to which each $R^+$ group is bound, form a 3-8-membered cycloalkyl, heterocyclyl, aryl, or heteroaryl ring having 0-3 heteroatoms independently selected from nitrogen, oxygen, or sulfur. Optional substituents on the aliphatic group or the phenyl ring of $R^+$ are selected from $NH_2$, NH($C_{1-4}$ aliphatic), N($C_{1-4}$ aliphatic)$_2$, halo, $C_{1-4}$ aliphatic, OH, O($C_{1-4}$ aliphatic), $NO_2$, CN, $CO_2H$ $CO_2$($C_{1-4}$ aliphatic), O(halo $C_{1-4}$ aliphatic), or halo($C_{1-4}$ aliphatic), wherein each of the foregoing $C_{1-4}$aliphatic groups of $R^+$ is unsubstituted.

As detailed above, in some embodiments, two independent occurrences of R (or any other variable similarly

defined herein), are taken together with the atom(s) to which each variable is bound to form a 3-8-membered cycloalkyl, heterocyclyl, aryl, or heteroaryl ring having 0-3 heteroatoms independently selected from nitrogen, oxygen, or sulfur. Exemplary rings that are formed when two independent occurrences of R (or any other variable similarly defined herein) are taken together with the atom(s) to which each variable is bound include, but are not limited to the following: a) two independent occurrences of R (or any other variable similarly defined herein) that are bound to the same atom and are taken together with that atom to form a ring, for example, $N(R')_2$, where both occurrences of R' are taken together with the nitrogen atom to form a piperidin-1-yl, piperazin-1-yl, or morpholin-4-yl group; and b) two independent occurrences of R (or any other variable similarly defined herein) that are bound to different atoms and are taken together with both of those atoms to form a ring, for example where a phenyl group is substituted with two occurrences of OR'

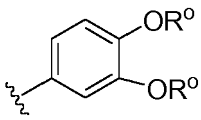,

these two occurrences of R° are taken together with the oxygen atoms to which they are bound to form a fused 6-membered oxygen containing ring:

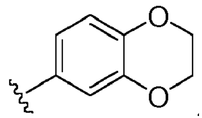.

It will be appreciated that a variety of other rings can be formed when two independent occurrences of R (or any other variable similarly defined herein) are taken together with the atom(s) to which each variable is bound and that the examples detailed above are not intended to be limiting.

A substituent bond in, e.g., a bicyclic ring system, as shown below, means that the substituent can be attached to any substitutable ring atom on either ring of the bicyclic ring system:

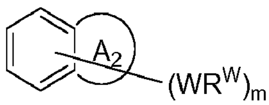

The term "protecting group" (PG) as used herein, represents those groups intended to protect a functional group, such as, for example, an alcohol, amine, carboxyl, carbonyl, etc., against undesirable reactions during synthetic procedures. Commonly used protecting groups are disclosed in Greene and Wuts, Protective Groups in Organic Synthesis, 3rd Edition (John Wiley & Sons, New York, 1999), which is incorporated herein by reference. Examples of nitrogen protecting groups include acyl, aroyl, or carbamyl groups such as formyl, acetyl, propionyl, pivaloyl, *t*-butylacetyl, 2-chloroacetyl, 2-bromoacetyl, trifluoroacetyl, trichloroacetyl, phthalyl, o-nitrophenoxyacetyl, $\alpha$-chlorobutyryl, benzoyl, 4-chlorobenzoyl, 4-bromobenzoyl, 4-nitrobenzoyl and chiral auxiliaries such as protected or unprotected D, L or D, L-amino acids such as alanine, leucine, phenylalanine and the like; sulfonyl groups such as benzenesulfonyl, p-toluenesulfonyl and the like; carbamate groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, $\alpha,\alpha$-dimethyl-3,5-dimethoxybenzyloxycarbonyl, benzhydryloxycarbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxy carbonyl, fluorenyl-9-methoxycarbonyl, cyclopentyloxycarbonyl, adamantyloxycarbonyl, cyclohexyloxycarbonyl, phenylthiocarbonyl and the like, arylalkyl groups such as benzyl, triphenylmethyl, benzyloxymethyl and the like and silyl groups such as trimethylsilyl and the like. Preferred *N*-protecting groups are *tert*-butyloxycarbonyl (Boc).

Examples of useful protecting groups for acids are substituted alkyl esters such as 9-fluorenylmethyl, methoxymethyl, methylthiomethyl, tetrahydropyranyl, tetrahydrofuranyl, methoxyethoxymethyl, 2-(trimethylsilyl)ethoxymethyl, benzyloxymethyl, pivaloyloxymethyl, phenylacetoxymethyl, triisopropropylsysilylmethyl, cyanomethyl, acetol, phenacyl, substituted phenacyl esters, 2,2,2- trichloroethyl, 2-haloethyl, $\omega$-chloroalkyl, 2-(trimethylsilyl)ethyl, 2-methylthioethyl, *t*-butyl, 3-methyl-3-pentyl, dicyclopropylmethyl, cyclopentyl, cyclohexyl, allyl, methallyl, cynnamyl, phenyl, silyl esters, benzyl and substituted benzyl esters, 2,6-dialkylphenyl esters such as pentafluorophenyl, 2,6-dialkylpyhenyl. Preferred protecting groups for acids are methyl or ethyl esters.

Methods of adding (a process generally referred to as "protection") and removing (process generally referred to as "deprotection") such amine and acid protecting groups are well-known in the art and available, for example in P.J.Kocienski, Protecting Groups, Thieme, 1994, which is hereby incorporated by reference in its entirety and in Greene and Wuts, Protective Groups in Organic Synthesis, 3rd Edition (John Wiley & Sons, New York, 1999).

Unless otherwise stated, structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. E.g., compounds of Formula I may exist as tautomers:

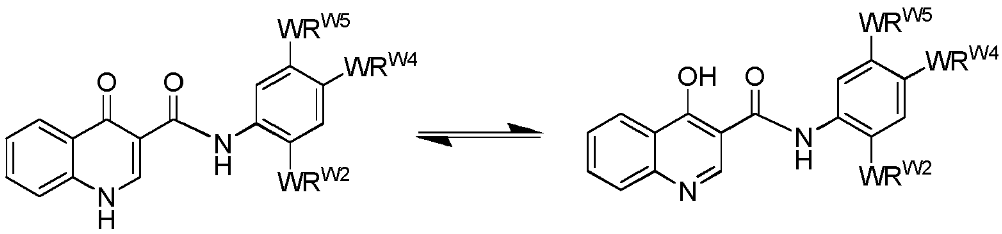

Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a $^{13}C$- or $^{14}C$-enriched carbon are within the scope of this invention. Such compounds are useful, for example, as analytical tools or probes in biological assays.

Examples of suitable solvents are, but not limited to, water, methanol, dichloromethane (DCM), acetonitrile, dimethylformamide (DMF), ethyl acetate (EtOAc), isopropyl alcohol (IPA), isopropyl acetate (IPAc), tetrahydrofuran (THF), methyl ethyl ketone (MEK), *t*-butanol and N-methyl pyrrolidone (NMP).

### II. Compounds of the Invention

In one aspect, the invention is directed to a pharmaceutical composition comprising a compound of Formula I in combination with a Compound of Formula II and/or a Compound of Formula III.

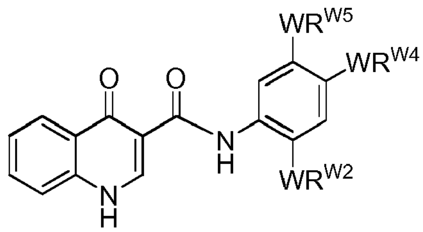

Formula I

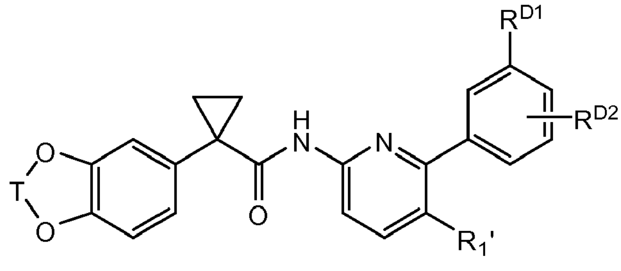

Formula II

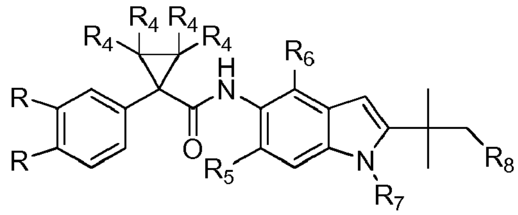

Formula III

**II.A. Compounds of Formula I**

**II.A.1. Embodiments of Compounds of Formula I**

In one aspect, the invention includes a composition comprising a compound of Formula I

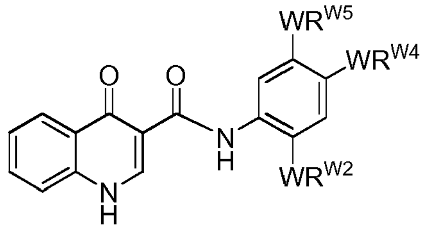

**Formula I**

or pharmaceutically acceptable salts thereof, wherein:

Each of $WR^{W2}$ and $WR^{W4}$ is independently selected from CN, $CF_3$, halo, $C_{2-6}$ straight or branched alkyl, $C_{3-12}$ membered cycloaliphatic, phenyl, a 5-10 membered heteroaryl or 3-7 membered heterocyclic, wherein said heteroaryl or heterocyclic has up to 3 heteroatoms selected from O, S, or N, wherein said $WR^{W2}$ and $WR^{W4}$ is independently and optionally substituted with up to three substituents selected from -OR', $-CF_3$, $-OCF_3$, SR', S(O)R', $SO_2R'$, $-SCF_3$, halo, CN, -COOR', -COR', $-O(CH_2)_2N(R')_2$, $-O(CH_2)N(R')_2$,$-CON(R')_2$, $-(CH_2)_2OR'$, $-(CH_2)OR'$, $-CH_2CN$, optionally substituted phenyl or phenoxy,$-N(R')_2$, -NR'C(O)OR', -NR'C(O)R', $-(CH_2)_2N(R')_2$, or $-(CH_2)N(R')_2$;

$WR^{W5}$ is selected from hydrogen, $-OCF_3$, $-CF_3$, -OH, $-OCH_3$, $-NH_2$, -CN, $-CHF_2$, -NHR', $-N(R')_2$, -NHC(O)R', -NHC(O)OR', $-NHSO_2R'$, $-CH_2OH$, $-CH_2N(R')_2$, -C(O)OR', $-SO_2NHR'$, $-SO_2N(R')_2$, or $-CH_2NHC(O)OR'$; and

Each R' is independently selected from an optionally substituted group selected from a $C_{1-8}$ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;

provided that:

ii) $WR^{W2}$ and $WR^{W4}$ are not both -Cl;

$WR^{W2}$, $WR^{W4}$ and $WR^{W5}$ are not $-OCH_2CH_2Ph$, $-OCH_2CH_2$(2-trifluoromethyl-phenyl),$-OCH_2CH_2$-(6,7-dimethoxy-1,2,3,4-tetrahydroisoquinolin-2-yl), or substituted 1*H*-pyrazol-3-yl.

In one embodiment of the compound of Formula I of the composition, each of $WR^{W2}$ and $WR^{W4}$ is independently selected from CN, $CF_3$, halo, $C_{2-6}$ straight or branched alkyl, $C_{3-12}$ membered cycloaliphatic, or phenyl, wherein said $WR^{W2}$ and $WR^{W4}$ is independently and optionally substituted with up to three substituents selected from-OR', $-CF_3$, $-OCF_3$, $-SCF_3$, halo, -COOR', -COR', $-O(CH_2)_2N(R')_2$, $-O(CH_2)N(R')_2$,$-CON(R')_2$, $-(CH_2)_2OR'$, $-(CH_2)OR'$, optionally substituted phenyl, $-N(R')_2$, -NC(O)OR',-NC(O)R', $-(CH_2)_2N(R')_2$, or $-(CH_2)N(R')_2$; and $WR^{W5}$ is selected from hydrogen, $-OCF_3$-$CF_3$, -OH, $-OCH_3$, $-NH_2$, -CN, -NHR', $-N(R')_2$, -NHC(O)R', -NHC(O)OR', $-NHSO_2R'$,$-CH_2OH$ -C(O)OR', $-SO_2NHR'$, or $-CH_2NHC(O)O$-R').

Alternatively, each of $WR^{W2}$ and $WR^{W4}$ is independently selected from -CN, $-CF_3$, $C_{2-6}$ straight or branched alkyl, $C_{3-12}$ membered cycloaliphatic, or phenyl, wherein each of said $WR^{W2}$ and $WR^{W4}$ is independently and optionally substituted with up to three substituents selected from -OR', $-CF_3$, $-OCF_3$, $-SCF_3$, halo, -COOR', -COR', $-O(CH_2)_2N(R')_2$, $-O(CH_2)N(R')_2$, $-CON(R')_2$, $-(CH_2)_2OR'$, $-(CH_2)O$ R', optionally substituted phenyl, $-N(R')_2$, -NC(O)OR', -NC(O)R', $-(CH_2)_2N(R')_2$, or $-(CH_2)N(R')_2$; and $WR^{W5}$ is selected from -OH, -CN, -NHR', $-N(R')_2$, -NHC(O)R', -NHC(O)OR', $-NHSO_2R'$, $-CH_2OH$, -C(O) OR', $-SO_2NHR'$, or $-CH_2NHC(O)O-(R')$.

In a further embodiment, $WR^{W2}$ is a phenyl ring optionally substituted with up to three substituents selected from -OR', $-CF_3$, $-OCF_3$, -SR', -S(O)R', $-SO_2R'$, $-SCF_3$, halo, -CN, -COOR', -COR', $-O(CH_2)_2N(R')_2$, $-O(CH_2)N(R')_2$, $-CON(R')_2$, $-(CH_2)_2OR'$, -(C $H_2$)OR', $-CH_2CN$, optionally substituted phenyl or phenoxy, $-N(R')_2$, -NR'C(O)OR', -NR'C(O)R', $-(CH_2)_2N(R')_2$, or $-(CH_2)N(R')_2$; $WR^{W4}$ is $C_{2-6}$ straight or branched alkyl; and $WR^{W5}$ is -OH.

In another embodiment, each of $WR^{W2}$ and $WR^{W4}$ is independently $-CF_3$, -CN, or a $C_{2-6}$ straight or branched alkyl.

In another embodiment, each of $WR^{W2}$ and $WR^{W4}$ is $C_{2-6}$ straight or branched alkyl optionally substituted with up to three substituents independently selected from -OR', $-CF_3$, $-OCF_3$, -SR', -S(O)R', $-SO_2R'$, $-SCF_3$, halo, -CN, -COOR', -COR', $-O(CH_2)_2N(R')_2$, $-O(CH_2)N(R')_2$, $-CON(R')_2$, $-(CH_2)_2OR'$, -(C $H_2$)OR', $-CH_2CN$, optionally substituted phenyl or phenoxy, $-N(R')_2$, -NR'C(O)OR', -NR'C(O)R', $-(CH_2)_2N(R')_2$, or $-(CH_2)N(R')_2$.

In another embodiment, each of $WR^{W2}$ and $WR^{W4}$ is independently selected from optionally substituted n-propyl, isopropyl, n-butyl, sec-butyl, t-butyl, 1,1-dimethyl-2-hydroxyethyl, 1,1-dimethyl-2-(ethoxycarbonyl)-ethyl, 1,1-dimethyl-3-(t-butoxycarbonylamino) propyl, or n-pentyl.

In another embodiment, $WR^{W5}$ is selected from -CN, -NHR', $-N(R')_2$, $-CH_2N(R')_2$, -NHC(O)R', -NHC(O)OR', -OH, C(O)OR', or $-SO_2NHR'$.

In another embodiment, $WR^{W5}$ is selected from -CN, -NH($C_{1-6}$ alkyl), -N($C_{1-6}$ alkyl)$_2$, -NHC(O)($C_{1-6}$ alkyl), $-CH_2NHC(O)O$($C_{1-6}$ alkyl), -NHC(O)O($C_{1-6}$ alkyl), -OH, -O($C_{1-6}$ alkyl), -C(O)O($C_{1-6}$ alkyl), $-CH_2O$($C_{1-6}$ alkyl), or $-SO_2NH_2$.

In another embodiment, $WR^{W5}$ is selected from -OH, $-CH_2OH$, -NHC(O)OMe, -NHC(O)OEt, -CN, $-CH_2NHC(O)O$(t-butyl), -C(O)OMe, or $-SO_2NH_2$.

In another embodiment:

a. $WR^{W2}$ is $C_{2-6}$ straight or branched alkyl;
b. $WR^{W4}$ is $C_{2-6}$ straight or branched alkyl or monocyclic or bicyclic aliphatic; and
c. $WR^{W5}$ is selected from -CN, -NH($C_{1-6}$ alkyl), -N($C_{1-6}$ alkyl)$_2$, -NHC(O)( $C_{1-6}$ alkyl), -NHC(O)O($C_{1-6}$ alkyl), $-CH_2C(O)O$($C_{1-6}$ alkyl), -OH, -O($C_{1-6}$ alkyl), -C(O)O($C_{1-6}$ alkyl), or $-SO_2NH_2$.

In another embodiment:

a. $WR^{W2}$ is $C_{2-6}$ alkyl, $-CF_3$, -CN, or phenyl optionally substituted with up to 3 substituents selected from $C_{1-4}$ alkyl, -O($C_{1-4}$ alkyl), or halo;
b. $WR^{W4}$ is $-CF_3$, $C_{2-6}$ alkyl, or $C_{6-10}$ cycloaliphatic; and
c. $WR^{W5}$ is -OH, -NH($C_{1-6}$ alkyl), or -N($C_{1-6}$ alkyl)$_2$.

In another embodiment, $WR^{W2}$ is *tert*-butyl.

In another embodiment, $WR^{W4}$ is *tert*-butyl.

In another embodiment, $WR^{W5}$ is -OH.

**II.A.2. Compound 1**

In another embodiment, the compound of Formula I is Compound 1.

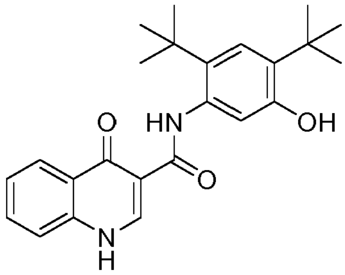

**Compound 1**

Compound 1 is known by the name N-[2,4-bis(1,1-dimethylethyl)-5-hydroxyphenyl]-1,4-dihydro-4-oxoquinoline-3-carboxamide and by the name N-(5-hydroxy-2,4-di-*tert*-butyl-phenyl)-4-oxo-1H-quinoline-3-carboxamide.

**II.A.3. Synthesis of the Compounds of Formula I**

Compounds of Formula I

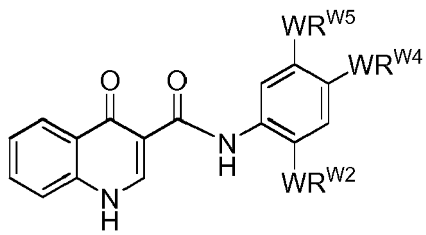

are readily prepared by combining an acid moiety

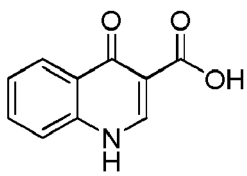

with an amine moiety

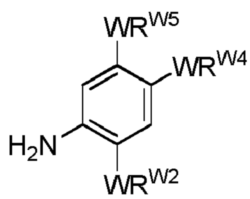

as described herein, wherein $WR^{W2}$, $WR^{W4}$, and $WR^{W5}$ are as defined previously.

**II.A.3.a. Synthesis of the Acid Moiety of Compounds of Formula I**

The acid precursor of compounds of Formula I, dihydroquinoline carboxylic acid, can be synthesized according to Scheme 1-1, by conjugate addition of $EtOCH=C(COOEt)_2$ to aniline, followed by thermal rearrangement and hydrolysis.

**Scheme 1-1: General Synthesis of Compound of Formula I Acid Moiety.**

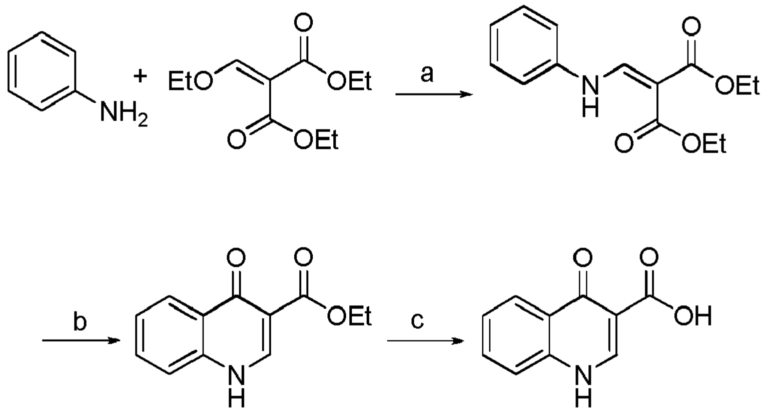


a) heat; b) PPA, $POCl_3$, 70 °C or diphenyl ether, 220 °C; c) i) 2N NaOH ii) 2N HCl; or HCl (aq)

**II.A.3.b. Synthesis of the Amine Moiety of Compounds of Formula I**

Amine precursors of compounds of Formula I are prepared as depicted in Scheme 1-2, wherein $WR^{W2}$, $WR^{W4}$, and $WR^{W5}$ are as defined previously. Thus, ortho alkylation of the para-substituted benzene in step (a) provides a trisubstituted intermediate. Optional protection when $WR^{W5}$ is OH (step (b) and nitration (step c) provides the trisubstituted nitrated intermediate. Optional deprotection (step d) and hydrogenation (step e) provides the desired amine moiety.

**Scheme 1-2: General Synthesis of the Amine Moiety.**

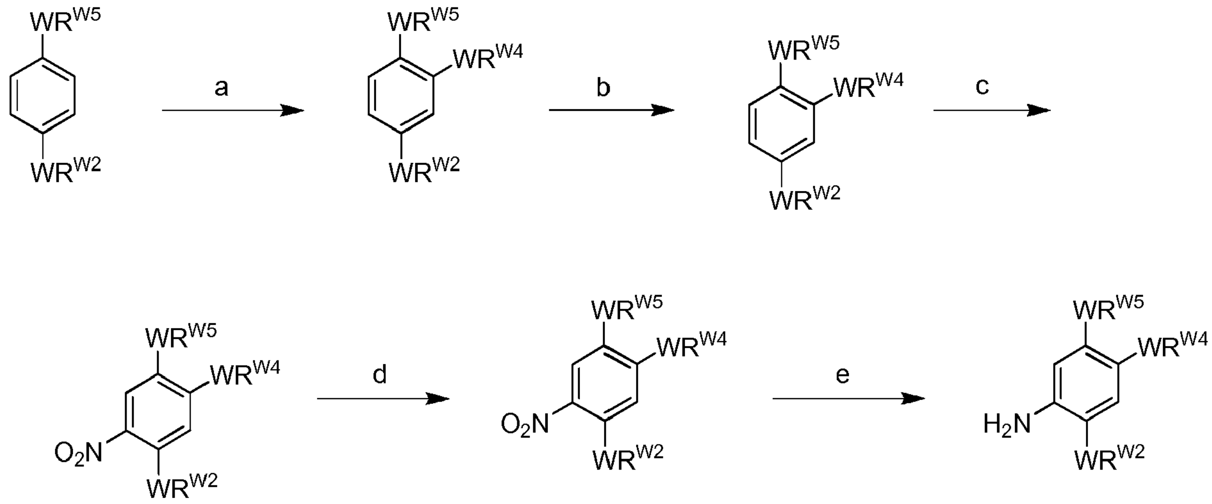


a) $WR^{W4}$-OH, $WR^{W4}$ = alkyl; b) $ClCO_2R$, TEA; c) $HNO_3$, $H_2SO_4$; d) base; e) hydrogenation.

**II.A.3.c. Synthesis of Compounds of Formula I by Acid and Amine Moiety Coupling**

Compounds of Formula I are prepared by coupling an acid moiety with an amine moiety as depicted in Scheme 1-3. In general, the coupling reaction requires a coupling reagent, a base, as well as a solvent. Examples of conditions used include HATU, DIEA; BOP, DIEA, DMF; HBTU, $Et_3N$, $CH_2Cl_2$; PFPTFA, pyridine.

## Scheme 1-3: Preparation of Compounds of Formula I.

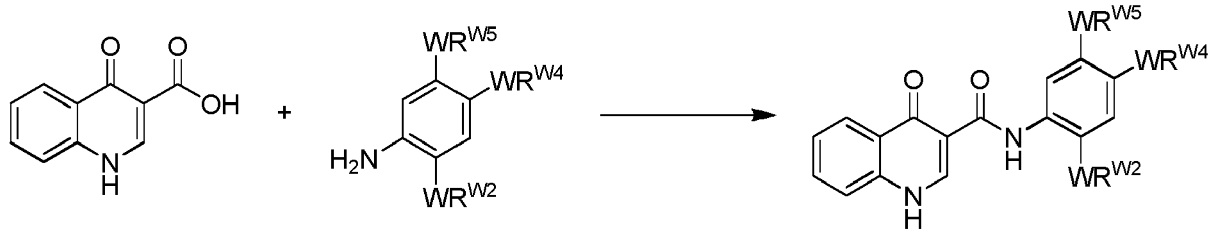

Formula I

### II.A.4. Examples: Synthesis of Compound 1

Compound 1 can be prepared generally as provided in Schemes 1-3 through 1-6, wherein an acid moiety

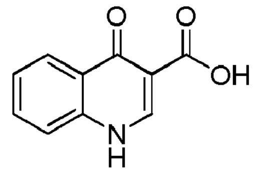

is coupled with an amine moiety

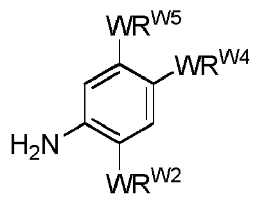

wherein $WR^{W2}$ and $WR^{W4}$ are t-butyl, and $WR^{W5}$ is OH. More detailed schemes and examples are provided below.

### II.A.4.a. Synthesis of Acid Moiety of Compound 1

The synthesis of the acid moiety 4-Oxo-1,4-dihydroquinoline-3-carboxylic acid **26,** is summarized in Scheme 1-4.

## Scheme 1-4: Synthesis of 4-Oxo-1,4-Dihydroquinoline-3-Carboxylic Acid.

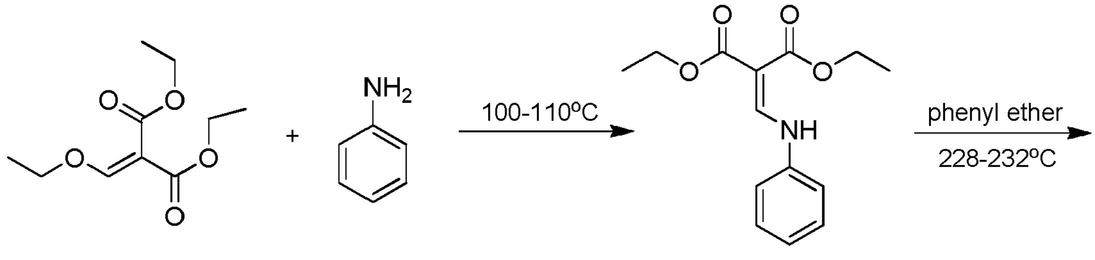


22 23 24

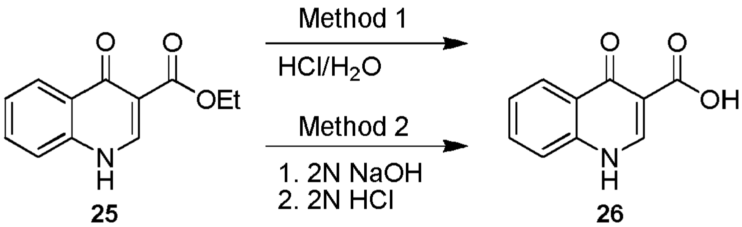


25 26

**Example 1a: Ethyl 4-oxo-1,4-dihydroquinoline-3-carboxylate (25).**

Compound **23** (4.77 g, 47.7 mmol) was added dropwise to Compound **22** (10 g, 46.3 mmol) with subsurface $N_2$ flow to drive out ethanol below 30 °C for 0.5 hours. The solution was then heated to 100-110 °C and stirred for 2.5 hours. After cooling the mixture to below 60 °C, diphenyl ether was added. The resulting solution was added dropwise to diphenyl ether that had been heated to 228-232 °C for 1.5 hours with subsurface $N_2$ flow to drive out ethanol. The mixture was stirred at 228-232 °C for another 2 hours, cooled to below 100 °C and then heptane was added to precipitate the product. The resulting slurry was stirred at 30 °C for 0.5 hours. The solids were then filtered, and the cake was washed with heptane and dried in vacuo to give Compound 25 as a brown solid. $^1H$ NMR (DMSO-$d_6$; 400 MHz) δ 12.25 (s), δ 8.49 (d), δ 8.10 (m), δ 7.64 (m), δ 7.55 (m), δ 7.34 (m), δ 4.16 (q), δ 1.23 (t).

**Example 1b: 4-Oxo-1,4-dihydroquinoline-3-carboxylic acid (26).**

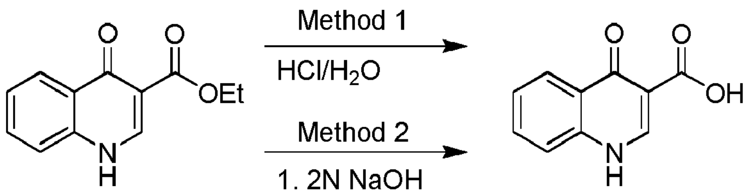


25 26

**Method 1**

Compound **25** (1.0 eq) was suspended in a solution of HCl (10.0 eq) and $H_2O$ (11.6 vol). The slurry was heated to 85 - 90 °C, although alternative temperatures are also suitable for this hydrolysis step. For example, the hydrolysis can alternatively be performed at a temperature of from about 75 to about 100 °C. In some instances, the hydrolysis is performed at a temperature of from about 80 to about 95 °C. In others, the hydrolysis step is performed at a temperature of from about 82 to about 93 °C (e.g., from about 82.5 to about 92.5 °C or from about 86 to about 89 °C). After stirring at 85 - 90 °C for approximately 6.5 hours, the reaction was sampled for reaction completion. Stirring may be performed under any of the temperatures suited for the hydrolysis. The solution was then cooled to 20 - 25 °C and filtered. The reactor/cake was rinsed with $H_2O$ (2 vol x 2). The cake was then washed with 2 vol $H_2O$ until the pH ≥ 3.0. The cake was then dried under vacuum at 60 °C to give Compound **26.**

**Method 2**

Compound **25** (11.3 g, 52 mmol) was added to a mixture of 10% NaOH (aq) (10 mL) and ethanol (100 mL). The solution was heated to reflux for 16 hours, cooled to 20-25 °C and then the pH was adjusted to 2-3 with 8% HCl. The mixture was then stirred for 0.5 hours and filtered. The cake was washed with water (50 mL) and then dried in vacuo to give Compound **26** as a brown solid. $^1H$ NMR (DMSO-$d_6$; 400 MHz) δ 15.33 (s), δ 13.39 (s), δ 8.87 (s), δ 8.26 (m), δ 7.87 (m), δ 7.80 (m), δ 7.56 (m).

**II.A.4.b. Synthesis of Amine Moiety of Compound 1**

The synthesis of the amine moiety **32,** is summarized in Scheme 1-5.

**Scheme 1-5: Synthesis of 5-Amino-2,4-Di-Tert-Butylphenyl Methyl Carbonate (32).**

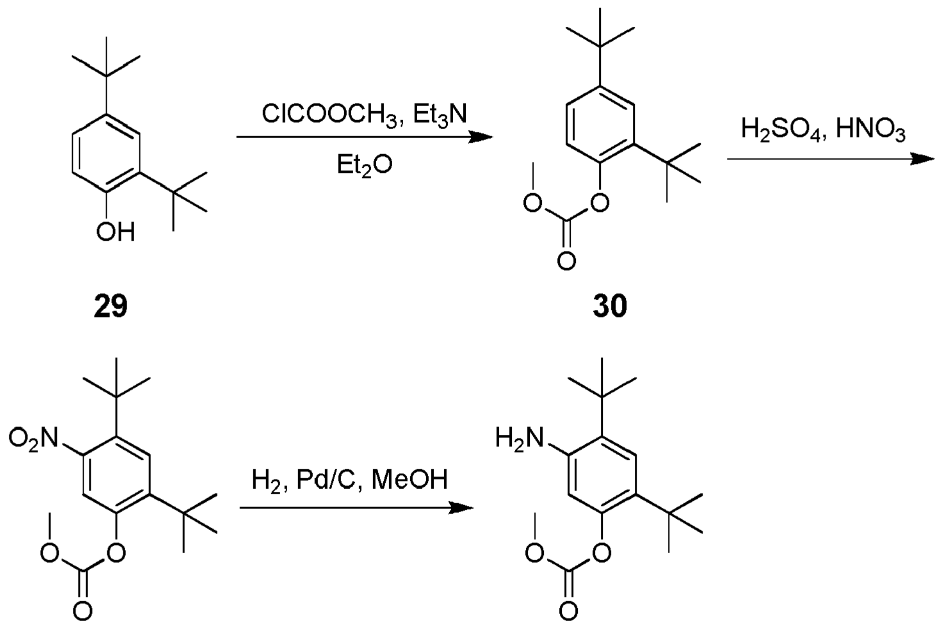


**29** **30**

**31** **32**

**Example 1c: 2,4-Di-tert-butylphenyl methyl carbonate (30).**

**Method 1**

To a solution of 2,4-di-*tert*-butyl phenol, **(29)** (10 g, 48.5mmol) in diethyl ether (100 mL) and triethylamine (10.1 mL, 72.8 mmol), was added methyl chloroformate (7.46 mL, 97 mmol) dropwise at 0 °C. The mixture was then allowed to warm to room temperature and stir for an additional 2 hours. An additional 5 mL triethylamine and 3.7 mL methyl chloroformate was then added and the reaction stirred overnight. The reaction was then filtered, the filtrate was cooled to 0 °C, and an additional 5 mL triethylamine and 3.7 mL methyl chloroformate was then added and the reaction was allowed to warm to room temperature and then stir for an additional 1 hour. At this stage, the reaction was almost complete and was worked up by filtering, then washing with water (2x), followed by brine. The solution was then concentrated to produce a yellow oil and purified using column chromatography to give Compound **30.** $^1H$ NMR (400 MHz, DMSO-$d_6$) δ 7.35 (d, $J$ = 2.4 Hz, 1H), 7.29 (dd, $J$ = 8.4, 2.4 Hz, 1H), 7.06 (d, $J$ = 8.4 Hz, 1H), 3.85 (s, 3H), 1.30 (s, 9H), 1.29 (s, 9H).

**Method 2**

To a reactor vessel charged with 4-dimethylaminopyridine (DMAP, 3.16 g, 25.7 mmol) and 2,4-di*tert*-butyl phenol (Compound **29,** 103.5 g, 501.6 mmol) was added methylene chloride (415 g, 313 mL) and the solution was

agitated until all solids dissolved. Triethylamine (76 g, 751 mmol) was then added and the solution was cooled to 0 - 5 °C. Methyl chloroformate (52 g, 550.3 mmol) was then added dropwise over 2.5 - 4 hours, while keeping the solution temperature between 0 - 5 °C. The reaction mixture was then slowly heated to 23 - 28 °C and stirred for 20 hours. The reaction was then cooled to 10 - 15 °C and charged with 150 mL water. The mixture was stirred at 15 - 20 °C for 35 - 45 minutes and the aqueous layer was then separated and extracted with 150 mL methylene chloride. The organic layers were combined and neutralized with 2.5% HCl (aq) at a temperature of 5 - 20 °C to give a final pH of 5 - 6. The organic layer was then washed with water and concentrated in vacuo at a temperature below 20 °C to 150 mL to give Compound **30.**

**Example 1d: 5-Nitro-2,4-di-tert-butylphenyl methyl carbonate (31).**

**Method 1**

To a stirred solution of Compound **30** (6.77g, 25.6 mmol) was added 6 mL of a 1:1 mixture of sulfuric acid and nitric acid at 0 °C dropwise. The mixture was allowed to warm to room temperature and stirred for 1 hour. The product was purified using liquid chromatography (ISCO, 120 g, 0-7% EtOAc/Hexanes, 38 min) producing about an 8:1 - 10:1 mixture of regioisomers of Compound **31** as a white solid. $^1$H NMR (400 MHz, DMSO-$d_6$) $\delta$ 7.63 (s, 1H), 7.56 (s, 1H), 3.87 (s, 3H), 1.36 (s, 9H), 1.32 (s, 9H). HPLC ret. time 3.92 min 10-99% $CH_3CN$, 5 min run; ESI-MS 310 m/z $(MH)^+$.

**Method 2**

To Compound **30** (100g, 378 mmol) was added DCM (540 g, 408 mL). The mixture was stirred until all solids dissolved, and then cooled to -5 - 0 °C. Concentrated sulfuric acid (163 g) was then added dropwise, while maintaining the initial temperature of the reaction, and the mixture was stirred for 4.5 hours. Nitric acid (62 g) was then added dropwise over 2-4 hours while maintaining the initial temperature of the reaction, and was then stirred at this temperature for an additional 4.5 hours. The reaction mixture was then slowly added to cold water, maintaining a temperature below 5 °C. The quenched reaction was then heated to 25 °C and the aqueous layer was removed and extracted with methylene chloride. The combined organic layers were washed with water, dried using $Na_2SO_4$, and concentrated to 124 - 155 mL. Hexane (48 g) was added and the resulting mixture was again concentrated to 124 - 155 mL. More hexane (160 g) was subsequently added to the mixture. The mixture was then stirred at 23 - 27 °C for 15.5 hours, and was then filtered. To the filter cake was added hexane (115 g), the resulting mixture was heated to reflux and stirred for 2 - 2.5 hours. The mixture was then cooled to 3 - 7 °C, stirred for an additional 1 - 1.5 hours, and filtered to give Compound **31** as a pale yellow solid.

**Example 1e: 5-Amino-2,4-di-tert-butylphenyl methyl carbonate (32).**

2,4-Di-tert-butyl-5-nitrophenyl methyl carbonate (1.00 eq) was charged to a suitable hydrogenation reactor, followed by 5% Pd/C (2.50 wt% dry basis, Johnson-Matthey Type 37). MeOH (15.0 vol) was charged to the reactor, and the system was closed. The system was purged with $N_2$ (g), and was then pressurized to 2.0 Bar with $H_2$ (g). The reaction was performed at a reaction temperature of 25 °C +/- 5 °C. When complete, the reaction was filtered, and the reactor/cake was washed with MeOH (4.00 vol). The resulting filtrate was distilled under vacuum at no more than 50 °C to 8.00 vol. Water (2.00 vol) was added at 45 °C +/- 5 °C. The resultant slurry was cooled to 0 °C +/- 5 . The slurry was held at 0 °C +/- 5 °C for no less than 1 hour, and filtered. The cake was washed once with 0 °C +/- 5 °C MeOH/$H_2O$ (8:2) (2.00 vol). The cake was dried under vacuum (-0.90 bar and -0.86 bar) at 35 °C - 40 °C to give Compound **32.** $^1$H NMR (400 MHz, DMSO-$d_6$) $\delta$ 7.05 (s, 1H), 6.39 (s, 1H), 4.80 (s, 2H), 3.82 (s, 3H), 1.33 (s, 9H), 1.23 (s, 9H).

Once the reaction was complete, the resulting mixture was diluted with from about 5 to 10 volumes of MeOH (e.g., from about 6 to about 9 volumes of MeOH, from about 7 to about 8.5 volumes of MeOH, from about 7.5 to about 8 volumes of MeOH, or about 7.7 volumes of MeOH), heated to a temperature of about 35 $\pm$ 5 °C, and filtered to remove palladium. The reactor cake was washed before combining the filtrate and wash, distilling, adding water, cooling, filtering, washing and drying the product cake as described above.

**II.A.4.c. Synthesis of Compound 1 by Acid and Amine Moiety Coupling**

The coupling of the acid moiety to the amine moiety is summarized in Scheme 1-6.

## Scheme 1-6: Synthesis of Compound 1

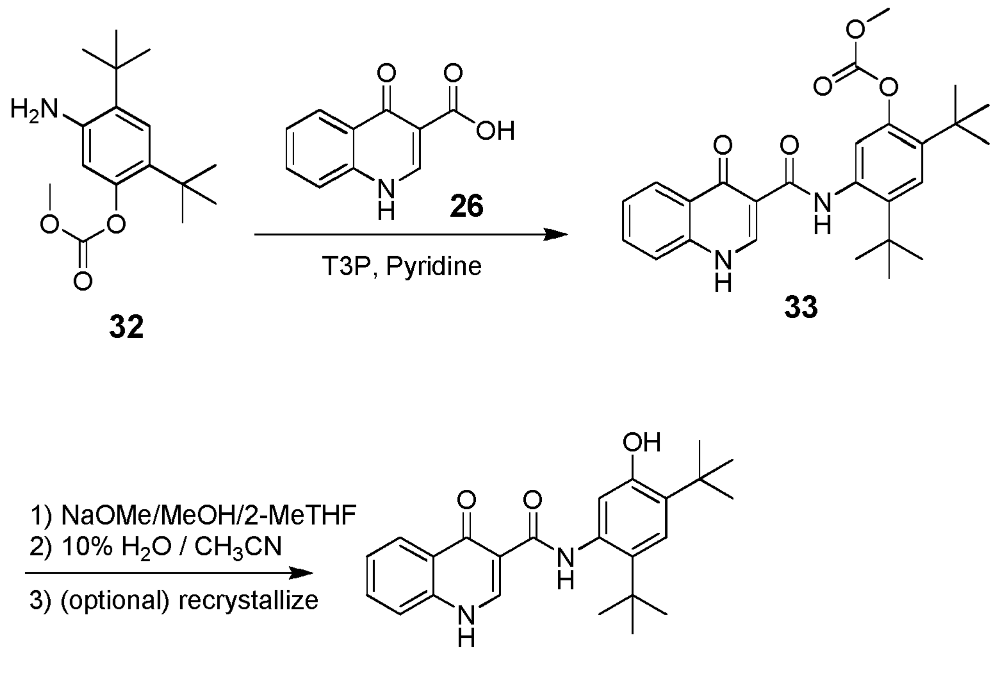


32

33

1

**Example If: N-(2,4-di-tert-butyl-5-hydroxyphenyl)-4-oxo-1,4-dihydroquinoline-3-carboxamide (1).**

4-Oxo-1,4-dihydroquinoline-3-carboxylic acid **(26)** (1.0 eq) and 5-amino-2,4-di-tert-butylphenyl methyl carbonate **(32)** (1.1 eq) were charged to a reactor. 2-MeTHF (4.0 vol, relative to the acid) was added followed by T3P® 50% solution in 2-MeTHF (1.7 eq). The T3P charged vessel was washed with 2-MeTHF (0.6 vol). Pyridine (2.0 eq) was then added, and the resulting suspension was heated to 47.5 +/- 5.0 °C and held at this temperature for 8 hours. A sample was taken and checked for completion by HPLC. Once complete, the resulting mixture was cooled to 25.0 °C +/- 2.5 °C. 2-MeTHF was added (12.5 vol) to dilute the mixture. The reaction mixture was washed with water (10.0 vol) 2 times. 2-MeTHF was added to bring the total volume of reaction to 40.0 vol (-16.5 vol charged). To this solution was added NaOMe/MeOH (1.7 equiv) to perform the methanolysis. The reaction was stirred for no less than 1.0 hour, and checked for completion by HPLC. Once complete, the reaction was quenched with 1 N HCl (10.0 vol), and washed with 0.1 N HCl (10.0 vol). The organic solution was polish filtered to remove any particulates and placed in a second reactor. The filtered solution was concentrated at no more than 45 °C (jacket temperature) and no less than 8.0 °C (internal reaction temperature) under reduced pressure to 20 vol. $CH_3CN$ was added to 40 vol and the solution concentrated at no more than 45 °C (jacket temperature) and no less than 8.0 °C (internal reaction temperature) to 20 vol. The addition of $CH_3CN$ and concentration cycle was repeated 2 more times for a total of 3 additions of $CH_3CN$ and 4 concentrations to 20 vol. After the final concentration to 20 vol, 16.0 vol of $CH_3CN$ was added followed by 4.0 vol of $H_2O$ to make a final concentration of 40 vol of 10% $H_2O/CH_3CN$ relative to the starting acid. This slurry was heated to 78.0 °C +/- 5.0 °C (reflux). The slurry was then stirred for no less than 5 hours. The slurry was cooled to 0.0 °C +/- 5 °C over 5 hours, and filtered. The cake was washed with 0.0 °C +/- 5.0 °C $CH_3CN$ (5 vol) 4 times. The resulting solid (Compound 1) was dried in a vacuum oven at no more than 50.0 °C. $^1H$ NMR (400 MHz, DMSO-$d_6$) δ 12.8 (s, 1H), 11.8 (s, 1H), 9.2 (s, 1H), 8.9 (s, 1H), 8.3 (s, 1H), 7.2 (s, 1H), 7.9 (t, 1H), 7.8 (d, 1H), 7.5 (t, 1H), 7.1 (s, 1H), 1.4 (s, 9H), 1.4 (s, 9H).

An alternative synthesis of Compound 1 is depicted in Scheme 1-7.

**Scheme 1-7: Alternate Synthesis of Compound 1.**

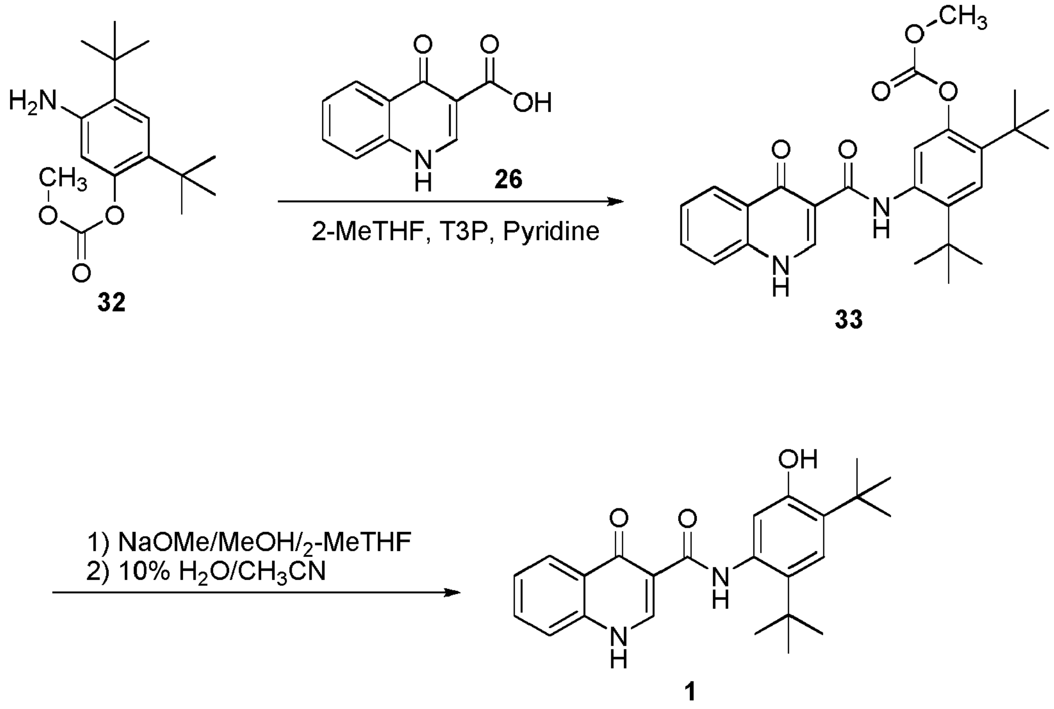


32 33 1

**Example 1g: N-(2,4-di-tert-butyl-5-hydroxyphenyl)-4-oxo-1,4-dihydroquinoline-3-carboxamide (1).** 4-Oxo-1,4-dihydroquinoline-3-carboxylic acid **26** (1.0 eq) and 5-amino-2,4-di-tert-butylphenyl methyl carbonate **32** (1.1 eq) were charged to a reactor. 2-MeTHF (4.0 vol, relative to the acid) was added followed by T3P® 50% solution in 2-MeTHF (1.7 eq). The T3P charged vessel was washed with 2-MeTHF (0.6 vol). Pyridine (2.0 eq) was then added, and the resulting suspension was heated to 47.5 +/-5.0 °C and held at this temperature for 8 hours. A sample was taken and checked for completion by HPLC. Once complete, the resulting mixture was cooled to 20 °C +/- 5 °C. 2-MeTHF was added (12.5 vol) to dilute the mixture. The reaction mixture was washed with water (10.0 vol) 2 times and 2-MeTHF (16.5 vol) was charged to the reactor. This solution was charged with 30% w/w NaOMe/MeOH (1.7 equiv) to perform the methanolysis. The reaction was stirred at 25.0 °C +/- 5.0 °C for no less than 1.0 hour, and checked for completion by HPLC. Once complete, the reaction was quenched with 1.2 N $HCl/H_2O$ (10.0 vol), and washed with 0.1 N $HCl/H_2O$ (10.0 vol). The organic solution was polish filtered to remove any particulates and placed in a second reactor.

The filtered solution was concentrated at no more than 45 °C (jacket temperature) and no less than 8.0 °C (internal reaction temperature) under reduced pressure to 20 vol. $CH_3CN$ was added to 40 vol and the solution concentrated at no more than 45 °C (jacket temperature) and no less than 8.0 °C (internal reaction temperature) to 20 vol. The addition of $CH_3CN$ and concentration cycle was repeated 2 more times for a total of 3 additions of $CH_3CN$ and 4 concentrations to 20 vol. After the final concentration to 20 vol, 16.0 vol of $CH_3CN$ was charged followed by 4.0 vol of $H_2O$ to make a final concentration of 40 vol of 10% $H_2O/CH_3CN$ relative to the starting acid. This slurry was heated to 78.0 °C +/- 5.0 °C (reflux). The slurry was then stirred for no less than 5 hours. The slurry was cooled to 20 to 25 °C over 5 hours, and filtered. The cake was washed with $CH_3CN$ (5 vol) heated to 20 to 25 °C 4 times. The resulting solid (Compound 1) was dried in a vacuum oven at more than 50.0 °C. $^1H$ NMR (400 MHz, DMSO-$d_6$) $\delta$ 12.8 (s, 1H), 11.8 (s, 1H), 9.2 (s, 1H), 8.9 (s, 1H), 8.3 (s, 1H), 7.2 (s, 1H), 7.9 (t, 1H), 7.8 (d, 1H), 7.5 (t, 1H), 7.1 (s, 1H), 1.4 (s, 9H), 1.4 (s, 9H).

**II.B. Compounds of Formula II**

**II.B.1. Embodiments of the Compounds of Formula II**

In one aspect the invention includes a pharmaceutical composition comprising a Compound of Formula II

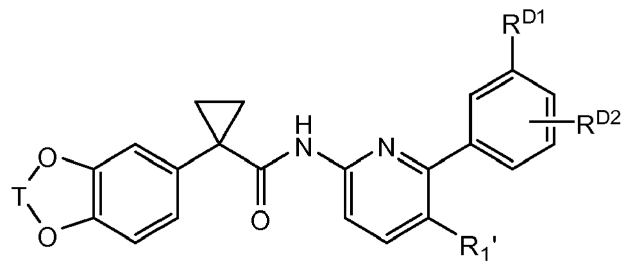

Formula II

or pharmaceutically acceptable salts thereof, wherein:

T is $-CH_2-$, $-CH_2CH_2-$, $-CF_2-$, $-C(CH_3)_2-$, or -C(O)-;
$R_1$' is H, $C_{1-6}$ aliphatic, halo, $CF_3$, $CHF_2$, O($C_{1-6}$ aliphatic); and
$R^{D1}$ or $R^{D2}$ is $Z^DR_9$
wherein:

$Z^D$ is a bond, CONH, $SO_2NH$, $SO_2N(C_{1-6}$ alkyl), $CH_2NHSO_2$, $CH_2N(CH_3)SO_2$, $CH_2NHCO$, COO, $SO_2$, or CO; and
$R_9$ is H, $C_{1-6}$ aliphatic, or aryl.

**II.B.2. Compound 2**

In another embodiment, the compound of Formula II is Compound 2, depicted below, which is also known by its chemical name 3-(6-(1-(2,2-difluorobenzo[d][1,3]dioxol-5-yl)cyclopropanecarboxamido)-3-methylpyridin-2-yl)benzoic acid.

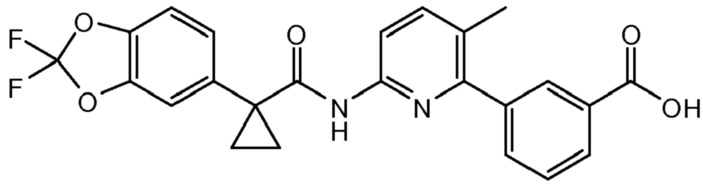

Compound 2

**II.B.3. Overview of the Synthesis of Compound 2**

Compounds of Formula II, as exemplified by Compound 2, can be prepared by coupling an acid chloride moiety with an amine moiety according to following Schemes 2-1 a to 2-3 .

**Scheme 2-1a: Synthesis of the Acid Chloride Moiety.**

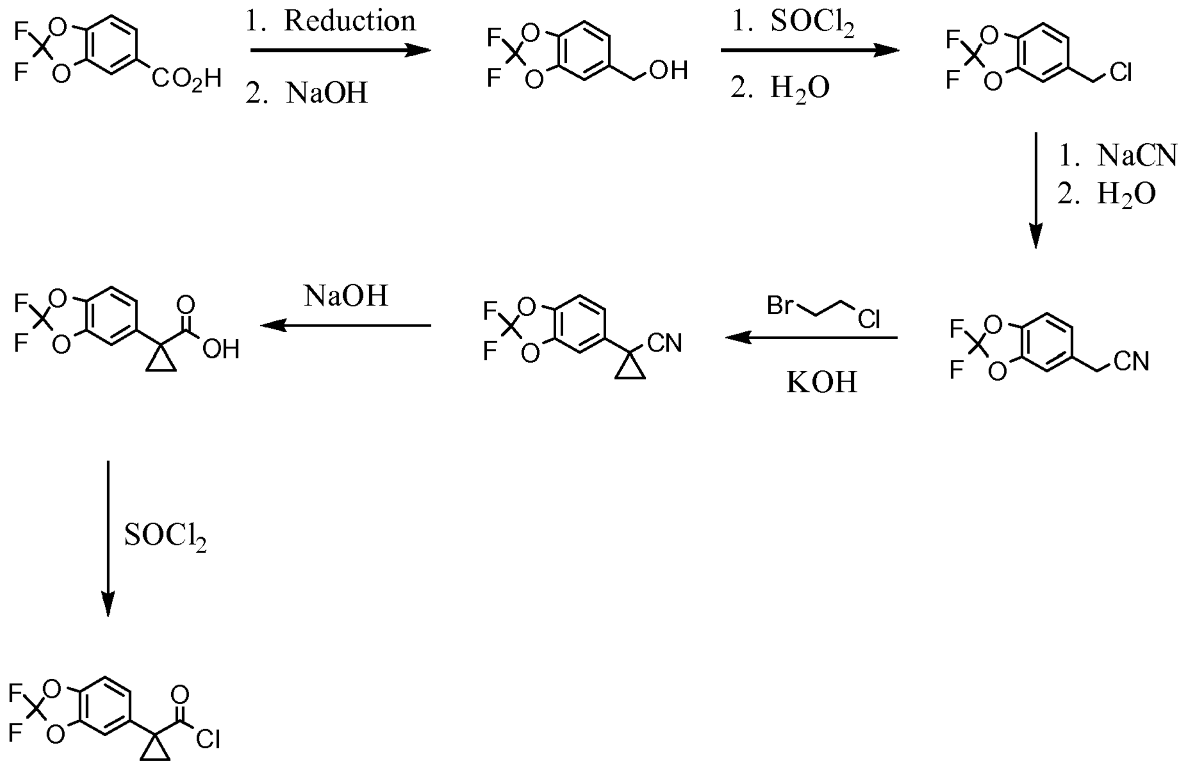


Scheme 2-1a depicts the preparation of 1-(2,2-difluorobenzo[d][1,3]dioxol-5-yl)cyclopropanecarbonyl chloride, which is used in Scheme 3 to make the amide linkage of Compound 2.

The starting material, 2,2-difluorobenzo[d][1,3]dioxole-5-carboxylic acid, is commercially available from Saltigo (an affiliate of the Lanxess Corporation). Reduction of the carboxylc acid moiety in 2,2-difluorobenzo[d][1,3]dioxole-5-carboxylic acid to the primary alcohol, followed by conversion to the corresponding chloride using thionyl chloride ($SOCl_2$), provides 5-(chloromethyl)-2,2-difluorobenzo[d][1,3]dioxole, which is subsequently converted to 2-(2,2-difluorobenzo[d][1,3]dioxol-5-yl)acetonitrile using sodium cyanide. Treatment of 2-(2,2-difluorobenzo[d][1,3]dioxol-5-yl)acetonitrile with base and 1-bromo-2-chloroethane provides 1-(2,2-difluorobenzo[d][1,3]dioxol-5-yl)cyclopropanecarbonitrile. The nitrile moiety in 1-(2,2-difluorobenzo[d][1,3]dioxol-5-yl)cyclopropanecarbonitrile is converted to a carboxylic acid using base to give 1-(2,2-difluorobenzo[d][1,3]dioxol-5-yl)cyclopropanecarboxylic acid, which is converted to the desired acid chloride using thionyl chloride.

**Scheme 2-1b. Alternative Synthesis of the Acid Chloride Moiety.**

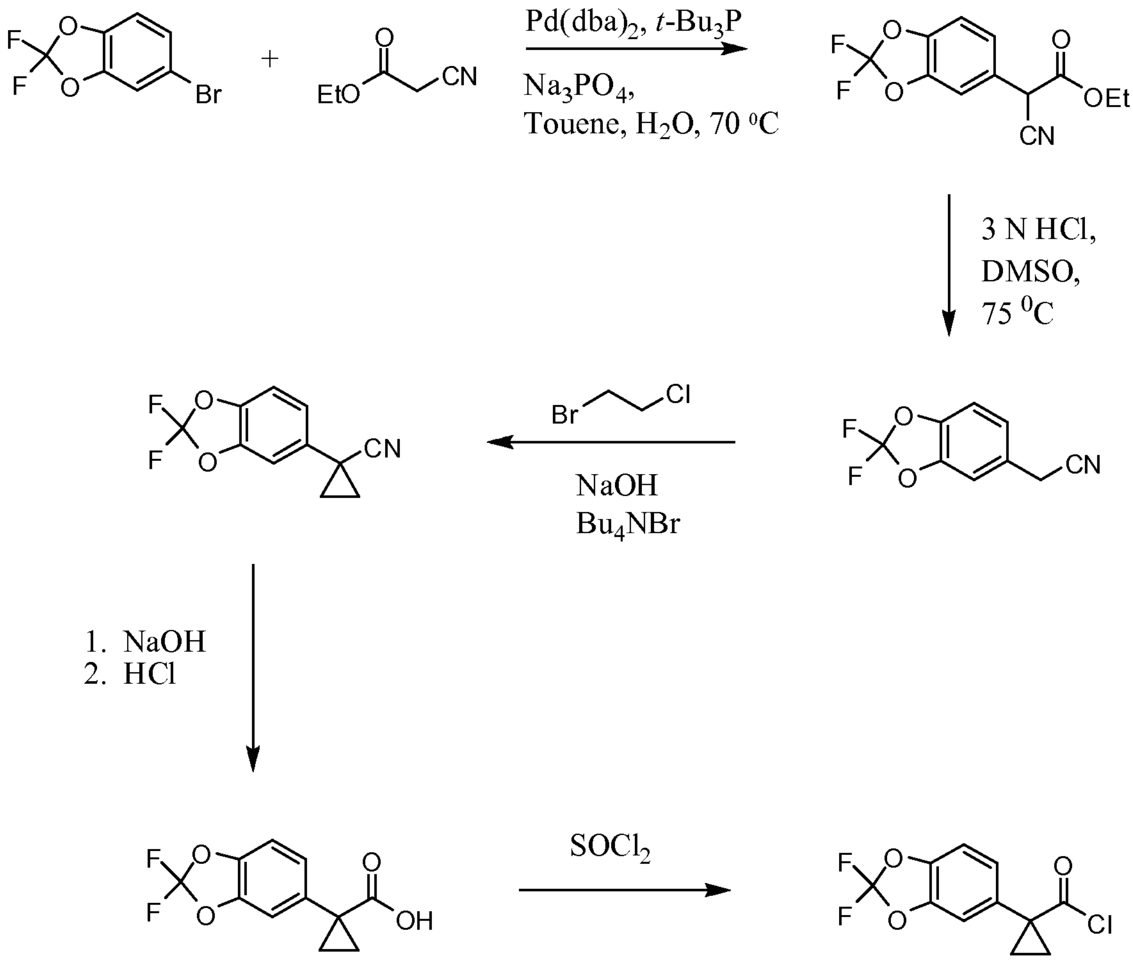


Scheme 2-1b provides an alternative synthesis of the requisite acid chloride. The compound 5-bromomethyl-2,2-difluoro-1,3-benzodioxole is coupled with ethyl cyanoacetate in the presence of a palladium catalyst to form the corresponding alpha cyano ethyl ester. Saponification of the ester moiety to the carboxylic acid gives the cyanoethyl compound. Alkylation of the cyanoethyl compound with 1-bromo-2-chloro ethane in the presence of base gives the cyanocyclopropyl compound. Treatment of the cyanocyclopropyl compound with base gives the carboxylate salt, which is converted to the carboxylic acid by treatment with acid. Conversion of the carboxylic acid to the acid chloride is then accomplished using a chlorinating agent such as thionyl chloride or the like.

## Scheme 2-2: Synthesis of the Amine Moiety.

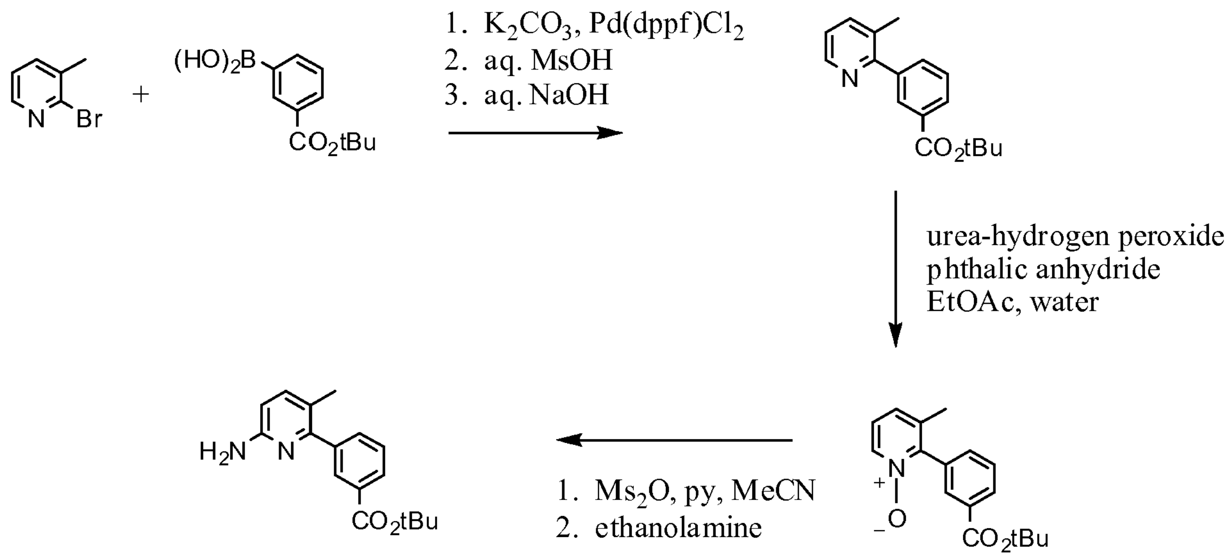


Scheme 2-2 depicts the preparation of the requisite tert-butyl 3-(6-amino-3-methylpyridin-2-yl)benzoate, which is coupled with 1-(2,2-difluorobenzo[d][1,3]dioxol-5-yl)cyclopropanecarbonyl chloride in Scheme 3 to give Compound 2. Palladium-catalyzed coupling of 2-bromo-3-methylpyridine with 3-(tert-butoxycarbonyl)phenylboronic acid gives tert-butyl 3-(3-methylpyridin-2-yl)benzoate, which is subsequently converted to the desired compound.

**Scheme 2-3: Formation of an acid salt of 3-(6-(1-(2,2-difluorobenzo[d][1,3]dioxol-5-yl) cyclopropanecarboxamido)-3-methylpyridin-2-yl)benzoic Acid.**

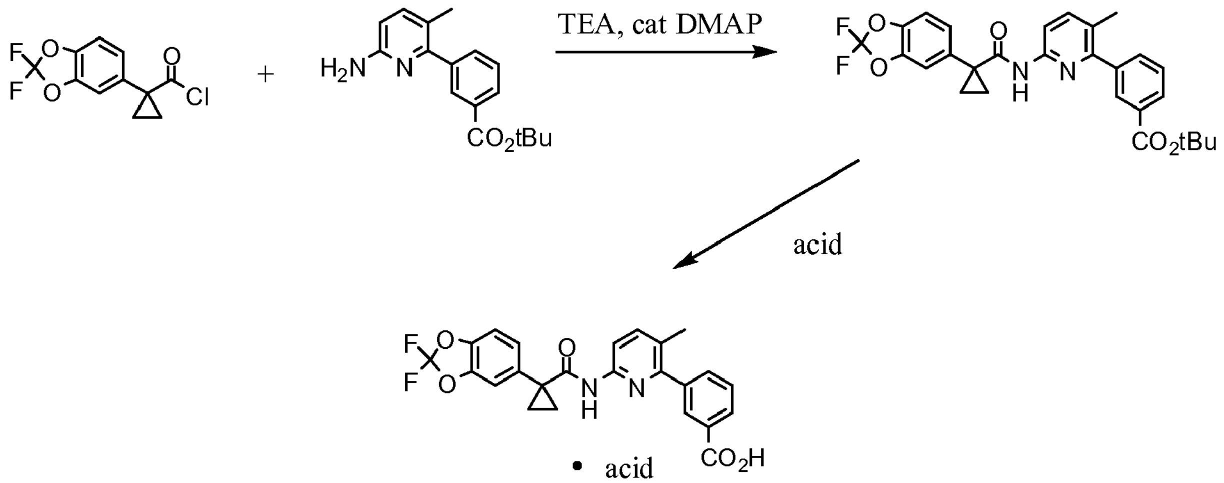


Scheme 2-3 depicts the coupling of 1-(2,2-difluorobenzo[d][1,3]dioxol-5-yl)cyclopropanecarbonyl chloride with tert-butyl 3-(6-amino-3-methylpyridin-2-yl)benzoate using triethyl amine and 4-dimethylaminopyridine to initially provide the tert-butyl ester of Compound 2. Treatment of the tert-butyl ester with an acid such as HCl, gives the HCl salt of Compound 2, which is typically a crystalline solid.

**II.B.4. Examples: Synthesis of Compound 2**

Vitride® (sodium bis(2-methoxyethoxy)aluminum hydride [or NaA1$H_2(OCH_2CH_2OCH_3)_2$], 65 wgt% solution in toluene) was purchased from Aldrich Chemicals. 2,2-Difluoro-1,3-benzodioxole-5-carboxylic acid was purchased from

Saltigo (an affiliate of the Lanxess Corporation).

**Example 2a: (2,2-Difluoro-1,3-benzodioxol-5-yl)-methanol.**

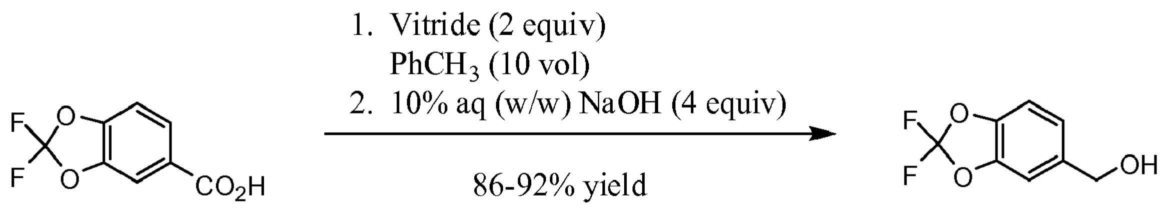


Commercially available 2,2-difluoro-1,3-benzodioxole-5-carboxylic acid (1.0 eq) was slurried in toluene (10 vol). Vitride® (2 eq) was added via addition funnel at a rate to maintain the temperature at 15-25 °C. At the end of the addition, the temperature was increased to 40 °C for 2 hours (h), then 10% (w/w) aqueous (aq) NaOH (4.0 eq) was carefully added via addition funnel, maintaining the temperature at 40-50 °C. After stirring for an additional 30 minutes (min), the layers were allowed to separate at 40 °C. The organic phase was cooled to 20 °C, then washed with water (2 x 1.5 vol), dried ($Na_2SO_4$), filtered, and concentrated to afford crude (2,2-difluoro-1,3-benzodioxol-5-yl)-methanol that was used directly in the next step.

**Example 2b: 5-Chloromethyl-2,2-difluoro-1,3-benzodioxole.**

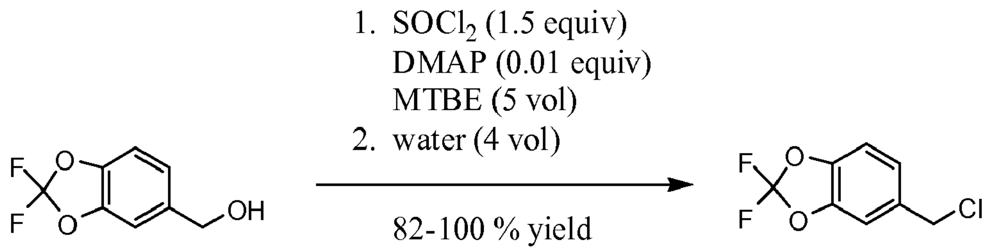


(2,2-Difluoro-1,3-benzodioxol-5-yl)-methanol (1.0 eq) was dissolved in MTBE (5 vol). A catalytic amount of 4-(N,N-dimethyl)aminopyridine (DMAP) (1 mol %) was added and $SOCl_2$ (1.2 eq) was added via addition funnel. The $SOCl_2$ was added at a rate to maintain the temperature in the reactor at 15-25 °C. The temperature was increased to 30 °C for 1 h, and then was cooled to 20 °C. Water (4 vol) was added via addition funnel while maintaining the temperature at less than 30 °C. After stirring for an additional 30 min, the layers were allowed to separate. The organic layer was stirred and 10% (w/v) aq NaOH (4.4 vol) was added. After stirring for 15 to 20 min, the layers were allowed to separate. The organic phase was then dried ($Na_2SO_4$), filtered, and concentrated to afford crude 5-chloromethyl-2,2-difluoro-1,3-benzodioxole that was used directly in the next step.

**Example 2c: (2,2-Difluoro-1,3-benzodioxol-5-yl)-acetonitrile.**

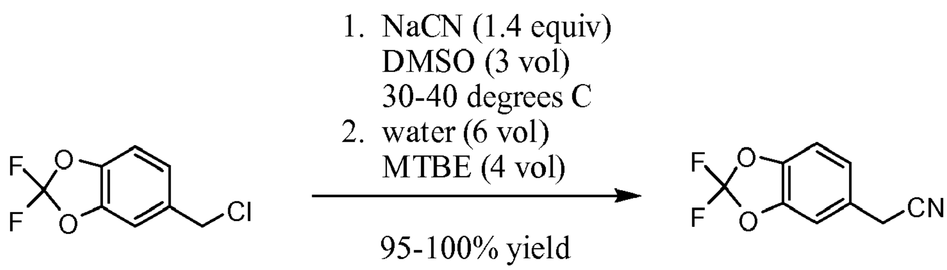

A solution of 5-chloromethyl-2,2-difluoro-1,3-benzodioxole (1 eq) in DMSO (1.25 vol) was added to a slurry of NaCN (1.4 eq) in DMSO (3 vol), while maintaining the temperature between 30-40 °C. The mixture was stirred for 1 h, and then water (6 vol) was added, followed by methyl *tert*-butyl ether (MTBE) (4 vol). After stirring for 30 min, the layers were separated. The aqueous layer was extracted with MTBE (1.8 vol). The combined organic layers were washed with water (1.8 vol), dried ($Na_2SO_4$), filtered, and concentrated to afford crude (2,2-difluoro-1,3-benzodioxol-5-yl)-acetonitrile (95%) that was used directly in the next step. $^1$H NMR (500 MHz, DMSO) δ 7.44 (br s, 1H), 7.43 (d, *J* = 8.4 Hz, 1H), 7.22 (dd, *J* = 8.2, 1.8 Hz, 1H), 4.07 (s, 2H).

**Example 2d: Alternate Synthesis of (2,2-difluoro-1,3-benzodioxol-5-yl)-1-ethylacetate-acetonitrile**

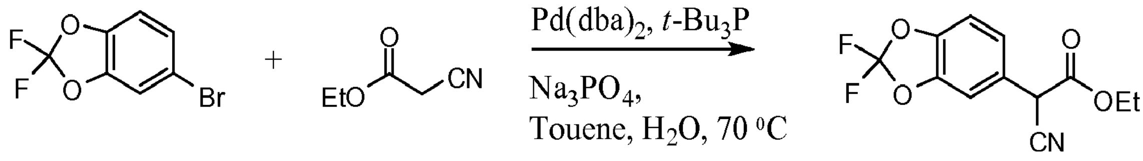


A reactor was purged with nitrogen and charged with toluene (900 mL). The solvent was degassed via nitrogen sparge for no less than 16 hours. To the reactor was then charged $Na_3PO_4$ (155.7 g, 949.5 mmol), followed by bis(dibenzylideneacetone) palladium (0) (7.28 g, 12.66 mmol). A 10% w/w solution of *tert*-butylphosphine in hexanes (51.23 g, 25.32 mmol) was charged over 10 minutes at 23 °C from a nitrogen purged addition funnel. The mixture was allowed to stir for 50 minutes, at which time 5-bromo-2,2-difluoro-1,3-benzodioxole (75 g, 316.5 mmol) was added over 1 minute. After stirring for an additional 50 minutes, the mixture was charged with ethyl cyanoacetate (71.6 g, 633.0 mmol) over 5 minutes, followed by water (4.5 mL) in one portion. The mixture was heated to 70 °C over 40 minutes and analyzed by HPLC every 1 to 2 hours for the percent conversion of the reactant to the product. After complete conversion was observed (typically 100% conversion after 5 to 8 hours), the mixture was cooled to 20 to 25 °C and filtered through a celite pad. The celite pad was rinsed with toluene (2 X 450 mL), and the combined organics were concentrated to 300 mL under vacuum at 60 to 65 °C. The concentrate was charged with DMSO (225mL) and concentrated under vacuum at 70 to 80 °C until active distillation of the solvent ceased. The solution was cooled to 20 to 25 °C and diluted to 900 mL with DMSO in preparation for Step 2. $^1$H NMR (500 MHz, $CDCl_3$) δ 7.16 - 7.10 (m, 2H), 7.03 (d, *J* = 8.2 Hz, 1H), 4.63 (s, 1H), 4.19 (m, 2H), 1.23 (t, *J* = 7.1 Hz, 3H).

**Example 2e: Alternate Synthesis of (2,2-difluoro-1,3-benzodioxol-5-yl)-acetonitrile.**

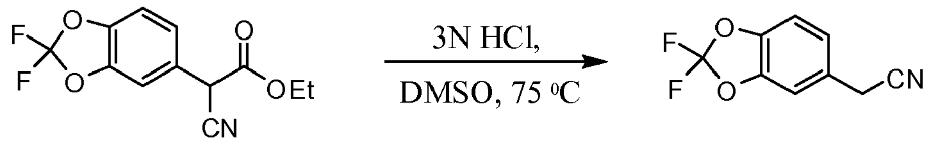


The DMSO solution of (2,2-difluoro-1,3-benzodioxol-5-yl)-1-ethylacetate-acetonitrile from above was charged with 3 N HCl (617.3 mL, 1.85 mol) over 20 minutes while maintaining an internal temperature less than 40 °C. The mixture was then heated to 75 °C over 1 hour and analyzed by HPLC every 1 to 2 hour for percent conversion. When a conversion of greater than 99% was observed (typically after 5 to 6 hours), the reaction was cooled to 20 to 25 °C and extracted with MTBE (2 X 525 mL), with sufficient time to allow for complete phase separation during the extractions. The combined organic extracts were washed with 5% NaCl (2 X 375 mL). The solution was then transferred to equipment appropriate for a 1.5 to 2.5 Torr vacuum distillation that was equipped with a cooled receiver flask. The solution was concentrated under vacuum at less than 60°C to remove the solvents. (2,2-Difluoro-1,3-benzodioxol-5-yl)-acetonitrile was then distilled from the resulting oil at 125 to 130 °C (oven temperature) and 1.5 to 2.0 Torr. (2,2-Difluoro-1,3-benzodioxol-5-yl)-acetonitrile was isolated as a clear oil in 66% yield from 5-bromo-2,2-difluoro-1,3-benzodioxole (2 steps) and with an HPLC purity of 91.5% AUC (corresponds to a w/w assay of 95%). $^1$H NMR (500 MHz, DMSO) δ 7.44 (br s, 1H), 7.43 (d, *J* = 8.4 Hz, 1H), 7.22 (dd, *J* = 8.2, 1.8 Hz, 1H), 4.07 (s, 2H).

**Example 2f: (2,2-Difluoro-1,3-benzodioxol-5-yl)-cyclopropanecarbonitrile.**

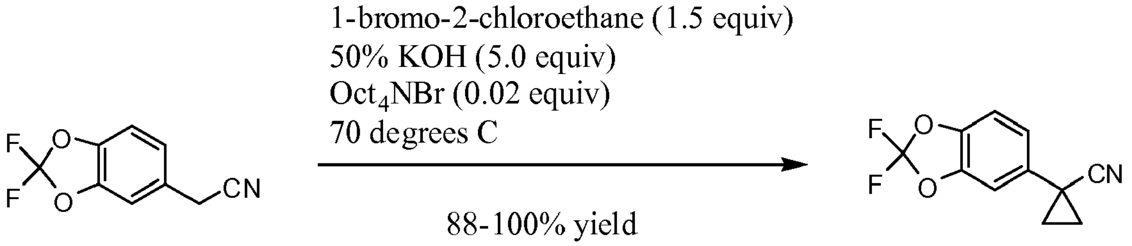


A mixture of (2,2-difluoro-1,3-benzodioxol-5-yl)-acetonitrile (1.0 eq), 50 wt % aqueous KOH (5.0 eq) 1-bromo-2-chloroethane (1.5 eq), and $Oct_4NBr$ (0.02 eq) was heated at 70 °C for 1 h. The reaction mixture was cooled, then worked up with MTBE and water. The organic phase was washed with water and brine. The solvent was removed to afford (2,2-difluoro-1,3-benzodioxol-5-yl)-cyclopropanecarbonitrile. $^1H$ NMR (500 MHz, DMSO) δ 7.43 (d, *J* = 8.4 Hz, 1H), 7.40 (d, *J* = 1.9 Hz, 1H), 7.30 (dd, *J*= 8.4, 1.9 Hz, 1H), 1.75 (m, 2H), 1.53 (m, 2H).

**Example 2g: 1-(2,2-Difluoro-1,3-benzodioxol-5-yl)-cyclopropanecarboxylic acid.**

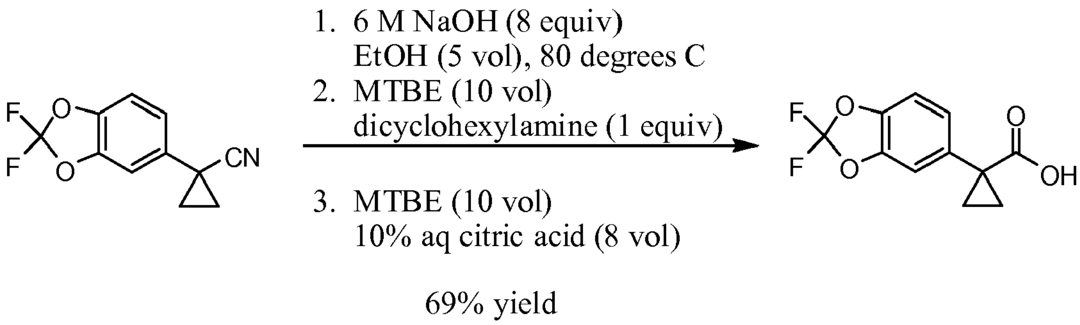


(2,2-Difluoro-1,3-benzodioxol-5-yl)-cyclopropanecarbonitrile was hydrolyzed using 6 M NaOH (8 equiv) in ethanol (5 vol) at 80 °C overnight. The mixture was cooled to room temperature and the ethanol was evaporated under vacuum. The residue was taken up in water and MTBE, 1 M HCl was added, and the layers were separated. The MTBE layer was then treated with dicyclohexylamine (DCHA) (0.97 equiv). The slurry was cooled to 0 °C, filtered and washed with heptane to give the corresponding DCHA salt. The salt was taken into MTBE and 10% citric acid and stirred until all the solids had dissolved. The layers were separated and the MTBE layer was washed with water and brine. A solvent swap to heptane followed by filtration gave 1-(2,2-difluoro-1,3-benzodioxol-5-yl)-cyclopropanecarboxylic acid after drying in a vacuum oven at 50 °C overnight. ESI-MS *m/z* calc. 242.04, found 241.58 $(M+1)^+$; $^1H$ NMR (500 MHz, DMSO) δ 12.40 (s, 1H), 7.40 (d, *J* = 1.6 Hz, 1H), 7.30 (d, *J* = 8.3 Hz, 1H), 7.17 (dd, *J* = 8.3, 1.7 Hz, 1H), 1.46 (m, 2H), 1.17 (m, 2H).

**Example 2h: 1-(2,2-Difluoro-1,3-benzodioxol-5-yl)-cyclopropanecarbonyl chloride.**

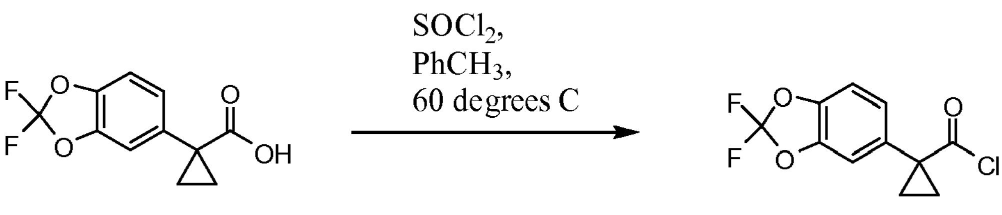


1-(2,2-Difluoro-1,3-benzodioxol-5-yl)-cyclopropanecarboxylic acid (1.2 eq) is slurried in toluene (2.5 vol) and

the mixture was heated to 60 °C. $SOCl_2$ (1.4 eq) was added via addition funnel. The toluene and $SOCl_2$ were distilled from the reaction mixture after 30 minutes. Additional toluene (2.5 vol) was added and the resulting mixture was distilled again, leaving the product acid chloride as an oil, which was used without further purification.

**Example 2i: *tert*-Butyl-3-(3-methylpyridin-2-yl)benzoate.**

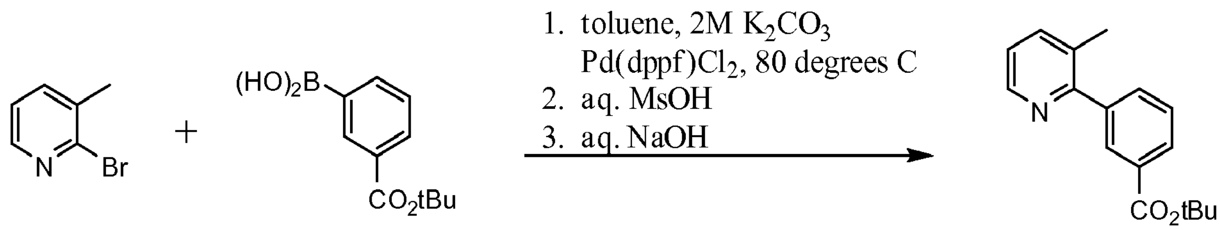


2-Bromo-3-methylpyridine (1.0 eq) was dissolved in toluene (12 vol). $K_2CO_3$ (4.8 eq) was added, followed by water (3.5 vol). The resulting mixture was heated to 65 °C under a stream of $N_2$ for 1 hour. 3-(*t*-Butoxycarbonyl)phenylboronic acid (1.05 eq) and Pd(dppf)$Cl_2$·$CH_2Cl_2$ (0.015 eq) were then added and the mixture was heated to 80 °C. After 2 hours, the heat was turned off, water was added (3.5 vol), and the layers were allowed to separate. The organic phase was then washed with water (3.5 vol) and extracted with 10% aqueous methanesulfonic acid (2 eq MsOH, 7.7 vol). The aqueous phase was made basic with 50% aqueous NaOH (2 eq) and extracted with EtOAc (8 vol). The organic layer was concentrated to afford crude *tert*-butyl-3-(3-methylpyridin-2-yl)benzoate (82%) that was used directly in the next step.

**Example 2j: 2-(3-(*tert*-Butoxycarbonyl)phenyl)-3-methylpyridine-1-oxide.**

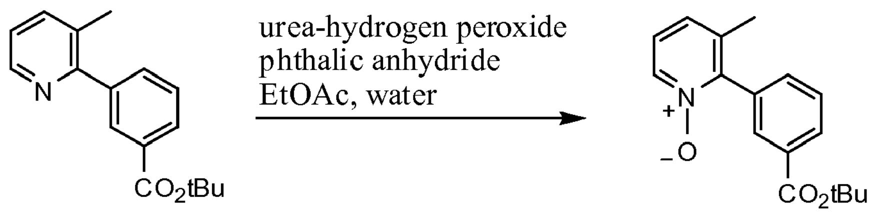


tert-Butyl-3-(3-methylpyridin-2-yl)benzoate (1.0 eq) was dissolved in EtOAc (6 vol). Water (0. 3 vol) was added, followed by urea-hydrogen peroxide (3 eq). Phthalic anhydride (3 eq) was then added portionwise to the mixture as a solid at a rate to maintain the temperature in the reactor below 45 °C. After completion of the phthalic anhydride addition, the mixture was heated to 45 °C. After stirring for an additional 4 hours, the heat was turned off. 10% w/w aqueous $Na_2SO_3$ (1.5 eq) was added via addition funnel. After completion of $Na_2SO_3$ addition, the mixture was stirred for an additional 30 min and the layers separated. The organic layer was stirred and 10% wt/wt aqueous. $Na_2CO_3$ (2 eq) was added. After stirring for 30 minutes, the layers were allowed to separate. The organic phase was washed 13% w/v aq NaCl. The organic phase was then filtered and concentrated to afford crude 2-(3-(*tert*-butoxycarbonyl)phenyl)-3-methylpyridine-1-oxide (95%) that was used directly in the next step.

**Example 2k: *tert*-Butyl-3-(6-amino-3-methylpyridin-2-yl)benzoate.**

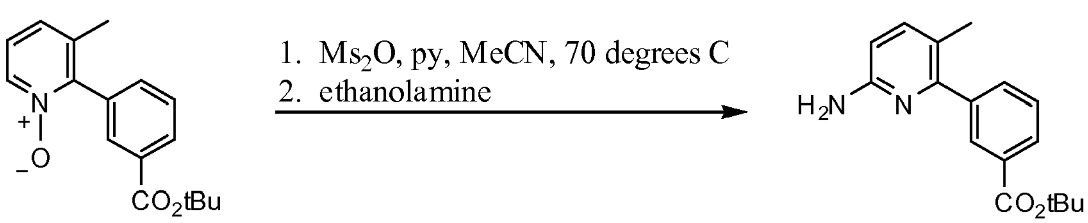

A solution of 2-(3-(*tert*-butoxycarbonyl)phenyl)-3-methylpyridine-1-oxide (1 eq) and pyridine (4 eq) in acetonitrile (8 vol) was heated to 70 °C. A solution of methanesulfonic anhydride (1.5 eq) in MeCN (2 vol) was added over 50 min via addition funnel while maintaining the temperature at less than 75 °C. The mixture was stirred for an additional 0.5 hours after complete addition. The mixture was then allowed to cool to ambient temperature. Ethanolamine (10 eq) was added via addition funnel. After stirring for 2 hours, water (6 vol) was added and the mixture was cooled to 10 °C. After stirring for 3 hours, the solid was collected by filtration and washed with water (3 vol), 2:1 acetonitrile/water (3 vol), and acetonitrile (2 x 1.5 vol). The solid was dried to constant weight (<1% difference) in a vacuum oven at 50 °C with a slight $N_2$ bleed to afford *tert*-butyl-3-(6-amino-3-methylpyridin-2-yl)benzoate as a red-yellow solid (53% yield).

**Example 21: 3-(6-(1-(2,2-Difluorobenzo[d] [1,3]dioxol-5-yl)-cyclopropanecarboxamido)-3-methylpyridin-2-yl)-t-butylbenzoate.**

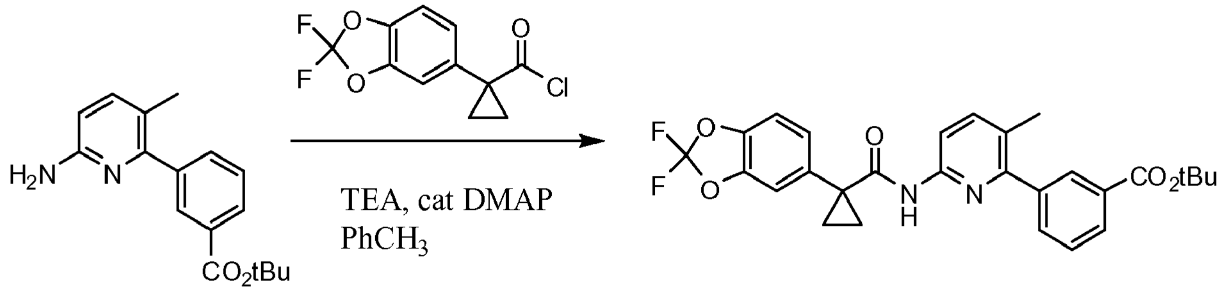


The crude acid chloride described above was dissolved in toluene (2.5 vol based on acid chloride) and added via addition funnel to a mixture of *tert*-butyl-3-(6-amino-3-methylpyridin-2-yl)benzoate (1 eq), DMAP, (0.02 eq), and triethylamine (3.0 eq) in toluene (4 vol based on *tert*-butyl-3-(6-amino-3-methylpyridin-2-yl)benzoate). After 2 hours, water (4 vol based on *tert*-butyl-3-(6-amino-3-methylpyridin-2-yl)benzoate) was added to the reaction mixture. After stirring for 30 minutes, the layers were separated. The organic phase was then filtered and concentrated to afford a thick oil of 3-(6-(1-(2,2-difluorobenzo[d][1,3]dioxol-5-yl) cyclopropanecarboxamido)-3-methylpyridin-2-yl)-t-butylbenzoate (quantitative crude yield). Acetonitrile (3 vol based on crude product) was added and distilled until crystallization occurs. Water (2 vol based on crude product) was added and the mixture stirred for 2 h. The solid was collected by filtration, washed with 1:1 (by volume) acetonitrile/water (2 x 1 volumes based on crude product), and partially dried on the filter under vacuum. The solid was dried to a constant weight (<1% difference) in a vacuum oven at 60 °C with a slight $N_2$ bleed to afford 3-(6-(1-(2,2-difluorobenzo[d][1,3]dioxol-5-yl) cyclopropanecarboxamido)-3-methylpyridin-2-yl)-t-butylbenzoate as a brown solid.

**Example 2m: 3-(6-(1-(2,2-Difluorobenzo[d] [1,3]dioxol-5-yl) cyclopropanecarboxamido)-3-methylpyridin-2-yl)benzoic acid • HCl salt.**

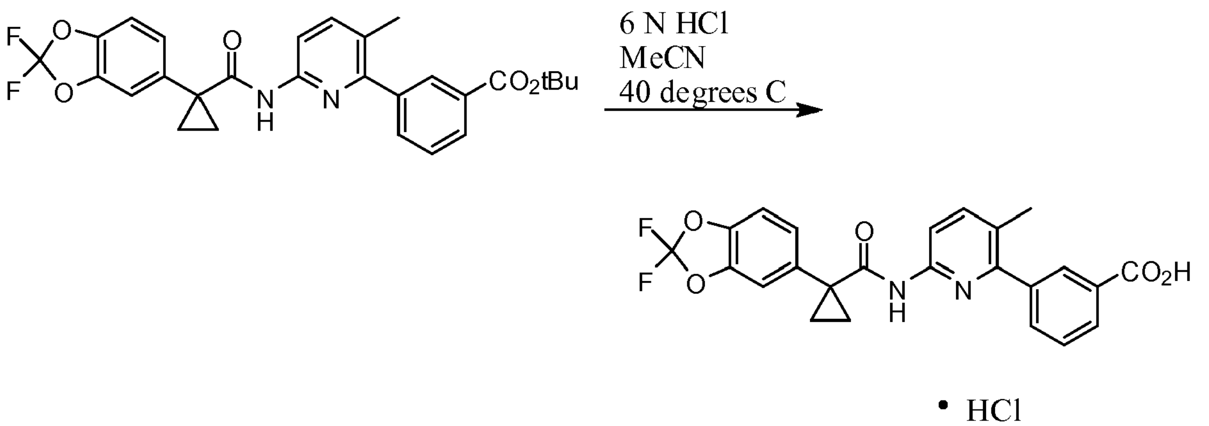

To a slurry of 3-(6-(1-(2,2-difluorobenzo[d][1,3]dioxol-5-yl) cyclopropanecarboxamido)-3-methylpyridin-2-yl)-t-butylbenzoate (1.0 eq) in MeCN (3.0 vol) was added water (0.83 vol) followed by concentrated aqueous HCl (0.83 vol). The mixture was heated to 45 ± 5 °C. After stirring for 24 to 48 h, the reaction was complete, and the mixture was allowed to cool to ambient temperature. Water (1.33 vol) was added and the mixture stirred. The solid was collected by filtration, washed with water (2 x 0.3 vol), and partially dried on the filter under vacuum. The solid was dried to a constant weight (<1% difference) in a vacuum oven at 60 °C with a slight $N_2$ bleed to afford 3-(6-(1-(2,2-difluorobenzo[d][1,3]dioxol-5-yl) cyclopropanecarboxamido)-3-methylpyridin-2-yl)benzoic acid HCl as an off-white solid.

Table 2-1 below recites physical data for Compound 2.

**Table 2-1.**

| Compound | LC/MS M + 1 | LC/RT minutes | NMR |
|---|---|---|---|
| Compound 2 | 453.3 | 1.93 | $^1$HNMR (400 MHz, DMSO-d6) 9.14 (s, 1H), 7.99-7.93 (m, 3H), 7.80-7.78 (m,1H), 7.74-7.72 (m,1H), 7.60-7.55 (m,2H), 7.41-7.33 (m,2H), 2.24 (s, 3H), 1.53-1.51 (m, 2H), 1.19-1.17 (m, 2H). |

**II.C. Compounds of Formula III**

**II.C.1. Embodiments of Compounds of Formula III**

In one aspect the invention includes a pharmaceutical composition comprising a Compound of Formula III

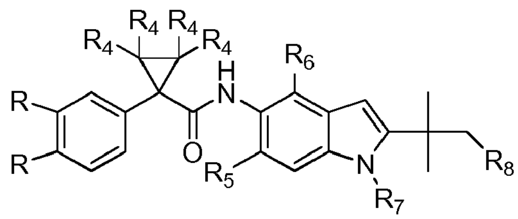

Formula III

or pharmaceutically acceptable salts thereof, wherein:

- R is H, OH, $OCH_3$ or two R taken together form $-OCH_2O-$ or $-OCF_2O-$;
- $R_4$ is H or alkyl;
- $R_5$ is H or F;
- $R_6$ is H or CN;
- $R_7$ is H, $-CH_2CH(OH)CH_2OH$, $-CH_2CH_2N^+(CH_3)_3$, or $-CH_2CH_2OH$;
- $R_8$ is H, OH, $-CH_2CH(OH)CH_2OH$, $-CH_2OH$, or $R_7$ and $R_8$ taken together form a five membered ring.

**II.C.2. Compound 3**

In another embodiment, the compound of Formula III is Compound 3, which is known by its chemical name (*R*)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide.

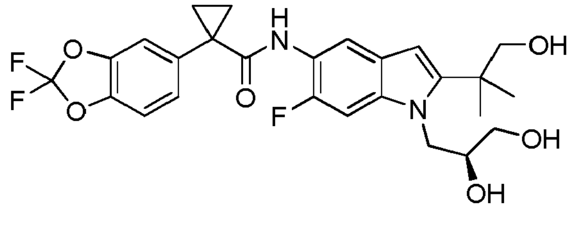

Compound 3

### II.C.3. Overview of the Synthesis of Compound 3

Compound 3 can be prepared by coupling an acid chloride moiety with an amine moiety according to the schemes below.

### II.C.3.a. Synthesis of the Acid Moiety of Compound 3

The acid moiety of Compound 3 can be synthesized as the acid chloride,

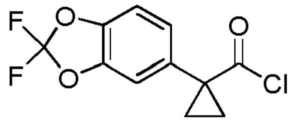

,

according to Scheme 2-1a, Scheme 2-1b and Examples 2a - 2h.

### II.C.3.b. Synthesis of the Amine Moiety of Compound 3

**Scheme 3-1: Synthesis of the Amine Moiety.**

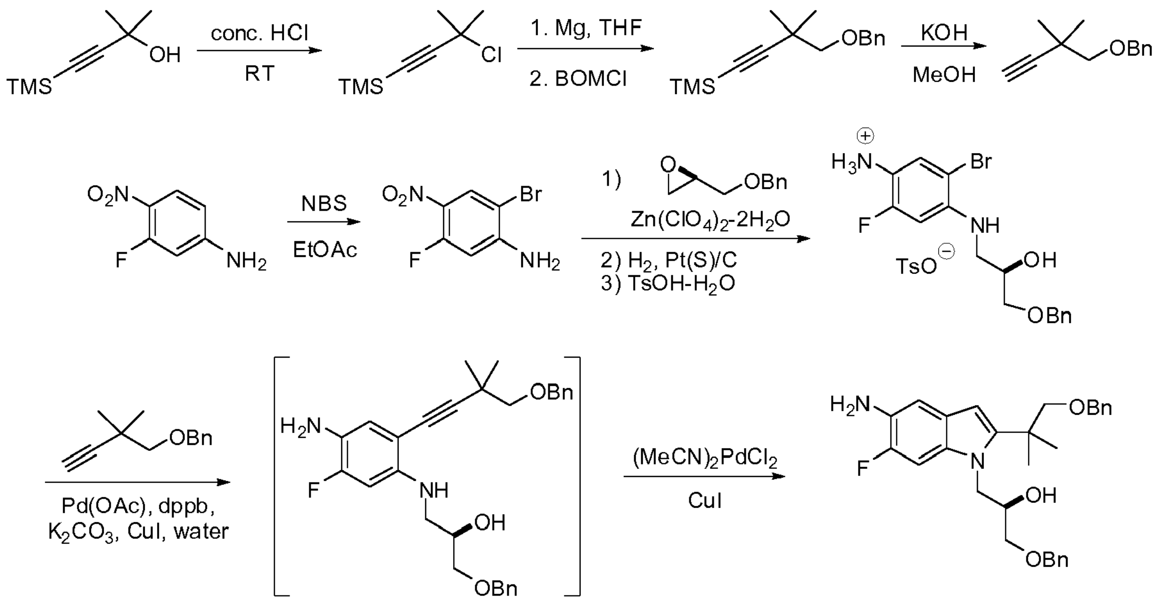


Scheme 3-1 provides an overview of the synthesis of the amine moiety of Compound 3. From the silyl protected propargyl alcohol shown, conversion to the propargyl chloride followed by formation of the Grignard reagent and subsequent nucleophilic substitution provides ((2,2-dimethylbut-3-ynyloxy)methyl)benzene, which is used in another step of the synthesis. To complete the amine moiety, 4-nitro-3-fluoroaniline is first brominated, and then converted to the toluenesulfonic acid salt of *(R)*-1-(4-amino-2-bromo-5-fluorophenylamino)-3-(benzyloxy)propan-2-ol in a two step process beginning with alkylation of the aniline amino group by (*R*)-2-(benzyloxymethyl)oxirane, followed by reduction of the nitro group to the corresponding amine. Palladium catalyzed coupling of the product with ((2,2-dimethylbut-3-ynyloxy)methyl)benzene (discussed above) provides the intermediate akynyl compound which is then cyclized to the indole moiety to produce the benzyl protected amine moiety of Compound 3.

### II.C.3.c. Synthesis of Compound 3 by Acid and Amine Moiety Coupling

## Scheme 3-2. Formation of Compound 3.

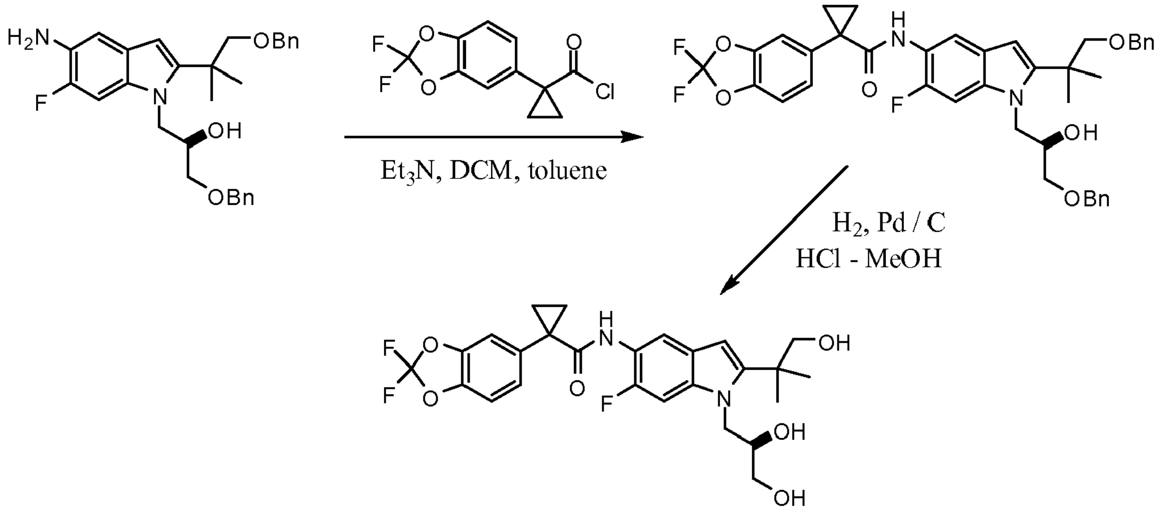


Scheme 3-2 depicts the coupling of the Acid and Amine moieties to produce Compound 3. In the first step, (*R*)-1-(5-amino-2-(1-(benzyloxy)-2-methylpropan-2-yl)-6-fluoro-1H-indol-1-yl)-3-(benzyloxy)propan-2-ol is coupled with 1-(2,2-difluorobenzo[d][1,3]dioxol-5-yl)cyclopropanecarbonyl chloride to provide the benzyl protected Compound 3. This step can be performed in the presence of a base and a solvent. The base can be an organic base such as triethylamine, and the solvent can be an organic solvent such as DCM or a mixture of DCM and toluene.

In the last step, the benzylated intermediate is deprotected to produce Compound 3. The deprotection step can be accomplished using reducing conditions sufficient to remove the benzyl group. The reducing conditions can be hydrogenation conditions such as hydrogen gas in the presence of a palladium catalyst.

### II.C.4. Examples: Synthesis of Compound 3

### II.C.4.a. Compound 3 Amine Moiety Synthesis

**Example 3a: 2-Bromo-5-fluoro-4-nitroaniline.**

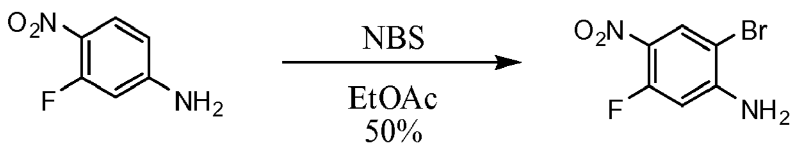


A flask was charged with 3-fluoro-4-nitroaniline (1.0 equiv) followed by ethyl acetate (10 vol) and stirred to dissolve all solids. N-Bromosuccinimide (1.0 equiv) was added portion-wise as to maintain an internal temperature of 22 °C. At the end of the reaction, the reaction mixture was concentrated in vacuo on a rotavap. The residue was slurried in distilled water (5 vol) to dissolve and remove succinimide. (The succinimide can also be removed by water workup procedure.) The water was decanted and the solid was slurried in 2-propanol (5 vol) overnight. The resulting slurry was filtered and the wetcake was washed with 2-propanol, dried in vacuum oven at 50 °C overnight with $N_2$ bleed until constant weight was achieved. A yellowish tan solid was isolated (50% yield, 97.5% AUC). Other impurities were a bromo-regioisomer (1.4% AUC) and a di-bromo adduct (1.1% AUC). $^1H$ NMR (500 MHz, DMSO) $\delta$ 8.19 (1 H, d, *J* = 8.1 Hz), 7.06 (br. s, 2 H), 6.64 (d, 1 H, *J* = 14.3 Hz).

**Example 3b: *p*-toluenesulfonic acid salt of (*R*)-1-((4-amino-2-bromo-5-fluorophenyl)amino)-3-(benzyloxy)propan-2-ol.**

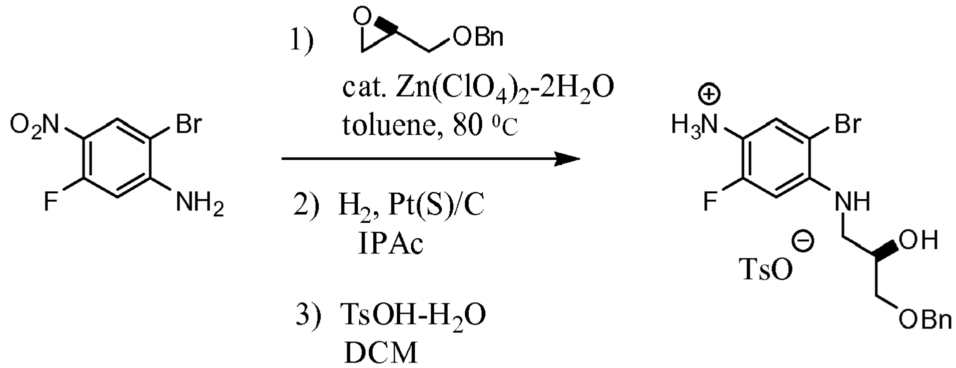


A thoroughly dried flask under $N_2$ was charged with the following: Activated powdered 4 Å molecular sieves (50 wt% based on 2-bromo-5-fluoro-4-nitroaniline), 2-Bromo-5-fluoro-4-nitroaniline (1.0 equiv), zinc perchlorate dihydrate (20 mol%), and toluene (8 vol). The mixture was stirred at room temperature for no more than 30 min. Lastly, (R)-benzyl glycidyl ether (2.0 equiv) in toluene (2 vol) was added in a steady stream. The reaction was heated to 80 °C (internal temperature) and stirred for approximately 7 hours or until 2-bromo-5-fluoro-4-nitroaniline was <5%AUC.

The reaction was cooled to room temperature and Celite® (50 wt%) was added, followed by ethyl acetate (10 vol). The resulting mixture was filtered to remove Celite® and sieves and washed with ethyl acetate (2 vol). The filtrate was washed with ammonium chloride solution (4 vol, 20% w/v). The organic layer was washed with sodium bicarbonate solution (4 vol x 2.5% w/v). The organic layer was concentrated in vacuo on a rotovap. The resulting slurry was dissolved in isopropyl acetate (10 vol) and this solution was transferred to a Buchi hydrogenator.

The hydrogenator was charged with 5wt% Pt(S)/C (1.5 mol%) and the mixture was stirred under $N_2$ at 30 °C (internal temperature). The reaction was flushed with $N_2$ followed by hydrogen. The hydrogenator pressure was adjusted to 1 Bar of hydrogen and the mixture was stirred rapidly (>1200 rpm). At the end of the reaction, the catalyst was filtered through a pad of Celite® and washed with dichloromethane (10 vol). The filtrate was concentrated in vacuo. Any remaining isopropyl acetate was chased with dichloromethane (2 vol) and concentrated on a rotavap to dryness.

The resulting residue was dissolved in dichloromethane (10 vol). *p*-Toluenesulfonic acid monohydrate (1.2 equiv) was added and stirred overnight. The product was filtered and washed with dichloromethane (2 vol) and suction dried. The wetcake was transferred to drying trays and into a vacuum oven and dried at 45 °C with $N_2$ bleed until constant weight was achieved. The *p*-toluenesulfonic acid salt of (*R*)-1-((4-amino-2-bromo-5-fluorophenyl)amino)-3-(benzyloxy)propan-2-ol was isolated as an off-white solid.

**Example 3c: (3-Chloro-3-methylbut-1-ynyl)trimethylsilane.**

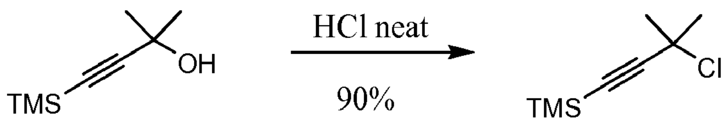


Propargyl alcohol (1.0 equiv) was charged to a vessel. Aqueous hydrochloric acid (37%, 3.75 vol) was added and stirring begun. During dissolution of the solid alcohol, a modest endotherm (5-6 °C) was observed. The resulting mixture was stirred overnight (16 h), slowly becoming dark red. A 30 L jacketed vessel was charged with water (5 vol) which was then cooled to 10 °C. The reaction mixture was transferred slowly into the water by vacuum, maintaining the internal temperature of the mixture below 25 °C. Hexanes (3 vol) was added and the resulting mixture was stirred for 0.5 h. The phases were settled and the aqueous phase (pH < 1) was drained off and discarded. The organic phase was concentrated in vacuo using a rotary evaporator, furnishing the product as red oil.

**Example 3d: (4-(Benzyloxy)-3,3-dimethylbut-1-ynyl)trimethylsilane.**

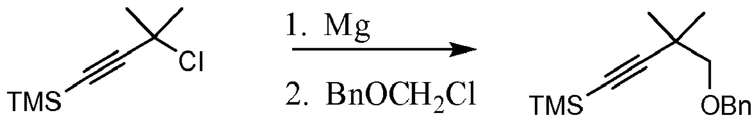


**Method A**

All equivalents and volume descriptors in this part are based on a 250g reaction. Magnesium turnings (69.5 g, 2.86 mol, 2.0 equiv) were charged to a 3 L 4-neck reactor and stirred with a magnetic stirrer under nitrogen for 0.5 h. The reactor was immersed in an ice-water bath. A solution of the propargyl chloride (250 g, 1.43 mol, 1.0 equiv) in THF (1.8 L, 7.2 vol) was added slowly to the reactor, with stirring, until an initial exotherm (about 10 °C) was observed. The Grignard reagent formation was confirmed by IPC using $^1$H-NMR spectroscopy. Once the exotherm subsided, the remainder of the solution was added slowly, maintaining the batch temperature <15 °C. The addition required about 3.5 h. The resulting dark green mixture was decanted into a 2 L capped bottle.

All equivalent and volume descriptors in this part are based on a 500g reaction. A 22 L reactor was charged with a solution of benzyl chloromethyl ether (95%, 375 g, 2.31 mol, 0.8 equiv) in THF (1.5 L, 3 vol). The reactor was cooled in an ice-water bath. Two Grignard reagent batches prepared as above were combined and then added slowly to the benzyl chloromethyl ether solution *via* an addition funnel, maintaining the batch temperature below 25 °C. The addition required 1.5 h. The reaction mixture was stirred overnight (16 h).

All equivalent and volume descriptors in this part are based on a 1 kg reaction. A solution of 15% ammonium chloride was prepared in a 30 L jacketed reactor (1.5 kg in 8.5 kg of water, 10 vol). The solution was cooled to 5 °C. Two Grignard reaction mixtures prepared as above were combined and then transferred into the ammonium chloride solution *via* a header vessel. An exotherm was observed in this quench, which was carried out at a rate such as to keep the internal temperature below 25 °C. Once the transfer was complete, the vessel jacket temperature was set to 25 °C. Hexanes (8 L, 8 vol) was added and the mixture was stirred for 0.5 h. After settling the phases, the aqueous phase (pH 9) was drained off and discarded. The remaining organic phase was washed with water (2 L, 2 vol). The organic phase was concentrated in vacuo using a 22 L rotary evaporator, providing the crude product as an orange oil.

**Method B**

Magnesium turnings (106 g, 4.35 mol, 1.0 eq) were charged to a 22 L reactor and then suspended in THF (760 mL, 1 vol). The vessel was cooled in an ice-water bath such that the batch temperature reached 2 °C. A solution of the propargyl chloride (760 g, 4.35 mol, 1.0 equiv) in THF (4.5 L, 6 vol) was added slowly to the reactor. After 100 mL was added, the addition was stopped and the mixture stirred until a 13 °C exotherm was observed, indicating the Grignard reagent initiation. Once the exotherm subsided, another 500 mL of the propargyl chloride solution was added slowly, maintaining the batch temperature <20 °C. The Grignard reagent formation was confirmed by IPC using $^1$H-NMR spectroscopy. The remainder of the propargyl chloride solution was added slowly, maintaining the batch temperature <20 °C. The addition required about 1.5 h. The resulting dark green solution was stirred for 0.5 h. The Grignard reagent formation was confirmed by IPC using $^1$H-NMR spectroscopy. Neat benzyl chloromethyl ether was charged to the reactor addition funnel and then added dropwise into the reactor, maintaining the batch temperature below 25 °C. The addition required 1.0 h. The reaction mixture was stirred overnight. The aqueous work-up and concentration was carried out using the same procedure and relative amounts of materials as in Method A to give the product as an orange oil.

**Example 3e: 4-Benzyloxy-3,3-dimethylbut-1-yne.**

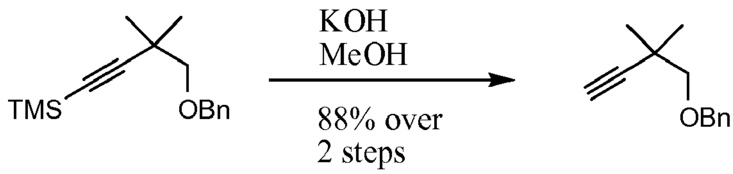

A 30 L jacketed reactor was charged with methanol (6 vol) which was then cooled to 5 °C. Potassium hydroxide (85%, 1.3 equiv) was added to the reactor. A 15-20 °C exotherm was observed as the potassium hydroxide dissolved. The jacket temperature was set to 25 °C. A solution of 4-benzyloxy-3,3-dimethyl-1-trimethylsilylbut-1-yne (1.0 equiv) in methanol (2 vol) was added and the resulting mixture was stirred until reaction completion, as monitored by HPLC. Typical reaction time at 25 °C was 3-4 h. The reaction mixture was diluted with water (8 vol) and then stirred for 0.5 h. Hexanes (6 vol) was added and the resulting mixture was stirred for 0.5 h. The phases were allowed to settle and then the aqueous phase (pH 10-11) was drained off and discarded. The organic phase was washed with a solution of KOH (85%, 0.4 equiv) in water (8 vol) followed by water (8 vol). The organic phase was then concentrated down using a rotary evaporator, yielding the title material as a yellow-orange oil. Typical purity of this material was in the 80% range with primarily a single impurity present. $^1H$ NMR (400 MHz, $C_6D_6$) δ 7.28 (d, 2 H, *J* = 7.4 Hz), 7.18 (t, 2 H, *J* = 7.2 Hz), 7.10 (d, 1H, *J* = 7.2 Hz), 4.35 (s, 2 H), 3.24 (s, 2 H), 1.91 (s, 1 H), 1.25 (s, 6 H).

**Example 3f: (*R*)-1-(4-amino-2-(4-(benzyloxy)-3,3-dimethylbut-1-ynyl)-5-fluorophenylamino)-3-(benzyloxy)propan-2-ol.**

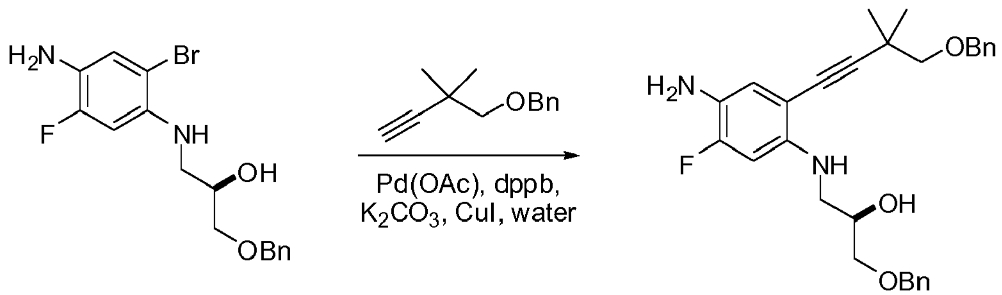


The tosylate salt of (*R*)-1-(4-amino-2-bromo-5-fluorophenylamino)-3-(benzyloxy)propan-2-ol was converted to the free base by stirring in dichloromethane (5 vol) and saturated $NaHCO_3$ solution (5 vol) until a clear organic layer was achieved. The resulting layers were separated and the organic layer was washed with saturated $NaHCO_3$ solution (5 vol) followed by brine and concentrated in vacuo to obtain (*R*)-1-(4-amino-2-bromo-5-fluorophenylamino)-3-(benzyloxy)propan-2-ol (free base) as an oil.

Palladium acetate (0.01 eq), dppb (0.015 eq), CuI (0.015 eq) and potassium carbonate (3 eq) were suspended in acetonitrile (1.2 vol). After stirring for 15 minutes, a solution of 4-benzyloxy-3,3-dimethylbut-1-yne (1.1 eq) in acetonitrile (0.2 vol) was added. The mixture was sparged with nitrogen gas for 1 h and then a solution of (*R*)-1-((4-amino-2-bromo-5-fluorophenyl)amino)-3-(benzyloxy)propan-2-ol free base (1 eq) in acetonitrile (4.1 vol) was added. The mixture was sparged with nitrogen gas for another hour and then was heated to 80 °C. Reaction progress was monitored by HPLC and the reaction was usually complete within 3-5 h. The mixture was cooled to room temperature and then filtered through Celite. The cake was washed with acetonitrile (4 vol). The combined filtrates were azeotroped to dryness and then the mixture was polish filtered into the next reactor. The acetonitrile solution of (*R*)-1-((4-amino-2-(4-(benzyloxy)-3,3-dimethylbut-1-yn-1-yl)-5-fluorophenyl)amino)-3-(benzyloxy)propan-2-ol thus obtained was used directly in the next procedure (cyclization) without further purification.

**Example 3g: (*R*)-1-(5-amino-2-(1-(benzyloxy)-2-methylpropan-2-yl)-6-fluoro-1H-indol-1-yl)-3-(benzyloxy)propan-2-ol.**

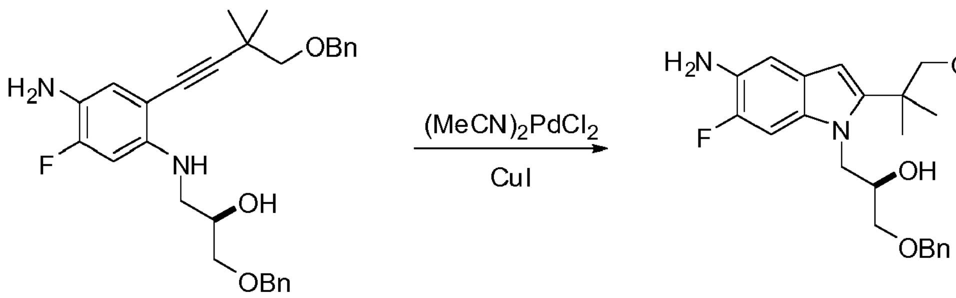


*Bis*-acetonitriledichloropalladium (0.1 eq) and CuI (0.1 eq) were charged to the reactor and then suspended in a solution of (*R*)-1-((4-amino-2-(4-(benzyloxy)-3,3-dimethylbut-1-yn-1-yl)-5-fluorophenyl)amino)-3-(benzyloxy)propan-2-ol obtained above (1 eq) in acetonitrile (9.5 vol total). The mixture was sparged with nitrogen gas for 1 h and then was heated to 80 °C. The reaction progress was monitored by HPLC and the reaction was typically complete within 1-3 h. The mixture was filtered through Celite and the cake was washed with acetonitrile. A solvent swap into ethyl acetate (7.5 vol) was performed. The ethyl acetate solution was washed with aqueous $NH_3$-$NH_4Cl$ solution (2 x 2.5 vol) followed by 10% brine (2.5 vol). The ethyl acetate solution was then stirred with silica gel (1.8 wt eq) and Si-TMT (0.1 wt eq) for 6 h. After filtration, the resulting solution was concentrated down. The residual oil was dissolved in DCM / heptane (4 vol) and then purified by column chromatography. The oil thus obtained was then crystallized from 25% EtOAc / heptane (4 vol). Crystalline (*R*)-1-(5-amino-2-(1-(benzyloxy)-2-methylpropan-2-yl)-6-fluoro-1H-indol-1-yl)-3-(benzyloxy)propan-2-ol was typically obtained in 27-38% yield. $^1H$ NMR (400 MHz, DMSO) 7.38-7.34 (m, 4 H), 7.32-7.23 (m, 6 H), 7.21 (d, 1 H, *J* = 12.8 Hz), 6.77 (d, 1H, *J* = 9.0 Hz), 6.06 (s, 1 H), 5.13 (d, 1H, *J* = 4.9 Hz), 4.54 (s, 2 H), 4.46 (br. s, 2 H), 4.45 (s, 2 H), 4.33 (d, 1 H, *J* = 12.4 Hz), 4.09-4.04 (m, 2 H), 3.63 (d, 1H, *J* = 9.2 Hz), 3.56 (d, 1H, *J* = 9.2 Hz), 3.49 (dd, 1H, *J* = 9.8, 4.4 Hz), 3.43 (dd, 1H, *J* = 9.8, 5.7 Hz), 1.40 (s, 6 H).

**II.C.4.b. Coupling**

**Example 3h: Synthesis of (*R*)-N-(1-(3-(benzyloxy)-2-hydroxypropyl)-2-(1-(benzyloxy)-2-methylpropan-2-yl)-6-fluoro-1*H*-indol-5-yl)-1-(2,2-difluorobenzo[*d*][1,3]dioxol-5-yl)cyclopropanecarboxamide.**

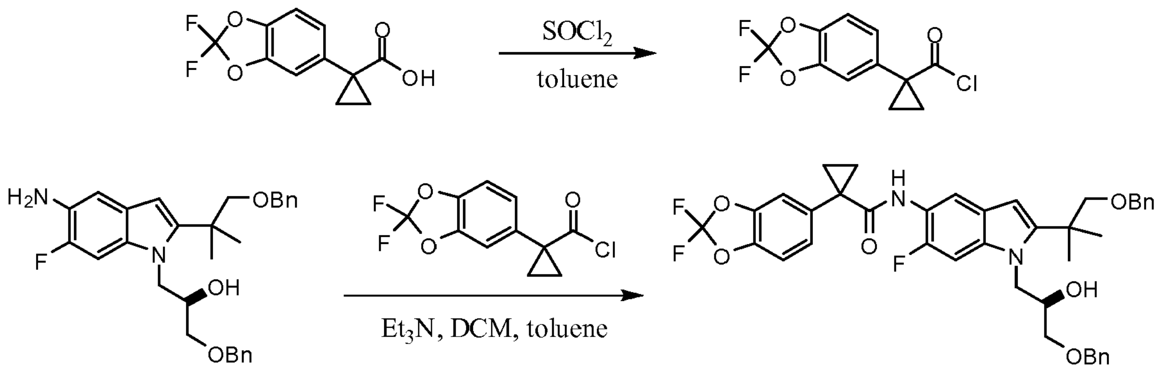


1-(2,2-Difluoro-1,3-benzodioxol-5-yl)-cyclopropanecarboxylic acid (1.3 equiv) was slurried in toluene (2.5 vol, based on 1-(2,2-difluoro-1,3-benzodioxol-5-yl)-cyclopropanecarboxylic acid). Thionyl chloride ($SOCl_2$, 1.7 equiv) was added *via* addition funnel and the mixture was heated to 60 °C. The resulting mixture was stirred for 2 h. The toluene and the excess $SOCl_2$ were distilled off using a rotavop. Additional toluene (2.5 vol, based on 1-(2,2-difluoro-1,3-benzodioxol-5-yl)-cyclopropanecarboxylic acid) was added and the mixture was distilled down to 1 vol of toluene. A solution of (*R*)-1-(5-amino-2-(1-(benzyloxy)-2-methylpropan-2-yl)-6-fluoro-1H-indol-1-yl)-3-(benzyloxy)propan-2-ol (1 eq) and triethylamine (3 eq) in DCM (4 vol) was cooled to 0 °C. The acid chloride solution in toluene (1 vol) was added while maintaining the batch temperature below 10 °C. The reaction progress was monitored by HPLC, and the reaction was usually complete within minutes. After warming to 25 °C, the reaction mixture was washed with 5% $NaHCO_3$ (3.5 vol), 1 M NaOH (3.5 vol) and 1 M HCl (5 vol). A solvent swap to into methanol (2 vol) was performed and the resulting solution of (*R*)-N-(1-(3-(benzyloxy)-2-hydroxypropyl)-2-(1-(benzyloxy)-2-methylpropan-2-yl)-6-fluoro-1*H*-indol-5-yl)-1-(2,2-difluorobenzo[*d*][1,3]dioxol-5-yl)cyclopropanecarboxamide in methanol was used without further purification in the next

step (hydrogenolysis).

**Example 3i: Synthesis of Compound 3.**

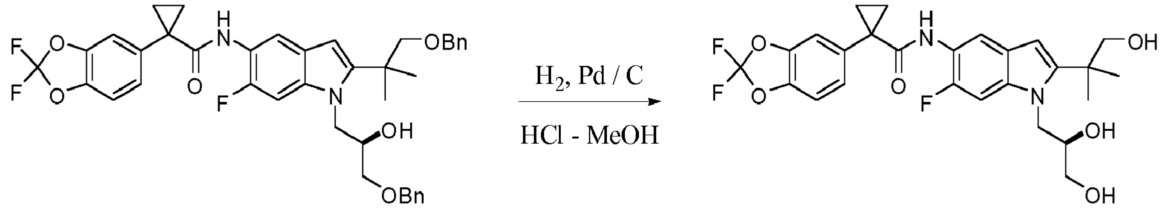


5% palladium on charcoal (~50% wet, 0.01 eq) was charged to an appropriate hydrogenation vessel. The (*R*)-N-(1-(3-(benzyloxy)-2-hydroxypropyl)-2-(1-(benzyloxy)-2-methylpropan-2-yl)-6-fluoro-1*H*-indol-5-yl)-1-(2,2-difluorobenzo[*d*][1,3]dioxol-5-yl)cyclopropanecarboxamide solution in methanol (2 vol) obtained above was added carefully, followed by a 3 M solution of HCl in methanol. The vessel was purged with nitrogen gas and then with hydrogen gas. The mixture was stirred vigorously until the reaction was complete, as determined by HPLC analysis. Typical reaction time was 3-5 h. The reaction mixture was filtered through Celite and the cake was washed with methanol (2 vol). A solvent swap into isopropanol (3 vol) was performed. Crude Compound 3 was crystallized from 75% IPA-heptane (4 vol, *ie.* 1 vol heptane added to the 3 vol of IPA) and the resulting crystals were matured in 50% IPA-heptane (*ie.* 2 vol of heptane added to the mixture). Typical yields of Compound 3 from the two-step acylation / hydrogenolysis procedure range from 68% to 84%. Compound 3 can be recrystallized from IPA-heptane following the same procedure just described.

Compound 3 may also be prepared by one of several synthetic routes disclosed in US published patent application US 2009/0131492, incorporated herein by reference.

**Table 3-1: Physical Data for Compound 3.**

| Cmpd. No. | LC/MS M+1 | LC/RT min | NMR |
|---|---|---|---|
| 3 | 521.5 | 1.69 | 1H NMR (400.0 MHz, $CD_3CN$) d 7.69 (d, J = 7.7 Hz, 1H), 7.44 (d, J = 1.6 Hz, 1H), 7.39 (dd, J = 1.7, 8.3 Hz, 1H), 7.31 (s, 1H), 7.27 (d, J = 8.3 Hz, 1H), 7.20 (d, J = 12.0 Hz, 1H), 6.34 (s, 1H), 4.32 (d, J = 6.8 Hz, 2H), 4.15 - 4.09 (m, 1H), 3.89 (dd, J = 6.0, 11.5 Hz, 1H), 3.63 - 3.52 (m, 3H), 3.42 (d, J = 4.6 Hz, 1H), 3.21 (dd, J = 6.2, 7.2 Hz, 1H), 3.04 (t, J = 5.8 Hz, 1H), 1.59 (dd, J = 3.8, 6.8 Hz, 2H), 1.44 (s, 3H), 1.33 (s, 3H) and 1.18 (dd, J = 3.7, 6.8 Hz, 2H) ppm. |

## III. Solid Forms

### III.A. Solid Forms of Compound 1

### III.A.1. Compound 1 Form C

### III.A.1.a. Characterization and Embodiments of Compound 1 Form C

XRPD (X-ray Powder Diffraction)

The XRPD patterns were acquired at room temperature in reflection mode using a Bruker D8 Advance diffractometer equipped with a sealed tube copper source and a Vantec-1 detector. The X-ray generator was operating at a voltage of 40 kV and a current of 40 mA. The data were recorded in a θ-θ scanning mode over the range of 3°-40° 2θ with a step size of 0.014° and the sample spinning at 15 rpm.

In one aspect, Compound 1 is in Form C. In one embodiment, of this aspect, the invention includes crystalline N-[2,4-bis(1,1-dimethylethyl)-5-hydroxyphenyl]-1,4-dihydro-4-oxoquinoline-3-carboxamide (Compound 1) characterized as Form C.

In one embodiment of this aspect, Form C is characterized by a peak having a 2-Theta value from about 6.0 to about 6.4 degrees in an XRPD pattern. In a further embodiment, Form C is characterized by a peak having a 2-Theta

value from about 7.3 to about 7.7 degrees in an XRPD pattern. In a further embodiment, Form C is characterized by a peak having a 2-Theta value from about 8.1 to about 8.5 degrees in an XRPD pattern. In a further embodiment, Form C is characterized by a peak having a 2-Theta value from about 12.2 to about 12.6 degrees in an XRPD pattern. In a further embodiment, Form C is characterized by a peak having a 2-Theta value from about 14.4 to about 14.8 degrees in an XRPD pattern. In a further embodiment, Form C is characterized by a peak having a 2-Theta value from about 17.7 to about 18.1 degrees in an XRPD pattern. In a further embodiment, Form C is characterized by a peak having a 2-Theta value from about 20.3 to about 20.7 degrees in an XRPD pattern. In a further embodiment, Form C is characterized by a peak having a 2-Theta value from about 20.7 to about 21.1 degrees in an XRPD pattern.

In another embodiment, Form C is characterized by a peak having a 2-Theta value of about 6.2 degrees in an XRPD pattern. In a further embodiment, Form C is characterized by a peak having a 2-Theta value of about 7.5 degrees in an XRPD pattern. In a further embodiment, Form C is characterized by a peak having a 2-Theta value of about 8.3 degrees in an XRPD pattern. In a further embodiment, Form C is characterized by a peak having a 2-Theta value of about 12.4 degrees in an XRPD pattern. In a further embodiment, Form C is characterized by a peak having a 2-Theta value of about 14.6 degrees in an XRPD pattern. In a further embodiment, Form C is characterized by a peak having a 2-Theta value of about 17.9 degrees in an XRPD pattern. In a further embodiment, Form C is characterized by a peak having a 2-Theta value of about 20.5 degrees in an XRPD pattern. In a further embodiment, Form C is characterized by a peak having a 2-Theta value of about 20.9 degrees in an XRPD pattern.

In another embodiment, Form C is characterized by one or more peaks in an XRPD pattern selected from about 6.2, about 7.5, about 8.3, about 12.4, about 14.6, about 17.9, about 20.5 and about 20.9 degrees as measured on a 2-Theta scale.

In still another embodiment, Form C is characterized by all of the following peaks in an XRPD pattern: about 6.2, about 7.5, about 8.3, about 12.4, about 14.6, about 17.9, about 20.5 and about 20.9 degrees as measured on a 2-Theta scale. Compound 1 Form C can be characterized by the X-Ray powder diffraction pattern depicted in Figure 1-1. Representative peaks as observed in the XRPD pattern are provided in Table 1-1a and Table 1-1b below. Each peak described in Table 1-1a also has a corresponding peak label (A - H), which are used to describe some embodiments of the invention.

**Table 1-1a:Representative XRPD peaks for Compound 1 Form C.**

| Peak # | Angle 2-θ (°) | Peak Label |
|---|---|---|
| 1 | 6.2 | A |
| 2 | 7.5 | B |
| 3 | 8.3 | C |
| 4 | 12.4 | D |
| 5 | 14.6 | E |
| 6 | 17.9 | F |
| 7 | 20.5 | G |
| 8 | 20.9 | H |

In another embodiment, Form C can be characterized by an X-Ray powder diffraction pattern having the representative peaks listed in Table 1-1b.

**Table 1-1b: Further representative XRPD peaks for Form C.**

| Peak # | Angle 2-θ (°) |
|---|---|
| 1 | 6.2 |
| 2 | 7.5 |
| 3 | 8.3 |
| 4 | 11.0 |
| 5 | 12.4 |
| 6 | 14.6 |
| 7 | 16.3 |
| 8 | 17.1 |
| 9 | 17.9 |
| 10 | 18.1 |
| 11 | 18.7 |

(continued)

| Peak # | Angle 2-θ (°) |
|---|---|
| 12 | 19.5 |
| 13 | 20.5 |
| 14 | 20.9 |
| 15 | 21.3 |
| 16 | 21.5 |
| 17 | 21.8 |
| 18 | 22.1 |
| 19 | 22.4 |
| 20 | 22.7 |

In one aspect, Compound 1 Form C can be characterized by an X-Ray powder diffraction pattern having one or more of peaks A, B, C, D, E, F, G and H as described in Table 1-1a.

In one embodiment of this aspect, Form C is characterized by peak A. In another embodiment, Form C is characterized by peak B. In another embodiment, Form C is characterized by peak B. In another embodiment, Form C is characterized by peak C. In another embodiment, Form C is characterized by peak D. In another embodiment, Form C is characterized by peak E. In another embodiment, Form C is characterized by peak F. In another embodiment, Form C is characterized by peak G. In another embodiment, Form C is characterized by peak H.

In another embodiment of this aspect, Form C is characterized by an X-Ray powder diffraction pattern having one of the following groups of peaks as described in Table 1-1a: A and B; A and C; A and D; A and E; A and F; A and G; A and H; B and C; B and D; B and E; B and F; B and G; B and H; C and D; C and E; C and F; C and G; C and H; D and E; D and F; D and G; D and H; E and F; E and G; E and H; F and G; F and H; and G and H.

In another embodiment of this aspect, Form C is characterized by an X-Ray powder diffraction pattern having one of the following groups of peaks as described in Table 1-1a: A, B and C; A, B and D; A, B and E; A, B and F; A, B and G; A, B and H; A, C and D; A, C and E; A, C and F; A, C and G; A, C and H; A, D and E; A, D and F; A, D and G; A, D and H; A, E and F; A, E and G; A, E and H; A, F and G; A, F and H; A, G and H; B, C and D; B, C and E; B, C and F; B, C and G; B, C and H; B, D and E; B, D and F; B, D and G; B, D and H; B, E and F; B, E and G; B, E and H; B, F and G; B, F and H; B, G and H; C, D and E; C, D F; C, D and G; C, D and H; C, E and F; C, E and G; C, E and H; C, F and G; C, F and H; C, G and H; D, E and F; D, E and G; D, E and H; D, F and G; D, F and H; D, G and H; E, F and G; E, F and H, E, G and H; and F, G and H.

In another embodiment of this aspect, Form C is characterized by an X-Ray powder diffraction pattern having one of the following groups of peaks as described in Table 1-1a: A, B, C and D; A, B, C and E, A, B, C and F; A, B, C and G; A, B, C and H; A, B, D and E; A, B, D and F; A, B, D and G; A, B, D and H; A, B, E and F; A, B, E and G; A, B, E and H; A, B, F and G; A, B, F and H; A, B, G and H; A, C, D and E; A, C, D and F; A, C, D and G; A, C, D and H; A, C, E and F; A, C, E and G; A, C, E and H; A, C, F and G; A, C, F and H; A, C, G and H; A, D, F and G; A, D, F and H; A, D, G and H; A, E, F and G; A, E, F and H; A, E, G and H; A, F, G and H; B, C, D and E; B, C, D and F; B, C, D and G; B, C, D and H; B, C, E and F; B, C, E and G; B, C, E and H; B, C, F and G; B, C, F and H; B, C, G and H; B, D, E and F; B, D, E and G; B, D, E and H; B, D, F and G; B, D, F and H; B, D, G and H; B, E, F and G; B, E, F and H; B, E, G and H; B, F, G and H; C, D, E and F; C, D, E and G; C, D, E and H; C, D, F and G; C, D, F and H; C, D, G and H; C, E, F and G; C, E, F and H; C, E, G and H; C, F, G and H; D, E, F and G; D, E, F and H; D, E, G and H; D, F, G and H; and E, F, G and H.

In another embodiment of this aspect, Form C is characterized by an X-Ray powder diffraction pattern having one of the following groups of peaks as described in Table 1-1a: A, B, C, D and E; A, B, C, D and F; A, B, C, D and G; A, B, C, D and H; A, B, C, E and F; A, B, C, E and G; A, B, C, E and H; A, B, C, F and G; A, B, C, F and H; A, B, C, G and H; A, B, C, E and F; A, B, C, E and G; A, B, C, E and H; A, B, C, F and G; A, B, C, F and H; A, B, C, G and H; A, B, D, E and F; A, B, D, E and G; A, B, D, E and H; A, B, D, F and G; A, B, D, F and H; A, B, D, G and H; A, B, E, F and G; A, B, E, F and H; A, B, E, G and H; A, B, F, G and H; A, C, D, E and F; A, C, D, E and G; A, C, D, E and H; A, C, D, F and G; A, C, D, F and H; A, C, D, G and H; A, C, E, F and G; A, C, E, F and H; A, C, E, G and H; A, C, F, G and H; A, D, E, F and G; A, D, E, F and H; A, D, E, G and H; A, D, F, G and H; A, E, F, G and H; B, C, D, E and F; B, C, D, E and G; B, C, D, E and H; B, C, D, F and G; B, C, D, F and H; B, C, D, G and H; B, C, E, F and G; B, C, E, F and H; B, C, E, G and H; B, C, F, G and H; B, D, E, F and G; B, D, E, F and H; B, D, E, G and H; B, D, F, G and H; B, E, F, G and H; C, D, E, F and G; C, D, E, F and H; C, D, E, G and H; C, D, F, G and H; C, E, F, G and H; and D, E, F, G and H.

In another embodiment of this aspect, Form C is characterized by an X-Ray powder diffraction pattern having one of the following groups of peaks as described in Table 1-1a: A, B, C, D, E and F; A, B, C, D, E and G; A, B, C, D, E and H; A, B, C, D, F and G; A, B, C, D, F and H; A, B, C, D, G and H; A, B, C, E, F and G; A, B, C, E, F and H; A, B,

C, E, G and H; A, B, C, F, G and H; A, B, D, E, F and G; A, B, D, E, F and H; A, B, D, E, G and H; A, B, D, F, G and H; A, B, E, F, G and H; A, C, D, E, F and G; A, C, D, E, F and H; A, C, D, E, G and H; A, C, D, F, G and H; A, C, E, F, G and H; A, D, E, F, G and H; B, C, D, E, F and G; B, C, D, E, F and H; B, C, D, E, G and H; B, C, D, F, G and H; B, C, E, F, G and H; B, D, E, F, G and H; and C, D, E, F, G and H.

In another embodiment of this aspect, Form C is characterized by an X-Ray powder diffraction pattern having one of the following groups of peaks as described in Table 1-1a: A, B, C, D, E, F and G; A, B, C, D, E, F and H; A, B, C, D, E, G and H; A, B, C, D, F, G and H; A, B, C, E, F, G and H; A, B, D, E, F, G and H; A, C, D, E, F, G and H; and B, C, D, E, F, G and H.

In another embodiment of this aspect, Form C is characterized by an X-Ray powder diffraction pattern having all of the following peaks as described in Table 1-1a: A, B, C, D, E, F, G and H.

In another aspect, Compound 1 Form C can be characterized by an X-Ray powder diffraction pattern having one or more of peaks that range in value within ±0.2 degrees of one or more of the peaks A, B, C, D, E, F, G and H as described in Table 1. In one embodiment of this aspect, Form C is characterized by a peak within ±0.2 degrees of A. In another embodiment, Form C is characterized by a peak within ±0.2 degrees of B. In another embodiment, Form C is characterized by a peak within ±0.2 degrees of B. In another embodiment, Form C is characterized by a peak within ±0.2 degrees of C. In another embodiment, Form C is characterized by a peak within ±0.2 degrees of D. In another embodiment, Form C is characterized by a peak within ±0.2 degrees of E. In another embodiment, Form C is characterized by a peak within ±0.2 degrees of F. In another embodiment, Form C is characterized by a peak within ±0.2 degrees of G. In another embodiment, Form C is characterized by a peak within ±0.2 degrees of H.

In another embodiment of this aspect, Form C is characterized by an X-Ray powder diffraction pattern having one of the following groups of peaks as described in Table 1-1a: A and B; A and C; A and D; A and E; A and F; A and G; A and H; B and C; B and D; B and E; B and F; B and G; B and H; C and D; C and E; C and F; C and G; C and H; D and E; D and F; D and G; D and H; E and F; E and G; E and H; F and G; F and H; and G and H, wherein each peak in the group is within ±0.2 degrees of the corresponding value described in Table 1-1a.

In another embodiment of this aspect, Form C is characterized by an X-Ray powder diffraction pattern having one of the following groups of peaks as described in Table 1-1a: A, B and C; A, B and D; A, B and E; A, B and F; A, B and G; A, B and H; A, C and D; A, C and E; A, C and F; A, C and G; A, C and H; A, D and E; A, D and F; A, D and G; A, D and H; A, E and F; A, E and G; A, E and H; A, F and G; A, F and H; A, G and H; B, C and D; B, C and E; B, C and F; B, C and G; B, C and H; B, D and E; B, D and F; B, D and G; B, D and H; B, E and F; B, E and G; B, E and H; B, F and G; B, F and H; B, G and H; C, D and E; C, D F; C, D and G; C, D and H; C, E and F; C, E and G; C, E and H; C, F and G; C, F and H; C, G and H; D, E and F; D, E and G; D, E and H; D, F and G; D, F and H; D, G and H; E, F and G; E, F and H, E, G and H; and F, G and H, wherein each peak in the group is within ±0.2 degrees of the corresponding value described in Table 1-1a.

In another embodiment of this aspect, Form C is characterized by an X-Ray powder diffraction pattern having one of the following groups of peaks as described in Table 1-1a: A, B, C and D; A, B, C and E, A, B, C and F; A, B, C and G; A, B, C and H; A, B, D and E; A, B, D and F; A, B, D and G; A, B, D and H; A, B, E and F; A, B, E and G; A, B, E and H; A, B, F and G; A, B, F and H; A, B, G and H; A, C, D and E; A, C, D and F; A, C, D and G; A, C, D and H; A, C, E and F; A, C, E and G; A, C, E and H; A, C, F and G; A, C, F and H; A, C, G and H; A, D, F and G; A, D, F and H; A, D, G and H; A, E, F and G; A, E, F and H; A, E, G and H; A, F, G and H; B, C, D and E; B, C, D and F; B, C, D and G; B, C, D and H; B, C, E and F; B, C, E and G; B, C, E and H; B, C, F and G; B, C, F and H; B, C, G and H; B, D, E and F; B, D, E and G; B, D, E and H; B, D, F and G; B, D, F and H; B, D, G and H; B, E, F and G; B, E, F and H; B, E, G and H; B, F, G and H; C, D, E and F; C, D, E and G; C, D, E and H; C, D, F and G; C, D, F and H; C, D, G and H; C, E, F and G; C, E, F and H; C, E, G and H; C, F, G and H; D, E, F and G; D, E, F and H; D, E, G and H; D, F, G and H; and E, F, G and H, wherein each peak in the group is within ±0.2 degrees of the corresponding value described in Table 1-1a.

In another embodiment of this aspect, Form C is characterized by an X-Ray powder diffraction pattern having one of the following groups of peaks as described in Table 1-1a: A, B, C, D and E; A, B, C, D and F; A, B, C, D and G; A, B, C, D and H; A, B, C, E and F; A, B, C, E and G; A, B, C, E and H; A, B, C, F and G; A, B, C, F and H; A, B, C, G and H; A, B, C, E and F; A, B, C, E and G; A, B, C, E and H; A, B, C, F and G; A, B, C, F and H; A, B, C, G and H; A, B, D, E and F; A, B, D, E and G; A, B, D, E and H; A, B, D, F and G; A, B, D, F and H; A, B, D, G and H; A, B, E, F and G; A, B, E, F and H; A, B, E, G and H; A, B, F, G and H; A, C, D, E and F; A, C, D, E and G; A, C, D, E and H; A, C, D, F and G; A, C, D, F and H; A, C, D, G and H; A, C, E, F and G; A, C, E, F and H; A, C, E, G and H; A, C, F, G and H; A, D, E, F and G; A, D, E, F and H; A, D, E, G and H; A, D, F, G and H; A, E, F, G and H; B, C, D, E and F; B, C, D, E and G; B, C, D, E and H; B, C, D, F and G; B, C, D, F and H; B, C, D, G and H; B, C, E, F and G; B, C, E, F and H; B, C, E, G and H; B, C, F, G and H; B, D, E, F and G; B, D, E, F and H; B, D, E, G and H; B, D, F, G and H; B, E, F, G and H; C, D, E, F and G; C, D, E, F and H; C, D, E, G and H; C, D, F, G and H; C, E, F, G and H; and D, E, F, G and H, wherein each peak in the group is within ±0.2 degrees of the corresponding value described in Table 1-1a.

In another embodiment of this aspect, Form C is characterized by an X-Ray powder diffraction pattern having

one of the following groups of peaks as described in Table 1-1a: A, B, C, D, E and F; A, B, C, D, E and G; A, B, C, D, E and H; A, B, C, D, F and G; A, B, C, D, F and H; A, B, C, D, G and H; A, B, C, E, F and G; A, B, C, E, F and H; A, B, C, E, G and H; A, B, C, F, G and H; A, B, D, E, F and G; A, B, D, E, F and H; A, B, D, E, G and H; A, B, D, F, G and H; A, B, E, F, G and H; A, C, D, E, F and G; A, C, D, E, F and H; A, C, D, E, G and H; A, C, D, F, G and H; A, C, E, F, G and H; A, D, E, F, G and H; B, C, D, E, F and G; B, C, D, E, F and H; B, C, D, E, G and H; B, C, D, F, G and H; B, C, E, F, G and H; B, D, E, F, G and H; and C, D, E, F, G and H, wherein each peak in the group is within ±0.2 degrees of the corresponding value described in Table 1-1a.

In another embodiment of this aspect, Form C is characterized by an X-Ray powder diffraction pattern having one of the following groups of peaks as described in Table 1-1a: A, B, C, D, E, F and G; A, B, C, D, E, F and H; A, B, C, D, E, G and H; A, B, C, D, F, G and H; A, B, C, E, F, G and H; A, B, D, E, F, G and H; A, C, D, E, F, G and H; and B, C, D, E, F, G and H, wherein each peak in the group is within ±0.2 degrees of the corresponding value described in Table 1-1a.

In another embodiment of this aspect, Form C is characterized by an X-Ray powder diffraction pattern having all of the following peaks as described in Table 1-1a: A, B, C, D, E, F, G and H, wherein each peak in the group is within ±0.2 degrees of the corresponding value described in Table 1-1a.

**Rietveld Refinement of Form C (Compound 1) from powder**

High resolution data were collected for a crystalline powder sample of Compound 1 Form C (Collection performed at the European Synchrotron Radiation Facility, Grenoble, France) at the beamline ID31. The X-rays are produced by three 11-mm-gap ex-vacuum undulators. The beam is monochromated by a cryogenically cooled double-crystal monochromator (Si 111 crystals). Water-cooled slits define the size of the beam incident on the monochromator, and of the monochromatic beam transmitted to the sample in the range of 0.5 - 2.5 mm (horizontal) by 0.1 - 1.5 mm (vertical). The wavelength used for the experiment was 1.29984(3) Å.

The powder diffraction data were processed and indexed using Materials Studio (Reflex module). The structure was solved using PowderSolve module of Materials Studio. The resulting solution was assessed for structural viability and subsequently refined using Rietveld refinement procedure.

The structure was solved and refined in a centrosymmetric space group $P2_1/c$ using simulated annealing algorithm. The main building block in form C is a dimer composed of two Compound 1 molecules related to each other by a crystallographic inversion center and connected via a pair of hydrogen bonds between the hydroxyl and the amide carbonyl group. These dimers are then further arranged into infinite chains and columns through hydrogen bonding, π-π stacking and van der Waals interactions. Two adjacent columns are oriented perpendicular to each other, one along the crystallographic direction a, the other along b. The columns are connected with each other through van der Waals interactions.

The 4-oxo-1H-quinoline group is locked in a nearly coplanar conformation with the amide group via an intramolecular hydrogen bond. Owing to the centrosymmetric space group, Form C structure contains two Compound 1 molecular conformations related to one another by rotation around the C1-N12 bond.

A powder pattern calculated from the crystal structure of form C and an experimental powder pattern recorded on powder diffractometer using a flat sample in reflectance mode have been compared. The peak positions are in excellent agreement. Some discrepancies in intensities of some peaks exist and are due to preferred orientation of crystallites in the flat sample.

The results of refinement, instrument setup, radiation details, lattice parameters of the resulting crystal are listed below.

**Table 1-2:** Results of refinement:

| | | | |
|---|---|---|---|
| Final $R_{wp}$: | 10.24% | Final $R_p$: | 7.27% |
| Final $R_{wp}$ (without background): | 15.98% | Final CMACS: | 0.09% |

**Table 1-3:** Results of further refinement:

| | | | |
|---|---|---|---|
| Final $R_{wp}$: | 10.50% | Final $R_p$: | 7.49% |
| Final $R_{wp}$ (without background): | 16.41% | Final CMACS: | 0.09% |

**Table 1-4:** Setup

| | | | |
|---|---|---|---|
| 2θ Range (degrees): | 1.00-50.00 | Step Size (degrees): | 0.003 |

(continued)

Excluded Regions:

**Table 1-5:** Radiation

| | | | |
|---|---|---|---|
| Type: | X-ray | Source: | Synchrotron |
| $\lambda_1$ (Å): | 1.299840 | Monochromator: | Double |
| Anom. Dispersion: | No | Angle: | 50.379 |
| | | Polarization: | 0.950 |

**Table 1-6:** Lattice Parameters (Lattice Type: Monoclinic; Space Group: $P2_1/c$

| **Parameter** | **Value** | **Refined?** |
|---|---|---|
| a | 12.211 Å | Yes |
| b | 5.961 Å | Yes |
| c | 32.662 Å | Yes |
| $\alpha$ | 90.00° | No |
| $\beta$ | 119.62° | Yes |
| $\gamma$ | 90.00° | No |

In one embodiment, the crystal structure of Compound 1 Form C has a monoclinic lattice type. In another embodiment, the crystal structure of Compound 1 Form C has a $P2_1/c$ space group. In another embodiment, the crystal structure of Compound 1 Form C has a monoclinic lattice type and a $P2_1/c$ space group.

In one embodiment, the crystal structure of Compound 1 Form C has the following unit cell dimensions:

a = 12.211 Angstroms
b = 5.961 Angstroms
c = 32.662 Angstroms
$\alpha$ = 90.00°
$\beta$ = 119.62°
$\gamma$ = 90.00°

In one aspect, the invention includes Pharmaceutical compositions including Compound 1 Form C and a pharmaceutically acceptable adjuvant or carrier. In one embodiment, Compound 1 Form C can be formulated in a pharmaceutical composition, in some instances, with another therapeutic agent, for example another therapeutic agent for treating cystic fibrosis or a symptom thereof.

Processes for preparing Compound 1 Form C are exemplified herein.

Methods of treating a CFTR mediated disease, such as cystic fibrosis, in a patient include administering to said patient Compound 1 Form C or a pharmaceutical composition comprising Compound 1 Form C.

Compound 1 Form C can be also characterized by an endotherm beginning at 292.78 °C, that plateaus slightly and then peaks at 293.83 °C as measured by DSC (Figure 1-2). Further, this endotherm preceeds an 85% weight loss, as measured by TGA (Figure 1-3), which is attributed to chemical degradation.

Compound 1 Form C can be characterized by a FT-IR spectrum as depicted in Figure 1-5 and by raman spectroscopy as depicted by Figure 1-4.

Compound 1 Form C can be characterize by solid state NMR spectrum as depicted in Figure 1-6.

Processes for preparing Compound 1 Form C are exemplified below.

**III.A.1.b. Synthesis of Compound 1 Form C**

Compound 1 Form C was prepared by adding an excess of optionally recrystallized Compound 1, prepared as provided in Section II.A.3, into acetonitrile, stirring at 90 °C for 3 days, and cooling to room temperature. The product was harvested by filtration, and the purity of the Compound was confirmed using SSNMR. The recrystallization procedure

is reproduced below for convenience.

**Recrystallization of Compound 1**

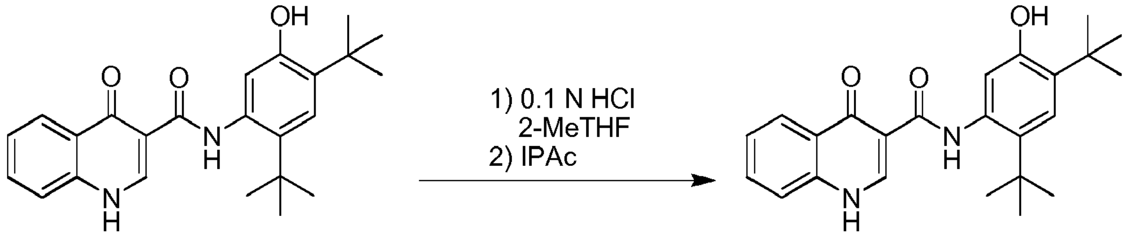


1 1

Compound 1 (1.0 eq) was charged to a reactor. 2-MeTHF (20.0 vol) was added followed by 0.1N HCl (5.0 vol). The biphasic solution was stirred and separated and the top organic phase was washed twice more with 0.1N HCl (5.0 vol). The organic solution was polish filtered to remove any particulates and placed in a second reactor. The filtered solution was concentrated at no more than 35 °C (jacket temperature) and no more than 8.0 °C (internal reaction temperature) under reduced pressure to 10 vol. Isopropyl acetate (IPAc) (10 vol) was added and the solution concentrated at no more than 35 °C (jacket temperature) and no more than 8.0 °C (internal reaction temperature) to 10 vol. The addition of IPAc and concentration was repeated 2 more times for a total of 3 additions of IPAc and 4 concentrations to 10 vol. After the final concentration, 10 vol of IPAc was charged and the slurry was heated to reflux and maintained at this temperature for 5 hours. The slurry was cooled to 0.0 °C +/- 5 °C over 5 hours and filtered. The cake was washed with IPAc (5 vol) once. The resulting solid was dried in a vacuum oven at 50.0 °C +/- 5.0 °C.

**Methods & Materials**

Differential Scanning Calorimetry (DSC)

The DSC traces of Form C were obtained using TA Instruments DSC Q2000 equipped with Universal Analysis 2000 software. An amount (3-8 mg) of Compound 1 Form C was weighed into an aluminum pan and sealed with a pinhole lid. The sample was heated from 25 °C to 325 °C at 10 °C/min. The sample exhibited high melting points which is consistent with highly crystalline material. In one embodiment, the melting range is about 293.3 to about 294.7 °C. In a further embodiment, the melting range is about 293.8 °C to about 294.2 °C. In another embodiment, the onset temperature range is about 292.2 °C to about 293.5 °C. In a further embodiment, the onset temperature range is about 292.7 °C to about 293.0 °C.

Thermogravimetric analysis (TGA)

TGA was conducted on a TA Instruments model Q5000. An amount (3-5 mg) of Compound 1 Form C was placed in a platinum sample pan and heated at 10 °C/min from room temperature to 400 °C. Data were collected by Thermal Advantage Q Series™ software and analyzed by Universal Analysis 2000 software.

XRPD (X-ray Powder Diffraction)

As stated previously, the XRPD patterns were acquired at room temperature in reflection mode using a Bruker D8 Advance diffractometer equipped with a sealed tube copper source and a Vantec-1 detector. The X-ray generator was operating at a voltage of 40 kV and a current of 40 mA. The data were recorded in a θ-θ scanning mode over the range of 3°-40° 2θ with a step size of 0.014° and the sample spinning at 15 rpm.

Raman and FTIR Spectroscopy

Raman spectra for Compound 1, Form C was acquired at room temperature using the VERTEX 70 FT-IR spectrometer coupled to a RAMII FT-Raman module. The sample was introduced into a clear vial, placed in the sample compartment and analyzed using the parameters outlined in the table below.

Raman Parameters

| Parameter | Setting |
|---|---|
| Beam splitter | $CaF_2$ |
| Laser frequency | 9395.0 $cm^{-1}$ |
| Laser power | 1000 mW |
| Save data from | 3501 to 2.94 $cm^{-1}$ |
| Resolution | 4 $cm^{-1}$ |
| Sample scan time | 64 scans |

The FTIR spectra for Compound 1, Form C was acquired at room temperature using the Bruker VERTEX 70 FT-IR spectrometer using the parameters described in the table below.

FTIR Parameters

| Parameter | Setting |
|---|---|
| Scan range | 4000 - 650 $cm^{-1}$ |
| Resolution | 4 $cm^{-1}$ |
| Scans sample | 16 |
| Scans background | 16 |
| Sampling mode | ATR, single reflection ZnSe |

**Table 1-7:** FTIR and Raman peak assignments for Compound 1, Form C: vs= very strong s= strong, m = medium, w= weak intensity.

| Peak assignments | FTIR Wavenumber Intensity | Raman Wavenumber Intensity |
|---|---|---|
| N-H str in -C(=O)-NHR trans | 3281 m | Not observed |
| Unsaturated C-H str -substituted aromatic and olefin | 3085 m, 3056 m | 3071 w, 2991 w |
| Aliphatic C-H str | 2991 m, 2955 m, 2907 m, 2876 m | 2959 w, 2913 w, 2878 w |
| Amide C=O str + Conjugated ketone C=O str | 1643 s | Not observed |
| Olefin C=C conjugated with C=O | Not observed | 1615 s |
| Amide II in -C(=O)-NHR trans | 1524 vs | 1528 s |
| Benzene ring str | 1475 s | Not observed |
| Amide III in -C(=O)-NHR trans | 1285 s | 1310 vs |
| Aromatic C-H wag | 765 vs | Not observed |
| Aromatic in-plane bend modes | Not observed | 748 s |

SSNMR (Solid State Nuclear Magnetic Resonance Spectroscopy)

Bruker-Biospin 400 MHz wide-bore spectrometer equipped with Bruker-Biospin 4mm HFX probe was used. Samples were packed into 4mm $ZrO_2$ rotors and spun under Magic Angle Spinning (MAS) condition with spinning speed of 12.0 kHz. The proton relaxation time was first measured using $^1H$ MAS $T_1$ saturation recovery relaxation experiment in order to set up proper recycle delay of the $^{13}C$ cross-polarization (CP) MAS experiment. The CP contact time of carbon CPMAS experiment was set to 2 ms. A CP proton pulse with linear ramp (from 50% to 100%) was employed. The Hartmann-Hahn match was optimized on external reference sample (glycine). TPPM15 decoupling sequence was used with the field strength of approximately 100 kHz. Some peaks from a $^{13}C$ SSNMR spectrum of Compound 1 Form C are given in Table 1-14.

**Table 1-14:** Listing of some of the SSNMR peaks for Form C.

| Compound 1 Form C | | | |
|---|---|---|---|
| Peak # | Chemical Shift [ppm] | Intensity | Peak Label |
| 1 | 176.5 | 17.95 | A |
| 2 | 165.3 | 23.73 | B |
| 3 | 152.0 | 47.53 | C |
| 4 | 145.8 | 33.97 | D |
| 5 | 139.3 | 30.47 | E |
| 6 | 135.4 | 21.76 | F |
| 7 | 133.3 | 35.38 | G |
| 8 | 131.8 | 21.72 | H |
| 9 | 130.2 | 21.45 | I |
| 10 | 129.4 | 29.31 | J |
| 11 | 127.7 | 31.54 | K |
| 12 | 126.8 | 25.44 | L |
| 13 | 124.8 | 20.47 | M |
| 14 | 117.0 | 42.4 | N |
| 15 | 112.2 | 61.08 | O |
| 16 | 34.5 | 33.34 | P |
| 17 | 32.3 | 14.42 | Q |
| 18 | 29.6 | 100 | R |

In some embodiments, the $^{13}C$ SSNMR spectrum of Compound 1 Form C is includes one or more of the following peaks: 176.5 ppm, 165.3 ppm, 152.0 ppm, 145.8 ppm, 139.3 ppm, 135.4 ppm, 133.3 ppm, 131.8 ppm, 130.2 ppm, 129.4 ppm, 127.7 ppm, 126.8 ppm, 124.8 ppm, 117.0 ppm, 112.2 ppm, 34.5 ppm, 32.3 ppm and 29.6 ppm.

In some embodiments, the $^{13}C$ SSNMR spectrum of Compound 1 Form C includes all of the following peaks: 152.0 ppm, 135.4 ppm, 131.8 ppm, 130.2 ppm, 124.8 ppm, 117.0 ppm and 34.5 ppm.

In some embodiments, the $^{13}C$ SSNMR spectrum of Compound 1 Form C includes all of the following peaks: 152.0 ppm, 135.4 ppm, 131.8 ppm and 117.0 ppm.

In some embodiments, the 13C SSNMR spectrum of Compound 1 Form C includes all of the following peaks: 135.4 ppm and 131.8 ppm.

In some embodiments, the SSNMR of Compound 1 Form C includes a peak at about 152.0 ppm, about 135.4, about 131.8 ppm, and about 117 ppm.

In one aspect, the invention includes Compound 1 Form C which is characterized by a $^{13}C$ SSNMR spectrum having one or more of the following peaks: C, F, H, I, M, N and P, as described by Table 1-14.

In one embodiment of this aspect, Form C is characterized by one peak in a $^{13}C$ SSNMR spectrum, wherein the peak is selected from C, F, H, I, M, N and P, as described by Table 1-14.

In another embodiment of this aspect, Form C is characterized by a $^{13}C$ SSNMR spectrum having a group of peaks selected from C and F; C and H; C and N; F and H; F and N; and H and N, as described by Table 1-14. In a further embodiment, the $^{13}C$ SSNMR spectrum includes the peaks I, M and P as described by Table 1-14.

In another embodiment of this aspect, Form C is characterized by a $^{13}C$ SSNMR spectrum having a group of peaks selected from C, F and H; C, H and N; and F, H and N, as described by Table 1-14. In a further embodiment, the $^{13}C$ SSNMR spectrum includes the peaks I, M and P as described by Table 1-14.

In another embodiment of this aspect, Form C is characterized by a $^{13}C$ SSNMR spectrum having the following group of peaks: C, F, H and N, as described by Table 1-14. In a further embodiment, the $^{13}C$ SSNMR spectrum includes the peaks I, M and P as described by Table 1-14.

In another embodiment of this aspect, Form C is characterized by a $^{13}C$ SSNMR spectrum having a group of peaks selected from C and F; C and H, C and N; C and I; C and M; or C and P, as described by Table 1-14. In another embodiment of this aspect, Form C is characterized by a $^{13}C$ SSNMR spectrum having a group of peaks selected from F and H; F and N; F and I; F and M; or F and P as described by Table 1-14. In another embodiment of this aspect, Form C is characterized by a $^{13}C$ SSNMR spectrum having a group of peaks selected from H and N; H and I; H and M; or H and P as described by Table 1-14. In another embodiment of this aspect, Form C is characterized by a $^{13}C$ SSNMR spectrum having a group of peaks selected from N and I; N and M; or N and P as described by Table 1-14. In another embodiment of this aspect, Form C is characterized by a $^{13}C$ SSNMR spectrum having a group of peaks selected from

I and M; I and P or M and P as described by Table 1-14.

In another embodiment of this aspect, Form C is characterized by a $^{13}C$ SSNMR spectrum having a group of peaks selected from C, F and H; C, F and N; C, F and I; C, F and M; or C, F and P as described by Table 1-14. In another embodiment of this aspect, Form C is characterized by a $^{13}C$ SSNMR spectrum having a group of peaks selected from C, H and N; C, H and I; C, H and M; or C, H and P as described by Table 1-14. In another embodiment of this aspect, Form C is characterized by a $^{13}C$ SSNMR spectrum having a group of peaks selected from C, N and I; C, N and M; or C, N and P as described by Table 1-14. In another embodiment of this aspect, Form C is characterized by a $^{13}C$ SSNMR spectrum having a group of peaks selected from C, I and M; or C, I and P as described by Table 1-14. In another embodiment of this aspect, Form C is characterized by a $^{13}C$ SSNMR spectrum having a group of peaks selected from C, M and P as described by Table 1-14. In another embodiment of this aspect, Form C is characterized by a $^{13}C$ SSNMR spectrum having a group of peaks selected from F, H, and N; F, H and I; F, H and M; or F, H and P as described by Table 1-14. In another embodiment of this aspect, Form C is characterized by a $^{13}C$ SSNMR spectrum having a group of peaks selected from F, N and I; F, N and M; or F, N and P as described by Table 1-14. In another embodiment of this aspect, Form C is characterized by a $^{13}C$ SSNMR spectrum having a group of peaks selected from F, I and M; or F, I and P as described by Table 1-14. In another embodiment of this aspect, Form C is characterized by a $^{13}C$ SSNMR spectrum having a group of peaks selected from F, M and P as described by Table 1-14. In another embodiment of this aspect, Form C is characterized by a $^{13}C$ SSNMR spectrum having a group of peaks selected from H, N and I; H, N and M; or H, N and P as described by Table 1-14. In another embodiment of this aspect, Form C is characterized by a $^{13}C$ SSNMR spectrum having a group of peaks selected from H, I and M; or H, I and P as described by Table 1-14. In another embodiment of this aspect, Form C is characterized by a $^{13}C$ SSNMR spectrum having a group of peaks selected from H, M and P as described by Table 1-14. In another embodiment of this aspect, Form C is characterized by a $^{13}C$ SSNMR spectrum having a group of peaks selected from N, I and M; or N, I and P as described by Table 1-14. In another embodiment of this aspect, Form C is characterized by a $^{13}C$ SSNMR spectrum having a group of peaks selected from N, M and P as described by Table 1-14. In another embodiment of this aspect, Form C is characterized by a $^{13}C$ SSNMR spectrum having a group of peaks selected from I, M and P as described by Table 1-14.

In another embodiment of this aspect, Form C is characterized by a $^{13}C$ SSNMR spectrum having a group of peaks selected from C, F, H, and N; C, F H, and I; C, F H, and M; or C, F H, and P as described by Table 1-14. In another embodiment of this aspect, Form C is characterized by a $^{13}C$ SSNMR spectrum having a group of peaks selected from F, H, N and I; F, H, N and M; or F, H, N and P as described by Table 1-14. In another embodiment of this aspect, Form C is characterized by a $^{13}C$ SSNMR spectrum having a group of peaks selected from H, N, I and M; H, N, I and P; or H, N, I and C as described by Table 1-14. In another embodiment of this aspect, Form C is characterized by a $^{13}C$ SSNMR spectrum having a group of peaks selected from N, I, M and P; N, I, M and C; or N, I, M and F as described by Table 1-14. In another embodiment of this aspect, Form C is characterized by a $^{13}C$ SSNMR spectrum having a group of peaks selected from I, M, P and C; I, M, P and F; I, M, P and H as described by Table 1-14.

In another embodiment of this aspect, Form C is characterized by a $^{13}C$ SSNMR spectrum having a group of peaks selected from C, H, N and I; C, H, N, and M; or C, H, N, and P as described by Table 1-14. In another embodiment of this aspect, Form C is characterized by a $^{13}C$ SSNMR spectrum having a group of peaks selected from C, N, I and M; C, N, I and P; or C, N, I and F as described by Table 1-14. In another embodiment of this aspect, Form C is characterized by a $^{13}C$ SSNMR spectrum having a group of peaks selected from C, I, M and P; C, I, M and F; or C, I, M and H as described by Table 1-14. In another embodiment of this aspect, Form C is characterized by a $^{13}C$ SSNMR spectrum having a group of peaks selected from C, M, P and F; C, M, P and H; or C, M, P and N as described by Table 1-14. In another embodiment of this aspect, Form C is characterized by a $^{13}C$ SSNMR spectrum having a group of peaks selected from F, N, I and M; F, N, I and P; or F, N, I and C as described by Table 1-14. In another embodiment of this aspect, Form C is characterized by a $^{13}C$ SSNMR spectrum having a group of peaks selected from F, I, M and P; F, I, M and C; F, I, M and H; or F, I, M and N as described by Table 1-14. In another embodiment of this aspect, Form C is characterized by a $^{13}C$ SSNMR spectrum having a group of peaks selected from F, M, P and C; F, M, P and H; or F, M, P and N as described by Table 1-14. In another embodiment of this aspect, Form C is characterized by a $^{13}C$ SSNMR spectrum having a group of peaks selected from H, I, M and P; H, I, M and C; or H, I, M and F as described by Table 1-14. In another embodiment of this aspect, Form C is characterized by a $^{13}C$ SSNMR spectrum having a group of peaks selected from N, M, P and C; N, M, P and F; or N, M, P and H as described by Table 1-14. In another embodiment of this aspect, Form C is characterized by a $^{13}C$ SSNMR spectrum having a group of peaks selected from N, M, C and F; or N, M, C and H as described by Table 1-14. In another embodiment of this aspect, Form C is characterized by a $^{13}C$ SSNMR spectrum having a group of peaks selected from N, M, F and P as described by Table 1-14. In another embodiment of this aspect, Form C is characterized by a $^{13}C$ SSNMR spectrum having a group of peaks selected from N, M, H and P as described by Table 1-14. In another embodiment of this aspect, Form C is characterized by a $^{13}C$ SSNMR spectrum having a group of peaks selected from C, H, I and P; C, F, I and P; C, F, N and P or F, H, I and P as described by Table 1-14.

In another embodiment of this aspect, Form C is characterized by a $^{13}C$ SSNMR spectrum having a group of peaks selected from C, F, H, N and I; C, F, H, N and M; or C, F, H, N and P; C, F, H, I and M; C, F, H, I and P; C, F, H,

M and P; C, F, N, I and M; C, F, N, I and P; C, F, N, M and P; C, H, N, I and M; C, H, N, I and P; C, H, N, M and P; C, H, I, M and P; F, H, N, I and M; F, H, N, I and P; F, H, N, M and P; F, H, I, M and P; F, N, I, M and P or H, N, I, M and P as described by Table 1-14.

In another embodiment of this aspect, Form C is characterized by a $^{13}C$ SSNMR spectrum having a group of peaks selected from C, F, H, N and I; C, F, H, N and M; or C, F, H, N and P as described by Table 1-14. In another embodiment of this aspect, Form C is characterized by a $^{13}C$ SSNMR spectrum having a group of peaks selected from C, H, N, I and M; or C, H, N, I and P as described by Table 1-14. In another embodiment of this aspect, Form C is characterized by a $^{13}C$ SSNMR spectrum having a group of peaks selected from C, N, I, M and P; or C, N, I, M and F as described by Table 1-14. In another embodiment of this aspect, Form C is characterized by a $^{13}C$ SSNMR spectrum having a group of peaks selected from C, I, M, P and F; or C, I, M, P and H as described by Table 1-14. In another embodiment of this aspect, Form C is characterized by a $^{13}C$ SSNMR spectrum having a group of peaks selected from C, M, P, F and H; or C, M, P, F and N as described by Table 1-14. In another embodiment of this aspect, Form C is characterized by a $^{13}C$ SSNMR spectrum having a group of peaks selected from C, P, F, H and I; or C, P, F, H and M as described by Table 1-14. In another embodiment of this aspect, Form C is characterized by a $^{13}C$ SSNMR spectrum having a group of peaks selected from F, H, N, I and M; or F, H, N, I and P as described by Table 1-14. In another embodiment of this aspect, Form C is characterized by a $^{13}C$ SSNMR spectrum having a group of peaks selected from F, N, I, M and P; or F, N, I, M and C as described by Table 1-14. In another embodiment of this aspect, Form C is characterized by a $^{13}C$ SSNMR spectrum having a group of peaks selected from F, I, M, C and H; F, I, M, C and N as described by Table 1-14. In another embodiment of this aspect, Form C is characterized by a $^{13}C$ SSNMR spectrum having a group of peaks selected from F, M, P, C and H; F, M, P, C and N , N, I and M; or F, H, N, I and P as described by Table 1-14. In another embodiment of this aspect, Form C is characterized by a $^{13}C$ SSNMR spectrum having a group of peaks selected from H, N, I M, and P as described by Table 1-14. In another embodiment of this aspect, Form C is characterized by a $^{13}C$ SSNMR spectrum having a group of peaks selected from H, I M, P and F as described by Table 1-14. In another embodiment of this aspect, Form C is characterized by a $^{13}C$ SSNMR spectrum having a group of peaks selected from H, M, P, C and F as described by Table 1-14. In another embodiment of this aspect, Form C is characterized by a $^{13}C$ SSNMR spectrum having a group of peaks selected from H, P, C, F and I as described by Table 1-14.

In another embodiment of this aspect, Form C is characterized by a $^{13}C$ SSNMR spectrum having a group of peaks selected from C, F, H, N, I, and M; or C, F, H, N, I and P as described by Table 1-14. In another embodiment of this aspect, Form C is characterized by a $^{13}C$ SSNMR spectrum having a group of peaks selected from F, H, N, I, M and P as described by Table 1-14. In another embodiment of this aspect, Form C is characterized by a $^{13}C$ SSNMR spectrum having a group of peaks selected from H, N, I, M, P and C as described by Table 1-14. In another embodiment of this aspect, Form C is characterized by a $^{13}C$ SSNMR spectrum having a group of peaks selected from N, I, M, P, C and F as described by Table 1-14. In another embodiment of this aspect, Form C is characterized by a $^{13}C$ SSNMR spectrum having a group of peaks selected from M, P, C, F, H and N as described by Table 1-14.

In another embodiment of this aspect, Form C is characterized by a $^{13}C$ SSNMR spectrum having a group of peaks selected from C, F, H, N, I, and M; C, F, H, N, I and P; C, F, H, N, M and P; C, F, H, I, M and P; C, F, N, I, M and P; C, H, N, I, M and P or F, H, N, I, M and P as described by Table 1-14.

In another embodiment of this aspect, Form C is characterized by a 13C SSNMR spectrum having a group of peaks selected from C, F, H, N, I, M and P as described by Table 1-14.

### III.B. Solid Forms of Compound 2

### III.B.1. Compound 2 Form I

### III.B.1.a. Embodiments of Compound 2 Form I

In one aspect of the composition, Compound 2 is in solid Form I (Compound 2 Form I).

In another embodiment, Compound 2 Form I is characterized by one or more peaks at 15.2 to 15.6 degrees, 16.1 to 16.5 degrees, and 14.3 to 14.7 degrees in an X-ray powder diffraction obtained using Cu K alpha radiation.

In another embodiment, Compound 2 Form I is characterized by one or more peaks at 15.4, 16.3, and 14.5 degrees.

In another embodiment, Compound 2 Form I is further characterized by a peak at 14.6 to 15.0 degrees.

In another embodiment, Compound 2 Form I is further characterized by a peak at 14.8 degrees.

In another embodiment, Compound 2 Form I is further characterized by a peak at 17.6 to 18.0 degrees.

In another embodiment, Compound 2 Form I is further characterized by a peak at 17.8 degrees.

In another embodiment, Compound 2 Form I is further characterized by a peak at 16.4 to 16.8 degrees.

In another embodiment, Compound 2 Form I is further characterized by a peak at 16.4 to 16.8 degrees.

In another embodiment, Compound 2 Form I is further characterized by a peak at 16.6 degrees.

In another embodiment, Compound 2 Form I is further characterized by a peak at 7.6 to 8.0 degrees.

In another embodiment, Compound 2 Form I is further characterized by a peak at 7.8 degrees.

In another embodiment, Compound 2 Form I is further characterized by a peak at 25.8 to 26.2 degrees.

In another embodiment, Compound 2 Form I is further characterized by a peak at 26.0 degrees.

In another embodiment, Compound 2 Form I is further characterized by a peak at 21.4 to 21.8 degrees.

In another embodiment, Compound 2 Form I is further characterized by a peak at 21.6 degrees.

In another embodiment, Compound 2 Form I is further characterized by a peak at 23.1 to 23.5 degrees.

In another embodiment, Compound 2 Form I is further characterized by a peak at 23.3 degrees.

In some embodiments, Compound 2 Form I is characterized by a diffraction pattern substantially similar to that of Figure 2-1.

In some embodiments, Compound 2 Form I is characterized by a diffraction pattern substantially similar to that of Figure 2-2.

In some embodiments, the particle size distribution of D90 is about 82 μm or less for Compound 2 Form I.

In some embodiments, the particle size distribution of D50 is about 30 μm or less for Compound 2 Form I.

In one aspect, the invention features a crystal form of Compound 2 Form I having a monoclinic crystal system, a $P2_1/n$ space group, and the following unit cell dimensions: a = 4.9626 (7) Å, b = 12.2994 (18) Å, c = 33.075 (4) Å, $\alpha$ = 90°, $\beta$ = 93.938 (9)°, and $\gamma$ = 90°.

**III.B.1.b. Synthesis of Compound 2 Form I**

**Preparation of Compound 2 Form I**

**Method A.**

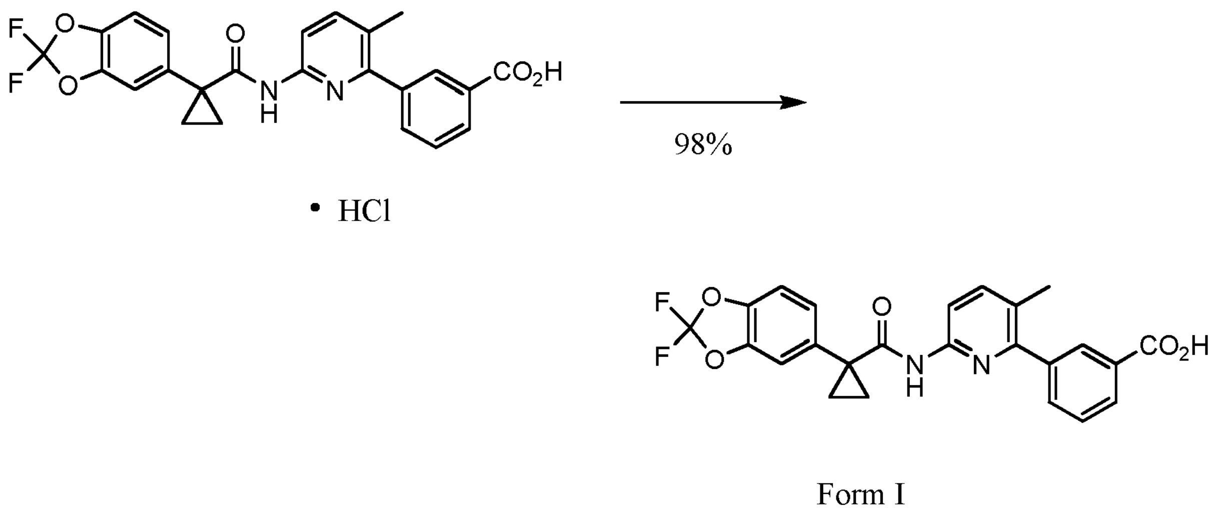


A slurry of 3-(6-(1-(2,2-difluorobenzo[d][1,3]dioxol-5-yl) cyclopropanecarboxamido)-3-methylpyridin-2-yl)benzoic acid • HCl (1 eq) in water (10 vol) was stirred at ambient temperature. A sample was taken after stirring for 24 h. The sample was filtered and the solid was washed with water (2 times). The solid sample was submitted for DSC analysis. When DSC analysis indicated complete conversion to Form I, the solid was collected by filtration, washed with water (2 x 1.0 vol), and partially dried on a filter under vacuum. The solid was then dried to a constant weight (<1% difference) in a vacuum oven at 60 °C with a slight $N_2$ bleed to afford Compound 2 Form I as an off-white solid (98% yield).

**Method B:**

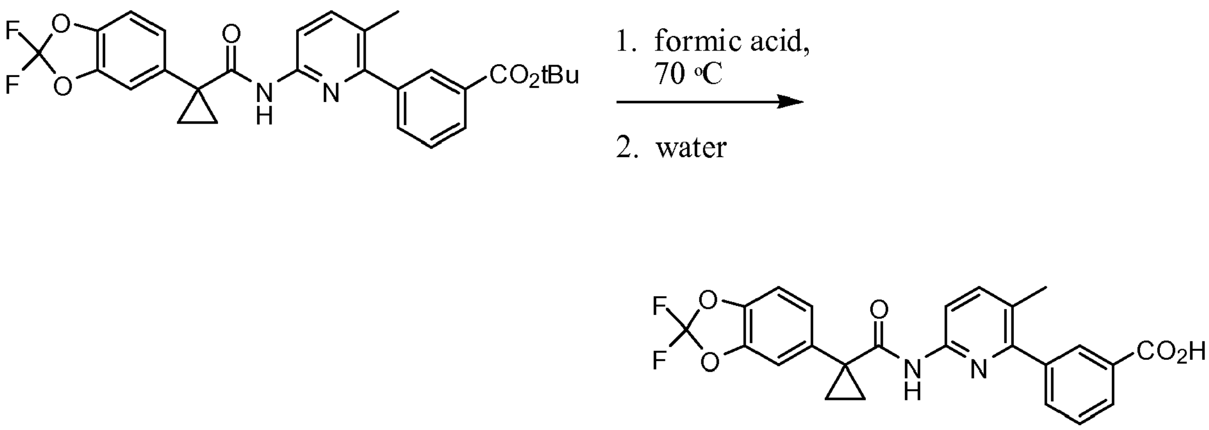


Form I

A solution of 3-(6-(1-(2,2-difluorobenzo[d][1,3]dioxol-5-yl) cyclopropanecarboxamido)-3-methylpyridin-2-yl)-t-butylbenzoate (1.0 eq) in formic acid (3.0 vol) was heated with stirring to 70 ± 10 °C , for 8 h. The reaction was deemed complete when no more than 1.0% AUC by chromatographic methods of 3-(6-(1-(2,2-difluorobenzo[d][1,3]dioxol-5-yl) cyclopropanecarboxamido)-3-methylpyridin-2-yl)-t-butylbenzoate) remained. The mixture was allowed to cool to ambient temperature. The solution was added to water (6 vol), heated at 50 °C, and the mixture was stirred. The mixture was then heated to 70 ± 10 °C until the level of 3-(6-(1-(2,2-difluorobenzo[d][1,3]dioxol-5-yl) cyclopropanecarboxamido)-3-methylpyridin-2-yl)-t-butylbenzoate was no more than 0.8% (AUC). The solid was collected by filtration, washed with water (2 x 3 vol), and partially dried on the filter under vacuum. The solid was dried to a constant weight (<1% difference) in a vacuum oven at 60 °C with a slight $N_2$ bleed to afford Compound 2 Form I as an off-white solid.

**III.B.1.c. Characterization of Compound 2 Form I**

**Methods & Materials**

**XRPD (X-ray Powder Diffraction)**

The X-Ray diffraction (XRD) data of Compound 2 Form I were collected on a Bruker D8 DISCOVER powder diffractometer with HI-STAR 2-dimensional detector and a flat graphite monochromator. Cu sealed tube with K$\alpha$ radiation was used at 40 kV, 35mA. The samples were placed on zero-background silicon wafers at 25°C. For each sample, two data frames were collected at 120 seconds each at 2 different $\theta_2$ angles: 8° and 26°. The data were integrated with GADDS software and merged with DIFFRACT$^{plus}$EVA software. Uncertainties for the reported peak positions are ± 0.2 degrees.

**Differential Scanning Calorimetry (DSC)**

The Differential scanning calorimetry (DSC) data of Compound 2 Form I were collected using a DSC Q100 V9.6 Build 290 (TA Instruments, New Castle, DE). Temperature was calibrated with indium and heat capacity was calibrated with sapphire. Samples of 3-6 mg were weighed into aluminum pans that were crimped using lids with 1 pin hole. The samples were scanned from 25°C to 350°C at a heating rate of 1.0°C/min and with a nitrogen gas purge of 50 ml/min. Data were collected by Thermal Advantage Q SeriesTM version 2.2.0.248 software and analyzed by Universal Analysis software version 4.1D (TA Instruments, New Castle, DE). The reported numbers represent single analyses.

**Compound 2 Form I Single Crystal Structure Determination**

Diffraction data were acquired on Bruker Apex II diffractometer equipped with sealed tube Cu K-alpha source and an Apex II CCD detector. The structure was solved and refined using SHELX program (Sheldrick, G.M., Acta Cryst., (2008) A64, 112-122). Based on systematic absences and intensities statistics the structure was solved and refined in $P2_1/n$ space group.

An X-ray diffraction pattern was calculated from a single crystal structure of Compound 2 Form I and is shown in Figure 2-1. Table 2-2 lists the calculated peaks for Figure 2-1.

**Table 2-2**

| Peak Rank | 2θ Angle [degrees] | Relative Intensity [%] |
|---|---|---|
| 11 | 14.41 | 48.2 |
| 8 | 14.64 | 58.8 |
| 1 | 15.23 | 100.0 |
| 2 | 16.11 | 94.7 |
| 3 | 17.67 | 81.9 |
| 7 | 19.32 | 61.3 |
| 4 | 21.67 | 76.5 |
| 5 | 23.40 | 68.7 |
| 9 | 23.99 | 50.8 |
| 6 | 26.10 | 67.4 |
| 10 | 28.54 | 50.1 |

An actual X-ray powder diffraction pattern of Compound 2 Form I is shown in Figure 2-2. Table 2-3 lists the actual peaks for Figure 2-2.

**Table 2-3**

| Peak Rank | 2θ Angle [degrees] | Relative Intensity [%] |
|---|---|---|
| 7 | 7.83 | 37.7 |
| 3 | 14.51 | 74.9 |
| 4 | 14.78 | 73.5 |
| 1 | 15.39 | 100.0 |
| 2 | 16.26 | 75.6 |
| 6 | 16.62 | 42.6 |
| 5 | 17.81 | 70.9 |
| 9 | 21.59 | 36.6 |
| 10 | 23.32 | 34.8 |
| 11 | 24.93 | 26.4 |
| 8 | 25.99 | 36.9 |

Colorless crystals of Compound 2 Form I were obtained by cooling a concentrated 1-butanol solution from 75°C to 10 °C at a rate of 0.2 °C/min. A crystal with dimensions of 0.50 *x* 0.08 *x* 0.03 mm was selected, cleaned with mineral oil, mounted on a MicroMount and centered on a Bruker *APEX* II system. Three batches of 40 frames separated in reciprocal space were obtained to provide an orientation matrix and initial cell parameters. Final cell parameters were obtained and refined based on the full data set.

A diffraction data set of reciprocal space was obtained to a resolution of 0.82 Å using 0.5° steps using 30 s exposure for each frame. Data were collected at 100 (2) K. Integration of intensities and refinement of cell parameters were accomplished using APEXII software. Observation of the crystal after data collection showed no signs of decomposition.

A conformational picture of Compound 2 Form I based on single crystal X-ray analysis is shown in Figure 2-3. Compound 2 Form I is monoclinic, $P_21/n$, with the following unit cell dimensions: a=4.9626(7) Å, b=12.299(2) Å, c=33.075 (4) Å, β=93.938(9)°, V=2014.0 $Å^3$, Z=4. Density of Compound 2 in Form I calculated from structural data is 1.492 $g/cm^3$ at 100 K.

Melting for Compound 2 in Form I occurs at about 204 °C.

**Compound 2 Form I SSNMR Characterization**

Bruker-Biospin 400 MHz wide-bore spectrometer equipped with Bruker-Biospin 4mm HFX probe was used. Samples were packed into 4mm $ZrO_2$ rotors and spun under Magic Angle Spinning (MAS) condition with spinning speed of 15.0 kHz. The proton relaxation time was first measured using $^{1}H$ MAS $T_1$ saturation recovery relaxation experiment in order to set up proper recycle delay of the $^{13}C$ cross-polarization (CP) MAS experiment. The fluorine relaxation time was measured using $^{19}F$ MAS $T_1$ saturation recovery relaxation experiment in order to set up proper recycle delay of the $^{19}F$ MAS experiment. The CP contact time of carbon CPMAS experiment was set to 2 ms. A CP proton pulse with linear ramp (from 50% to 100%) was employed. The carbon Hartmann-Hahn match was optimized on external reference sample (glycine). The fluorine MAS and CPMAS spectra were recorded with proton decoupling. TPPM 15 proton decoupling sequence was used with the field strength of approximately 100 kHz for both $^{13}C$ and $^{19}F$ acquisitions.

Figure 2-23 shows the $^{13}C$ CPMAS NMR spectrum of Compound 2 Form I. Some peaks of this spectrum are summarized in Table 2-4.

**Table 2-4**

| Compound 2 Form I $^{13}C$ Chem. Shifts | | |
|---|---|---|
| Peak # | [ppm] | Intensity |
| 1 | 172.1 | 8.59 |
| 2 | 170.8 | 4.3 |
| 3 | 157.0 | 4.04 |
| 4 | 148.0 | 3.46 |
| 5 | 144.3 | 6.1 |
| 6 | 140.9 | 9.9 |
| 7 | 135.6 | 7.21 |
| 8 | 131.8 | 6.94 |
| 9 | 131.0 | 7.78 |
| 10 | 130.4 | 5.49 |
| 11 | 128.9 | 5.72 |
| 12 | 128.4 | 7.26 |
| 13 | 128.0 | 8.43 |
| 14 | 126.6 | 6.3 |
| 15 | 113.3 | 7.52 |
| 16 | 111.1 | 9.57 |
| 17 | 31.5 | 9.14 |
| 18 | 19.3 | 6.51 |
| 19 | 18.1 | 10 |
| 20 | 15.1 | 6.16 |

Figure 2-24 shows the $^{19}F$ MAS NMR spectrum of Compound 2 Form I. The peaks marked with an asterisk (*) are spinning side bands (15.0 kHz spinning speed). Some peaks of this spectrum are summarized in Table 2-5.

**Table 2-5**

| Compound 2 Form I $^{19}F$ Chem. Shifts* | | |
|---|---|---|
| Peak # | [ppm] | Intensity |
| 1 | -42.3 | 12.5 |
| 2 | -47.6 | 10.16 |

### III.B.2. Compound 2 Solvate Form A

### III.B.2.a. Embodiments of Compound 2 Solvate Form A

In one aspect, the invention includes compositions comprising various combinations of Compound 2.

In one aspect of the composition, Compound 2 is characterized as an isostructural solvate form referred to as Compound 2 Solvate Form A.

Compound 2 Solvate Form A as disclosed herein comprises a crystalline lattice of Compound 2 in which voids in the crystalline lattice are occupied by one or more molecules of a suitable solvent. Suitable solvents include, but are not limited to, methanol, ethanol, acetone, 2-propanol, acetonitrile, tetrahydrofuran, methyl acetate, 2-butanone, ethyl formate, and 2-methyl tetrahydrofuran. Certain physical characteristics of Compound 2 isostructural solvate forms, such as X-ray powder diffraction, melting point and DSC, are not substantially affected by the particular solvent molecule in question.

In one embodiment, Compound 2 Solvate Form A is characterized by one or more peaks at 21.50 to 21.90 degrees, 8.80 to 9.20 degrees, and 10.80 to 11.20 degrees in an X-ray powder diffraction obtained using Cu K alpha radiation.

In another embodiment, Compound 2 Solvate Form A is characterized by one or more peaks at 21.50 to 21.90 degrees, 8.80 to 9.20 degrees, 10.80 to 11.20 degrees, 18.00 to 18.40 degrees, and 22.90 to 23.30 degrees in an X-ray powder diffraction obtained using Cu K alpha radiation.

In another embodiment, Compound 2 Solvate Form A is characterized by one or more peaks at 21.70, 8.98, and 11.04 degrees.

In another embodiment, Compound 2 Solvate Form A is characterized by one or more peaks at 21.70, 8.98, 11.04, 18.16, and 23.06 degrees.

In another embodiment, Compound 2 Solvate Form A is characterized by a peak at 21.50 to 21.90 degrees.

In another embodiment, Compound 2 Solvate Form A is further characterized by a peak at 21.70 degrees.

In another embodiment, Compound 2 Solvate Form A is further characterized by a peak at 8.80 to 9.20 degrees.

In another embodiment, Compound 2 Solvate Form A is further characterized by a peak at 8.98 degrees.

In another embodiment, Compound 2 Solvate Form A is further characterized by a peak at 10.80 to 11.20 degrees.

In another embodiment, Compound 2 Solvate Form A is further characterized by a peak at 11.04.

In another embodiment, Compound 2 Solvate Form A is further characterized by a peak at 18.00 to 18.40 degrees.

In another embodiment, Compound 2 Solvate Form A is further characterized by a peak at 18.16 degrees.

In another embodiment, Compound 2 Solvate Form A is further characterized by a peak at 22.90 to 23.30 degrees.

In another embodiment, Compound 2 Solvate Form A is further characterized by a peak at 23.06 degrees.

In another embodiment, Compound 2 Solvate Form A is further characterized by a peak at 20.40 to 20.80 degrees.

In another embodiment, Compound 2 Solvate Form A is further characterized by a peak at 20.63 degrees.

In another embodiment, Compound 2 Solvate Form A is further characterized by a peak at 22.00 to 22.40 degrees.

In another embodiment, Compound 2 Solvate Form A is further characterized by a peak at 22.22 degrees.

In another embodiment, Compound 2 Solvate Form A is further characterized by a peak at 18.40 to 18.80 degrees.

In another embodiment, Compound 2 Solvate Form A is further characterized by a peak at 18.57 degrees.

In another embodiment, Compound 2 Solvate Form A is further characterized by a peak at 16.50 to 16.90 degrees.

In another embodiment, Compound 2 Solvate Form A is further characterized by a peak at 16.66 degrees.

In another embodiment, Compound 2 Solvate Form A is further characterized by a peak at 19.70 to 20.10 degrees.

In another embodiment, Compound 2 Solvate Form A is further characterized by a peak at 19.86 degrees.

In some embodiments, Compound 2 Solvate Form A is characterized by a diffraction pattern substantially similar to that of Figure 2-4.

In some embodiments, Compound 2 Solvate Form A is characterized by diffraction patterns substantially similar to those provided in Figure 2-5.

In other embodiments, the solvate or solvate mixture that forms Solvate Form A with Compound 2 is selected from the group consisting of an organic solvent of sufficient size to fit in the voids in the crystalline lattice of Compound 2. In some embodiments, the solvate is of sufficient size to fit in voids measuring about 100 $Å^3$.

In another embodiment, the solvate that forms Compound 2 Solvate Form A is selected from the group consisting of methanol, ethanol, acetone, 2-propanol, acetonitrile, tetrahydrofuran, methyl acetate, 2-butanone, ethyl formate, and 2-methyl tetrahydrofuran. Diffraction patterns are provided for the following Compound 2, Solvate A forms: methanol (Figure 2-6), ethanol (Figure 2-7), acetone (Figure 2-8), 2-propanol (Figure 2-9), acetonitrile (Figure 2-10), tetrahydrofuran (Figure 2-11), methyl acetate (Figure 2-12), 2-butanone (Figure 2-13), ethyl formate (Figure 2-14), and 2-methytetrahydrofuran (Figure 2-15).

In another embodiment, the invention features crystalline Compound 2 Acetone Solvate Form A having a $P2_1/n$ space group, and the following unit cell dimensions: a = 16.5235 (10) Å, b = 12.7425 (8) Å, c = 20.5512 (13) Å, $\alpha$ = 90°, $\beta$ = 103.736 (4)°, and $\gamma$ = 90°.

In another embodiment, the invention provides Compound 2 Solvate Form A which exhibits two or more phase transitions as determined by DSC or a similar analytic method known to the skilled artisan.

In another embodiment of this aspect, the DSC gives two phase transitions.

In another embodiment, the DSC gives three phase transitions.

In another embodiment, one of the phase transitions occurs between 200 and 207 °C. In another embodiment, one of the phase transitions occurs between 204 and 206 °C. In another embodiment, one of the phase transitions occurs between 183 and 190 °C. In another embodiment, one of the phase transitions occurs between 185 and 187 °C.

In another embodiment, the melting point of Compound 2 Solvate Form A is between 183 °C to 190 °C. In another embodiment, the melting point of Compound 2 Solvate Form A is between 185 °C to 187 °C.

In another embodiment, Compound 2 Solvate Form A comprises 1 to 10 weight percent (wt. %) solvate as determined by TGA.

In another embodiment, Compound 2 Solvate Form A comprises 2 to 5 wt. % solvate as determined by TGA or a similar analytic method known to the skilled artisan.

In another embodiment, the conformation of Compound 2 Acetone Solvate Form A is substantially similar to that depicted in Figure 2-16, which is based on single X-ray analysis.

In one aspect, the present invention features a process for preparing Compound 2 Solvate Form A. Accordingly, an amount of Compound 2 Form I is slurried in an appropriate solvent at a sufficient concentration for a sufficient time. The slurry is then filtered centrifugally or under vacuum and dried at ambient conditions for sufficient time to yield Compound 2 Solvate Form A.

In some embodiments, about 20 to 40 mg of Compound 2 Form I is slurried in about 400 to 600 $\mu$L of an appropriate solvent. In another embodiment, about 25 to 35 mg of Compound 2 Form I is slurried in about 450 to 550 $\mu$L of an appropriate solvent. In another embodiment, about 30 mg of Compound 2 Form I is slurried in about 500 $\mu$L of an appropriate solvent.

In some embodiments, the time that Compound 2 Form I is allowed to slurry with the solvent is from1 hour to four days. More particularly, the time that Compound 2 Form I is allowed to slurry with the solvent is from1 to 3 days. More particularly, the time is 2 days.

In some embodiments, the appropriate solvent is selected from an organic solvent of sufficient size to fit the voids in the crystalline lattice of Compound 2. In other embodiments, the solvate is of sufficient size to fit in voids measuring about 100 $Å^3$.

In other embodiments, the solvent is selected from the group consisting of methanol, ethanol, acetone, 2-propanol, acetonitrile, tetrahydrofuran, methyl acetate, 2-butanone, ethyl formate, and 2-methyl tetrahydrofuran.

In other embodiments, a mixture of two or more of these solvents may be used to obtain Compound 2 Solvate Form A. Alternatively, Compound 2 Solvate Form A may be obtained from a mixture comprising one or more of these solvents and water.

In some embodiments, the effective amount of time for drying Compound 2 Solvate Form A is 1 to 24 hours. More particularly, the time is 6 to 18 hours. More particularly, the time is about 12 hours.

In another embodiment, Compound 2 HCl salt is used to prepare Compound 2 Solvate Form A. Compound 2 Solvate Form A is prepared by dispersing or dissolving a salt form, such as the HCl salt, in an appropriate solvent for an effective amount of time.

### III.B.2.b. Synthesis of Compound 2 Solvate Form A

### Preparation of Compound 2 Solvate Form A

Compound 2 Form I (approximately 30 mg) was slurried in 500 $\mu$L of an appropriate solvent (for example, methanol, ethanol, acetone, 2-propanol, acetonitrile, tetrahydrofuran, methyl acetate, 2-butanone, ethyl formate, and -methyl tetrahydrofuran for two days. The slurry was then filtered centrifugally or under vacuum and was left to dry at ambient temperature overnight to yield Compound 2 Solvate Form A.

### III.B.2.c. Characterization of Compound 2 Solvate Form A

#### Methods & Materials

#### Differential Scanning Calorimetry (DSC)

The Differential scanning calorimetry (DSC) data for Compound 2 Solvate Form A were collected using a DSC Q100 V9.6 Build 290 (TA Instruments, New Castle, DE). Temperature was calibrated with indium and heat capacity was calibrated with sapphire. Samples of 3-6 mg were weighed into aluminum pans that were crimped using lids with 1 pin hole. The samples were scanned from 25°C to 350°C at a heating rate of 1.0°C/min and with a nitrogen gas purge of 50 ml/min. Data were collected by Thermal Advantage Q Series™ version 2.2.0.248 software and analyzed by Universal Analysis software version 4.1D (TA Instruments, New Castle, DE). The reported numbers represent single analyses.

#### XRPD (X-ray Powder Diffraction)

X-Ray diffraction (XRD) data were collected on either a Bruker D8 DISCOVER or Bruker APEX II powder diffractometer. The Bruker D8 DISCOVER Diffractomer with HI-STAR 2-dimensional detector and a flat graphite monochromator. Cu sealed tube with Kα radiation was used at 40 kV, 35mA. The samples were placed on zero-background silicon wafers at 25°C. For each sample, two data frames were collected at 120 seconds each at 2 different $\theta_2$ angles: 8° and 26°. The data were integrated with GADDS software and merged with DIFFRACT$^{plus}$EVA software. Uncertainties for the reported peak positions are ± 0.2 degrees. equipped with sealed tube Cu Kα source and an Apex II CCD detector.

The Bruker II powder diffractomer was equipped with a sealed tube CuK source and an APEX II CCD detector. Structures were solved and refined using the *SHELX* program. (Sheldrick, G.M., Acta Cryst. (2008) A64, 112-122).

The melting point for Compound 2 Acetone Solvate Form A occurs at about 188 °C and 205 °C.

An actual X-ray powder diffraction pattern of Compound 2 Solvate Form A is shown in Figure 2-4. Table 2-6 lists the actual peaks for Figure 2-4 in descending order of relative intensity.

**Table 2-6**

| 2θ Angle [degrees] | Relative Intensity [%] |
|---|---|
| 21.70 | 100.0 |
| 8.98 | 65.5 |
| 11.04 | 57.4 |
| 18.16 | 55.9 |
| 23.06 | 55.4 |
| 20.63 | 53.1 |
| 22.22 | 50.2 |
| 18.57 | 49.1 |
| 16.66 | 47.2 |
| 19.86 | 35.0 |

Conformational depictions of Compound 2 Acetone Solvate Form A based on single crystal X-ray analysis are shown in Figures 2-16 through 2-19. Figure 2-16 shows a conformational image of Compound 2 Acetone Solvate Form A, based on single crystal X-ray analysis. Figure 2-17 provides a conformational image of Compound 2 Acetone Solvate Form A as a dimer showing hydrogen bonding between the carboxylic acid groups based on single X-ray crystal analysis. Figure 2-18 provides a conformational image of a tetramer of Compound 2 Acetone Solvate Form A. Figure 2-19 provides a confirmation of Compound 2 Acetone Solvate Form A, based on single crystal X-ray analysis. The stoichiometry between Compound 2 Solvate Form A and acetone is approximately 4.4:1 (4.48:1 calculated from $^1$H NMR; 4.38:1 from X-ray). The crystal structure reveals a packing of the molecules where there are two voids or pockets per unit cell, or 1 void per host molecule. In the acetone solvate, approximately 92 percent of voids are occupied by acetone molecules. Compound 2 Solvate Form A is a monoclinic $P2_1/n$ space group with the following unit cell dimensions: a = 16.5235(10) Å, b = 12.7425(8) Å, c = 20.5512 (13) Å, α = 90°, β = 103.736(4)°, γ = 90°, V = 4203.3(5) Å$^3$, = 4. The density of Compound 2 in Compound 2 Solvate Form A calculated from structural data is 1.430/cm$^3$ at 100 K.

**Compound 2 Acetone Solvate Form A SSNMR Characterization**

Bruker-Biospin 400 MHz wide-bore spectrometer equipped with Bruker-Biospin 4mm HFX probe was used. Samples were packed into 4mm $ZrO_2$ rotors and spun under Magic Angle Spinning (MAS) condition with spinning speed of 15.0 kHz. The proton relaxation time was first measured using $^1H$ MAS $T_1$ saturation recovery relaxation experiment in order to set up proper recycle delay of the $^{13}C$ cross-polarization (CP) MAS experiment. The fluorine relaxation time was measured using $^{19}F$ MAS $T_1$ saturation recovery relaxation experiment in order to set up proper recycle delay of the $^{19}F$ MAS experiment. The CP contact time of carbon CPMAS experiment was set to 2 ms. A CP proton pulse with linear ramp (from 50% to 100%) was employed. The carbon Hartmann-Hahn match was optimized on external reference sample (glycine). The fluorine MAS and CPMAS spectra were recorded with proton decoupling. TPPM 15 proton decoupling sequence was used with the field strength of approximately 100 kHz for both $^{13}C$ and $^{19}F$ acquisitions.

Figure 2-25 shows the $^{13}C$ CPMAS NMR spectrum of Compound 2 Acetone Solvate Form A. Some peaks of this spectrum are summarized in Table 2-7.

**Table 2-7**

| Compound 2 Acetone Solvate Form A $^{13}C$ Chem. Shifts | | |
|---|---|---|
| Peak # | [ppm] | Intensity |
| 1 | 202.8 | 6.05 |
| 2 | 173.3 | 62.66 |
| 3 | 171.9 | 20.53 |
| 4 | 153.5 | 28.41 |
| 5 | 150.9 | 21.68 |
| 6 | 150.1 | 19.49 |
| 7 | 143.2 | 45.74 |
| 8 | 142.3 | 42.68 |
| 9 | 140.1 | 37.16 |
| 10 | 136.6 | 26.82 |
| 11 | 135.9 | 30.1 |
| 12 | 134.6 | 39.39 |
| 13 | 133.2 | 23.18 |
| 14 | 131.0 | 60.92 |
| 15 | 128.5 | 84.58 |
| 16 | 116.0 | 34.64 |
| 17 | 114.2 | 23.85 |
| 18 | 112.4 | 25.3 |
| 19 | 110.9 | 24.12 |
| 20 | 107.8 | 18.21 |
| 21 | 32.0 | 54.41 |
| 22 | 22.2 | 20.78 |
| 23 | 18.8 | 100 |

Figure 2-26 shows the $^{19}F$ MAS NMR spectrum of Compound 2 Acetone Solvate Form A. The peaks marked with an asterisk (*) are spinning side bands (15.0 kHz spinning speed). Some peaks of this spectrum are summarized in Table 2-8.

**Table 2-8**

| Compound 2 Acetone Solvate Form A $^{19}F$ Chem. Shifts* | | |
|---|---|---|
| Peak # | [ppm] | Intensity |
| 1 | -41.6 | 12.5 |
| 2 | -46.4 | 6.77 |
| 3 | -51.4 | 9.05 |

### III.B.3. Compound 2 HCl Salt Form A

### III.B.3.a. Embodiments of Compound 2 HCl Salt Form A

In one aspect of the composition, Compound 2 is characterized as Compound 2 HCl Salt Form A.
In one embodiment, Compound 2 HCl Salt Form A is characterized by one or more peaks at 8.80 to 9.20 degrees, 17.30 to 17.70 degrees, and 18.20 to 18.60 degrees in an X-ray powder diffraction obtained using Cu K alpha radiation.
In another embodiment, Compound 2 HCl Salt Form A is characterized by one or more peaks at 8.80 to 9.20 degrees, 17.30 to 17.70 degrees, 18.20 to 18.60 degrees, 10.10 to 10.50, and 15.80 to 16.20 degrees in an X-ray powder diffraction obtained using Cu K alpha radiation.
In another embodiment, Compound 2 HCl Salt Form A is characterized by one or more peaks at 8.96, 17.51, and 18.45 degrees.
In another embodiment, Compound 2 HCl Salt Form A is characterized by one or more peaks at 8.96, 17.51, 18.45. 10.33, and 16.01 degrees.
In another embodiment, Compound 2 HCl Salt Form A is characterized by a peak at 8.80 to 9.20 degrees.
In another embodiment, Compound 2 HCl Salt Form A is characterized by a peak at 8.96 degrees.
In another embodiment, Compound 2 HCl Salt Form A is further characterized by a peak at 17.30 to 17.70 degrees.
In another embodiment, Compound 2 HCl Salt Form A is characterized by a peak at 17.51 degrees.
In another embodiment, Compound 2 HCl Salt Form A is further characterized by a peak at 18.20 to 18.60 degrees.
In another embodiment, Compound 2 HCl Salt Form A is further characterized by a peak at 18.45degrees.
In another embodiment, Compound 2 HCl Salt Form A is further characterized by a peak at 10.10 to 10.50 degrees.
In another embodiment, Compound 2 HCl Salt Form A is further characterized by a peak at 10.33 degrees.
In another embodiment, Compound 2 HCl Salt Form A is further characterized by a peak at 15.80 to 16.20 degrees.
In another embodiment, Compound 2 HCl Salt Form A is further characterized by a peak at 16.01 degrees.
In another embodiment, Compound 2 HCl Salt Form A is further characterized by a peak at 11.70 to 12.10 degrees.
In another embodiment, Compound 2 HCl Salt Form A is further characterized by a peak at 11.94 degrees.
In another embodiment, Compound 2 HCl Salt Form A is further characterized by a peak at 7.90 to 8.30 degrees.
In another embodiment, Compound 2 HCl Salt Form A is further characterized by a peak at 8.14 degrees.
In another embodiment, Compound 2 HCl Salt Form A is further characterized by a peak at 9.90 to 10.30 degrees.
In another embodiment, Compound 2 HCl Salt Form A is further characterized by a peak at 10.10 degrees.
In another embodiment, Compound 2 HCl Salt Form A is further characterized by a peak at 16.40 to 16.80 degrees.
In another embodiment, Compound 2 HCl Salt Form A is further characterized by a peak at 16.55 degrees.
In another embodiment, Compound 2 HCl Salt Form A is further characterized by a peak at 9.30 to 9.70 degrees.
In another embodiment, Compound 2 HCl Salt Form A is further characterized by a peak at 9.54 degrees.
In another embodiment, Compound 2 HCl Salt Form A is further characterized by a peak at 16.40 to 16.80 degrees.
In another embodiment, Compound 2 HCl Salt Form A is further characterized by a peak at 16.55 degrees.
In some embodiments, Compound 2 HCl Salt Form A is characterized as a dimer as depicted in Figure 2-20.
In some embodiments, Compound 2 HCl Salt Form A is characterized by the packing diagram depicted in Figure 2-21.
In some embodiments, Compound 2 HCl Salt Form A is characterized by a diffraction pattern substantially

similar to that of Figure 2-22.

In another embodiment, the invention features crystalline Compound 2 HCl Salt Form A having a P-1 space group, and the following unit cell dimensions: a = 10.2702 (2) Å, b = 10.8782 (2) Å, c = 12.4821 (3) Å, $\alpha$ = 67.0270 (10)°, $\beta$ = 66.1810 (10)°, and $\gamma$ = 72.4760 (10)°.

In one embodiment, Compound 2 HCl Salt Form A was prepared from the HCl salt of Compound 2, by dissolving the HCl salt of Compound 2 in a minimum of solvent and removing the solvent by slow evaporation. In another embodiment, the solvent is an alcohol. In a further embodiment, the solvent is ethanol. In one embodiment, slow evaporation includes dissolving the HCl salt of Compound 2 in a partially covered container.

### III.B.3.b. Synthesis of Compound 2 HCl Salt Form A

#### Preparation of Compound 2 HCl Salt Form A

Colorless crystals of Compound 2 HCl Salt Form A was obtained by slow evaporation from a concentrated solution in ethanol. A crystal with dimensions of 0.30 *x* 1/5*x* 0.15 mm was selected, cleaned using mineral oil, mounted on a MicroMount and centered on a Bruker *APEX*II diffractometer. Three batches of 40 frames separated in reciprocal space were obtained to provide an orientation matrix and initial cell parameters. Final cell parameters were obtained and refined based on the full data set.

### III.B.3.c. Characterization of Compound 2 HCl Salt Form A

#### Methods & Materials

#### Differential Scanning Calorimetry (DSC)

The Differential scanning calorimetry (DSC) data for Compound 2 Solvate Form A were collected using a DSC Q100 V9.6 Build 290 (TA Instruments, New Castle, DE). Temperature was calibrated with indium and heat capacity was calibrated with sapphire. Samples of 3-6 mg were weighed into aluminum pans that were crimped using lids with 1 pin hole. The samples were scanned from 25°C to 350°C at a heating rate of 1.0°C/min and with a nitrogen gas purge of 50 ml/min. Data were collected by Thermal Advantage Q Series™ version 2.2.0.248 software and analyzed by Universal Analysis software version 4.1D (TA Instruments, New Castle, DE). The reported numbers represent single analyses.

#### XRPD (X-ray Powder Diffraction)

X-Ray diffraction (XRD) data were collected on either a Bruker D8 DISCOVER or Bruker APEX II powder diffractometer. The Bruker D8 DISCOVER Diffractomer with HI-STAR 2-dimensional detector and a flat graphite monochromator. Cu sealed tube with K$\alpha$ radiation was used at 40 kV, 35mA. The samples were placed on zero-background silicon wafers at 25°C. For each sample, two data frames were collected at 120 seconds each at 2 different $\theta_2$ angles: 8° and 26°. The data were integrated with GADDS software and merged with DIFFRACT$^{plus}$EVA software. Uncertainties for the reported peak positions are ± 0.2 degrees. equipped with sealed tube Cu K$\alpha$ source and an Apex II CCD detector.

The Bruker II powder diffractomer was equipped with a sealed tube CuK source and an APEX II CCD detector. Structures were solved and refined using the *SHELX* program. (Sheldrick, G.M., Acta Cryst. (2008) A64, 112-122).

Figure 2-20 provides a conformational image of Compound 2 HCl Salt Form A as a dimer, based on single crystal analysis. Figure 2-21 provides a packing diagram of Compound 2 HCl Salt Form A, based on single crystal analysis. An X-ray diffraction pattern of Compound 2 HCl Salt Form A calculated from the crystal structure is shown in Figure 2-22. Table 2-9 contains the calculated peaks for Figure 2-22 in descending order of relative intensity.

**Table 2-9**

| 2$\theta$ [degrees] | Relative Intensity [%] |
|---|---|
| 8.96 | 100.00 |
| 17.51 | 48.20 |
| 18.45 | 34.60 |
| 10.33 | 32.10 |
| 16.01 | 18.90 |
| 11.94 | 18.40 |
| 8.14 | 16.20 |
| 10.10 | 13.90 |

(continued)

| 2θ [degrees] | Relative Intensity [%] |
|---|---|
| 16.55 | 13.30 |
| 9.54 | 10.10 |
| 16.55 | 13.30 |

### III.C. Solid Forms of Compound 3

### III.C.1. Compound 3 Form A

### III.C.1.a. Embodiments of Compound 3 Form A

In one aspect, the invention features Compound 3 characterized as crystalline Form A.

In another embodiment, Compound 3 Form A is characterized by one or more peaks at 19.3 to 19.7 degrees, 21.5 to 21.9 degrees, and 16.9 to 17.3 degrees in an X-ray powder diffraction obtained using Cu K alpha radiation. In another embodiment, Compound 3 Form A is characterized by one or more peaks at about 19.5, 21.7, and 17.1 degrees. In another embodiment, Compound 3 Form A is further characterized by a peak at 20.2 to 20.6 degrees. In another embodiment, Compound 3 Form A is further characterized by a peak at about 20.4 degrees. In another embodiment, Compound 3 Form A is further characterized by a peak at 18.6 to 19.0 degrees. In another embodiment, Compound 3 Form A is further characterized by a peak at about 18.8 degrees. In another embodiment, Compound 3 Form A is further characterized by a peak at 24.5 to 24.9 degrees. In another embodiment, Compound 3 Form A is further characterized by a peak at about 24.7 degrees. In another embodiment, Compound 3 Form A is further characterized by a peak at 9.8 to 10.2 degrees. In another embodiment, Compound 3 Form A is further characterized by a peak at about 10.0 degrees. In another embodiment, Compound 3 Form A is further characterized by a peak at 4.8 to 5.2 degrees. In another embodiment, Compound 3 Form A is further characterized by a peak at about 5.0 degrees. In another embodiment, Compound 3 Form A is further characterized by a peak at 24.0 to 24.4 degrees. In another embodiment, Compound 3 Form A is further characterized by a peak at about 24.2 degrees. In another embodiment, Compound 3 Form A is further characterized by a peak at 18.3 to 18.7 degrees. In another embodiment, Compound 3 Form A is further characterized by a peak at about 18.5 degrees.

In another embodiment, Compound 3 Form A is characterized by a diffraction pattern substantially similar to that of Figure 3-1. In another embodiment, Compound 3 Form A is characterized by a diffraction pattern substantially similar to that of Figure 3-2.

In another aspect, the invention features a crystal form of Compound 3 Form A having a monoclinic crystal system, a C2 space group, and the following unit cell dimensions: a = 21.0952(16) Å, $\alpha$ = 90°, b = 6.6287(5) Å, $\beta$ = 95.867(6)°, c = 17.7917(15) Å, and $\gamma$ = 90°.

In another aspect, the invention features a process of preparing Compound 3 Form A comprising slurrying Compound 3 in a solvent for an effective amount of time. In another embodiment, the solvent is ethyl acetate, dichloromethane, MTBE, isopropyl acetate, water/ethanol, water/acetonitrile, water/methanol, or water/isopropyl alcohol. In another embodiment, the effective amount of time is 24 hours to 2 weeks. In another embodiment, the effective amount of time is 24 hours to 1 week. In another embodiment, the effective amount of time is 24 hours to 72 hours.

In another aspect, the invention features a process of preparing Compound 3 Form A comprising dissolving Compound 3 in a solvent and evaporating the solvent. In another embodiment, the solvent is acetone, acetonitrile, methanol, or isopropyl alcohol.

In another aspect, the invention features a process of preparing Compound 3 Form A comprising dissolving Compound 3 in a first solvent and adding a second solvent that Compound 3 is not soluble in. In another embodiment, the first solvent is ethyl acetate, ethanol, isopropyl alcohol, or acetone. In another embodiment, the second solvent is heptane or water. In another embodiment, the addition of the second solvent is done while stirring the solution of the first solvent and Compound 3.

In another aspect, the invention features a kit comprising Compound 3 Form A, and instructions for use thereof.

In one embodiment, Compound 3 Form A is prepared by slurrying Compound 3 in an appropriate solvent for an effective amount of time. In another embodiment, the appropriate solvent is ethyl acetate, dichloromethane, MTBE, isopropyl acetate, various ratios of water/ethanol solutions, various ratios of water/acetonitrile solutions, various ratios of water/methanol solutions, or various ratios of water/isopropyl alcohol solutions. For example, various ratios of water/ethanol solutions include water/ethanol 1:9 (vol/vol), water/ethanol 1:1 (vol/vol), and water/ethanol 9:1 (vol/vol). Various ratios of water/acetonitrile solutions include water/acetonitrile 1:9 (vol/vol), water/acetonitrile 1:1 (vol/vol), and water/acetonitrile 9:1 (vol/vol). Various ratios of water/methanol solutions include water/methanol 1:9 (vol/vol), water/methanol 1:1 (vol/vol), and water/methanol 9:1 (vol/vol). Various ratios of water/isopropyl alcohol solutions include water/isopropyl alcohol 1:9 (vol/vol), water/isopropyl alcohol 1:1 (vol/vol), and water/isopropyl alcohol 9:1 (vol/vol).

Generally, about 40 mg of Compound 3 is slurred in about 1.5 mL of an appropriate solvent (target concentration at 26.7 mg/mL) at room temperature for an effective amount of time. In some embodiments, the effective amount of time is about 24 hours to about 2 weeks. In some embodiments, the effective amount of time is about 24 hours to about 1 week. In some embodiments, the effective amount of time is about 24 hours to about 72 hours. The solids are then collected.

In another embodiment, Compound 3 Form A is prepared by dissolving Compound 3 in an appropriate solvent and then evaporating the solvent. In one embodiment, the appropriate solvent is one in which Compound 3 has a solubility of greater than 20 mg/mL. For example, these solvents include acetonitrile, methanol, ethanol, isopropyl alcohol, acetone, and the like.

Generally, Compound 3 is dissolved in an appropriate solvent, filtered, and then left for either slow evaporation or fast evaporation. An example of slow evaporation is covering a container, such as a vial, comprising the Compound 3 solution with parafilm having one hole poked in it. An example of fast evaporation is leaving a container, such as a vial, comprising the Compound 3 solution uncovered. The solids are then collected.

In another aspect, the invention features a process of preparing Compound 3 Form A comprising dissolving Compound 3 in a first solvent and adding a second solvent that Compound 3 has poor solubility in (solubility < 1 mg/mL). For example, the first solvent may be a solvent that Compound 3 has greater than 20 mg/mL solubility in, e.g. ethyl acetate, ethanol, isopropyl alcohol, or acetone. The second solvent may be, for example, heptane or water.

Generally, Compound 3 is dissolved in the first solvent and filtered to remove any seed crystals. The second solvent is added slowly while stirring. The solids are precipitated and collected by filtering.

**III.C.1.b. Synthesis of Compound 3 Form A**

**Preparation of Compound 3 Form A**

**Slurry Method**

For EtOAc, MTBE, Isopropyl acetate, or DCM, approximately 40 mg of Compound 3 was added to a vial along with 1-2 mL of any one of the above solvents. The slurry was stirred at room temperature for 24 h to 2 weeks and Compound 3 Form A was collected by centrifuging the suspension (with filter). Figure 3-2 discloses an XRPD pattern of Compound 3 Form A obtained by this method with DCM as the solvent.

For EtOH/water solutions, approximately 40 mg of Compound 3 was added to three separate vials. In the first vial, 1.35 mL of EtOH and 0.15 mL of water were added. In the second vial, 0.75 mL of EtOH and 0.75 mL of water were added. In the third vial, 0.15 mL of EtOH and 1.35 mL of water were added. All three vials were stirred at room temperature for 24 h. Each suspension was then centrifuged separately (with filter) to collect Compound 3 Form A.

For isopropyl alcohol/water solutions, approximately 40 mg of Compound 3 was added to three separate vials. In the first vial, 1.35 mL of isopropyl alcohol and 0.15 mL of water were added. In the second vial, 0.75 mL of isopropyl alcohol and 0.75 mL of water were added. In the third vial, 0.15 mL of isopropyl alcohol and 1.35 mL of water were added. All three vials were stirred at room temperature for 24 h. Each suspension was then centrifuged separately (with filter) to collect Compound 3 Form A.

For methanol/water solutions, approximately 40 mg of Compound 3 was added to a vial. 0.5 mL of methanol and 1 mL of water were added and the suspension was stirred at room temperature for 24 h. The suspension was centrifuged (with filter) to collect Compound 3 Form A.

For acetonitrile, approximately 50 mg of Compound 3 was added to a vial along with 2.0 mL of acetonitrile. The suspension was stirred at room temperature for 24 h and Compound 3 Form A was collected by centrifuge (with filter).

For acetonitrile/water solutions, approximately 50 mg of Compound 3 was dissolved in 2.5 mL of acetonitrile to give a clear solution after sonication. The solution was filtered and 1 mL withdrawn to a vial. 2.25 mL of water was added to give a cloudy suspension. The suspension was stirred at room temperature for 24 h and Compound 3 Form A was collected by centrifuge (with filter).

**Slow Evaporation Method**

Approximately 55 mg of Compound 3 was dissolved in 0.5 mL of acetone to give a clear solution after sonication. The solution was filtered and 0.2 mL was withdrawn to a vial. The vial was covered with parrafilm with one hole poked in it and allowed to stand. Recrystallized Compound 3 Form A was collected by filtering.

**Fast Evaporation Method**

For isopropyl alcohol, approximately 43 mg of Compound 3 was dissolved in 2.1 mL of isopropyl alcohol to give a clear solution after sonication. The solution was filtered into a vial and allowed to stand uncovered. Recrystallized Compound 3 Form A was collected by filtering.

For methanol, approximately 58 mg of Compound 3 was dissolved in 0.5 mL of methanol to give a clear solution after sonication. The solution was filtered and 0.2 mL was withdrawn to an uncovered vial and allowed to stand. Recrystallized Compound 3 Form A was collected by filtering.

For acetonitrile, approximately 51 mg of Compound 3 was dissolved in 2.5 mL of acetonitrile to give a clear solution after sonication. The solution was filtered and half the solution was withdrawn to an uncovered vial and allowed to stand. Recrystallized Compound 3 Form A was collected by filtering. Figure 3-3 discloses an XRPD pattern of Compound 3 Form A prepared by this method.

**Anti-solvent Method**

For EtOAc/heptane, approximately 30 mg of Compound 3 was dissolved in 1.5 mL of EtOAc to give a clear solution after sonicating. The solution was filtered and 2.0 mL of heptane was added to the filtered solution while slowly stirring. The solution was stirred for an additional 10 minutes and allowed to stand. Recrystallized Compound 3 Form A was collected by filtering. Figure 3-4 discloses an XRPD pattern of Compound 3 Form A prepared by this method.

For isopropyl alcohol/water, approximately 21 mg of Compound 3 was dissolved in 1.0 mL of isopropyl alcohol to give a clear solution after sonicating. The solution was filtered to give 0.8 mL of solution. 1.8 mL of water was added while slowly stirring. An additional 0.2 mL of water was added to give a cloudy suspension. Stirring was stopped for 5 minutes to give a clear solution. The solution was stirred for an additional 2 minutes and allowed to stand. Recrystallized Compound 3 Form A was collected by filtering.

For ethanol/water, approximately 40 mg of Compound 3 was dissolved in 1.0 mL of ethanol to give a clear solution after sonicating. The solution was filtered and 1.0 mL of water was added. The solution was stirred for 1 day at room temperature. Recrystallized Compound 3 Form A was collected by filtering.

For acetone/water, approximately 55 mg of Compound 3 was dissolved in 0.5 mL of acetone to give a clear solution after sonicating. The solution was filtered and 0.2 mL was withdrawn to a vial. 1.5 mL of water was added, and then an additional 0.5 mL of water to give a cloudy suspension. The suspension was stirred for 1 day at room temperature. Compound 3 Form A was collected by filtering.

Table 3-2 summarizes the various techniques to form Compound 3 Form A.

**Table 3-2**

| Vehicle | Re-crystallization method | Results of residue solid |
|---|---|---|
| ACN | Fast Evaporation | Form A |
| Methanol | Fast Evaporation | Form A |
| Ethanol | N/A | N/A |
| IPA | Fast Evaporation | Form A |
| Acetone | Slow Evaporation | Form A |
| EtOAc | Slurry | Form A |
| DCM | Slurry | Form A |
| MTBE | Slurry | Form A |
| Isopropyl acetate | Slurry | Form A |
| Water / Ethanol 1:9 | N/A | N/A |
| Water / Ethanol 1:1 | Slurry | Form A |
| Water / Ethanol 9:1 | Slurry | Form A |
| Water/ ACN 9:4 | Slurry | Form A |
| Water / Methanol 2:1 | Slurry | Form A |
| Water/IPA 1:9 | N/A | N/A |

(continued)

| Vehicle | Re-crystallization method | Results of residue solid |
|---|---|---|
| Water / IPA 9:1 | Slurry | Form A |
| Water / IPA 7:3 | Slurry | Form A |
| Methanol/Water 4:3 | Slurry | Form A |
| EtOAc/ Heptane 3:4 | Anti-solvent | Form A |
| IPA/Water 2:5 | Anti-solvent | Form A |
| Ethanol /Water 1:1 | Anti-solvent | Form A |
| Acetone/water 1:10 | Anti-solvent | Form A |
| Ethanol /Water 5:6 | Anti-solvent | N/A |
| Toluene | N/A | N/A |
| MEK | N/A | N/A |
| Water | N/A | N/A |

**III.C.1.c. Characterization of Compound 3 Form A**

*Methods & Materials*

*XRPD (X-ray Powder Diffraction)*

X-ray Powder Diffraction was used to characterize the physical form of the lots produced to date and to characterize different polymorphs identified. The XRPD data of a compound were collected on a PANalytical X'pert Pro Powder X-ray Diffractometer (Almelo, the Netherlands). The XRPD pattern was recorded at room temperature with copper radiation (1.54060 A). The X-ray was generated using Cu sealed tube at 45 kV, 40 mA with a Nickel Kβ suppression filter. The incident beam optic was comprised of a variable divergence slit to ensure a constant illuminated length on the sample and on the diffracted beam side; a fast linear solid state detector was used with an active length of 2.12 degrees 2 theta measured in a scanning mode. The powder sample was packed on the indented area of a zero background silicon holder and spinning was performed to achieve better statistics. A symmetrical scan was measured from 4 - 40 degrees 2 theta with a step size of 0.017 degrees and a scan step time of 15.5 seconds. The data collection software is X'pert Data Collector (version 2.2e). The data analysis software is either X'pert Data Viewer (version 1.2d) or X'pert Highscore (version: 2.2c).

*Compound 3 Form A Single Crystal Structure Determination*

Diffraction data were acquired on Bruker Apex II diffractometer equipped with sealed tube Cu Kα source and an Apex II CCD detector. The structure was solved and refined using *SHELX* program (Sheldrick,G.M.,Acta Cryst., (2008) A64, 112-122). Based on intensities statistics and systematic absences the structure was solved and refined in *C2* space group. The absolute configuration was determined using anomalous diffraction. Flack parameter refined to 0.00 (18) indicating that the model represent the correct enantiomer [(R)].

*Solid State NMR*

Solid state NMR was conducted on a Bruker-Biospin 400 MHz wide-bore spectrometer equipped with a Bruker-Biospin 4mm HFX probe. Samples were packed into 4mm $ZrO_2$ rotors and spun under Magic Angle Spinning (MAS) condition with spinning speed of 12.5 kHz. The proton relaxation time was first measured using $^1H$ MAS $T_1$ saturation recovery relaxation experiment in order to set up proper recycle delay of the $^{13}C$ cross-polarization (CP) MAS experiment. The CP contact time of carbon CPMAS experiment was set to 2 ms. A CP proton pulse with linear ramp (from 50% to 100%) was employed. The Hartmann-Hahn match was optimized on external reference sample (glycine). The fluorine MAS spectrum was recorded with proton decoupling. TPPM15 decoupling sequence was used with the field strength of approximately 100 kHz for both $^{13}C$ and $^{19}F$ acquisitions.

An X-ray diffraction pattern was calculated from a single crystal structure of Compound 3 Form A and single

crystal structure of Compound 3 Form A is depicted in Figure 3-5. Table 3-3 lists the calculated peaks for Figure 3-5.

**Table 3-3**

| Peak Rank | 2θ Angle [degrees] | Relative Intensity [%] |
|---|---|---|
| 1 | 19.4 | 100.0 |
| 2 | 21.6 | 81.9 |
| 3 | 17.1 | 71.4 |
| 4 | 5.0 | 56.1 |
| 5 | 20.3 | 49.6 |
| 6 | 18.8 | 43.4 |
| 7 | 24.7 | 36.6 |
| 8 | 18.4 | 33.9 |
| 9 | 10.0 | 31.2 |
| 10 | 24.2 | 24.0 |
| 11 | 14.0 | 20.7 |
| 12 | 20.9 | 19.9 |
| 13 | 8.4 | 18.4 |
| 14 | 14.7 | 18.2 |
| 15 | 18.0 | 16.0 |
| 16 | 12.4 | 14.9 |

An actual X-ray powder diffraction pattern of Compound 3 Form A is shown in Figure 3-2. Table 3-4 lists the actual peaks for Figure 3-2.

**Table 3-4**

| Peak Rank | 2θ Angle [degrees] | Relative Intensity [%] |
|---|---|---|
| 1 | 19.5 | 100.0 |
| 2 | 21.7 | 88.2 |
| 3 | 17.1 | 85.1 |
| 4 | 20.4 | 80.9 |
| 5 | 18.8 | 51.0 |
| 6 | 24.7 | 40.8 |
| 7 | 10.0 | 40.7 |
| 8 | 5.0 | 39.0 |
| 9 | 24.2 | 35.4 |
| 10 | 18.5 | 35.0 |
| 11 | 18.0 | 29.0 |
| 12 | 20.9 | 27.0 |
| 13 | 14.8 | 19.9 |
| 14 | 14.1 | 19.2 |
| 15 | 12.4 | 18.2 |
| 16 | 8.4 | 14.1 |

Single crystal data were obtained for Compound 3 Form A, providing additional detail about the crystal structure, including lattice size and packing.

<u>Crystal Preparation</u>

Crystals of Compound 3 Form A were obtained by slow evaporation from a concentrated solution of methanol (10 mg/mL). A colorless crystal of Compound 3 Form A with dimensions of 0.20 × 0.05 × 0.05 mm was selected, cleaned using mineral oil, mounted on a MicroMount and centered on a Bruker *APEX*II diffractometer. Three batches of 40 frames separated in reciprocal space were obtained to provide an orientation matrix and initial cell parameters. Final cell parameters were obtained and refined based on the full data set.

Experimental

A diffraction data set of reciprocal space was obtained to a resolution of 0.83 Å using 0.5° steps with 30 s exposure for each frame. Data were collected at room temperature [295 (2) K]. Integration of intensities and refinement of cell parameters were accomplished using APEXII software. Observation of the crystal after data collection showed no signs of decomposition.

Geometry: All esds (except the esd in the dihedral angle between two l.s. planes) are estimated using the full covariance matrix. The cell esds are taken into account individually in the estimation of esds in distances, angles and torsion angles; correlations between esds in cell parameters are only used when they are defined by crystal symmetry. An approximate (isotropic) treatment of cell esds is used for estimating esds involving l.s. planes.

Data collection: Apex II; cell refinement: Apex II; data reduction: Apex II; program(s) used to solve structure: SHELXS97 (Sheldrick, 1990); program(s) used to refine structure: *SHELXL97* (Sheldrick, 1997); molecular graphics: Mercury; software used to prepare material for publication: publCIF.

Refinement: Refinement of $F^2$ against ALL reflections. The weighted R-factor wR and goodness of fit S are based on $F^2$, conventional R-factors R are based on F, with F set to zero for negative $F^2$. The threshold expression of $F^2 > 2sigma(F^2)$ is used only for calculating R-factors(gt) etc. and is not relevant to the choice of reflections for refinement. R-factors based on $F^2$ are statistically about twice as large as those based on F, and R-factors based on ALL data will be even larger.

Conformational pictures of Compound 3 Form A based on single crystal X-ray analysis are shown in Figures 3-5 and 3-6. The terminal -OH groups are connected via hydrogen bond networks to form a tetrameric cluster with four adjacent molecules (Figure 3-6). The other hydroxyl group acts as a hydrogen bond donor to form a hydrogen bond with a carbonyl group from an adjacent molecule. The crystal structure reveals a dense packing of the molecules. Compound 3 Form A is monoclinic, C2 space group, with the following unit cell dimensions: a = 21.0952(16) Å, b = 6.6287(5) Å, c = 17.7917(15) Å, $\beta$ = 95.867(6)°, $\gamma$ = 90°.

A solid state $^{13}C$ NMR spectrum of Compound 3 Form A is shown in Figure 3-7. Table 3-5 provides chemical shifts of the relevant peaks.

**Table 3-5**

| | Compound 3 Form A $^{13}C$ Chem. Shifts | |
|---|---|---|
| Peak # | F1 [ppm] | Intensity |
| 1 | 175.3 | 2.9 |
| 2 | 155.4 | 0.54 |
| 3 | 153.3 | 0.81 |
| 4 | 144.3 | 3.35 |
| 5 | 143.7 | 4.16 |
| 6 | 143.0 | 4.24 |
| 7 | 139.0 | 2.86 |
| 8 | 135.8 | 5.19 |
| 9 | 128.2 | 5.39 |
| 10 | 123.3 | 5.68 |
| 11 | 120.0 | 4.55 |
| 12 | 115.8 | 2.66 |
| 13 | 114.9 | 4.2 |
| 14 | 111.3 | 5.17 |
| 15 | 102.8 | 5.93 |
| 16 | 73.8 | 10 |
| 17 | 69.8 | 7.06 |
| 18 | 64.5 | 8.29 |

(continued)

| | Compound 3 Form A $^{13}C$ Chem. Shifts | |
|---|---|---|
| Peak # | F1 [ppm] | Intensity |
| 19 | 51.6 | 4.96 |
| 20 | 39.1 | 9.83 |
| 21 | 30.5 | 7.97 |
| 22 | 26.8 | 6.94 |
| 23 | 24.4 | 9.19 |
| 24 | 16.3 | 5.58 |
| 25 | 15.8 | 6.33 |

A solid state $^{19}F$ NMR spectrum of Compound 3 Form A is shown in Figure 3-8. Peaks with an asterisk denote spinning side bands. Table 3-6 provides chemical shifts of the relevant peaks.

**Table 3-6**

| | Compound 3 Form A $^{19}F$ Chem. Shifts | |
|---|---|---|
| Peak # | F1 [ppm] | Intensity |
| 1 | -45.9 | 9.48 |
| 2 | -51.4 | 7.48 |
| 3 | -53.3 | 4.92 |
| 4 | -126.5 | 11.44 |
| 5 | -128.4 | 12.5 |

**III.C.2. Compound 3 Amorphous Form**

**III.C.2.a. Embodiments of Compound 3 Amorphous Form**

In another aspect, the invention features a solid substantially amorphous Compound 3. In another embodiment, the amorphous Compound 3 comprises less than about 5% crystalline Compound 3.

In another aspect, the invention features a pharmaceutical composition comprising the amorphous Compound 3 and a pharmaceutically acceptable carrier. In another embodiment, the pharmaceutical composition further comprises an additional therapeutic agent. In another embodiment, the additional therapeutic agent is selected from a mucolytic agent, bronchodialator, an anti-biotic, an anti-infective agent, an anti-inflammatory agent, a CFTR potentiator, or a nutritional agent.

In another aspect, the invention features a process of preparing the amorphous Compound 3 comprising dissolving Compound 3 in a suitable solvent and removing the solvent by rotary evaporation. In another embodiment, the solvent is methanol.

In another aspect, the invention features a solid dispersion comprising the amorphous Compound 3 and a polymer. In another embodiment, the polymer is hydroxypropylmethylcellulose (HPMC). In another embodiment, the polymer is hydroxypropylmethylcellulose acetate succinate (HPMCAS).

In another embodiment, the polymer is present in an amount from 10% by weight to 80% by weight. In another embodiment, the polymer is present in an amount from 30% by weight to 60% by weight. In another embodiment, the polymer is present in an amount of about 49.5% by weight.

In another embodiment, Compound 3 is present in an amount from 10% by weight to 80% by weight. In another embodiment, Compound 3 is present in an amount from 30% by weight to 60% by weight. In another embodiment, Compound 3 is present in an amount of about 50% by weight.

In another embodiment, the solid dispersion further comprises a surfactant. In another embodiment, the surfactant is sodium lauryl sulfate. In another embodiment, the surfactant is present in an amount from 0.1% by weight to 5% by weight. In another embodiment, the surfactant is present in an amount of about 0.5% by weight.

In another embodiment, the polymer is hydroxypropylmethylcellulose acetate succinate (HPMCAS) in the amount of 49.5% by weight, the surfactant is sodium lauryl sulfate in the amount of 0.5% by weight, and Compound 3 is present in the amount of 50% by weight.

In another aspect, the invention features a pharmaceutical composition comprising the solid dispersion and a pharmaceutically acceptable carrier. In another embodiment, the pharmaceutical composition further comprises an additional therapeutic agent. In another embodiment, the additional therapeutic agent is selected from a mucolytic agent, bronchodialator, an anti-biotic, an anti-infective agent, an anti-inflammatory agent, a CFTR potentiator, or a nutritional agent.

In another aspect, the invention features a process of preparing amorphous Compound 3 comprising spray drying Compound 3.

In another embodiment, the process comprises combining Compound 3 and a suitable solvent and then spray drying the mixture to obtain amorphous Compound 3. In another embodiment, the solvent is an alcohol. In another embodiment, the solvent is methanol.

In another embodiment, the process comprises: a) forming a mixture comprising Compound 3, a polymer, and a solvent; and b) spray drying the mixture to form a solid dispersion.

In another embodiment, the polymer is hydroxypropylmethylcellulose acetate succinate (HPMCAS). In another embodiment, the polymer is in an amount of from 10% by weight to 80% by weight of the solid dispersion. In another embodiment, the polymer is in an amount of about 49.5% by weight of the solid dispersion. In another embodiment, the solvent is methanol. In another embodiment, the mixture further comprises a surfactant. In another embodiment, the surfactant is sodium lauryl sulfate (SLS). In another embodiment, the surfactant is in an amount of from 0.1% by weight to 5% by weight of the solid dispersion. In another embodiment, the surfactant is in an amount of about 0.5% by weight of the solid dispersion.

In another embodiment, the polymer is hydroxypropylmethylcellulose acetate succinate (HPMCAS) in the amount of about 49.5% by weight of the solid dispersion, the solvent is methanol, and the mixture further comprises sodium lauryl sulfate in an amount of about 0.5% by weight of the solid dispersion.

Starting from Compound 3 or Compound 3 Form A, the amorphous form of Compound 3 may be prepared by rotary evaporation or by spray dry methods.

Dissolving Compound 3 in an appropriate solvent like methanol and rotary evaporating the methanol to leave a foam produces Compound 3 amorphous form. In some embodiments, a warm water bath is used to expedite the evaporation.

Compound 3 amorphous form may also be prepared from Compound 3 Form A using spray dry methods. Spray drying is a process that converts a liquid feed to a dried particulate form. Optionally, a secondary drying process such as fluidized bed drying or vacuum drying, may be used to reduce residual solvents to pharmaceutically acceptable levels. Typically, spray drying involves contacting a highly dispersed liquid suspension or solution, and a sufficient volume of hot air to produce evaporation and drying of the liquid droplets. The preparation to be spray dried can be any solution, coarse suspension, slurry, colloidal dispersion, or paste that may be atomized using the selected spray drying apparatus. In a standard procedure, the preparation is sprayed into a current of warm filtered air that evaporates the solvent and conveys the dried product to a collector (e.g. a cyclone). The spent air is then exhausted with the solvent, or alternatively the spent air is sent to a condenser to capture and potentially recycle the solvent. Commercially available types of apparatus may be used to conduct the spray drying. For example, commercial spray dryers are manufactured by Buchi Ltd. And Niro (e.g., the PSD line of spray driers manufactured by Niro) (see, US 2004/0105820; US 2003/0144257).

Spray drying typically employs solid loads of material from about 3% to about 30% by weight, (i.e., drug and excipients), for example about 4% to about 20% by weight, preferably at least about 10%. In general, the upper limit of solid loads is governed by the viscosity of (e.g., the ability to pump) the resulting solution and the solubility of the components in the solution. Generally, the viscosity of the solution can determine the size of the particle in the resulting powder product.

Techniques and methods for spray drying may be found in Perry's Chemical Engineering Handbook, 6th Ed., R. H. Perry, D. W. Green & J. O. Maloney, eds.), McGraw-Hill book co. (1984); and Marshall "Atomization and Spray-Drying" 50, Chem. Eng. Prog. Monogr. Series 2 (1954). In general, the spray drying is conducted with an inlet temperature of from about 60 °C to about 200 °C, for example, from about 95 °C to about 185 °C, from about 110 °C to about 182 °C, from about 96 °C to about 180 °C, e.g., about 145 °C. The spray drying is generally conducted with an outlet temperature of from about 30 °C to about 90 °C, for example from about 40 °C to about 80 °C, about 45 °C to about 80 °C e.g., about 75 °C. The atomization flow rate is generally from about 4 kg/h to about 12 kg/h, for example, from about 4.3 kg/h to about 10.5 kg/h, e.g., about 6 kg/h or about 10.5 kg/h. The feed flow rate is generally from about 3 kg/h to about 10 kg/h, for example, from about 3.5 kg/h to about 9.0 kg/h, e.g., about 8 kg/h or about 7.1 kg/h. The atomization ratio is generally from about 0.3 to 1.7, e.g., from about 0.5 to 1.5, e.g., about 0.8 or about 1.5.

Removal of the solvent may require a subsequent drying step, such as tray drying, fluid bed drying (e.g., from about room temperature to about 100 °C), vacuum drying, microwave drying, rotary drum drying or biconical vacuum drying (e.g., from about room temperature to about 200 °C).

In one embodiment, the solid dispersion is fluid bed dried.

In one process, the solvent includes a volatile solvent, for example a solvent having a boiling point of less than about 100 °C. In some embodiments, the solvent includes a mixture of solvents, for example a mixture of volatile solvents or a mixture of volatile and non-volatile solvents. Where mixtures of solvents are used, the mixture can include one or more non-volatile solvents, for example, where the non-volatile solvent is present in the mixture at less than about 15%, e.g., less than about 12%, less than about 10%, less than about 8%, less than about 5%, less than about 3%, or less than about 2%.

Preferred solvents are those solvents where Compound 3 has a solubility of at least about 10 mg/mL, (e.g., at least about 15 mg/mL, 20 mg/mL, 25 mg/mL, 30 mg/mL, 35 mg/mL, 40 mg/mL, 45 mg/mL, 50 mg/mL, or greater). More preferred solvents include those where Compound 3 has a solubility of at least about 20 mg/mL.

Exemplary solvents that could be tested include acetone, cyclohexane, dichloromethane, N,N-dimethylacetamide (DMA), N,N-dimethylformamide (DMF), 1,3-dimethyl-2-imidazolidinone (DMI), dimethyl sulfoxide (DMSO), dioxane, ethyl acetate, ethyl ether, glacial acetic acid (HAc), methyl ethyl ketone (MEK), N-methyl-2-pyrrolidinone (NMP), methyl tert-butyl ether (MTBE), tetrahydrofuran (THF), pentane, acetonitrile, methanol, ethanol, isopropyl alcohol, isopropyl acetate, and toluene. Exemplary co-solvents include acetone/DMSO, acetone/DMF, acetone/water, MEK/water, THF/water, dioxane/water. In a two solvent system, the solvents can be present in of from about 0.1 % to about 99.9%. In some preferred embodiments, water is a co-solvent with acetone where water is present from about 0.1% to about 15%, for example about 9% to about 11%, e.g., about 10%. In some preferred embodiments, water is a co-solvent with MEK where water is present from about 0.1% to about 15%, for example about 9% to about 11%, e.g., about 10%. In some embodiments the solvent solution include three solvents. For example, acetone and water can be mixed with a third solvent such as DMA, DMF, DMI, DMSO, or HAc. In instances where amorphous Compound 3 is a component of a solid amorphous dispersion, preferred solvents dissolve both Compound 3 and the polymer. Suitable solvents include those described above, for example, MEK, acetone, water, methanol, and mixtures thereof.

The particle size and the temperature drying range may be modified to prepare an optimal solid dispersion. As would be appreciated by skilled practitioners, a small particle size would lead to improved solvent removal. Applicants have found however, that smaller particles can lead to fluffy particles that, under some circumstances do not provide optimal solid dispersions for downstream processing such as tabletting. At higher temperatures, crystallization or chemical degradation of Compound 3 may occur. At lower temperatures, a sufficient amount of the solvent may not be removed. The methods herein provide an optimal particle size and an optimal drying temperature.

In general, particle size is such that D10 (μm) is less than about 5, e.g., less than about 4.5, less than about 4.0, or less than about 3.5, D50 (μm) is generally less than about 17, e.g., less than about 16, less than about 15, less than about 14, less than about 13, and D90 (μm) is generally less than about 175, e.g., less than about 170, less than about 170, less than about 150, less than about 125, less than about 100, less than about 90, less than about 80, less than about 70, less than about 60, or less than about less than about 50. In general bulk density of the spray dried particles is from about 0.08 g/cc to about 0.20 g/cc, e.g., from about 0.10 to about 0.15 g/cc, e.g., about 0.11 g/cc or about 0.14 g/cc. Tap density of the spray dried particles generally ranges from about 0.08 g/cc to about 0.20 g/cc, e.g., from about 0.10 to about 0.15 g/cc, e.g., about 0.11 g/cc or about 0.14 g/cc, for 10 taps; 0.10 g/cc to about 0.25 g/cc, e.g., from about 0.11 to about 0.21 g/cc, e.g., about 0.15 g/cc, about 0.19 g/cc, or about 0.21 g/cc for 500 taps; 0.15 g/cc to about 0.27 g/cc, e.g., from about 0.18 to about 0.24 g/cc, e.g., about 0.18 g/cc, about 0.19 g/cc, about 0.20 g/cc, or about 0.24 g/cc for 1250 taps; and 0.15 g/cc to about 0.27 g/cc, e.g., from about 0.18 to about 0.24 g/cc, e.g., about 0.18 g/cc, about 0.21 g/cc, about 0.23 g/cc, or about 0.24 g/cc for 2500 taps.

## Polymers

Solid dispersions including amorphous Compound 3 and a polymer (or solid state carrier) also are included herein. For example, Compound 3 is present as an amorphous compound as a component of a solid amorphous dispersion. The solid amorphous dispersion, generally includes Compound 3 and a polymer. Exemplary polymers include cellulosic polymers such as HPMC or HPMCAS and pyrrolidone containing polymers such as PVP/VA. In some embodiments, the solid amorphous dispersion includes one or more additional exipients, such as a surfactant.

In one embodiment, a polymer is able to dissolve in aqueous media. The solubility of the polymers may be pH-independent or pH-dependent. The latter include one or more enteric polymers. The term "enteric polymer" refers to a polymer that is preferentially soluble in the less acidic environment of the intestine relative to the more acid environment of the stomach, for example, a polymer that is insoluble in acidic aqueous media but soluble when the pH is above 5-6. An appropriate polymer should be chemically and biologically inert. In order to improve the physical stability of the solid dispersions, the glass transition temperature (Tg) of the polymer should be as high as possible. For example, preferred

polymers have a glass transition temperature at least equal to or greater than the glass transition temperature of the drug (i.e., Compound 3). Other preferred polymers have a glass transition temperature that is within about 10 to about 15 °C of the drug (i.e., Compound 3). Examples of suitable glass transition temperatures of the polymers include at least about 90 °C, at least about 95 °C, at least about 100 °C, at least about 105 °C, at least about 110 °C, at least about 115 °C, at least about 120 °C, at least about 125 °C, at least about 130 °C, at least about 135 °C, at least about 140 °C, at least about 145 °C, at least about 150 °C, at least about 155 °C, at least about 160 °C, at least about 165 °C, at least about 170 °C, or at least about 175 °C (as measured under dry conditions). Without wishing to be bound by theory, it is believed that the underlying mechanism is that a polymer with a higher $T_g$ generally has lower molecular mobility at room temperature, which can be a crucial factor in stabilizing the physical stability of the amorphous solid dispersion.

Additionally, the hygroscopicity of the polymers should be as low, e.g., less than about 10%. For the purpose of comparison in this application, the hygroscopicity of a polymer or composition is characterized at about 60% relative humidity. In some preferred embodiments, the polymer has less than about 10% water absorption, for example less than about 9%, less than about 8%, less than about 7%, less than about 6%, less than about 5%, less than about 4%, less than about 3%, or less than about 2% water absorption. The hygroscopicity can also affect the physical stability of the solid dispersions. Generally, moisture adsorbed in the polymers can greatly reduce the $T_g$ of the polymers as well as the resulting solid dispersions, which will further reduce the physical stability of the solid dispersions as described above.

In one embodiment, the polymer is one or more water-soluble polymer(s) or partially water-soluble polymer(s). Water-soluble or partially water-soluble polymers include but are not limited to, cellulose derivatives (e.g., hydroxypropylmethylcellulose (HPMC), hydroxypropylcellulose (HPC)) or ethylcellulose; polyvinylpyrrolidones (PVP); polyethylene glycols (PEG); polyvinyl alcohols (PVA); acrylates, such as polymethacrylate (e.g., Eudragit® E); cyclodextrins (e.g., β-cyclodextin) and copolymers and derivatives thereof, including for example PVP-VA (polyvinylpyrollidone-vinyl acetate).

In some embodiments, the polymer is hydroxypropylmethylcellulose (HPMC), such as HPMC E50, HPMCE15, or HPMC60SH50).

As discussed herein, the polymer can be a pH-dependent enteric polymer. Such pH-dependent enteric polymers include, but are not limited to, cellulose derivatives (e.g., cellulose acetate phthalate (CAP)), hydroxypropyl methyl cellulose phthalates (HPMCP), hydroxypropyl methyl cellulose acetate succinate (HPMCAS), carboxymethylcellulose (CMC) or a salt thereof (e.g., a sodium salt such as (CMC-Na)); cellulose acetate trimellitate (CAT), hydroxypropylcellulose acetate phthalate (HPCAP), hydroxypropylmethyl-cellulose acetate phthalate (HPMCAP), and methylcellulose acetate phthalate (MCAP), or polymethacrylates (e.g., Eudragit® S). In some embodiments, the polymer is hydroxypropyl methyl cellulose acetate succinate (HPMCAS). In some embodiments, the polymer is hydroxypropyl methyl cellulose acetate succinate HG grade (HPMCAS-HG).

In yet another embodiment, the polymer is a polyvinylpyrrolidone co-polymer, for example, avinylpyrrolidone/vinyl acetate co-polymer (PVP/VA).

In embodiments where Compound 3 forms a solid dispersion with a polymer, for example with an HPMC, HPMCAS, or PVP/VA polymer, the amount of polymer relative to the total weight of the solid dispersion ranges from about 0.1% to 99% by weight. Unless otherwise specified, percentages of drug, polymer and other excipients as described within a dispersion are given in weight percentages. The amount of polymer is typically at least about 20%, and preferably at least about 30%, for example, at least about 35%, at least about 40%, at least about 45%, or about 50% (e.g., 49.5%). The amount is typically about 99% or less, and preferably about 80% or less, for example about 75% or less, about 70% or less, about 65% or less, about 60% or less, or about 55% or less. In one embodiment, the polymer is in an amount of up to about 50% of the total weight of the dispersion (and even more specifically, between about 40% and 50%, such as about 49%, about 49.5%, or about 50%). HPMC and HPMCAS are available in a variety of grades from ShinEtsu, for example, HPMCAS is available in a number of varieties, including AS-LF, AS-MF, AS-HF, AS-LG, AS-MG, AS-HG. Each of these grades vary with the percent substitution of acetate and succinate.

In some embodiments, Compound 3 and polymer are present in roughly equal amounts, for example each of the polymer and the drug make up about half of the percentage weight of the dispersion. For example, the polymer is present in about 49.5% and the drug is present in about 50%.

In some embodiments, Compound 3 and the polymer combined represent 1% to 20% w/w total solid content of the non-solid dispersion prior to spray drying. In some embodiments, Compound 3 and the polymer combined represent 5% to 15% w/w total solid content of the non-solid dispersion prior to spray drying. In some embodiments, Compound 3 and the polymer combined represent about 11% w/w total solid content of the non-solid dispersion prior to spray drying.

In some embodiments, the dispersion further includes other minor ingredients, such as a surfactant (e.g., SLS). In some embodiments, the surfactant is present in less than about 10% of the dispersion, for example less than about 9%, less than about 8%, less than about 7%, less than about 6%, less than about 5%, less than about 4%, less than about 3%, less than about 2%, about 1%, or about 0.5%.

In embodiments including a polymer, the polymer should be present in an amount effective for stabilizing the solid dispersion. Stabilizing includes inhibiting or preventing, the crystallization of Compound 3. Such stabilizing would

inhibit the conversion Compound 3 from amorphous to crystalline form. For example, the polymer would prevent at least a portion (e.g., about 5%, about 10%, about 15%, about 20%, about 25%, about 30%, about 35%, about 40%, about 45%, about 50%, about 55%, about 60%, about 65%, about 70%, about 75%, or greater) of Compound 3 from converting from an amorphous to a crystalline form. Stabilization can be measured, for example, by measuring the glass transition temperature of the solid dispersion, measuring the rate of relaxation of the amorphous material, or by measuring the solubility or bioavailability of Compound 3.

Suitable polymers for use in combination with Compound 3, for example to form a solid dispersion such as an amorphous solid dispersion, should have one or more of the following properties:

The glass transition temperature of the polymer should have a temperature of no less than about 10-15 °C lower than the glass transition temperature of Compound 3. Preferably, the glass transition temperature of the polymer is greater than the glass transition temperature of Compound 3, and in general at least 50°C higher than the desired storage temperature of the drug product. For example, at least about 100 °C, at least about 105 °C, at least about 105 °C, at least about 110 °C, at least about 120 °C, at least about 130 °C, at least about 140 °C, at least about 150 °C, at least about 160 °C, at least about 160 °C, or greater.

The polymer should be relatively non-hygroscopic. For example, the polymer should, when stored under standard conditions, absorb less than about 10% water, for example, less than about 9%, less than about 8%, less than about 7%, less than about 6%, or less than about 5%, less than about 4%, or less than about 3% water. Preferably the polymer will, when stored under standard conditions, be substantially free of absorbed water.

The polymer should have similar or better solubility in solvents suitable for spray drying processes relative to that of Compound 3. In preferred embodiments, the polymer will dissolve in one or more of the same solvents or solvent systems as Compound 3. It is preferred that the polymer is soluble in at least one non-hydroxy containing solvent such as methylene chloride, acetone, or a combination thereof.

The polymer, when combined with Compound 3, for example in a solid dispersion or in a liquid suspension, should increase the solubility of Compound 3 in aqueous and physiologically relative media either relative to the solubility of Compound 3 in the absence of polymer or relative to the solubility of Compound 3 when combined with a reference polymer. For example, the polymer could increase the solubility of amorphous Compound 3 by reducing the amount of amorphous Compound 3 that converts to crystalline Compound 3, either from a solid amorphous dispersion or from a liquid suspension.

The polymer should decrease the relaxation rate of the amorphous substance.

The polymer should increase the physical and/or chemical stability of Compound 3.

The polymer should improve the manufacturability of Compound 3.

The polymer should improve one or more of the handling, administration or storage properties of Compound 3.

The polymer should not interact unfavorably with other pharmaceutical components, for example excipients.

The suitability of a candidate polymer (or other component) can be tested using the spray drying methods (or other methods) described herein to form an amorphous composition. The candidate composition can be compared in terms of stability, resistance to the formation of crystals, or other properties, and compared to a reference preparation, e.g., a preparation of neat amorphous Compound 3 or crystalline Compound 3. For example, a candidate composition could be tested to determine whether it inhibits the time to onset of solvent mediated crystallization, or the percent conversion at a given time under controlled conditions, by at least 50 %, 75 %, 100%, or 110% as well as the reference preparation, or a candidate composition could be tested to determine if it has improved bioavailability or solubility relative to crystalline Compound 3.

Surfactants

A solid dispersion or other composition may include a surfactant. A surfactant or surfactant mixture would generally decrease the interfacial tension between the solid dispersion and an aqueous medium. An appropriate surfactant or surfactant mixture may also enhance aqueous solubility and bioavailability of Compound 3 from a solid dispersion. The surfactants for use in connection with the present invention include, but are not limited to, sorbitan fatty acid esters (e.g., Spans®), polyoxyethylene sorbitan fatty acid esters (e.g., Tweens®), sodium lauryl sulfate (SLS), sodium dodecylbenzene sulfonate (SDBS) dioctyl sodium sulfosuccinate (Docusate), dioxycholic acid sodium salt (DOSS), Sorbitan Monostearate, Sorbitan Tristearate, hexadecyltrimethyl ammonium bromide (HTAB), Sodium N-lauroylsarcosine, Sodium Oleate, Sodium Myristate, Sodium Stearate, Sodium Palmitate, Gelucire 44/14, ethylenediamine tetraacetic acid (EDTA), Vitamin E d-alpha tocopheryl polyethylene glycol 1000 succinate (TPGS), Lecithin, MW 677-692, Glutanic acid monosodium monohydrate, Labrasol, PEG 8 caprylic/capric glycerides, Transcutol, diethylene glycol monoethyl ether, Solutol HS-15, polyethylene glycol/hydroxystearate, Taurocholic Acid, Pluronic F68, Pluronic F108, and Pluronic F127 (or any other polyoxyethylene-polyoxypropylene co-polymers (Pluronics®) or saturated polyglycolized glycerides (Gelucirs®)). Specific example of such surfactants that may be used in connection with this invention include, but are not limited to, Span 65, Span 25, Tween 20, Capryol 90, Pluronic F108, sodium lauryl sulfate (SLS), Vitamin E

TPGS, pluronics and copolymers. SLS is generally preferred.

The amount of the surfactant (e.g., SLS) relative to the total weight of the solid dispersion may be between 0.1-15%. Preferably, it is from about 0.5% to about 10%, more preferably from about 0.5 to about 5%, e.g., about 0.5 to 4%, about 0.5 to 3%, about 0.5 to 2%, about 0.5 to 1%, or about 0.5%.

In certain embodiments, the amount of the surfactant relative to the total weight of the solid dispersion is at least about 0.1%, preferably about 0.5%. In these embodiments, the surfactant would be present in an amount of no more than about 15%, and preferably no more than about 12%, about 11%, about 10%, about 9%, about 8%, about 7%, about 6%, about 5%, about 4%, about 3%, about 2% or about 1%. An embodiment wherein the surfactant is in an amount of about 0.5% by weight is preferred.

Candidate surfactants (or other components) can be tested for suitability for use in the invention in a manner similar to that described for testing polymers.

**III.C.2.b. Synthesis of Compound 3 Amorphous Form**

**Preparation of Compound 3 Amorphous Form**

**Rotary Evaporation Method**

Compound 3 Amorphous Form was achieved via rotary evaporation. Compound 3 (approximately 10 g) was dissolved in 180 mL of MeOH and rotary evaporated under reduced pressure in a 50 °C bath to a foam. XRPD (Figure 3-9) confirmed amorphous form of Compound 3.

**Spray-Dried Method**

9.95g of Hydroxypropylmethylcellulose acetate succinate HG grade (HPMCAS-HG) was weighed into a 500 mL beaker, along with 50 mg of sodium lauryl sulfate (SLS). MeOH (200 mL) was mixed with the solid. The material was allowed to stir for 4 h. To insure maximum dissolution, after 2 h of stirring the solution was sonicated for 5 mins, then allowed to continue stirring for the remaining 2 h. A very fin suspension of HPMCAS remained in solution. However, visual observation determined that no gummy portions remained on the walls of the vessel or stuck to the bottom after tilting the vessel.

Compound 3 Form A (10g) was poured into the 500 mL beaker, and the system was allowed to continue stirring. The solution was spray dried using the following parameters:

Formulation Description: Compound 3 Form A/HPMCAS/SLS (50/49.5/0.5)

| Buchi Mini Spray Dryer | |
|---|---|
| T inlet (setpoint) | 145 °C |
| T outlet (start) | 75 °C |
| T outlet (end) | 55 °C |
| Nitrogen Pressure | 75 psi |
| Aspirator | 100 % |
| Pump | 35 % |
| Rotometer | 40 mm |
| Filter Pressure | 65 mbar |
| Condenser Temp | -3 °C |
| Run Time | 1 h |

Approximately 16g of Compound 3 Amorphous Form (80% yield) was recovered. Compound 3 Amorphous Form was confirmed by XRPD (Figure 3-10).

**III.C.2.c. Characterization of Compound 3 Amorphous Form**

*Methods & Materials*

XRPD (X-ray Powder Diffraction)

X-ray Powder Diffraction was used to characterize the physical form of the lots produced to date and to characterize different polymorphs identified. The XRPD data of a compound were collected on a PANalytical X'pert Pro Powder X-ray Diffractometer (Almelo, the Netherlands). The XRPD pattern was recorded at room temperature with copper radiation (1.54060 A). The X-ray was generated using Cu sealed tube at 45 Kv, 40 Ma with a Nickel Kβ suppression filter. The incident beam optic was comprised of a variable divergence slit to ensure a constant illuminated length on the sample and on the diffracted beam side; a fast linear solid state detector was used with an active length of 2.12 degrees 2 theta measured in a scanning mode. The powder sample was packed on the indented area of a zero background silicon holder and spinning was performed to achieve better statistics. A symmetrical scan was measured from 4-40 degrees 2 theta with a step size of 0.017 degrees and a scan step time of 15.5 seconds. The data collection software is X'pert Data Collector (version 2.2e). The data analysis software is either X'pert Data Viewer (version 1.2d) or X'pert Highscore (version: 2.2c).

A solid state $^{13}C$ NMR spectrum of Compound 3 amorphous form is shown in Figure 3-11. Table 3-7 provides chemical shifts of the relevant peaks.

**Table 3-7**

| | Compound 3 amorphous form $^{13}C$ Chem. Shifts | |
|---|---|---|
| Peak # | F1 [ppm] | Intensity |
| 1 | 171.6 | 26.33 |
| 2 | 147.9 | 41.9 |
| 3 | 144.0 | 100 |
| 4 | 135.8 | 70.41 |
| 5 | 127.3 | 38.04 |
| 6 | 123.8 | 62.66 |
| 7 | 119.8 | 42.09 |
| 8 | 111.2 | 68.11 |
| 9 | 102.4 | 37.01 |
| 10 | 97.5 | 37.47 |
| 11 | 70.0 | 65.02 |
| 12 | 64.7 | 37.94 |
| 13 | 48.3 | 38.16 |
| 14 | 39.1 | 80.54 |
| 15 | 31.1 | 92.01 |
| 16 | 25.1 | 58.68 |
| 17 | 16.5 | 78.97 |

A solid state $^{19}F$ NMR spectrum of Compound 3 amorphous form is shown in Figure 3-12. Peaks with an asterisk denote spinning side bands. To avoid extensive spinning side bands overlap, $^{19}F$ MAS spectrum of Compound 3 amorphous form was collected with spinning speed of 21.0 kHz using a Bruker-Biospin 2.5 mm probe and corresponding 2.5 mm $ZrO_2$ rotors. Table 3-8 provides chemical shifts of the relevant peaks.

**Table 3-8**

| | Compound 3 amorphous form $^{19}F$ Chem. Shifts | |
|---|---|---|
| Peak # | F1 [ppm] | Intensity |
| 1 | -46.1 | 100 |
| 2 | -53.1 | 94.9 |

(continued)

| | Compound 3 amorphous form $^{19}F$ Chem. Shifts | |
|---|---|---|
| Peak # | F1 [ppm] | Intensity |
| 3 | -139.4 | 76.05 |

**IV. Formulations**

In one aspect, the invention features a formulation comprising a component selected from any embodiment described in Column A of Table I in combination with a component selected from any embodiment described in Column B and/or a component selected from any embodiment described in Column C of Table I.

Table I is reproduced here for convenience.

**Table I**

| Column A Embodiments | | Column B Embodiments | | Column C Embodiments | |
|---|---|---|---|---|---|
| **Section** | **Heading** | **Section** | **Heading** | **Section** | **Heading** |
| **II.A.1.** | Compounds of Formula I | **II.B.1.** | Compounds of Formula II | **II.C.1.** | Compounds of Formula III |
| **II.A.2.** | Compound 1 | **II.B.2.** | Compound 2 | **II.C.2.** | Compound 3 |
| **III.A.1.a.** | Compound 1 Form C | **III.B.1.a.** | Compound 2 Form I | **III.C.1.a.** | Compound 3 Form A |
| **IV.A.1.a.** | Compound 1 First Formulation | **III.B.2.a.** | Compound 2 Solvate Form A | **III.C.2.a.** | Compound 3 Amorphous Form |
| **IV.A.2.a.** | Compound 1 Tablet and SDD Formulation | **III.B.3.a.** | Compound 2 HCl Salt Form A | **IV.B.1.a.** | Compound 3 Tablet Formulation |

In one embodiment of this aspect, the formulation comprises an embodiment described in Column A in combination with an embodiment described in Column B. In another embodiment, the formulation comprises an embodiment described in Column A in combination with an embodiment described in Column C. In another embodiment, the formulation comprises a combination of an embodiment described in Column A, an embodiment described in Column B, and an embodiment described in Column C.

In one embodiment of this aspect, the Column A component is a compound of Formula I. In another embodiment, the Column A component is Compound 1. In another embodiment, the Column A component is Compound 1 Form C. In another embodiment, the Column A component is Compound 1 First Formulation. In another embodiment, the Column A component is Compound 1 Tablet and SDD Formulation.

In one embodiment of this aspect, the Column B component is a compound of Formula II. In another embodiment, the Column B component is Compound 2. In another embodiment, the Column B component is Compound 2 Form I. In another embodiment, the Column B component is Compound 2 Solvate Form A. In another embodiment, the Column B component is Compound 2 HCl Salt Form A.

In one embodiment of this aspect, the Column C component is a compound of Formula III. In another embodiment, the Column C component is Compound 3. In another embodiment, the Column C component is Compound 3 Form A. In another embodiment, the Column C component is Compound 3 Amorphous Form. In another embodiment, the Column C component is Compound 3 Tablet Formulation.

In one embodiment, the formulation comprises a homogeneous mixture comprising a composition according to Table I. In another embodiment, the formulation comprises a non-homogeneous mixture comprising a composition according to Table I.

The pharmaceutical composition of Table I can be administered in one vehicle or separately.

In some embodiments, the pharmaceutical composition optionally comprises a pharmaceutically acceptable carrier, adjuvant or vehicle. In certain embodiments, these compositions optionally further comprise one or more additional therapeutic agents.

It will also be appreciated that certain of the Compounds of present invention can exist in free form for treatment, or where appropriate, as a pharmaceutically acceptable derivative or a prodrug thereof. According to the present invention, a pharmaceutically acceptable derivative or a prodrug includes, but is not limited to, pharmaceutically acceptable salts, esters, salts of such esters, or any other adduct or derivative which upon administration to a patient in need thereof is

capable of providing, directly or indirectly, a Compound as otherwise described herein, or a metabolite or residue thereof.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. A "pharmaceutically acceptable salt" means any non-toxic salt or salt of an ester of a Compound of this invention that, upon administration to a recipient, is capable of providing, either directly or indirectly, a Compound of this invention or an inhibitorily active metabolite or residue thereof.

Pharmaceutically acceptable salts are well known in the art. For example, S. M. Berge, *et al.* describe pharmaceutically acceptable salts in detail in J. Pharmaceutical Sciences, 1977, 66, 1-19, incorporated herein by reference. Pharmaceutically acceptable salts of the Compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and $N^+(C_{1-4}alkyl)_4$ salts. This invention also envisions the quaternization of any basic nitrogen-containing groups of the Compounds disclosed herein. Water or oil-soluble or dispersable products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As described above, the pharmaceutically acceptable compositions of the present invention additionally comprise a pharmaceutically acceptable carrier, adjuvant, or vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with the Compounds of the invention, such as by producing any undesirable biological effect or otherwise interacting in a deleterious manner with any other component(s) of the pharmaceutically acceptable composition, its use is contemplated to be within the scope of this invention. Some examples of materials which can serve as pharmaceutically acceptable carriers include, but are not limited to, ion exchangers, alumina, aluminum stearate, lecithin, serum proteins, such as human serum albumin, buffer substances such as phosphates, glycine, sorbic acid, or potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, polyacrylates, waxes, polyethylene-polyoxypropylene-block polymers, wool fat, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol or polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

The pharmaceutically acceptable compositions of this invention can be administered to humans and other animals orally, rectally, parenterally, intracisternally, intravaginally, intraperitoneally, topically (as by powders, ointments, or drops), bucally, as an oral or nasal spray, or the like, depending on the severity of the infection being treated. In certain embodiments, the compositions of the invention may be administered orally or parenterally at dosage levels of about 0.01 mg/kg to about 50 mg/kg and preferably from about 1 mg/kg to about 25 mg/kg, of subject body weight per day, one or more times a day, to obtain the desired therapeutic effect.

Liquid dosage forms for oral administration include, but are not limited to, pharmaceutically acceptable emulsions, microemulsions, solutions, suspensions, syrups and elixirs. In addition to the active Compounds of the composition, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that maybe employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a composition of the present invention, it is often desirable to slow the absorption of the composition from subcutaneous or intramuscular injection. This may be accomplished by the use of a liquid suspension of crystalline or amorphous material with poor water solubility. The rate of absorption of the composition then depends upon its rate of dissolution that, in turn, may depend upon crystal size and crystalline form. Alternatively, delayed absorption of a parenterally administered composition form is accomplished by dissolving or suspending the composition in an oil vehicle. Injectable depot forms are made by forming microencapsule matrices of the composition in biodegradable polymers such as polylactide-polyglycolide. Depending upon the ratio of composition to polymer and the nature of the particular polymer employed, the rate of composition release can be controlled. Examples of other biodegradable polymers include poly(orthoesters) and poly(anhydrides). Depot injectable formulations are also prepared by entrapping the composition in liposomes or microemulsions that are compatible with body tissues.

Compositions for rectal or vaginal administration are preferably suppositories which can be prepared by mixing the Compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active Compound.

Solid dosage forms for oral administration include capsules, tablets, pills, powders, and granules. In such solid dosage forms, the active Compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium Compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

The active Compounds can also be in microencapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active Compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain

part of the intestinal tract, optionally, in a delayed manner. Examples of embedding compositions that can be used include polymeric substances and waxes.

Dosage forms for topical or transdermal administration of a Compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the present invention contemplates the use of transdermal patches, which have the added advantage of providing controlled delivery of a Compound to the body. Such dosage forms are prepared by dissolving or dispensing the Compound in the proper medium. Absorption enhancers can also be used to increase the flux of the Compound across the skin. The rate can be controlled by either providing a rate controlling membrane or by dispersing the Compound in a polymer matrix or gel.

In some aspects, the dosage form includes a composition as described herein comprising about 250 mg of Compound 1. In one embodiment of this aspect, the composition also includes Compound 2. In another embodiment, the composition also includes Compound 3. In another embodiment, the composition also includes Compound 2 and Compound 3. In one embodiment of this aspect, the dosage form comprising about 250 mg of Compound 1 is a tablet. In a further embodiment, the dosage form comprising about 250 mg of Compound 1 is divided into two or more tablets. In still a further embodiment, the dosage form comprising about 250 mg of Compound 1 is a tablet including about 100 mg of Compound 1, plus a tablet including about 150 mg Compound 1.

It will also be appreciated that the compositions disclosed herein can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive Compound may be administered concurrently with another agent used to treat the same disorder), or they may achieve different effects (e.g., control of any adverse effects). As used herein, additional therapeutic agents that are normally administered to treat or prevent a particular disease, or condition, are known as "appropriate for the disease, or condition, being treated."

In one embodiment, the additional agent is selected from a mucolytic agent, bronchodialator, an anti-biotic, an anti-infective agent, an anti-inflammatory agent, a CFTR modulator other than a Compound of the present invention, or a nutritional agent.

In one embodiment, the additional agent is an antibiotic. Exemplary antibiotics useful herein include tobramycin, including tobramycin inhaled powder (TIP), azithromycin, aztreonam, including the aerosolized form of aztreonam, amikacin, including liposomal formulations thereof, ciprofloxacin, including formulations thereof suitable for administration by inhalation, levoflaxacin, including aerosolized formulations thereof, and combinations of two antibiotics, e.g., fosfomycin and tobramycin.

In another embodiment, the additional agent is a mucolyte. Exemplary mucolytes useful herein includes Pulmozyme®.

In another embodiment, the additional agent is a bronchodialator. Exemplary bronchodialtors include albuterol, metaprotenerol sulfate, pirbuterol acetate, salmeterol, or tetrabuline sulfate.

In another embodiment, the additional agent is effective in restoring lung airway surface liquid. Such agents improve the movement of salt in and out of cells, allowing mucus in the lung airway to be more hydrated and, therefore, cleared more easily. Exemplary such agents include hypertonic saline, denufosol tetrasodium ([[(3S, 5R)-5-(4-amino-2-oxopyrimidin-1-yl)-3-hydroxyoxolan-2-yl]methoxy-hydroxyphosphoryl] [[[(2R,3S,4R,5R)-5-(2,4-dioxopyrimidin-1-yl)-3, 4-dihydroxyoxolan-2-yl]methoxy-hydroxyphosphoryl]oxy-hydroxyphosphoryl] hydrogen phosphate), or bronchitol (inhaled formulation of mannitol).

In another embodiment, the additional agent is an anti-inflammatory agent, i.e., an agent that can reduce the inflammation in the lungs. Exemplary such agents useful herein include ibuprofen, docosahexanoic acid (DHA), sildenafil, inhaled glutathione, pioglitazone, hydroxychloroquine, or simavastatin.

In another embodiment, the additional agent is a CFTR modulator other than Compound 1, i.e., an agent that has the effect of modulating CFTR activity. Exemplary such agents include ataluren ("PTC124®"; 3-[5-(2-fluorophenyl)-1,2,4-oxadiazol-3-yl]benzoic acid), sinapultide, lancovutide, depelestat (a human recombinant neutrophil elastase inhibitor), cobiprostone (7-{(2R, 4aR, 5R, 7aR)-2-[(3S)-1,1-difluoro-3-methylpentyl]-2-hydroxy-6-oxooctahydrocyclopenta[b]pyran-5-yl}heptanoic acid), or (3-(6-(1-(2,2-difluorobenzo[d][1,3]dioxol-5-yl) cyclopropanecarboxamido)-3-methylpyridin-2-yl)benzoic acid. In another embodiment, the additional agent is (3-(6-(1-(2,2-difluorobenzo[d][1,3]dioxol-5-yl) cyclopropanecarboxamido)-3-methylpyridin-2-yl)benzoic acid.

In another embodiment, the additional agent is a nutritional agent. Exemplary such agents include pancrelipase (pancreating enzyme replacement), including Pancrease®, Pancreacarb®, Ultrase®, or Creon®, Liprotomase® (formerly Trizytek®), Aquadeks®, or glutathione inhalation. In one embodiment, the additional nutritional agent is pancrelipase.

The amount of additional therapeutic agent present in the compositions of this invention will be no more than

the amount that would normally be administered in a composition comprising that therapeutic agent as the only active agent. Preferably the amount of additional therapeutic agent in the presently disclosed compositions will range from about 50% to 100% of the amount normally present in a composition comprising that agent as the only therapeutically active agent.

A composition of the invention as disclosed herein may also be incorporated into compositions for coating an implantable medical device, such as prostheses, artificial valves, vascular grafts, stents and catheters. Accordingly, the present invention, in another aspect, includes a composition for coating an implantable device comprising a composition as disclosed herein or a pharmaceutically acceptable composition thereof, and in classes and subclasses herein, and a carrier suitable for coating said implantable device. In still another aspect, the present invention includes an implantable device coated with a composition comprising a composition as described herein or a pharmaceutically acceptable composition thereof, and a carrier suitable for coating said implantable device. Suitable coatings and the general preparation of coated implantable devices are described in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccarides, polyethylene glycol, phospholipids or combinations thereof to impart controlled release characteristics in the composition.

In order that the invention described herein may be more fully understood, the following examples are set forth. It should be understood that these examples are for illustrative purposes only and are not to be construed as limiting this invention in any manner.

### IV.A. Formulations of Compound 1

In some embodiments, Compound 1 is formulated as provided herein, and is administered together with Compound 2 or as provided in Table I. As a note, Compound 1 may be in any of the solid forms specified herein.

### IV.A.1. Compound 1 First Formulation

#### IV.A.1.a. Embodiments of Compound 1 First Formulation

In one embodiment, the Compound 1 Formulation comprises:

(i) Compound 1;
(ii) PEG 400; and
(iii) PVP K30.

In another embodiment, the Compound 1 Formulation comprises:

(i) Compound 1 or a pharmaceutically acceptable salt thereof;
(ii) A liquid PEG (polyethylene glycol polymer) that has an average molecular weight of between about 200 and about 600; and
(iii) Optionally, PVP.

In another embodiment, the Compound 1 Formulation comprises:

(i) Compound 1 or a pharmaceutically acceptable salt thereof;
(ii) a suitable liquid PEG; and
(iii) optionally, a suitable viscosity enhancing agent.

As used herein, the phrase "suitable liquid PEG" means a polyethylene glycol polymer that is in liquid form at ambient temperature and is amenable for use in a pharmaceutical composition. Such suitable polyethylene glycols are well known in the art; see, e.g., http://www.medicinescomplete.com/mc/excipients/current, which is incorporated herein by reference. Exemplary PEGs include low molecular weight PEGs such as PEG 200, PEG 300, PEG 400, etc. The number that follows the term "PEG" indicates the average molecular weight of that particular polymer. E.g., PEG 400 is a polyethylene glycol polymer wherein the average molecular weight of the polymer therein is about 400.

In one embodiment, said suitable liquid PEG has an average molecular weight of from about 200 to about 600. In another embodiment, said suitable liquid PEG is PEG 400 (for example a PEG having a molecular weight of from about 380 to about 420 g/mol).

In another embodiment, the present invention provides a pharmaceutical composition comprising Compound

1 or a pharmaceutically acceptable salt thereof; propylene glycol; and, optionally, a suitable viscosity enhancing agent.

In another embodiment, the pharmaceutical formulations of the present invention comprise a suitable viscosity enhancing agent. In one embodiment, the suitable viscosity enhancing agent is a polymer soluble in PEG. Such suitable viscosity enhancing agents are well known in the art, e.g., polyvinyl pyrrolidine (hereinafter "PVP"). PVP is characterized by its viscosity in aqueous solution, relative to that of water, expressed as a *K*-value (denoted as a suffix, e.g., PVP K20), in the range of from about 10 to about 120. See, e.g., http://www.medicinescomplete.com/mc/excipients/current. Embodiments of PVP useful in the present invention have a K-value of about 90 or less. An exemplary such embodiment is PVP K30.

In one embodiment, the Compound 1 formulation comprises:

(i) Compound 1 or a pharmaceutically acceptable salt thereof;
(ii) PEG 400; and
(iii) PVP K30.

In another embodiment, Compound 1 is present in an amount from about 0.01 % w/w to about 6.5 % w/w.

In another embodiment, the present invention provides a pharmaceutical formulation, wherein said PEG is present in an amount from about 87.5 % w/w to about 99.99 % w/w.

In another embodiment, the PVP K30 is present in an amount between 0% w/w to about 6 % w/w.

In another embodiment, the formulation comprises PEG 400 (e.g., from about 97.8 to about 98.0 % w/w, for example, about 97.88 % w/w), PVP K30 (e.g., from about 1.9 to about 2.1 % w/w, for example, about 2.0 % w/w), and Compound 1 (e.g., from about 0.10 to about 0.15 % w/w, for example, about 0.13 % w/w).

In another embodiment, the formulation comprises PEG 400 (e.g., from about 97.5 to about 98.0 % w/w, for example, about 97.75 % w/w), PVP K30 (e.g., from about 1.8 to about 2.2 % w/w, for example, about 2.0 % w/w), and Compound 1 (e.g., from about 0.2 to about 0.3 % w/w, for example, about 0.25 % w/w).

In another embodiment, the formulation comprises PEG 400 (e.g., from about 97.2 to about 97.8, for example, about 97.50 % w/w), PVP K30 (e.g., from about 1.8 to about 2.2 % w/w, for example, about 2.0 % w/w), and Compound 1 (e.g., from about 0.4 to about 0.6 % w/w, for example, about 0.50 % w/w).

In another embodiment, the formulation comprises PEG 400 (e.g., from about 96.5 to about 97.5 % w/w, for example, about 97.0 % w/w), PVP K30 (e.g., from about 1.8 to about 2.2 % w/w, for example, about 2.0 % w/w), and Compound 1 (e.g., from about 0.9 to about 1.1 % w/w, for example, about 1.0 % w/w).

In another embodiment, formulation comprises PEG 400 (e.g., from about 96.60 to about 96.65 % w/w, for example, about 96.63 % w/w), PVP K30 (e.g., from about 1.8 to about 2.2 % w/w, for example, about 2.0 % w/w), and Compound 1 (e.g., from about 1.30 to about 1.45 % w/w, for example, about 1.38 % w/w).

In another embodiment, the formulation comprises PEG 400 (e.g., from about 96.0 to about 96.3 % w/w, for example, about 96.12 % w/w), PVP K30 (e.g., from about 1.8 to about 2.0 % w/w, for example, about 2.0 % w/w), and Compound 1 (e.g., from about 1.8 to about 2.2 % w/w, for example, about 1.88 % w/w).

In another embodiment, the formulation comprises PEG 400 (e.g., from about 95.5 to about 96.0 % w/w, for example, about 95.75 % w/w), PVP K30 (e.g., from about 1.8 to about 2.2 % w/w, for example, about 2.0 % w/w), and Compound 1 (e.g., from about 2.0 to about 2.5 % w/w, for example, about 2.25 % w/w).

In another embodiment, the formulation comprises PEG 400 (e.g., from about 95 to about 96 % w/w, for example, about 95.5 % w/w), PVP K30 (e.g., from about 1.8 to about 2.2 % w/w, for example, about 2.0 % w/w), and Compound 1 (e.g., from about 2.3 to about 2.7 %w/w, for example, about 2.50 % w/w).

In another embodiment, the formulation comprises PEG 400 (e.g., from about 94.5 to about 94.8, for example, about 94.63 % w/w), PVP K30 (e.g., from about 1.8 to about 2.2 % w/w, for example, about 2.0 % w/w), and Compound 1 (e.g., from about 3.5 to about 4.0 % w/w, for example, about 3.38 % w/w).

In another embodiment, the formulation comprises PEG 400 (e.g., from about 93.5 to about 94.5 % w/w, for example, about 94.0 % w/w), PVP K30 (e.g., from about 1.8 to about 2.2 % w/w, for example, about 2.0 % w/w), and Compound 1 (e.g., from about 3.7 to about 4.3 % w/w, for example, about 4.0 % w/w).

In one embodiment, the formulation comprises:

(i) Compound 1 or a pharmaceutically acceptable salt thereof;
(ii) a suitable PEG lipid; and
(iii) PVP.

In some embodiments, the PEG lipid has an average molecular weight of from about 400 to about 600, for example, PEG 400. In some embodiments, the PVP is PVP K30.

The formulation comprises a therapeutically effective amount of Compound 1. The phrase "therapeutically effective amount" is that amount effective for treating or lessening the severity of any of the diseases, conditions, or

disorders recited below.

**IV.A.1.b. Preparation of Compound 1 First Formulation**

**Materials:**

- A Glass bottle for formulation preparation (250 cc amber glass with teflon lined lid)
- Glass bottle for dose confirmation sample (30 cc amber glass with Teflon lined lid)
- Stir Plate with temperature probe (ensure probe has been cleaned)
- New magnetic stir bar
- Spatulas for dispensing excipient and active.

**Step 1:**

To a clean 250 cc amber glass bottle add the stir bar to the bottle and record the tare weight of the bottle, stir bar, label and cap. Tare the bottle with the label and stir bar.

**Step 2:**

Dispense targeted amount of PEG400 into the bottle and accurately weigh. Place the bottle on stir plate and stir to form a small vortex at the surface of the liquid (~300-500rpm or as necessary). Insert the cleaned temperature probe into the liquid to a depth of ~1cm and raise the setpoint of the heater to 40°C. Cover the bottle opening with aluminum foil. Allow the PEG400 to stabilize at 40+/-5°C.

**Step 3:**

Dispense the required amount of PVP K30 and add to the stirring PEG400. Add the PVP in a slow stream (over ~2-3 minutes) and allow the particles to disperse. If the particles clump, the dissolution will take longer. Cover the bottle opening with foil and continue stirring the mixture at 40+/-5°C. The mixture should be sampled at 10 minutes using a small transfer pipette to determine if the PVP has completely dissolved. The stirring solution should also be examined for large, undissolved clumps. If the solution is clear, proceed to the next step. If undissolved polymer remains, continue stirring. Check for dissolution every 10 minutes, with a maximum stirring time of 30 minutes total. When complete dissolution is observed, proceed to the next step. If complete dissolution is not observed within 30 minutes after PVP addition, terminate preparation, discard the material, and start the preparation from the beginning.

**Step 4:**

Dispense the required amount of Compound 1 and add to the stirred PEG/PVP solution in a slow stream. Cover the bottle opening with foil and continue stirring the mixture at 40+/-5°C. The mixture should be sampled after 30 minutes using a small transfer pipette to determine if the Compound 1 has completely dissolved. If the solution is clear after 30 minutes, proceed to the next step. If undissolved Compound 1 remains, continue stirring. Check for dissolution every 30 minutes with a maximum stirring time of 300 minutes (5 hours) after addition of Compound 1. If complete dissolution is not observed within 300 minutes (5 hours) after addition of Compound 1, terminate preparation, discard the material, and start the preparation from the beginning.

Upon complete dissolution of the Compound 1, remove from the stir plate, and cap the bottle. The formulation should be maintained at room temperature until dosing, but must be dosed within 24 hours of preparation. If precipitation of Compound 1 is observed, do not dose the solution.

Using the above method, the following ten pharmaceutical formulations in Table 1-A were prepared.

**Table 1-A**

| Composition # | % PEG 400 w/w | % PVP K30 w/w | % Cmpd 1 w/w | Amount of Cmpd 1 per 20 g dose (mg) |
|---|---|---|---|---|
| 1 | 97.875 | 2.0 | 0.125 | 25 |
| 2 | 97.750 | 2.0 | 0.250 | 50 |
| 3 | 97.500 | 2.0 | 0.500 | 100 |

(continued)

| Composition # | % PEG 400 w/w | % PVP K30 w/w | % Cmpd 1 w/w | Amount of Cmpd 1 per 20 g dose (mg) |
|---|---|---|---|---|
| 4 | 97.000 | 2.0 | 1.000 | 200 |
| 5 | 96.625 | 2.0 | 1.375 | 275 |
| 6 | 96.125 | 2.0 | 1.875 | 375 |
| 7 | 95.750 | 2.0 | 2.25 | 450 |
| 8 | 95.500 | 2.0 | 2.500 | 500 |
| 9 | 94.625 | 2.0 | 3.375 | 675 |
| 10 | 94.000 | 2.0 | 4.000 | 800 |

**IV.A.2. Compound 1 Tablet and SDD Formulation**

**IV.A.2.a. Embodiments of Compound 1 Tablet and SDD Formulation**

In one embodiment, the present invention provides a pharmaceutical composition comprising:

a. a solid dispersion of substantially amorphous Compound 1 and HPMCAS;
b. a filler;
c. a disintegrant;
d. a surfactant;
e. a binder;
f. a glidant; and
g. a lubricant,

wherein the solid dispersion comprises about 100 mg of substantially amorphous Compound 1.

In one embodiment, the present invention provides a pharmaceutical composition comprising:

a. a solid dispersion of substantially amorphous Compound 1 and HPMCAS;
b. a filler;
c. a disintegrant;
d. a surfactant;
e. a binder;
f. a glidant; and
g. a lubricant,

wherein the solid dispersion comprises about 150 mg of substantially amorphous Compound 1.

In one embodiment, the present invention provides a pharmaceutical composition comprising:

a. a solid dispersion of amorphous Compound 1 and HPMCAS;
b. a filler;
c. a disintegrant;
d. a surfactant;
e. a binder;
f. a glidant; and
g. a lubricant,

wherein the solid dispersion comprises about 100 mg of amorphous Compound 1.

In one embodiment, the present invention provides a pharmaceutical composition comprising:

a. a solid dispersion of amorphous Compound 1 and HPMCAS;
b. a filler;
c. a disintegrant;
d. a surfactant;

e. a binder;
f. a glidant; and
g. a lubricant,

wherein the solid dispersion comprises about 150 mg of amorphous Compound 1.

In some embodiments, the pharmaceutical composition comprises a solid dispersion a filler, a disintegrant, a surfactant, a binder, a glidant, and a lubricant, wherein the solid dispersion comprises from about 75 wt% to about 95 wt% (e.g., about 80 wt%) of Compound 1 by weight of the dispersion and a polymer.

In one embodiment, the pharmaceutical composition of the present invention comprises a solid dispersion of Compound 1. For example, the solid dispersion comprises substantially amorphous Compound 1, where Compound 1 is less than about 15% (e.g., less than about 10% or less than about 5%) crystalline, and at least one polymer. In another example, the solid dispersion comprises amorphous Compound 1, i.e., Compound 1 has about 0% crystallinity. The concentration of Compound 1 in the solid dispersion depends on several factors such as the amount of pharmaceutical composition needed to provide a desired amount of Compound 1 and the desired dissolution profile of the pharmaceutical composition.

In another embodiment, the pharmaceutical composition comprises a solid dispersion that contains substantially amorphous Compound 1 and HPMCAS, in which the solid dispersion has a mean particle diameter, measured by light scattering (e.g., using a Malvern Mastersizer available from Malvern Instruments in England) of greater than about 5 μm (e.g., greater than about 6 μm, greater than about 7 μm, greater than about 8 μm, or greater than about 10 μm). For example, the pharmaceutical composition comprises a solid dispersion that contains amorphous Compound 1 and HPMCAS, in which the solid dispersion has a mean particle diameter, measured by light scattering, of greater than about 5 μm (e.g., greater than about 6 μm, greater than about 7 μm, greater than about 8 μm, or greater than about 10 μm). In another example, the pharmaceutical composition comprises a solid dispersion comprising substantially amorphous Compound 1 and HPMCAS, in which the solid dispersion has a mean particle diameter, measured by light scattering, of from about 7 μm to about 25 μm. For instance, the pharmaceutical composition comprises a solid dispersion comprising amorphous Compound 1 and HPMCAS, in which the solid dispersion has a mean particle diameter, measured by light scattering, of from about 7 μm to about 25 μm. In yet another example, the pharmaceutical composition comprises a solid dispersion comprising substantially amorphous Compound 1 and HPMCAS, in which the solid dispersion has a mean particle diameter, measured by light scattering, of from about 10 μm to about 35 μm. For instance, the pharmaceutical composition comprises a solid dispersion comprising amorphous Compound 1 and HPMCAS, in which the solid dispersion has a mean particle diameter, measured by light scattering, of from about 10 μm to about 35 μm. In another example, the pharmaceutical composition comprises a solid dispersion comprising substantially amorphous Compound 1 and HPMCAS, in which the solid dispersion has a bulk density of about 0.10 g/cc or greater (e.g., 0.15 g/cc or greater, 0.17 g/cc or greater). For instance, the pharmaceutical composition comprising a solid dispersion comprising amorphous Compound 1 and HPMCAS, in which the solid dispersion has a bulk density of about 0.10 g/cc or greater (e.g., 0.15 g/cc or greater, 0.17 g/cc or greater). In another instance, the pharmaceutical composition comprises a solid dispersion that comprises substantially amorphous Compound 1 and HPMCAS, in which the solid dispersion has a bulk density of from about 0.10 g/cc to about 0.45 g/cc (e.g., from about 0.15 g/cc to about 0.42 g/cc, or from about 0.17 g/cc to about 0.40 g/cc). In still another instance, the pharmaceutical composition comprises a solid dispersion that includes amorphous Compound 1 and HPMCAS, in which the solid dispersion has a bulk density of from about 0.10 g/cc to about 0.45 g/cc (e.g., from about 0.15 g/cc to about 0.42 g/cc, or from about 0.17 g/cc to about 0.40 g/cc). In another example, the pharmaceutical composition comprises a solid dispersion that comprises substantially amorphous Compound 1 and HPMCAS, in which the solid dispersion has a bulk density of from about 0.10 g/cc to about 0.45 g/cc (e.g., from about 0.15 g/cc to about 0.42 g/cc, or from about 0.17 g/cc to about 0.40 g/cc). For instance, the pharmaceutical composition includes a solid dispersion that comprises amorphous Compound 1 and HPMCAS, in which the solid dispersion has a bulk density of from about 0.10 g/cc to about 0.45 g/cc (e.g., from about 0.15 g/cc to about 0.42 g/cc, or from about 0.17 g/cc to about 0.40 g/cc).

Other solid dispersions comprise from about 65 wt% to about 95 wt% (e.g., from about 67 wt% to about 92 wt%, from about 70 wt% to about 90 wt%, or from about 72 wt% to about 88 wt%) of substantially amorphous Compound 1 by weight of the solid dispersion and from about 45 wt% to about 5 wt% of polymer (e.g., HPMCAS). For instance, the solid dispersion comprises from about 65 wt% to about 95 wt% (e.g., from about 67 wt% to about 92 wt%, from about 70 wt% to about 90 wt%, or from about 72 wt% to about 88 wt%) of amorphous Compound 1 by weight of the solid dispersion and from about 45 wt% to about 5 wt% of polymer (e.g., HPMCAS).

Suitable surfactants include sodium lauryl sulfate (SLS), sodium stearyl fumarate (SSF), polyoxyethylene 20 sorbitan mono-oleate (e.g., Tween™), any combination thereof, or the like. In one example, the solid dispersion comprises less than 5 wt% (less than 3.0 wt%, less than 1.5 wt%, or less than 1.0 wt%) of surfactant by weight of solid dispersion. In another example, the solid dispersion comprises from about 0.30 wt% to about 0.80 wt% (e.g., from about 0.35 wt% to about 0.70 wt%, from about 0.40 wt% to about 0.60 wt%, or from about 0.45 wt% to about 0.55 wt%) of surfactant by

weight of solid dispersion.

In alternative embodiments, the solid dispersion comprises from about 45 wt% to about 85 wt% of substantially amorphous or amorphous Compound 1, from about 0.45 wt% to about 0.55 wt% of SLS, and from about 14.45 wt% to about 55.55 wt% of HPMCAS by weight of the solid dispersion. One exemplary solid dispersion contains about 80 wt% of substantially amorphous or amorphous Compound 1, about 19.5 wt% of HPMCAS, and about 0.5 wt% of SLS.

Fillers suitable for the present invention are compatible with the ingredients of the pharmaceutical composition, i.e., they do not substantially reduce the solubility, the hardness, the chemical stability, the physical stability, or the biological activity of the pharmaceutical composition. Exemplary fillers include lactose, sorbitol, celluloses, calcium phosphates, starches, sugars (e.g., mannitol, sucrose, or the like), or any combination thereof. In one embodiment, the pharmaceutical composition comprises at least one filler in an amount of at least about 10 wt% (e.g., at least about 20 wt%, at least about 25 wt%, or at least about 27 wt%) by weight of the composition. For example, the pharmaceutical composition comprises from about 10 wt% to about 60 wt% (e.g., from about 20 wt% to about 55 wt%, from about 25 wt% to about 50 wt%, or from about 27 wt% to about 45 wt%) of filler, by weight of the composition. In another example, the pharmaceutical composition comprises at least about 20 wt% (e.g., at least 25 wt% or at least 27 wt%) of lactose, by weight of the composition. In yet another example, the pharmaceutical composition comprises from about 20 wt% to about 60 wt% (e.g., from about 25 wt% to about 55 wt% or from about 27 wt% to about 45 wt%) of lactose, by weight of the composition.

Disintegrants suitable for the present invention enhance the dispersal of the pharmaceutical composition and are compatible with the ingredients of the pharmaceutical composition, i.e., they do not substantially reduce the chemical stability, the physical stability, the hardness, or the biological activity of the pharmaceutical composition. Exemplary disintegrants include sodium croscarmellose, sodium starch glycolate, or a combination thereof. In one embodiment, the pharmaceutical composition comprises disintegrant in an amount of about 10 wt% or less (e.g., about 7 wt% or less, about 6 wt% or less, or about 5 wt% or less) by weight of the composition. For example, the pharmaceutical composition comprises from about 1 wt% to about 10 wt% (e.g., from about 1.5 wt% to about 7.5 wt% or from about 2.5 wt% to about 6 wt%) of disintegrant, by weight of the composition. In another example, the pharmaceutical composition comprises about 10 wt% or less (e.g., 7 wt% or less, 6 wt% or less, or 5 wt% or less) of sodium croscarmellose, by weight of the composition. In yet another example, the pharmaceutical composition comprises from about 1 wt% to about 10 wt% (e.g., from about 1.5 wt% to about 7.5 wt% or from about 2.5 wt% to about 6 wt%) of sodium croscarmellose, by weight of the composition. In some examples, the pharmaceutical composition comprises from about 0.1% to about 10 wt% (e.g., from about 0.5 wt% to about 7.5 wt% or from about 1.5 wt% to about 6 wt%) of disintegrant, by weight of the composition. In still other examples, the pharmaceutical composition comprises from about 0.5% to about 10 wt% (e.g., from about 1.5 wt% to about 7.5 wt% or from about 2.5 wt% to about 6 wt%) of disintegrant, by weight of the composition.

Surfactants suitable for the present invention enhance the solubility of the pharmaceutical composition and are compatible with the ingredients of the pharmaceutical composition, i.e., they do not substantially reduce the chemical stability, the physical stability, the hardness, or the biological activity of the pharmaceutical composition. Exemplary surfactants include sodium lauryl sulfate (SLS), sodium stearyl fumarate (SSF), polyoxyethylene 20 sorbitan mono-oleate (e.g., Tween™), any combination thereof, or the like. In one embodiment, the pharmaceutical composition comprises a surfactant in an amount of about 10 wt% or less (e.g., about 5 wt% or less, about 2 wt% or less, about 1 wt% or less, about 0.8 wt% or less, or about 0.6 wt% or less) by weight of the composition. For example, the pharmaceutical composition includes from about 10 wt% to about 0.1 wt% (e.g., from about 5 wt% to about 0.2 wt% or from about 2 wt% to about 0.3 wt%) of surfactant, by weight of the composition. In another example, the pharmaceutical composition comprises 10 wt% or less (e.g., about 5 wt% or less, about 2 wt% or less, about 1 wt% or less, about 0.8 wt% or less, or about 0.6 wt% or less) of sodium lauryl sulfate, by weight of the composition. In yet another example, the pharmaceutical composition comprises from about 10 wt% to about 0.1 wt% (e.g., from about 5 wt% to about 0.2 wt% or from about 2 wt% to about 0.3 wt%) of sodium lauryl sulfate, by weight of the composition.

Binders suitable for the present invention enhance the tablet strength of the pharmaceutical composition and are compatible with the ingredients of the pharmaceutical composition, i.e., they do not substantially reduce the chemical stability, the physical stability, or the biological activity of the pharmaceutical composition. Exemplary binders include microcrystalline cellulose, dibasic calcium phosphate, sucrose, corn (maize) starch, modified cellulose (e.g., hydroxymethyl cellulose), or any combination thereof. In one embodiment, the pharmaceutical composition comprises a binder in an amount of at least about 1 wt% (e.g., at least about 10 wt%, at least about 15 wt%, at least about 20 wt%, or at least about 22 wt%) by weight of the composition. For example, the pharmaceutical composition comprises from about 5 wt% to about 50 wt% (e.g., from about 10 wt% to about 45 wt% or from about 20 wt% to about 45 wt%) of binder, by weight of the composition. In another example, the pharmaceutical composition comprises at least about 1 wt% (e.g., at least about 10 wt%, at least about 15 wt%, at least about 20 wt%, or at least about 22 wt%) of microcrystalline cellulose, by weight of the composition. In yet another example, the pharmaceutical composition comprises from about 5 wt% to about 50 wt% (e.g., from about 10 wt% to about 45 wt% or from about 20 wt% to about 45 wt%) of microcrystalline cellulose, by weight of the composition.

Glidants suitable for the present invention enhance the flow properties of the pharmaceutical composition and are compatible with the ingredients of the pharmaceutical composition, i.e., they do not substantially reduce the solubility, the hardness, the chemical stability, the physical stability, or the biological activity of the pharmaceutical composition. Exemplary glidants include colloidal silicon dioxide, talc, or a combination thereof. In one embodiment, the pharmaceutical composition comprises a glidant in an amount of 2 wt% or less (e.g., 1.75 wt%, 1.25 wt% or less, or 1.00 wt% or less) by weight of the composition. For example, the pharmaceutical composition comprises from about 2 wt% to about 0.05 wt% (e.g., from about 1.5 wt% to about 0.07 wt% or from about 1.0 wt% to about 0.09 wt%) of glidant, by weight of the composition. In another example, the pharmaceutical composition comprises 2 wt% or less (e.g., 1.75 wt%, 1.25 wt% or less, or 1.00 wt% or less) of colloidal silicon dioxide, by weight of the composition. In yet another example, the pharmaceutical composition comprises from about 2 wt% to about 0.05 wt% (e.g., from about 1.5 wt% to about 0.07 wt% or from about 1.0 wt% to about 0.09 wt%) of colloidal silicon dioxide, by weight of the composition.

Lubricants suitable for the present invention improve the compression and ejection of compressed pharmaceutical compositions from a die press and are compatible with the ingredients of the pharmaceutical composition, i.e., they do not substantially reduce the solubility, the hardness, or the biological activity of the pharmaceutical composition. Exemplary lubricants include magnesium stearate, stearic acid (stearin), hydrogenated oil, sodium stearyl fumarate, or any combination thereof. In one embodiment, the pharmaceutical composition comprises a lubricant in an amount of 2 wt% or less (e.g., 1.75 wt%, 1.25 wt% or less, or 1.00 wt% or less) by weight of the composition. For example, the pharmaceutical composition comprises from about 2 wt% to about 0.10 wt% (e.g., from about 1.5 wt% to about 0.15 wt% or from about 1.3 wt% to about 0.30 wt%) of lubricant, by weight of the composition. In another example, the pharmaceutical composition comprises 2 wt% or less (e.g., 1.75 wt%, 1.25 wt% or less, or 1.00 wt% or less) of magnesium stearate, by weight of the composition. In yet another example, the pharmaceutical composition comprises from about 2 wt% to about 0.10 wt% (e.g., from about 1.5 wt% to about 0.15 wt% or from about 1.3 wt% to about 0.30 wt%) of magnesium stearate, by weight of the composition.

Pharmaceutical compositions of the present invention can optionally comprise one or more colorants, flavors, and/or fragrances to enhance the visual appeal, taste, and/or scent of the composition. Suitable colorants, flavors, or fragrances are compatible with the ingredients of the pharmaceutical composition, i.e., they do not substantially reduce the solubility, the chemical stability, the physical stability, the hardness, or the biological activity of the pharmaceutical composition. In one embodiment, the pharmaceutical composition comprises a colorant, a flavor, and/or a fragrance. For example, the pharmaceutical composition comprises less than about 1 wt% (e.g., less than about 0.75 wt% or less than about 0.5 wt%) of each optionally ingredient, i.e., colorant, flavor and/or fragrance, by weight of the composition. In another example, the pharmaceutical composition comprises less than about 1 wt% (e.g., less than about 0.75 wt% or less than about 0.5 wt%) of a colorant. In still another example, the pharmaceutical composition comprises less than about 1 wt% (e.g., less than about 0.75 wt% or less than about 0.5 wt%) of a blue colorant (e.g., FD&C Blue #1 and/or FD&C Blue #2 Aluminum Lake, commercially available from Colorcon, Inc. of West Point, PA.)

In some embodiments, the pharmaceutical composition can be made into tablets and the tablets can be coated with a colorant and optionally labeled with a logo, other image and/or text using a suitable ink. In still other embodiments, the pharmaceutical composition can be made into tablets and the tablets can be coated with a colorant, waxed, and optionally labeled with a logo, other image and/or text using a suitable ink. Suitable colorants and inks are compatible with the ingredients of the pharmaceutical composition, i.e., they do not substantially reduce the solubility, the chemical stability, the physical stability, the hardness, or the biological activity of the pharmaceutical composition. The suitable colorants and inks can be any color and are water based or solvent based. In one embodiment, tablets made from the pharmaceutical composition are coated with a colorant and then labeled with a logo, other image, and/or text using a suitable ink. For example, tablets comprising pharmaceutical composition as described herein can be coated with about 3 wt% (e.g., less than about 6 wt% or less than about 4 wt%) of film coating comprising a colorant. The colored tablets can be labeled with a logo and text indicating the strength of the active ingredient in the tablet using a suitable ink. In another example, tablets comprising pharmaceutical composition as described herein can be coated with about 3 wt% (e.g., less than about 6 wt% or less than about 4 wt%) of a film coating comprising a blue colorant (e.g., OPADRY® II, commercially available from Colorcon, Inc. of West Point, PA.). The colored tablets can be labeled with a logo and text indicating the strength of the active ingredient in the tablet using a black ink (e.g., Opacode® WB, commercially available from Colorcon, Inc. of West Point, PA.). In another embodiment, tablets made from the pharmaceutical composition are coated with a colorant, waxed, and then labeled with a logo, other image, and/or text using a suitable ink. For example, tablets comprising pharmaceutical composition as described herein can be coated with about 3 wt% (e.g., less than about 6 wt% or less than about 4 wt%) of film coating comprising a colorant. The colored tablets can be waxed with Carnauba wax powder weighed out in the amount of about 0.01% w/w of the starting tablet core weight. The waxed tablets can be labeled with a logo and text indicating the strength of the active ingredient in the tablet using a suitable ink. In another example, tablets comprising pharmaceutical composition as described herein can be coated with about 3 wt% (e.g., less than about 6 wt% or less than about 4 wt%) of a film coating comprising a blue colorant (e.g., OPADRY® II, commercially available from Colorcon, Inc. of West Point, PA.). The colored tablets can be waxed with Carnauba wax

powder weighed out in the amount of about 0.01% w/w of the starting tablet core weight. The waxed tablets can be labeled with a logo and text indicating the strength of the active ingredient in the tablet using a black ink (e.g., Opacode® S-1-17823 - a solvent based ink, commercially available from Colorcon, Inc. of West Point, PA.).

Another exemplary pharmaceutical composition comprises from about 5 wt% to about 50 wt% (e.g., from about 5 wt% to about 25 wt%, from about 15 wt% to about 40 wt%, or from about 30 wt% to about 50 wt%) of a solid dispersion, by weight of the composition, comprising from about 70 wt% to about 90 wt% of substantially amorphous Compound 1, by weight of the dispersion, and from about 30 wt% to about 10 wt% of a polymer, by weight of the dispersion; from about 25 wt% to about 50 wt% of a filler; from about 1 wt% to about 10 wt% of a disintegrant; from about 2 wt% to about 0.3 wt% of a surfactant; from about 5 wt% to about 50 wt% of a binder; from about 2 wt% to about 0.05 wt% of a glidant; and from about 2 wt% to about 0.1 wt% of a lubricant. Or, the pharmaceutical composition comprises from about 5 wt% to about 50 wt% (e.g., from about 5 wt% to about 25 wt%, from about 15 wt% to about 40 wt%, or from about 30 wt% to about 50 wt%) of a solid dispersion, by weight of the composition, comprising from about 70 wt% to about 90 wt% of amorphous Compound 1, by weight of the dispersion, and from about 30 wt% to about 10 wt% of a polymer, by weight of the dispersion; from about 25 wt% to about 50 wt% of a filler; from about 1 wt% to about 10 wt% of a disintegrant; from about 2 wt% to about 0.3 wt% of a surfactant; from about 5 wt% to about 50 wt% of a binder; from about 2 wt% to about 0.05 wt% of a glidant; and from about 2 wt% to about 0.1 wt% of a lubricant.

In another pharmaceutical composition of the present invention, a caplet shaped pharmaceutical tablet composition having an initial hardness of between about 6 and 16 Kp comprises about 34.1 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30.5 wt% of microcrystalline cellulose by weight of the composition; about 30.4 wt% of lactose by weight of the composition; about 3 wt% of sodium croscarmellose by weight of the composition; about 0.5 wt% of SLS by weight of the composition; about 0.5 wt% of colloidal silicon dioxide by weight of the composition; and about 1 wt% of magnesium stearate by weight of the composition. In some aspects, the caplet shaped pharmaceutical tablet composition contains 100 mg of Compound 1. In some further aspects, the caplet shaped pharmaceutical tablet composition comprises a colorant coated, a wax coating, and a printed logo or text. In some embodiments of this aspect, the caplet shaped pharmaceutical tablet includes a blue OPADRY® II coating and a water or solvent based ink logo or text. In some instances, the colorant coating is blue OPADRY® II. In some instances, the wax coating comprises Carnauba wax. In certain aspects, the ink for the printed logo or text is a solvent based ink. In some aspects, the caplet shaped pharmaceutical tablet composition contains 150 mg of Compound 1.

In still another pharmaceutical composition of the present invention, a pharmaceutical tablet composition having an initial hardness of between about 9 and 21 Kp comprises about 34.1 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30.5 wt% of microcrystalline cellulose by weight of the composition; about 30.4 wt% of lactose by weight of the composition; about 3 wt% of sodium croscarmellose by weight of the composition; about 0.5 wt% of SLS by weight of the composition; about 0.5 wt% of colloidal silicon dioxide by weight of the composition; and about 1 wt% of magnesium stearate by weight of the composition. In some embodiments, the caplet shaped pharmaceutical tablet composition contains 150 mg of Compound 1. In some aspects, the caplet shaped pharmaceutical tablet composition further comprises a colorant coated, a wax coating, and a printed logo or text. In some instances, the tablet includes a blue OPADRY® II coating and a water or solvent based ink logo or text. In still other instances, the wax coating comprises Carnauba wax. In some embodiments, the ink for the printed logo or text is a solvent based ink. In some aspects, the caplet shaped pharmaceutical tablet composition contains 100 mg of Compound 1.

In another pharmaceutical composition of the present invention, a pharmaceutical composition comprises about 34.1 wt% of a solid dispersion by weight of the composition, wherein the dispersion comprises about 80 wt% of substantially amorphous Compound 1 by weight of the dispersion, about 19.5 wt% of HPMCAS by weight of the dispersion, and about 0.5 wt% SLS by weight of the dispersion; about 30.5 wt% of microcrystalline cellulose by weight of the composition; about 30.4 wt% of lactose by weight of the composition; about 3 wt% of sodium croscarmellose by weight of the composition; about 0.5 wt% of SLS by weight of the composition; about 0.5 wt% of colloidal silicon dioxide by weight of the composition; and about 1 wt% of magnesium stearate by weight of the composition. In some aspects, the pharmaceutical tablet contains 100 mg of Compound 1. In other embodiments, the pharmaceutical composition contains 150 mg of Compound 1. In some further aspects, the pharmaceutical composition is formed as a tablet and comprises a colorant coated, a wax coating, and a printed logo or text. In some embodiments of this aspect, the pharmaceutical tablet includes a blue OPADRY® II coating and a water or solvent based ink logo or text. In some instances, the colorant coating is blue OPADRY® II. In some instances, the wax coating comprises Carnauba wax. In certain aspects, the ink for the printed logo or text is a solvent based ink.

Another aspect of the present invention provides a pharmaceutical composition consisting of a tablet that includes a CF potentiator API (e.g., a solid dispersion of N-[2,4-bis(1,1-dimethylethyl)-5-hydroxyphenyl]-1,4-dihydro-4-oxoquinoline 3-carboxamide) and other excipients (e.g., a filler, a disintegrant, a surfactant, a binder, a glidant, a colorant, a lubricant, or any combination thereof), each of which is described above and in the Examples below, wherein the tablet has a dissolution of at least about 50% (e.g., at least about 60%, at least about 70%, at least about 80%, at least about 90%, or at least about 99%) in about 30 minutes. In one example, the pharmaceutical composition consists of a tablet that includes a CF potentiator API (e.g., a solid dispersion of Compound 1) and other excipients (e.g., a filler, a disintegrant, a surfactant, a binder, a glidant, a colorant, a lubricant, or any combination thereof), each of which is described above and in the Examples below, wherein the tablet has a dissolution of from about 50% to about 100% (e.g., from about 55% to about 95% or from about 60% to about 90%) in about 30 minutes. In another example, the pharmaceutical composition consists of a tablet that comprises a solid dispersion comprising substantially amorphous or amorphous Compound 1 and HPMCAS; and, a filler, a disintegrant, a surfactant, a binder, a glidant, and a lubricant, wherein the tablet has a dissolution of at least about 50% (e.g., at least about 60%, at least about 70%, at least about 80%, at least about 90%, or at least about 99%) in about 30 minutes. In still another example, the pharmaceutical composition consists of a tablet that comprises a solid dispersion comprising substantially amorphous or amorphous Compound 1 and HPMCAS; and, a filler, a disintegrant, a surfactant, a binder, a glidant, and a lubricant, wherein the tablet has a dissolution of from about 50% to about 100% (e.g., from about 55% to about 95% or from about 60% to about 90%) in about 30 minutes.

In one embodiment, the tablet comprises a solid dispersion comprising at least about 100 mg, or at least 150 mg of substantially amorphous or amorphous Compound 1; and HPMCAS and SLS.

Dissolution can be measured with a standard USP Type II apparatus that employs a dissolution media of 0.6% sodium lauryl sulfate dissolved in 900 mL of DI water, stirring at about 50-75 rpm at a temperature of about 37 °C. A single experimental tablet is tested in each test vessel of the apparatus. Dissolution can also be measured with a standard USP Type II apparatus that employs a dissolution media of 0.7% sodium lauryl sulfate dissolved in 900 mL of 50 mM sodium phosphate buffer (pH 6.8), stirring at about 65 rpm at a temperature of about 37 °C. A single experimental tablet is tested in each test vessel of the apparatus. Dissolution can also be measured with a standard USP Type II apparatus that employs a dissolution media of 0.5% sodium lauryl sulfate dissolved in 900 mL of 50 mM sodium phosphate buffer (pH 6.8), stirring at about 65 rpm at a temperature of about 37 °C. A single experimental tablet is tested in each test vessel of the apparatus.

Another aspect of the present invention provides a pharmaceutical composition consisting of a tablet that comprises a CF potentiator API (e.g., a solid dispersion of Compound 1) and other excipients (e.g., a filler, a disintegrant, a surfactant, a binder, a glidant, a colorant, a lubricant, or any combination thereof), each of which is described above and in the Examples below, wherein the tablet has a hardness of at least about 5 Kp. In one example, the pharmaceutical composition consists of a tablet that comprises a CF potentiator API (e.g., a solid dispersion of Compound 1) and other excipients (e.g., a filler, a disintegrant, a surfactant, a binder, a glidant, a colorant, a lubricant, or any combination thereof), each of which is described above and in the Examples below, wherein the tablet has a hardness of at least about 5 Kp (e.g., at least about 5.5, at least about 6 Kp, or at least about 7 Kp).

### IV.A.2.b. Preparation of Compound 1 Tablet and SDD Formulation

Another aspect of the present invention provides a method of producing a pharmaceutical composition comprising providing an admixture of a solid dispersion of substantially amorphous or amorphous Compound 1, a binder, a glidant, a surfactant, a lubricant, a disintegrant, and a filler, and compressing the admixture into a tablet having a dissolution of at least about 50% in about 30 minutes.

Each of the ingredients of this admixture is described above and in the Examples below. Furthermore, the admixture can comprise optional additives such as one or more colorants, one or more flavors, and/or one or more fragrances as described above and in the Examples below. And, the relative concentrations (e.g., wt%) of each of these ingredients (and any optional additives) in the admixture is also presented above and in the Examples below. The ingredients constituting the admixture can be provided sequentially or in any combination of additions; and, the ingredients or combination of ingredients can be provided in any order. In one embodiment the lubricant is the last component added to the admixture.

In one embodiment, the admixture comprises a solid dispersion of substantially amorphous Compound 1, a binder, a glidant, a surfactant, a lubricant, a disintegrant, and a filler, wherein each of these ingredients is provided in a powder form (e.g., provided as particles having a mean diameter, measured by light scattering, of 250 μm or less (e.g., 150 μm or less, 100 μm or less, 50 μm or less, 45 μm or less, 40 μm or less, or 35 μm or less)). For instance, the admixture comprises a solid dispersion of amorphous Compound 1, a binder, a glidant, a surfactant, a lubricant, a disintegrant, and a filler, wherein each of these ingredients is provided in a powder form (e.g., provided as particles having a mean diameter, measured by light scattering, of 250 μm or less (e.g., 150 μm or less, 100 μm or less, 50 μm or less, 45 μm or less, 40 μm or less, or 35 μm or less)).

In another embodiment, the admixture comprises a solid dispersion of substantially amorphous Compound 1, a binder, a glidant, a surfactant, a lubricant, a disintegrant, and a filler, wherein each of these ingredients is substantially

free of water. Each of the ingredients comprises less than 5 wt% (e.g., less than 2 wt%, less than 1 wt%, less than 0.75 wt%, less than 0.5 wt%, or less than 0.25 wt%) of water by weight of the ingredient. For instance, the admixture comprises a solid dispersion of amorphous Compound 1, a binder, a glidant, a surfactant, a lubricant, a disintegrant, and a filler, wherein each of these ingredients is substantially free of water. Each of the ingredients comprises less than 5 wt% (e.g., less than 2 wt%, less than 1 wt%, less than 0.75 wt%, less than 0.5 wt%, or less than 0.25 wt%) of water by weight of the ingredient.

In another embodiment, compressing the admixture into a tablet is accomplished by filling a form (e.g., a mold) with the admixture and applying pressure to admixture. This can be accomplished using a die press or other similar apparatus. It is also noted that the application of pressure to the admixture in the form can be repeated using the same pressure during each compression or using different pressures during the compressions. In another example, the admixture is compressed using a die press that applies sufficient pressure to form a tablet having a dissolution of about 50% or more at about 30 minutes (e.g., about 55% or more at about 30 minutes or about 60% or more at about 30 minutes). For instance, the admixture is compressed using a die press to produce a tablet hardness of at least about 5 Kp (at least about 5.5 Kp, at least about 6 Kp, at least about 7 Kp, at least about 11 Kp, or at least 21Kp). In some instances, the admixture is compressed to produce a tablet hardness of between about 6 and 21 Kp.

In some embodiments, tablets comprising a pharmaceutical composition as described herein can be coated with about 3.0 wt% of a film coating comprising a colorant by weight of the tablet. In certain instances, the colorant suspension or solution used to coat the tablets comprises about 20%w/w of solids by weight of the colorant suspension or solution. In still further instances, the coated tablets can be labeled with a logo, other image or text.

In another embodiment, the method of producing a pharmaceutical composition comprises providing an admixture of a solid dispersion of substantially amorphous Compound 1, a binder, a glidant, a surfactant, a lubricant, a disintegrant, and a filler; mixing the admixture until the admixture is substantially homogenous, and compressing the admixture into a tablet as described above or in the Examples below. Or, the method of producing a pharmaceutical composition comprises providing an admixture of a solid dispersion of amorphous Compound 1, a binder, a glidant, a surfactant, a lubricant, a disintegrant, and a filler; mixing the admixture until the admixture is substantially homogenous, and compressing the admixture into a tablet as described above or in the Examples below. For example, the admixture is mixed by stirring, blending, shaking, or the like using hand mixing, a mixer, a blender, any combination thereof, or the like. When ingredients or combinations of ingredients are added sequentially, mixing can occur between successive additions, continuously throughout the ingredient addition, after the addition of all of the ingredients or combinations of ingredients, or any combination thereof. The admixture is mixed until it has a substantially homogenous composition.

**Intermediate F**

A solvent system of MEK and DI water, formulated according to the ratio 90 wt% MEK / 10 wt% DI water, was heated to a temperature of 20 - 30 °C in a reactor, equipped with a magnetic stirrer and thermal circuit. Into this solvent system, hypromellose acetate succinate polymer (HPMCAS)(HG grade), SLS, and Compound 1 were added according to the ratio 19.5 wt% hypromellose acetate succinate / 0.5 wt% SLS / 80 wt% Compound 1. The resulting mixture contained 10.5 wt% solids. The actual amounts of ingredients and solvents used to generate this mixture are recited in Table 1-F1.

**Table 1-F1: Solid Spray Dispersion Ingredients for Intermediate F.**

| | Units | Batch |
|---|---|---|
| Compound 1 | Kg | 70.0 |
| HPMCAS | Kg | 17.1 |
| SLS | Kg | 0.438 |
| **Total Solids** | **Kg** | **87.5** |
| MEK | Kg | 671 |
| Water | Kg | 74.6 |
| **Total Solvents** | **Kg** | **746** |
| **Total Spray Solution Weight** | **Kg** | **833** |

The mixture temperature was adjusted to a range of 20 - 45 °C and mixed until it was substantially homogenous and all components were substantially dissolved.

A spray drier, Niro PSD4 Commercial Spray Dryer, fitted with pressure nozzle (Spray Systems Maximum Passage series SK-MFP having orifice/core size 54/21) equipped with anti-bearding cap, was used under normal spray drying mode, following the dry spray process parameters recited in Table 1-F2.

**Table 1-F2: Dry Spray Process Parameters Used to Generate Intermediate F.**

| **Parameter** | **Value** |
|---|---|
| Feed Pressure | 20 bar |
| Feed Flow Rate | 92 - 100 Kg/hr |
| Inlet Temperature | 93 - 99 °C |
| Outlet Temperature | 53 - 57 °C |
| Vacuum Dryer Temperature | 80 °C for 2 hours then 110 °C (+/-5 °C) |
| Vacuum Drying Time | 20 - 24 hours |

A high efficiency cyclone separated the wet product from the spray gas and solvent vapors. The wet product contained 8.5 - 9.7% MEK and 0.56 - 0.83% Water and had a mean particle size of 17 - 19um and a bulk density of 0.27 - 0.33g/cc. The wet product was transferred to a 4000L stainless steel double cone vacuum dryer for drying to reduce residual solvents to a level of less than about 5000 ppm and to generate dry Intermediate F. The dry Intermediate F contained <0.03% MEK and 0.3% Water.

**Intermediate G**

A solvent system of MEK and DI water, formulated according to the ratio 90 wt% MEK / 10 wt% DI water, was heated to a temperature of 20 - 30 °C in a reactor, equipped with a magnetic stirrer and thermal circuit. Into this solvent system, hypromellose acetate succinate polymer (HPMCAS)(HG grade), SLS, and Compound 1 were added according to the ratio 19.5 wt % hypromellose acetate succinate / 0.5 wt % SLS / 80 wt% Compound 1. The resulting mixture contained 10.5 wt% solids. The actual amounts of ingredients and solvents used to generate this mixture are recited in Table 1-G1.

**Table 1-G1: Solid Spray Dispersion Ingredients for Intermediate G.**

| | Units | Batch |
|---|---|---|
| Compound 1 | Kg | 24.0 |
| HPMCAS | Kg | 5.85 |
| SLS | Kg | 0.15 |
| **Total Solids** | **Kg** | **30.0** |
| MEK | Kg | 230.1 |
| Water | Kg | 25.6 |
| **Total Solvents** | **Kg** | **255.7** |
| **Total Spray Solution Weight** | **Kg** | **285.7** |

The mixture temperature was adjusted to a range of 20 - 45 °C and mixed until it was substantially homogenous and all components were substantially dissolved.

A spray drier, Niro Production Minor Spray Dryer, fitted with pressure nozzle (Spray Systems Maximum Passage series SK-MFP having orifice size 72) was used under normal spray drying mode, following the dry spray process parameters recited in Table 1-G2.

**Table 1-G2: Dry Spray Process Parameters Used to Generate Intermediate G.**

| **Parameter** | **Value** |
|---|---|
| Feed Pressure | 33 bar |

(continued)

| Parameter | Value |
|---|---|
| Feed Flow Rate | 18 - 24 Kg/hr |
| Inlet Temperature | 82 - 84 °C |
| Outlet Temperature | 44 - 46 °C |
| Vacuum Dryer Temperature | 80 °C for 2 hours then 110 °C (+/-5 °C) |
| Vacuum Drying Time | 48 hours |

A high efficiency cyclone separated the wet product from the spray gas and solvent vapors. The wet product contained 10.8% MEK and 0.7% Water and had a mean particle size of 19um and a bulk density of 0.32g/cc. The wet product was transferred to a 4000L stainless steel double cone vacuum dryer for drying to reduce residual solvents to a level of less than about 5000 ppm and to generate dry Intermediate. The dry Intermediate G contained <0.05% MEK and 0.7% Water.

**Intermediate H**

A solvent system of MEK and DI water, formulated according to the ratio 90 wt% MEK / 10 wt% DI water, was heated to a temperature of 20 - 30 °C in a reactor, equipped with a magnetic stirrer and thermal circuit. Into this solvent system, hypromellose acetate succinate polymer (HPMCAS)(HG grade), SLS, and Compound 1 were added according to the ratio 19.5 wt % hypromellose acetate succinate / 0.5 wt % SLS / 80 wt% Compound 1. The actual amounts of ingredients and solvents used to generate this mixture are recited in Table 1-H1:

**Table 1-H1: Solid Spray Dispersion Ingredients for Intermediate H.**

| | Units | Batch |
|---|---|---|
| Compound 1 | Kg | 56.0 |
| HPMCAS | Kg | 13.65 |
| SLS | Kg | 0.35 |
| **Total Solids** | **Kg** | **70.0** |
| MEK | Kg | 509.73 |
| Water | Kg | 56.64 |
| **Total Solvents** | **Kg** | **566.40** |
| **Total Spray Solution Weight** | **Kg** | **636.40** |

The mixture temperature was adjusted to a range of 20 - 30 °C and mixed until it was substantially homogenous and all components were substantially dissolved.

A spray drier, Niro Production Minor Spray Dryer, fitted with pressure nozzle (Spray Systems Maximum Passage series SK-MFP having orifice size # 52 or # 54, e.g., about 1.39-1.62 mm) was used under normal spray drying mode, following the dry spray process parameters recited in Table 1-H2.

**Table 1-H2: Dry Spray Process Parameters Used to Generate Intermediate H.**

| Parameter | Value |
|---|---|
| Feed Pressure | 20-50 bar |
| Feed Flow Rate | 18 - 24 Kg/hr |
| Inlet Temperature | -7 to 7 °C |
| Outlet Temperature | 30 - 70 °C |

A high efficiency cyclone separated the wet product from the spray gas and solvent vapors. The wet product

contained approximately 10.8% MEK and 0.7% Water and had a mean particle size of about 19μm and a bulk density of about 0.33g/cc.

An inertial cyclone is used to separate the spray dried intermediate from the process gas and solvent vapors. Particle size is monitored on-line. The spray dried intermediate is collected in an intermediate bulk container. The process gas and solvent vapors are passed through a filter bag to collect the fine particles not separated by the cyclone. The resultant gas is condensed to remove process vapors and recycled back to the heater and spray dryer. The spray dried intermediate will be stored at less than 30°C, if secondary drying will occur in less than 24 hours or between 2-8°C, if secondary drying will occur in more than 24 hours.

Secondary drying occurs by charging a 4000-L biconical dryer having a jacket temperature between about 20-30°C with the spray dried intermediate. The vacuum pressure, jacket temperature, and nitrogen bleed are set at between about -0.8 psig and about -1.0 psig, between about 80 - 120°C, and between about 0.5 - 8.0 $m^3$/h, respectively. Agitation is set at 1rpm. Bulk samples of the spray dried intermediate are tested for MEK (GC), every 4 hours until dry. The MEK drying rate is monitored on-line by GC-MS, calibrated for MEK concentration. Upon reaching a plateau in the drying of the residual MEK, heating in the biconical dryer is discontinued while continuing rotation until the spray dried intermediate reaches a temperature less than or equal to 50°C.

Although Intermediates F through H are described above as being formed, in part, by admixing the solid spray dispersion ingredients with application of heat to form a homogeneuos mixture, the solid spray dispersion ingredients can also be mixed without application of heat to form a mixture of the solid spray dispersion ingredients.

**Tablets:**

**Example 8. Exemplary Tablet 9 (Formulated with HPMCAS Polymer to have 100 mg of Compound 1)**

A batch of caplet-shaped tablets was formulated to have about 100 mg of Compound 1 per tablet using the amounts of ingredients recited in Table 1-8.

**Table 1-8: Ingredients for Exemplary Tablet 9.**

| Tablet Formulation | Percent Dose %Wt./Wt. | Dose (mg) | Batch (g) |
|---|---|---|---|
| Intermediate F | 34.09% | 125.1 | 23.86 |
| Microcrystalline cellulose | 30.51% | 112.0 | 21.36 |
| Lactose | 30.40% | 111.6 | 21.28 |
| Sodium croscarmellose | 3.000% | 11.01 | 2.100 |
| SLS | 0.500% | 1.835 | 0.3500 |
| Colloidal silicon dioxide | 0.500% | 1.835 | 0.3500 |
| Magnesium stearate | 1.000% | 3.670 | 0.7000 |
| **Total** | **100%** | **367** | **70** |

The colloidal silicon dioxide (Cabot Cab-O-Sil® M-5P Fumed Silicon Dioxide) and the microcrystalline cellulose (FMC MCC Avicel® PH102) were passed through a 30 mesh screen.

The sodium croscarmellose (FMC Ac-Di-Sol®), SLS, Intermediate F, and lactose (Foremost FastFlo® Lactose #316) were also passed, individually in the preceding order, through the same 30 mesh screen. A nitrogen purge was used when screening Intermediate F. The screened components were loaded into a 10 cubic feet V-blender, which was purged with nitrogen, and blended for about 180 (+/- 10) inversions.

The Magnesium Stearate was filtered through a 40 mesh screen sieve into the blending container and mixed to provide about 54 inversions.

The resulting mixture was compressed into tablets using a fully tooled 36 Fette 2090 press with 0.568" x 0.2885" caplet type B tooling set to produce a tablet having an initial target hardness of about 10 Kp ± 20%.

**Example 9. Exemplary Tablet 10 (Tablet 9 with Spray-Coating)**

A batch of caplet-shaped tablets from Example 8 was spray-coated with OPADRY® II (Blue, Colorcon) to a weight gain of about 3.0% using a 24" coating pan configured with the parameters in Table 1-9 followed by wax coating and then printing using Opacode® S-1-17823 (Solvent based Black, Colorcon).

**Table 1-9: Spray-Coating Process Parameters**

| Coating Parameters 24" Pan | Target |
|---|---|
| Pan Load (kg) | 14 |
| Inlet Temperature (°C)* | * |
| Pan Speed (rpm) | 10 |
| Jog Time (sec) | |
| # of Spray Guns | 2 |
| Solids Content (%w/w) | 20 |
| Gun to Bed Distance (inches) | 6 |
| Inlet Air Flow (cfm) | 300 |
| Spray Rate (g/min) | 35 |
| Exhaust Temperature (°C) | 50 |
| Atomization Pressure (psi) | 42 |

* Inlet temperature is monitored to achieve target exhaust temperature. Initial inlet temperature should be set at about 75°C to achieve target exhaust temp.

The OPADRY® II suspension was prepared by measuring an amount of deionized water which when combined with OPADRY® II would produce a total solids content of 20 %w/w. The water is mixed to a vortex followed by addition of OPADRY® II over a period of approximately 5 minutes. Once the OPADRY® II powder was wetted, mixing was continued to ensure that all solid material is well-dispersed. The suspension is then charged into a Thomas 24" pan coating instrument using coating conditions outlined in Table 1-9.

Uncoated tablets are placed into the coating pan and pre-warmed. The inlet was increased from room temperature to about 55°C and then increased as necessary to provide the exhaust temperature in Table 1-9. The coating process was performed with 20% w/w OPADRY® II (85 Series Blue) coating dispersion to obtain a target weight gain of about 3%. The coated tablets were then allowed to tumble for about 2 minutes without spraying. The bed temperature was then allowed to cool to about 35°C.

Upon cooling, the Carnauba wax powder was weighed out in the amount of about 0.01% w/w of the starting tablet core weight. With the air flow off, the carnauba wax powder was sprinkled evenly on the tablet bed. The pan bed was turned on to the speed indicated in Table 1-9. After 5 minutes, the air flow was turned on (without heating) to the setting indicated in Table 1-9. After about one minute the air flow and pan were turned off.

Once coated with OPADRY® II, the tablets are then labeled using a Hartnett Delta tablet printer charged with Opacode® S-1-17823.

**Example 10. Exemplary Tablet 11 (Formulated with HPMCAS Polymer to have 150 mg of Compound 1)**

A batch of caplet-shaped tablets was formulated to have about 150 mg of Compound 1 per tablet using the amounts of ingredients recited in Table 1-10.

**Table 1-10: Ingredients for Exemplary Tablet 11.**

| Tablet Formulation | Percent Dose %Wt./Wt. | Dose (mg) | Batch (g) |
|---|---|---|---|
| Intermediate F | 34.09% | 187.5 | 23.86 |
| Microcrystalline cellulose | 30.51% | 167.8 | 21.36 |
| Lactose | 30.40% | 167.2 | 21.28 |
| Sodium croscarmellose | 3.000% | 16.50 | 2.100 |
| SLS | 0.500% | 2.750 | 0.3500 |
| Colloidal silicon dioxide | 0.500% | 2.750 | 0.3500 |
| Magnesium stearate | 1.000% | 5.500 | 0.7000 |
| **Total** | **100%** | **550** | **70** |

The colloidal silicon dioxide (Cabot Cab-O-Sil® M-5P Fumed Silicon Dioxide) and the microcrystalline cellulose

(FMC MCC Avicel® PH102) were passed through a 30 mesh screen.

The sodium croscarmellose (FMC Ac-Di-Sol®), SLS, Intermediate F, and lactose (Foremost FastFlo® Lactose #316) were also passed, individually in the preceding order, through the same 30 mesh screen. A nitrogen purge was used when screening Intermediate F. The screened components were loaded into a 10 cubic feet V-blender, which was purged with nitrogen, and blended for about 180 (+/- 10) inversions.

The Magnesium Stearate was filtered through a 40 mesh screen sieve into the blending container and mixed to provide about 54 inversions.

The resulting mixture was compressed into tablets using a fully tooled 36 Fette 2090 press with 0.568" x 0.2885" caplet type B tooling set to produce a tablet having an initial target hardness of about 10 Kp ± 20%.

**Example 11. Exemplary Tablet 12 (Tablet 11 with Spray-Coating)**

A batch of caplet-shaped tablets from Example 10 was spray-coated with OPADRY® II (Blue, Colorcon) to a weight gain of about 3.0% using a 24" coating pan configured with the parameters in Table 1-11 followed by wax coating and then printing using Opacode® S-1-17823 (Solvent based Black, Colorcon).

**Table 1-11: Spray-Coating Process Parameters**

| Coating Parameters 24" Pan | Target |
|---|---|
| Pan Load (kg) | 14 |
| Inlet Temperature (°C)* | * |
| Pan Speed (rpm) | 10 |
| Jog Time (sec) | 2-5 sec every 60 sec |
| # of Spray Guns | 2 |
| Solids Content (%w/w) | 20 |
| Gun to Bed Distance (inches) | 6 |
| Inlet Air Flow (cfm) | 300 |
| Spray Rate (g/min) | 35 |
| Exhaust Temperature (°C) | 50 |
| Atomization Pressure (psi) | 42 |

* Inlet temperature is monitored to achieve target exhaust temperature. Initial inlet temperature should be set at about 75°C to achieve target exhaust temp.

The OPADRY® II suspension was prepared by measuring an amount of deionized water which when combined with OPADRY® II would produce a total solids content of 20 %w/w. The water is mixed to a vortex followed by addition of OPADRY® II over a period of approximately 5 minutes. Once the OPADRY® II powder was wetted, mixing was continued to ensure that all solid material is well-dispersed. The suspension is then charged into a Thomas 24" pan coating instrument using coating conditions outlined in Table 1-11.

Uncoated tablets are placed into the coating pan and pre-warmed. The inlet was increased from room temperature to about 55°C and then increased as necessary to provide the exhaust temperature in Table 1-11. The coating process was performed with 20% w/w OPADRY® II (85 Series Blue) coating dispersion to obtain a target weight gain of about 3%. The coated tablets were then allowed to tumble for about 2 minutes without spraying. The bed temperature was then allowed to cool to about 35°C.

Upon cooling, the Carnauba wax powder was weighed out in the amount of about 0.01% w/w of the starting tablet core weight. With the air flow off, the carnauba wax powder was sprinkled evenly on the tablet bed. The pan bed was turned on to the speed indicated in Table 1-11. After 5 minutes, the air flow was turned on (without heating) to the setting indicated in Table 1-11. After about one minute the air flow and pan were turned off.

Once coated with OPADRY® II, the tablets are then labeled using a Hartnett Delta tablet printer charged with Opacode® S-1-17823.

**Example 12. Exemplary Tablet 13 (Formulated with HPMCAS Polymer to have 150 mg of Compound 1)**

A batch of caplet-shaped tablets is formulated to have about 150 mg of Compound 1 per tablet using the amounts of ingredients recited in Table 1-12.

**Table 1-12: Ingredients for Exemplary Tablet 13.**

| Tablet Formulation | Percent Dose %Wt./Wt. |
|---|---|
| Intermediate H | 34.1% |
| Microcrystalline cellulose | 30.5% |
| Lactose | 30.4% |
| Sodium croscarmellose | 3.000% |
| SLS | 0.500% |
| Colloidal silicon dioxide | 0.500% |
| Magnesium stearate | 1.000% |
| **Total** | **100%** |

The colloidal silicon dioxide (Cabot Cab-O-Sil® M-5P Fumed Silicon Dioxide) and the microcrystalline cellulose (FMC MCC Avicel® PH102) are passed through a 30 mesh screen.

The sodium croscarmellose (FMC Ac-Di-Sol®), SLS, Intermediate H, and lactose (Foremost FastFlo® Lactose #316) are also passed, individually in the preceding order, through the same 30 mesh screen. A nitrogen purge is used when screening Intermediate H. The screened components are loaded into a 10 cubic feet V-blender, which is purged with nitrogen, and blended for about 180 (+/- 10) inversions.

The Magnesium Stearate is filtered through a 40 mesh screen sieve into the blending container and mixed to provide about 54 inversions.

The resulting mixture is compressed into tablets using a fully tooled 36 Fette 2090 press with 0.568" x 0.2885" caplet type B tooling set to produce a tablet having an initial target hardness of about 10 Kp ± 20%.

**Example 13. Exemplary Tablet 14 (Tablet 13 with Spray-Coating)**

A batch of caplet-shaped tablets from Example 12 is spray-coated with OPADRY® II (Blue, Colorcon) to a weight gain of about 3.0% using a Thomas 48" coating pan configured with the parameters in Table 1-13 followed by wax coating and then printing using Opacode® S-1-17823 (Solvent based Black, Colorcon).

**Table 1-13: Spray-Coating Process Parameters**

| Coating Parameters 48" Pan | Target |
|---|---|
| Pan Load (kg) | up to 120 |
| Inlet Temperature (°C)* | * |
| # of Spray Guns | 4 |
| Solids Content (%w/w) | 20 |
| Gun to Bed Distance (inches) | 7-7.5 |
| Inlet Air Flow (cfm) | 1050-2400 |
| Spray Rate (ml/min) | 203-290 |
| Exhaust Temperature (°C) | 40-65 |
| Atomization Pressure (slpm) | 145 |

* Inlet temperature is monitored to achieve target exhaust temperature. Initial inlet temperature should be set at about 50-75°C to achieve target exhaust temp.

The OPADRY® II suspension is prepared by measuring an amount of deionized water which when combined with OPADRY® II would produce a total solids content of 20 %w/w. The water is mixed to a vortex followed by addition of OPADRY® II over a period of approximately 5 minutes. Once the OPADRY® II powder is wetted, mixing is continued to ensure that all solid material is well-dispersed. The suspension is then charged into a Thomas 48" pan coating instrument using coating conditions outlined in Table 1-13. In other examples, the suspension can be coated with a Thomas 24" pan coating instrument.

Uncoated tablets are placed into the coating pan and pre-warmed. The inlet is increased from room temperature to about 55°C and then increased as necessary to provide the exhaust temperature in Table 1-13. The coating process

is performed with 20% w/w OPADRY® II (85 Series Blue) coating dispersion to obtain a target weight gain of about 3%. The coated tablets are then allowed to tumble for about 2 minutes without spraying. The bed temperature is then allowed to cool to about 35°C.

Upon cooling, the Carnauba wax powder is weighed out in the amount of about 0.01% w/w of the starting tablet core weight. With the air flow off, the carnauba wax powder is sprinkled evenly on the tablet bed. The pan bed is turned on to the speed indicated in Table 1-13. After 5 minutes, the air flow is turned on (without heating) to the setting indicated in Table 1-13. After about one minute the air flow and pan is turned off.

Once coated with OPADRY® II, the tablets are then labeled using a Hartnett Delta tablet printer charged with Opacode® S-1-17823.

Another aspect of the present invention provides a method of producing a pharmaceutical composition comprising providing an admixture of a solid dispersion of substantially amorphous or amorphous Compound 1, a binder, a glidant, a surfactant, a lubricant, a disintegrant, and a filler, and compressing the admixture into a tablet having a dissolution of at least about 50% in about 30 minutes.

Each of the ingredients of this admixture is described above and in the Examples below. Furthermore, the admixture can comprise optional additives such as one or more colorants, one or more flavors, and/or one or more fragrances as described above and in the Examples below. And, the relative concentrations (e.g., wt%) of each of these ingredients (and any optional additives) in the admixture is also presented above and in the Examples below. The ingredients constituting the admixture can be provided sequentially or in any combination of additions; and, the ingredients or combination of ingredients can be provided in any order. In one embodiment the lubricant is the last component added to the admixture.

In one embodiment, the admixture comprises a solid dispersion of substantially amorphous Compound 1, a binder, a glidant, a surfactant, a lubricant, a disintegrant, and a filler, wherein each of these ingredients is provided in a powder form (e.g., provided as particles having a mean diameter, measured by light scattering, of 250 $\mu$m or less (e.g., 150 $\mu$m or less, 100 $\mu$m or less, 50 $\mu$m or less, 45 $\mu$m or less, 40 $\mu$m or less, or 35 $\mu$m or less)). For instance, the admixture comprises a solid dispersion of amorphous Compound 1, a binder, a glidant, a surfactant, a lubricant, a disintegrant, and a filler, wherein each of these ingredients is provided in a powder form (e.g., provided as particles having a mean diameter, measured by light scattering, of 250 $\mu$m or less (e.g., 150 $\mu$m or less, 100 $\mu$m or less, 50 $\mu$m or less, 45 $\mu$m or less, 40 $\mu$m or less, or 35 $\mu$m or less)).

In another embodiment, the admixture comprises a solid dispersion of substantially amorphous Compound 1, a binder, a glidant, a surfactant, a lubricant, a disintegrant, and a filler, wherein each of these ingredients is substantially free of water. Each of the ingredients comprises less than 5 wt% (e.g., less than 2 wt%, less than 1 wt%, less than 0.75 wt%, less than 0.5 wt%, or less than 0.25 wt%) of water by weight of the ingredient. For instance, the admixture comprises a solid dispersion of amorphous Compound 1, a binder, a glidant, a surfactant, a lubricant, a disintegrant, and a filler, wherein each of these ingredients is substantially free of water. Each of the ingredients comprises less than 5 wt% (e.g., less than 2 wt%, less than 1 wt%, less than 0.75 wt%, less than 0.5 wt%, or less than 0.25 wt%) of water by weight of the ingredient.

In another embodiment, compressing the admixture into a tablet is accomplished by filling a form (e.g., a mold) with the admixture and applying pressure to admixture. This can be accomplished using a die press or other similar apparatus. It is also noted that the application of pressure to the admixture in the form can be repeated using the same pressure during each compression or using different pressures during the compressions. In another example, the admixture is compressed using a die press that applies sufficient pressure to form a tablet having a dissolution of about 50% or more at about 30 minutes (e.g., about 55% or more at about 30 minutes or about 60% or more at about 30 minutes). For instance, the admixture is compressed using a die press to produce a tablet hardness of at least about 5 Kp (at least about 5.5 Kp, at least about 6 Kp, at least about 7 Kp, at least about 11 Kp, or at least 21Kp). In some instances, the admixture is compressed to produce a tablet hardness of between about 6 and 21 Kp.

In some embodiments, tablets comprising a pharmaceutical composition as described herein can be coated with about 3.0 wt% of a film coating comprising a colorant by weight of the tablet. In certain instances, the colorant suspension or solution used to coat the tablets comprises about 20%w/w of solids by weight of the colorant suspension or solution. In still further instances, the coated tablets can be labeled with a logo, other image or text.

In another embodiment, the method of producing a pharmaceutical composition comprises providing an admixture of a solid dispersion of substantially amorphous Compound 1, a binder, a glidant, a surfactant, a lubricant, a disintegrant, and a filler; mixing the admixture until the admixture is substantially homogenous, and compressing the admixture into a tablet as described above or in the Examples below. Or, the method of producing a pharmaceutical composition comprises providing an admixture of a solid dispersion of amorphous Compound 1, a binder, a glidant, a surfactant, a lubricant, a disintegrant, and a filler; mixing the admixture until the admixture is substantially homogenous, and compressing the admixture into a tablet as described above or in the Examples below. For example, the admixture is mixed by stirring, blending, shaking, or the like using hand mixing, a mixer, a blender, any combination thereof, or the like. When ingredients or combinations of ingredients are added sequentially, mixing can occur between successive

additions, continuously throughout the ingredient addition, after the addition of all of the ingredients or combinations of ingredients, or any combination thereof. The admixture is mixed until it has a substantially homogenous composition.

### IV.A.2.c. Administration of Compound 1 Tablet and SDD Formulation

Another aspect of the present invention provides a method of administering a pharmaceutical composition by orally administering to a patient at least once per day the composition comprising a solid dispersion of substantially amorphous or amorphous Compound 1, in which the solid dispersion comprises at least about 100mg of substantially amorphous or amorphous Compound 1.

Another aspect of the present invention provides a method of administering a pharmaceutical composition by orally administering to a patient at least once per day the composition comprising a solid dispersion of substantially amorphous or amorphous Compound 1, in which the solid dispersion comprises at least about 150 mg of substantially amorphous or amorphous Compound 1.

Another aspect of the present invention provides a method of administering a pharmaceutical composition by orally administering to a patient twice per day the composition comprising a solid dispersion of substantially amorphous or amorphous Compound 1, in which the solid dispersion comprises at least about 100 mg of substantially amorphous or amorphous Compound 1.

Another aspect of the present invention provides a method of administering a pharmaceutical composition by orally administering to a patient twice per day the composition comprising a solid dispersion of substantially amorphous or amorphous Compound 1, in which the solid dispersion comprises at least about 150 mg of substantially amorphous or amorphous Compound 1.

Another aspect of the present invention provides a method of administering a pharmaceutical composition by orally administering to a patient once every 12 hours day. The composition comprising a solid dispersion of substantially amorphous or amorphous Compound 1, in which the solid dispersion comprises at least about 100 mg of substantially amorphous or amorphous Compound 1.

Another aspect of the present invention provides a method of administering a pharmaceutical composition by orally administering to a patient once every 12 hours. The composition comprising a solid dispersion of substantially amorphous or amorphous Compound 1, in which the solid dispersion comprises at least about 150 mg of substantially amorphous or amorphous Compound 1.

In still other aspects of the present invention, a pharmaceutical composition as described herein is orally administered to a patient once every 24 hours.

Another aspect of the present invention provides a method of administering a pharmaceutical composition by orally administering to a patient once per day the composition comprising a solid dispersion of substantially amorphous or amorphous Compound 1, in which the solid dispersion comprises at least about 100 mg of substantially amorphous or amorphous Compound 1.

Another aspect of the present invention provides a method of administering a pharmaceutical composition by orally administering to a patient once per day the composition comprising a solid dispersion of substantially amorphous or amorphous Compound 1, in which the solid dispersion comprises at least about 150 mg of substantially amorphous or amorphous Compound 1.

In some embodiments, the present invention provides a method of administering a pharmaceutical composition comprising orally administering to a patient at least one tablet comprising:

a. a solid dispersion comprising about 100 mg of substantially amorphous or amorphous Compound 1 and HPMCAS;
b. a filler;
c. a disintegrant;
d. a surfactant;
e. a binder;
f. a glidant; and
g. a lubricant.

In some embodiments, the present invention provides a method of administering a pharmaceutical composition comprising orally administering to a patient at least one tablet comprising:

a. a solid dispersion comprising about 150 mg of substantially amorphous or amorphous Compound 1 and HPMCAS;
b. a filler;
c. a disintegrant;
d. a surfactant;
e. a binder;

f. a glidant; and
g. a lubricant.

In some embodiments, the present invention provides for a method of orally administering the pharmaceutical composition described herein once a day. In other embodiments, the present invention provides for a method of orally administering the pharmaceutical composition described herein twice a day.

Another aspect of the present invention provides a method of administering a pharmaceutical composition by orally administering to a patient at least once per day at least one tablet comprising a solid dispersion of substantially amorphous or amorphous Compound 1, a filler, a binder, a glidant, a disintegrant, a surfactant, and a lubricant, in which the solid dispersion comprises at least about 100 mg of substantially amorphous or amorphous Compound 1. In some embodiments, the tablet is orally administered to the patient once per day. In another method, the administration comprises orally administering to a patient twice per day at least one tablet comprising a solid dispersion of substantially amorphous or amorphous Compound 1, a filler, a binder, a glidant, a disintegrant, a surfactant, and a lubricant, in which the solid dispersion contains at least about 100 mg of substantially amorphous or amorphous Compound 1. Other tablets useful in this method comprise a solid dispersion containing at least about 150 mg of substantially amorphous or amorphous Compound 1. In another method, the administration includes orally administering to a patient twice per day at least one tablet comprising a solid dispersion of substantially amorphous or amorphous Compound 1, a filler, a binder, a glidant, a disintegrant, a surfactant, and a lubricant, in which the solid dispersion contains at least about 150 mg of substantially amorphous or amorphous Compound 1.

In another embodiment, the method of administering a pharmaceutical composition includes orally administering to a patient once per day at least one tablet comprising a pharmaceutical composition containing a solid dispersion of Compound 1, a filler, a binder, a glidant, a disintegrant, a surfactant, and a lubricant, each of which is described above and in the Examples below, wherein the solid dispersion comprises at least about 100 mg, or at least about 150 mg) of substantially amorphous Compound 1 or amorphous Compound 1. For example, the method of administering a pharmaceutical composition includes orally administering to a patient once per day one tablet comprising a pharmaceutical composition containing a solid dispersion of Compound 1, a filler, a binder, a glidant, a disintegrant, a surfactant, and a lubricant, wherein the solid dispersion comprises at least 100 mg, or at least 150 mg of substantially amorphous Compound 1 or amorphous Compound 1.

In another embodiment, the method of administering a pharmaceutical composition includes orally administering to a patient twice per day one tablet comprising a pharmaceutical composition containing a solid dispersion of Compound 1, a filler, a binder, a glidant, a disintegrant, a surfactant, and a lubricant, wherein the solid dispersion comprises at least 100 mg or at least 150 mg of substantially amorphous Compound 1 or amorphous Compound 1.

In one embodiment, the method of administering a pharmaceutical composition includes orally administering to a patient a formulation comprising from about 25 mg to about 300 mg of Compound 1. In one embodiment, the method of administering a pharmaceutical composition includes orally administering to a patient one or more tablets, each tablet comprising about 100 mg, about 150 mg, or about 250 mg of Compound 1. In some embodiments, the method includes administering a tablet comprising about 250 mg of Compound 1. In some embodiments, the method includes administering a tablet comprising about 150 mg of Compound 1 and a tablet comprising about 100 mg of Compound 1. In one embodiment, the method includes administering to a patient a tablet comprising about 100 mg of Compound 1 as described in Example 8 or Example 9 of Section IV.A.2.b, entitled "Preparation of Compound 1 Tablet and SDD Formulation." In another embodiment, the method includes administering to a patient a tablet comprising about 150 mg of Compound 1 as described in Example 10, Example 11, Example 12 or Example 13 of Section IV.A.2.b, entitled "Preparation of Compound 1 Tablet and SDD Formulation." In a further embodiment, the method includes administering to a patient a tablet comprising about 100 mg of Compound 1 as described in Example 8 or Example 9 of Section IV.A.2.b, entitled "Preparation of Compound 1 Tablet and SDD Formulation" and a tablet comprising about 150 mg of Compound 1 as described in Example 10, Example 11, Example 12 or Example 13 of Section IV.A.2.b, entitled "Preparation of Compound 1 Tablet and SDD Formulation." In some embodiments, the method includes administering the tablet comprising 100 mg of Compound 1 and the tablet comprising 150 mg of Compound 1 in the same vehicle. In some embodiments, the method includes administering the tablet comprising 100 mg of Compound 1 and the tablet comprising 150 mg of Compound 1 in separate vehicles.

In one embodiment, the method of administering a pharmaceutical composition includes orally administering to a patient a formulation comprising from about 25 mg to about 300 mg of Compound 1 and a formulation comprising from about 25 mg to about 250 mg of Compound 3. In one embodiment, the method of administering a pharmaceutical composition includes orally administering to a patient one or more tablets, each tablet comprising about 100 mg, about 150 mg, or about 250 mg of Compound 1 and one or more of a tablet comprising from about 25 mg to about 250 mg of Compound 3. In some embodiments, the method includes administering a tablet comprising about 250 mg of Compound 1 and one or more of the following: a tablet comprising about 100 mg of Compound 3 or a tablet comprising about 50 mg of Compound 3. In some embodiments, the method includes administering a tablet comprising about 150 mg of

Compound 1 and one or more of the following: a tablet comprising about 100 mg of Compound 3 or a tablet comprising about 50 mg of Compound 3. In some embodiments, the method includes administering a tablet comprising about 100 mg of Compound 1 and one or more of the following: a tablet comprising about 100 mg of Compound 3 or a tablet comprising about 50 mg of Compound 3. In some embodiments, the method includes administering a tablet comprising about 150 mg of Compound 1; a tablet comprising about 100 mg of Compound 1; and one or more of the following: a tablet comprising about 100 mg of Compound 3 or a tablet comprising about 50 mg of Compound 3. In one embodiment, the method includes administering to a patient a tablet comprising about 100 mg of Compound 1 as described in Example 8 or Example 9 of Section IV.A.2.b, entitled "Preparation of Compound 1 Tablet and SDD Formulation" and a tablet comprising Compound 3 as described in Tables 3-9 or 3-10. In another embodiment, the method includes administering to a patient a tablet comprising about 150 mg of Compound 1 as described in Example 10, Example 11, Example 12 or Example 13 of Section IV.A.2.b, entitled "Preparation of Compound 1 Tablet and SDD Formulation" and a tablet comprising Compound 3 as described in Tables 3-9 or 3-10. In a further embodiment, the method includes administering to a patient a tablet comprising about 100 mg of Compound 1 as described in Example 8 or Example 9 of Section IV.A.2.b, entitled "Preparation of Compound 1 Tablet and SDD Formulation;" a tablet comprising about 150 mg of Compound 1 as described in Example 10, Example 11, Example 12 or Example 13 of Section IV.A.2.b, entitled "Preparation of Compound 1 Tablet and SDD Formulation;" and a tablet comprising Compound 3 as described in Tables 3-9 or 3-10. In some embodiments, the method includes administering the tablet comprising 100 mg of Compound 1, the tablet comprising 150 mg of Compound 1 and the tablet comprising Compound 3 in the same vehicle. In some embodiments, the method includes administering the tablet comprising 100 mg of Compound 1, the tablet comprising 150 mg of Compound 1 and the tablet comprising Compound 3 in separate vehicles.

It is noted that the methods of administration of the present invention can optionally include orally administering a beverage (water, milk, or the like), food, and/or additional pharmaceutical compositions including additional APIs. When the method of administration includes orally administering a beverage (water, milk, or the like), food (including a standard high fat high calorie CF meal or snack), and/or additional pharmaceutical compositions including additional APIs, the oral administration of the beverage, food, and/or additional API can occur concurrently with the oral administration of the tablet, prior to the oral administration of the tablet, and/or after the administration of the tablet. For instance, in one example, the method of administering a pharmaceutical composition includes orally administering to a patient at least once per day at least one tablet comprising a pharmaceutical composition containing a solid dispersion of substantially amorphous Compound 1 or amorphous Compound 1, a filler, a binder, a glidant, a disintegrant, a surfactant, a lubricant, and a second API. In still other examples, the method of administering a pharmaceutical composition includes orally administering to a patient every 12 hours at least one tablet comprising a pharmaceutical composition as described herein, in which the tablet is administered about 30 minutes after consuming a high fat, high calorie CF meal or snack.

**IV.B. Formulations of Compound 3**

**IV.B.1. Compound 3 Tablet Formulation**

**IV.B.1.a. Embodiments of Compound 3 Tablet Formulation**

In one aspect, the invention features a tablet for oral administration comprising: a) Compound 3; b) a filler; c) a diluent; d) a disintegrant; e) a lubricant; and f) a glidant.

In some embodiments, Compound 3 is in a substantially amorphous form (Compound 3 Amorphous Form). In other embodiments, Compound 3 is in a substantially crystalline solid form. In one embodiment, Compound 3 is in substantially crystalline Form A (Compound 3 Form A). In other embodiments, Compound 3 is in a mixture of solid (i.e., amorphous and crystalline) forms.

In one embodiment, Compound 3 or Compound 3 Amorphous Form is present in the tablet in an amount ranging from about 25 mg to about 250 mg. In one embodiment, Compound 3 or Compound 3 Amorphous Form is present in the tablet in an amount of about 50 mg to about 200 mg. In one embodiment, Compound 3 or Compound 3 Amorphous Form is present in the tablet in an amount of about 100 mg.

In one embodiment, the amount of Compound 3 or Compound 3 Amorphous Form in the tablet ranges from about 10 wt% to about 50 wt% by weight of the tablet. In one embodiment, the amount of Compound 3 or Compound 3 Amorphous Form in the tablet ranges from about 20 wt% to about 30 wt% by weight of the tablet. In one embodiment, the amount of Compound 3 or Compound 3 Amorphous Form in the tablet is about 25 wt% of the tablet.

In one embodiment, the filler is selected from cellulose, modified cellulose, sodium carboxymethyl cellulose, ethyl cellulose hydroxymethyl cellulose, hydroxypropylcellulose, cellulose acetate, microcrystalline cellulose, dibasic calcium phosphate, sucrose, lactose, corn starch, potato starch, or any combination thereof. In one embodiment, the filler is microcrystalline cellulose (MCC) and is present in the tablet in an amount ranging from about 10 wt% to about 30 wt% by weight of the tablet.

In one embodiment, the diluent is selected from lactose monohydrate, mannitol, sorbitol, cellulose, calcium phosphate, starch, sugar or any combination thereof. In one embodiment, the diluent is lactose monohydrate and is present in the tablet in an amount ranging from about 10 wt% to about 30 wt% by weight of the tablet.

In one embodiment, the disintegrant is selected from agar-agar, algins, calcium carbonate, carboxmethylcellulose, cellulose, hydroxypropylcellulose, low substituted hydroxypropylcellulose, clays, croscarmellose sodium, crospovidone, gums, magnesium aluminum silicate, methylcellulose, polacrilin potassium, sodium alginate, sodium starch glycolate, maize starch, potato starch, tapioca starch, or any combination thereof. In one embodiment, the disintegrant is croscarmellose sodium and is present in the tablet at a concentration of 5 wt% or less by weight of the tablet.

In one embodiment, the lubricant is selected from magnesium stearate, calcium stearate, zinc stearate, sodium stearate, stearic acid, aluminum stearate, leucine, glyceryl behenate, hydrogenated vegetable oil or any combination thereof. In one embodiment, the lubricant is magnesium stearate and has a concentration of less than 2 wt% by weight of the tablet.

In one embodiment, the glidant is selected from colloidal silicon dioxide, talc, corn starch, or a combination thereof. In one embodiment, the glidant is colloidal silicon dioxide and has a concentration of 3 wt% or less by weight of the tablet.

In one embodiment, the tablet further comprises a colorant.

In one aspect, the invention features a tablet comprising a plurality of granules, the composition comprising: a) Compound 3 Amorphous Form in an amount ranging from about 10 wt% to about 50 wt% by weight of the composition; b) a filler in an amount ranging from about 10 wt% to about 30 wt% by weight of the composition; c) a diluent in an amount ranging from about 10 wt% to about 30 wt% by weight of the composition; d) a disintegrant in an amount ranging from about 1 wt% to about 5 wt% by weight of the composition; e) a lubricant in an amount ranging from about 0.3 wt% to about 3 wt% by weight of the composition; and f) a glidant in an amount ranging from about 0.3 wt% to about 3 wt% by weight of the composition.

In one embodiment, Compound 3 is Compound 3 Amorphous Form and is in a spray dried dispersion. In one embodiment, the spray dried dispersion comprises a polymer. In one embodiment, the polymer is hydroxypropylmethylcellulose (HPMC). In one embodiment, the polymer is hydroxypropylmethylcellulose acetate succinate (HPMCAS).

In one embodiment, the polymer is present in an amount from 20% by weight to 70% by weight. In one embodiment, the polymer is present in an amount from 30% by weight to 60% by weight. In one embodiment, the polymer is present in an amount of about 49.5% by weight.

In one embodiment, the tablet further comprises a surfactant. In one embodiment, the surfactant is sodium lauryl sulfate. In one embodiment, the surfactant is present in an amount from 0.1% by weight to 5% by weight. In one embodiment, the surfactant is present in an amount of about 0.5% by weight.

In another aspect, the invention features a tablet of the formulation set forth in Table 3-9.

**Table 3-9**

| Component | Function | Final Blend Composition %w/w | Tablet (mg/tablet) |
|---|---|---|---|
| 50% Compound 3 /49.5% HPMCAS-HG/0.5% sodium lauryl sulfate | Active as a spray dried dispersion (SSD) | 50.00 | 200.0 SDD (100.00 Compound 3) |
| Microcrystalline cellulose | Filler | 22.63 | 90.5 |
| Lactose Monohydrate | Diluent | 22.63 | 90.5 |
| Crosscarmelose Sodium | Disintegrant | 3.00 | 12.0 |
| Magnesium Stearate | Lubricant | 0.25 | 1.0 |
| Colloidal Silica Dioxide | Glidant | 1.00 | 4.0 |
| | **Intragranular content** | 99.5 | |
| ***Extragranular*** *Blend* Colloidal Silica | | | |
| Colloidal Silica Dioxide | Glidant | 0.25 | 1.0 |
| Magnesium Stearate | Lubricant | 0.25 | 1.0 |

(continued)

| Component | Function | Final Blend Composition %w/w | Tablet (mg/tablet) |
|---|---|---|---|
| | **Extragranular content** | 0.5 | |
| | ***Total*** | **100.00** | **400.0** |

In another aspect, the invention features a tablet of the formulation set forth in Table 3-10.

**Table 3-10**

| Component | Function | Final Blend Composition %w/w | Tablet (mg/tablet) |
|---|---|---|---|
| 50% Compound 3/49.5% HPMCAS-HG/0.5% sodium | Active as a spray dried dispersion | 50.00 | 100.0 SDD (50.00 Compound 3) |
| lauryl sulfate | (SSD) | | |
| Microcrystalline cellulose | Filler | 22.63 | 45.25 |
| Lactose Monohydrate | Diluent | 22.63 | 45.25 |
| Crosscarmelose Sodium | Disintegrant | 3.00 | 6.0 |
| Magnesium Stearate | Lubricant | 0.25 | 0.5 |
| Colloidal Silica Dioxide | Glidant | 1.00 | 2.0 |
| | **Intragranular content** | **99.5** | |
| ***Extragranular Blend*** | | | |
| Colloidal Silica Dioxide | Glidant | 0.25 | 0.5 |
| Magnesium Stearate | Lubricant | 0.25 | 0.5 |
| | **Extragranular content** | 0.5 | |
| | ***Total*** | **100.00** | **200.0** |

In another aspect, the invention provides a pharmaceutical composition in the form of a tablet that comprises Compound 3, and one or more pharmaceutically acceptable excipients, for example, a filler, a disintegrant, a surfactant, a diluent, a glidant, and a lubricant and any combination thereof, where the tablet has a dissolution of at least about 50% in about 30 minutes. In another embodiment, the dissolution rate is at least about 75% in about 30 minutes. In another embodiment, the dissolution rate is at least about 90% in about 30 minutes.

In another aspect, the invention provides a pharmaceutical composition in the form of a tablet that comprises a powder blend or granules comprising Compound 3, and, one or more pharmaceutically acceptable excipients, for example, a filler, a disintegrant, a surfactant, a diluent, a glidant, and a lubricant, wherein the tablet has a hardness of at least about 5 kP (kP = kilo Ponds; 1 kP = ~9.8 N). In another embodiment, the tablet has a target friability of less than 1.0% after 400 revolutions.

In another aspect, the invention provides a tablet as described herein further comprising an additional therapeutic agent. In one embodiment, the additional therapeutic agent is a mucolytic agent, bronchodialator, an antibiotic, an anti-infective agent, an antiinflammatory agent, a CFTR modulator other than Compound 3, or a nutritional agent. In some embodiments, the additional therapeutic agent is Compound 1.

In one aspect, the invention features a method of administering a tablet comprising orally administering to a patient at least once per day a tablet comprising: a) about 25 to 200 mg of Compound 3 Amorphous Form; b) a filler; c) a diluent; d) a disintegrant; e) a surfactant; f) a glidant; and g) a lubricant. In one embodiment, the tablet comprises about 25 mg of Compound 3 Amorphous Form. In one embodiment, the tablet comprises about 50 mg of Compound 3 Amorphous Form. In one embodiment, the tablet comprises about 100 mg of Compound 3 Amorphous Form. In one embodiment, the tablet comprises about 150 mg of Compound 3 Amorphous Form. In one embodiment, the tablet comprises about 200 mg of Compound 3 Amorphous Form.

In one aspect, the invention features a method of administering a tablet comprising orally administering to a patient twice per day a tablet comprising: a) about 25 to 200 mg of Compound 3 Amorphous Form; b) a filler; c) a diluent;

d) a disintegrant; e) a surfactant; f) a glidant; and g) a lubricant. In one embodiment, the tablet comprises about 25 mg of Compound 3 Amorphous Form. In one embodiment, the tablet comprises about 50 mg of Compound 3 Amorphous Form. In one embodiment, the tablet comprises about 100 mg of Compound 3 Amorphous Form. In one embodiment, the tablet comprises about 150 mg of Compound 3 Amorphous Form. In one embodiment, the tablet comprises about 200 mg of Compound 3 Amorphous Form.

In one aspect, the invention features a method for administering a tablet comprising orally administering to a patient once every 12 hours a tablet comprising: a) about 25 to 200 mg of Compound 3 Amorphous Form; b) a filler; c) a diluent; d) a disintegrant; e) a surfactant; f) a glidant; and g) a lubricant. In one embodiment, the tablet comprises about 25 mg of Compound 3 Amorphous Form. In one embodiment, the tablet comprises about 50 mg of Compound 3 Amorphous Form. In one embodiment, the tablet comprises about 100 mg of Compound 3 Amorphous Form. In one embodiment, the tablet comprises about 200 mg of Compound 3 Amorphous Form.

**Compound 3 Pharmaceutical Compositions**

The invention provides pharmaceutical compositions, pharmaceutical formulations and solid dosage forms such as tablets comprising Compound 3 Amorphous Form or Compound 3 Form A. In some embodiments of this aspect, the amount of Compound 3 that is present in the pharmaceutical composition is 25 mg, 50 mg, 75 mg, 100 mg, 125 mg, 150 mg, or 200 mg. In some embodiments of this aspect, weight/weight relative percent of Compound 3 that is present in the pharmaceutical composition is from 10 to 50 percent. In these and other embodiments, Compound 3 is present as substantially pure Compound 3 Amorphous Form. "Substantially pure" means greater than ninety percent pure; preferably greater than 95 percent pure; more preferably greater than 99.5 percent pure (i.e., not mixed with crystalline forms of Compound 3).

Thus in one aspect, the invention provides a pharmaceutical composition comprising:

a. Compound 3 Amorphous Form;

b. a filler;

c. a disintegrant;

d. a diluent;

e. a lubricant; and

g. a glidant.

In one embodiment of this aspect, the pharmaceutical composition comprises 25 mg of Compound 3 Amorphous Form. In another embodiment of this aspect, the pharmaceutical composition comprises 50 mg of Compound 3 Amorphous Form. In another embodiment of this aspect, the pharmaceutical composition comprises 100 mg of Compound 3 Amorphous Form. In another embodiment of this aspect, the pharmaceutical composition comprises 125 mg of Compound 3 Amorphous Form. In another embodiment of this aspect, the pharmaceutical composition comprises 150 mg of Compound 3 Amorphous Form. In another embodiment of this aspect, the pharmaceutical composition comprises 200 mg of Compound 3 Amorphous Form.

In some embodiments, the pharmaceutical compositions comprises Compound 3 Amorphous Form, wherein Compound 3 Amorphous Form is present in an amount of at least 15 wt% (e.g., at least 20 wt%, at least 30 wt%, at least 40 wt%, at least 50 wt%, or at least 60 wt%) by weight of the composition.

In some embodiments, the pharmaceutical composition comprises Compound 3 Amorphous Form, a filler, a diluent, a disintegrant, a glidant, and a lubricant. In this embodiment, the composition comprises from about 10 wt% to about 50 wt% (e.g., about 15-45 wt%) of Compound 3 Amorphous Form by weight of the composition, and more typically, from 20 wt% to about 40 wt% (e.g., about 25-30 wt%) of Compound 3 Amorphous Form by weight of the composition.

In some embodiments, the pharmaceutical composition comprises Compound 3 Amorphous Form, a filler, a diluent, a disintegrant, a glidant, and a lubricant. In this embodiment, the composition comprises from about 10 wt% to about 50 wt% (e.g., about 15-45 wt%) of Compound 3 Amorphous Form by weight of the composition, and more typically from 20 wt% to about 40 wt% (e.g., about 25-30 wt%) of Compound 3 Amorphous Form by weight of the composition.

The concentration of Compound 3 Amorphous Form in the composition depends on several factors such as the amount of pharmaceutical composition needed to provide a desired amount of Compound 3 Amorphous Form and the desired dissolution profile of the pharmaceutical composition.

In another embodiment, the pharmaceutical composition comprises Compound 3 in which the Compound 3 in

its solid form has a mean particle diameter, measured by light scattering (e.g., using a Malvern Mastersizer available from Malvern Instruments in England) of 0.1 microns to 10 microns. In another embodiment, the particle size of Compound 3 is 1 micron to 5 microns. In another embodiment, Compound 3 has a particle size D50 of 2.0 microns.

As indicated, in addition to Compound 3 Amorphous Form, in some embodiments of the invention, the pharmaceutical compositions which are oral formulations also comprise one or more excipients such as fillers, disintegrants, surfactants, diluents, glidants, lubricants, colorants, or fragrances and any combination thereof.

Fillers suitable for the invention are compatible with the ingredients of the pharmaceutical composition, i.e., they do not substantially reduce the solubility, the hardness, the chemical stability, the physical stability, or the biological activity of the pharmaceutical composition. Exemplary fillers include: celluloses, modified celluloses, (e.g. sodium carboxymethyl cellulose, ethyl cellulose hydroxymethyl cellulose, hydroxypropylcellulose), cellulose acetate, microcrystalline cellulose, calcium phosphates, dibasic calcium phosphate, starches (e.g. corn starch, potato starch), sugars (e.g., sorbitol) lactose, sucrose, or the like), or any combination thereof.

Thus, in one embodiment, the pharmaceutical composition comprises at least one filler in an amount of at least 5 wt% (e.g., at least about 20 wt%, at least about 30 wt%, or at least about 40 wt%) by weight of the composition. For example, the pharmaceutical composition comprises from about 10 wt% to about 60 wt% (e.g., from about 10 wt% to about 55 wt%, from about 15 wt% to about 30 wt%, or from about 20 wt% to about 25 wt%) of filler, by weight of the composition. In another example, the pharmaceutical composition comprises at least about 20 wt% (e.g., at least 20 wt% or at least 20 wt%) of microcrystalline cellulose, for example MCC Avicel PH102, by weight of the composition.

Disintegrants suitable for the invention enhance the dispersal of the pharmaceutical composition and are compatible with the ingredients of the pharmaceutical composition, i.e., they do not substantially reduce the chemical stability, the physical stability, the hardness, or the biological activity of the pharmaceutical composition. Exemplary disintegrants include croscarmellose sodium, sodium starch glycolate, or a combination thereof.

Thus, in one embodiment, the pharmaceutical composition comprises disintegrant in an amount of about 10 wt% or less (e.g., about 7 wt% or less, about 6 wt% or less, or about 5 wt% or less) by weight of the composition. For example, the pharmaceutical composition comprises from about 1 wt% to about 10 wt% (e.g., from about 1.5 wt% to about 7.5 wt% or from about 2.5 wt% to about 6 wt%) of disintegrant, by weight of the composition. In some examples, the pharmaceutical composition comprises from about 0.1% to about 10 wt% (e.g., from about 0.5 wt% to about 7.5 wt% or from about 1.5 wt% to about 6 wt%) of disintegrant, by weight of the composition. In still other examples, the pharmaceutical composition comprises from about 0.5% to about 10 wt% (e.g., from about 1.5 wt% to about 7.5 wt% or from about 2.5 wt% to about 6 wt%) of disintegrant, by weight of the composition.

Surfactants suitable for the invention enhance the wettability of the pharmaceutical composition and are compatible with the ingredients of the pharmaceutical composition, i.e., they do not substantially reduce the chemical stability, the physical stability, the hardness, or the biological activity of the pharmaceutical composition. Exemplary surfactants include sodium lauryl sulfate (SLS), sodium stearyl fumarate (SSF), polyoxyethylene 20 sorbitan mono-oleate (e.g., Tween™), any combination thereof, or the like.

Thus, in one embodiment, the pharmaceutical composition comprises a surfactant in an amount of about 10 wt% or less (e.g., about 5 wt% or less, about 2 wt% or less, about 1 wt% or less, about 0.8 wt% or less, or about 0.6 wt% or less) by weight of the composition. For example, the pharmaceutical composition includes from about 10 wt% to about 0.1 wt% (e.g., from about 5 wt% to about 0.2 wt% or from about 2 wt% to about 0.3 wt%) of surfactant, by weight of the composition. In yet another example, the pharmaceutical composition comprises from about 10 wt% to about 0.1 wt% (e.g., from about 5 wt% to about 0.2 wt% or from about 2 wt% to about 0.3 wt%) of sodium lauryl sulfate, by weight of the composition.

Diluents suitable for the invention may add necessary bulk to a formulation to prepare tablets of the desired size and are generally compatible with the ingredients of the pharmaceutical composition, i.e., they do not substantially reduce the solubility, the hardness, the chemical stability, the physical stability, or the biological activity of the pharmaceutical composition. Exemplary diluents include: sugars, for example, confectioner's sugar, compressible sugar, dextrates, dextrin, dextrose, lactose, lactose monohydrate, mannitol, sorbitol, cellulose, and modified celluloses, for example, powdered cellulose, talc, calcium phosphate, starch, or any combination thereof.

Thus, in one embodiment, the pharmaceutical composition comprises a diluent in an amount of 40 wt% or less (e.g., 35 wt% or less, 30 wt% or less, or 25 wt% or less, or 20 wt% or less, or 15 wt% or less, or 10 wt% or less) by weight of the composition. For example, the pharmaceutical composition comprises from about 40 wt% to about 1 wt% (e.g., from about 35 wt% to about 5 wt% or from about 30 wt% to about 7 wt%, from about 25 wt% to about 15 wt%) of diluent, by weight of the composition. In another example, the pharmaceutical composition comprises 40 wt% or less (e.g., 35 wt% or less, or 25 wt% or less) of lactose monohydrate, by weight of the composition. In yet another example, the pharmaceutical composition comprises from about 35 wt% to about 1 wt% (e.g., from about 30 wt% to about 5 wt% or from about 25 wt% to about 10 wt%) of lactose monohydrate, by weight of the composition.

Glidants suitable for the invention enhance the flow properties of the pharmaceutical composition and are compatible with the ingredients of the pharmaceutical composition, i.e., they do not substantially reduce the solubility,

the hardness, the chemical stability, the physical stability, or the biological activity of the pharmaceutical composition. Exemplary glidants include colloidal silicon dioxide, talc, or a combination thereof.

Thus, in one embodiment, the pharmaceutical composition comprises a glidant in an amount of 2 wt% or less (e.g., 1.75 wt%, 1.25 wt% or less, or 1.00 wt% or less) by weight of the composition. For example, the pharmaceutical composition comprises from about 2 wt% to about 0.05 wt% (e.g., from about 1.5 wt% to about 0.07 wt% or from about 1.0 wt% to about 0.09 wt%) of glidant, by weight of the composition. In another example, the pharmaceutical composition comprises 2 wt% or less (e.g., 1.75 wt%, 1.25 wt% or less, or 1.00 wt% or less) of colloidal silicon dioxide, by weight of the composition. In yet another example, the pharmaceutical composition comprises from about 2 wt% to about 0.05 wt% (e.g., from about 1.5 wt% to about 0.07 wt% or from about 1.0 wt% to about 0.09 wt%) of colloidal silicon dioxide, by weight of the composition.

In some embodiments, the pharmaceutical composition can include an oral solid pharmaceutical dosage form which can comprise a lubricant that can prevent adhesion of a granulate-bead admixture to a surface (e.g., a surface of a mixing bowl, a compression die and/or punch). A lubricant can also reduce interparticle friction within the granulate and improve the compression and ejection of compressed pharmaceutical compositions from a die press. The lubricant is also compatible with the ingredients of the pharmaceutical composition, i.e., they do not substantially reduce the solubility, the hardness, or the biological activity of the pharmaceutical composition. Exemplary lubricants include magnesium stearate, calcium stearate, zinc stearate, sodium stearate, stearic acid, aluminum stearate, leucine, glyceryl behenate, hydrogenated vegetable oil or any combination thereof. In one embodiment, the pharmaceutical composition comprises a lubricant in an amount of 5 wt% or less (e.g., 4.75 wt%, 4.0 wt% or less, or 3.00 wt% or less, or 2.0 wt% or less) by weight of the composition. For example, the pharmaceutical composition comprises from about 5 wt% to about 0.10 wt% (e.g., from about 4.5 wt% to about 0.5 wt% or from about 3 wt% to about 0.5 wt%) of lubricant, by weight of the composition. In another example, the pharmaceutical composition comprises 5 wt% or less (e.g., 4.0 wt% or less, 3.0 wt% or less, or 2.0 wt% or less, or 1.0 wt% or less) of magnesium stearate, by weight of the composition. In yet another example, the pharmaceutical composition comprises from about 5 wt% to about 0.10 wt% (e.g., from about 4.5 wt% to about 0.15 wt% or from about 3.0 wt% to about 0.50 wt%) of magnesium stearate, by weight of the composition.

Pharmaceutical compositions of the invention can optionally comprise one or more colorants, flavors, and/or fragrances to enhance the visual appeal, taste, and/or scent of the composition. Suitable colorants, flavors, or fragrances are compatible with the ingredients of the pharmaceutical composition, i.e., they do not substantially reduce the solubility, the chemical stability, the physical stability, the hardness, or the biological activity of the pharmaceutical composition. In one embodiment, the pharmaceutical composition comprises a colorant, a flavor, and/or a fragrance. In one embodiment, the pharmaceutical compositions provided by the invention are purple.

In some embodiments, the pharmaceutical composition includes or can be made into tablets and the tablets can be coated with a colorant and optionally labeled with a logo, other image and/or text using a suitable ink. In still other embodiments, the pharmaceutical composition includes or can be made into tablets and the tablets can be coated with a colorant, waxed, and optionally labeled with a logo, other image and/or text using a suitable ink. Suitable colorants and inks are compatible with the ingredients of the pharmaceutical composition, i.e., they do not substantially reduce the solubility, the chemical stability, the physical stability, the hardness, or the biological activity of the pharmaceutical composition. The suitable colorants and inks can be any color and are water based or solvent based. In one embodiment, tablets made from the pharmaceutical composition are coated with a colorant and then labeled with a logo, other image, and/or text using a suitable ink. For example, tablets comprising pharmaceutical composition as described herein can be coated with about 3 wt% (e.g., less than about 6 wt% or less than about 4 wt%) of film coating comprising a colorant. The colored tablets can be labeled with a logo and text indicating the strength of the active ingredient in the tablet using a suitable ink. In another example, tablets comprising pharmaceutical composition as described herein can be coated with about 3 wt% (e.g., less than about 6 wt% or less than about 4 wt%) of a film coating comprising a colorant.

In another embodiment, tablets made from the pharmaceutical composition are coated with a colorant, waxed, and then labeled with a logo, other image, and/or text using a suitable ink. For example, tablets comprising pharmaceutical composition as described herein can be coated with about 3 wt% (e.g., less than about 6 wt% or less than about 4 wt%) of film coating comprising a colorant. The colored tablets can be waxed with Carnauba wax powder weighed out in the amount of about 0.01% w/w of the starting tablet core weight. The waxed tablets can be labeled with a logo and text indicating the strength of the active ingredient in the tablet using a suitable ink. In another example, tablets comprising pharmaceutical composition as described herein can be coated with about 3 wt% (e.g., less than about 6 wt% or less than about 4 wt%) of a film coating comprising a colorant The colored tablets can be waxed with Carnauba wax powder weighed out in the amount of about 0.01% w/w of the starting tablet core weight. The waxed tablets can be labeled with a logo and text indicating the strength of the active ingredient in the tablet using a pharmaceutical grade ink such as a black ink (e.g., Opacode® S-1-17823, a solvent based ink, commercially available from Colorcon, Inc. of West Point, PA.).

One exemplary pharmaceutical composition comprises from about 15 wt% to about 70 wt% (e.g., from about 15 wt% to about 60 wt%, from about 15 wt% to about 50 wt%, or from about 25 wt% to about 50 wt%, or from about 20 wt% to about 70 wt%, or from about 30 wt% to about 70 wt%, or from about 40 wt% to about 70 wt%, or from about 50

wt% to about 70 wt%) of Compound 3 Amorphous Form, by weight of the composition. The aforementioned compositions can also include one or more pharmaceutically acceptable excipients, for example, from about 20 wt% to about 50 wt% of a filler; from about 1 wt% to about 5 wt% of a disintegrant; from about 2 wt% to about 0.25 wt% of a surfactant; from about 1 wt% to about 30 wt% of a diluent; from about 2 wt% to about 0.05 wt% of a glidant; and from about 5 wt% to about 0.1 wt% of a lubricant. Or, the pharmaceutical composition comprises a composition containing from about 15 wt% to about 70 wt% (e.g., from about 20 wt% to about 60 wt%, from about 25 wt% to about 55 wt%, or from about 30 wt% to about 50 wt%) of Compound 3 Amorphous Form, by weight of the composition; and one or more excipients, for example, from about 20 wt% to about 50 wt% of a filler; from about 1 wt% to about 5 wt% of a disintegrant; from about 2 wt% to about 0.25 wt% of a surfactant; from about 1 wt% to about 30 wt% of a diluent; from about 2 wt% to about 0.05 wt% of a glidant; and from about 5 wt% to about 0.1 wt% of a lubricant.

Another exemplary pharmaceutical composition comprises from about 15 wt% to about 70 wt% (e.g., from about 15 wt% to about 60 wt%, from about 15 wt% to about 50 wt%, or from about 25 wt% to about 50 wt% or from about 20 wt% to about 70 wt%, or from about 30 wt% to about 70 wt%, or from about 40 wt% to about 70 wt%, or from about 50 wt% to about 70 wt%) of Compound 3 Amorphous Form by weight of the composition, and one or more excipients, for example, from about 20 wt% to about 50 wt% of a filler; from about 1 wt% to about 5 wt% of a disintegrant; from about 2 wt% to about 0.25 wt% of a surfactant; from about 1 wt% to about 30 wt% of a diluent; from about 2 wt% to about 0.05 wt% of a glidant; and from about 2 wt% to about 0.1 wt% of a lubricant.

In one embodiment, the invention is a granular pharmaceutical composition comprising:

a. about 25 wt% of Compound 3 Amorphous Form by weight of the composition;

b. about 22.5 wt% of microcrystalline cellulose by weight of the composition;

c. about 22.5 wt% of lactose monohydrate by weight of the composition;

d. about 3 wt% of sodium croscarmellose sodium by weight of the composition;

e. about 0.25 wt% of sodium lauryl sulfate by weight of the composition;

f. about 0.5 wt% of magnesium stearate by weight of the composition; and

g. about 1.25 wt% of colloidal silica by weight of the composition.

In one embodiment, the invention is a granular pharmaceutical composition comprising:

a. about 25 wt% of Compound 3 Amorphous Form by weight of the composition;

b. about 22.5 wt% of microcrystalline cellulose by weight of the composition;

c. about 22.5 wt% of lactose monohydrate by weight of the composition;

d. about 3 wt% of sodium croscarmellose sodium by weight of the composition;

e. about 0.25 wt% of sodium lauryl sulfate by weight of the composition;

f. about 0.5 wt% of magnesium stearate by weight of the composition;

g. about 1.25 wt% of colloidal silica by weight of the composition; and

h. about 25 wt% of a polymer.

In another embodiment, the polymer is HPMCAS.

The pharmaceutical compositions of the invention can be processed into a tablet form, capsule form, pouch form, lozenge form, or other solid form that is suited for oral administration. Thus in some embodiments, the pharmaceutical compositions are in tablet form.

In still another pharmaceutical oral formulation of the invention, a shaped pharmaceutical tablet composition having an initial hardness of 5-21 kP ± 20 percent comprises: about 25 wt% of Compound 3 Amorphous Form; about 22.5 wt% of microcrystalline cellulose by weight of the composition; about 22.5 wt% of lactose monohydrate by weight

of the composition; about 3 wt% of sodium croscarmellose sodium by weight of the composition; about 0.25 wt% of sodium lauryl sulfate by weight of the composition; about 0.5 wt% of magnesium stearate by weight of the composition; and about 1.25 wt% of colloidal silica by weight of the composition. Wherein the amount of Compound 3 Amorphous Form in the shaped pharmaceutical tablet ranges from about 25 mg to about 200 mg, for example, 50 mg, or 75 mg, or 100 mg, or 150 mg or 200 mg Compound 3 Amorphous Form per tablet.

In certain embodiments, the shaped pharmaceutical tablet contains about 100 mg of Compound 3 Amorphous Form.

Another aspect of the invention provides a pharmaceutical formulation consisting of a tablet or capsule that includes a Compound 3 Amorphous Form and other excipients (e.g., a filler, a disintegrant, a surfactant, a glidant, a colorant, a lubricant, or any combination thereof), each of which is described above and in the Examples below, wherein the tablet has a dissolution of at least about 50% (e.g., at least about 60%, at least about 70%, at least about 80%, at least about 90%, or at least about 99%) in about 30 minutes. In one example, the pharmaceutical composition consists of a tablet that includes Compound 3 Amorphous Form in an amount ranging from 25 mg to 200 mg, for example, 25 mg, or 50 mg, or 75 mg, or 100 mg, or 150 mg, or 200 mg and one or more excipients (e.g., a filler, a disintegrant, a surfactant, a glidant, a colorant, a lubricant, or any combination thereof), each of which is described above and in the Examples below, wherein the tablet has a dissolution of from about 50% to about 100% (e.g., from about 55% to about 95% or from about 60% to about 90%) in about 30 minutes.

In one embodiment, the tablet comprises a composition comprising at least about 25 mg (e.g., at least about 30 mg, at least about 40 mg, or at least about 50 mg) of Compound 3 Amorphous Form; and one or more excipients from: a filler, a diluent, a disintegrant, a surfactant, a glidant, and a lubricant. In another embodiment, the tablet comprises a composition comprising at least about 25 mg (e.g., at least about 30 mg, at least about 40 mg, at least about 50 mg, at least about 100 mg, or at least 150 mg) of Compound 3 Amorphous Form and one or more excipients from: a filler, a diluent, a disintegrant, a surfactant, a glidant, and a lubricant.

Dissolution can be measured with a standard USP Type II apparatus that employs a dissolution media of 0.1% CTAB dissolved in 900 mL of DI water, buffered at pH 6.8 with 50 mM potassium phosphate monoasic, stirring at about 50-75 rpm at a temperature of about 37 °C. A single experimental tablet is tested in each test vessel of the apparatus. Dissolution can also be measured with a standard USP Type II apparatus that employs a dissolution media of 0.7% sodium lauryl sulfate dissolved in 900 mL of 50 mM sodium phosphate buffer (pH 6.8), stirring at about 65 rpm at a temperature of about 37 °C. A single experimental tablet is tested in each test vessel of the apparatus. Dissolution can also be measured with a standard USP Type II apparatus that employs a dissolution media of 0.5% sodium lauryl sulfate dissolved in 900 mL of 50 mM sodium phosphate buffer (pH 6.8), stirring at about 65 rpm at a temperature of about 37 °C. A single experimental tablet is tested in each test vessel of the apparatus.

### IV.B.1.b. Preparation of Compound 3 Tablet Formulation

The dosage unit forms of the invention can be produced by compacting or compressing an admixture or composition, for example, a powder or granules, under pressure to form a stable three-dimensional shape (e.g., a tablet). As used herein, "tablet" includes compressed pharmaceutical dosage unit forms of all shapes and sizes, whether coated or uncoated.

The expression "dosage unit form" as used herein refers to a physically discrete unit of agent appropriate for the patient to be treated. In general, a compacted mixture has a density greater than that of the mixture prior to compaction. A dosage unit form of the invention can have almost any shape including concave and/or convex faces, rounded or angled corners, and a rounded to rectilinear shape. In some embodiments, the compressed dosage forms of the invention comprise a rounded tablet having flat faces. The solid pharmaceutical dosage forms of the invention can be prepared by any compaction and compression method known by persons of ordinary skill in the art of forming compressed solid pharmaceutical dosage forms. In particular embodiments, the formulations provided herein may be prepared using conventional methods known to those skilled in the field of pharmaceutical formulation, as described, e.g., in pertinent textbooks. See, e.g., Remington: The Science and Practice of Pharmacy, 21st Ed., Lippincott Williams & Wilkins, Baltimore, Md. (2003); Ansel et al., Pharmaceutical Dosage Forms And Drug Delivery Systems, 7th Edition, Lippincott Williams & Wilkins, (1999); The Handbook of Pharmaceutical Excipients, 4th edition, Rowe et al., Eds., American Pharmaceuticals Association (2003); Gibson, Pharmaceutical Preformulation And Formulation, CRC Press (2001), these references hereby incorporated herein by reference in their entirety.

### V. Granulation and Compression

In some embodiments, solid forms, including powders comprising the active agent, Compound 3 Amorphous Form, and the included pharmaceutically acceptable excipients (e.g. filler, diluent, disintegrant, surfactant, glidant, lubricant, or any combination thereof) can be subjected to a dry granulation process. The dry granulation process causes

the powder to agglomerate into larger particles having a size suitable for further processing. Dry granulation can improve the flowability of a mixture in order to be able to produce tablets that comply with the demand of mass variation or content uniformity.

Formulations as described herein may be produced using one or more mixing and dry granulations steps. The order and the number of the mixing and granulation steps do not seem to be critical. However, at least one of the excipients and Compound 3 can be been subject to dry granulation or wet high shear granulation before compression into tablets. Dry granulation of Compound 3 Amorphous Form and the excipients made together prior to tablet compression seem, surprisingly, to be a simple, inexpensive and efficient way of providing close physical contact between the ingredients of the present compositions and formulations and thus results in a tablet formulation with good stability properties. Dry granulation can be carried out by a mechanical process, which transfers energy to the mixture without any use of any liquid substances (neither in the form of aqueous solutions, solutions based on organic solutes, or mixtures thereof) in contrast to wet granulation processes, also contemplated herein. Generally, the mechanical process requires compaction such as the one provided by roller compaction. An example of an alternative method for dry granulation is slugging.

In some embodiments, roller compaction is a granulation process comprising highly intensive mechanical compacting of one or more substances. In some embodiments, a pharmaceutical composition comprising an admixture of powders is pressed, that is roller compacted, between 2 counter rotating rollers to make a solid sheet which is subsequently crushed in a sieve to form a particulate matter. In this particulate matter, a close mechanical contact between the ingredients can be obtained. An example of roller compaction equipment is Minipactor® a Gerteis 3W-Polygran from Gerteis Maschinen+Processengineering AG.

In some embodiments, tablet compression according to the invention can occur without any use of any liquid substances (neither in the form of aqueous solutions, solutions based on organic solutes, or mixtures thereof), i.e. a dry granulation process. In a typical embodiment the resulting core or tablet has a compressive strength in the range of 1 to 15 kP; such as 1.5 to 12.5 kP, preferably in the range of 2 to 10 kP.

### VI. Brief Manufacturing Procedure

In some embodiments, the ingredients are weighed according to the formula set herein. Next, all of the intragranular ingredients are sifted and mixed well. The ingredients can be lubricated with a suitable lubricant, for example, magnesium stearate. The next step can comprise compaction/slugging of the powder admixture and sized ingredients. Next, the compacted or slugged blends are milled into granules and sifted to obtain the desired size. Next, the granules can be further lubricated with, for example, magnesium stearate. Next the granular composition of the invention can be compressed on suitable punches into various pharmaceutical formulations in accordance with the invention. Optionally the tablets can be coated with a film, colorant or other coating.

Another aspect of the invention provides a method for producing a pharmaceutical composition comprising providing an admixture of a composition comprising Compound 3 Amorphous Form and one or more excipients selected from: a filler, a diluent, a glidant, a surfactant, a lubricant, a disintegrant, and compressing the composition into a tablet having a dissolution of at least about 50% in about 30 minutes.

In another embodiment, a wet granulation process is performed to yield the pharmaceutical formulation of the invention from an admixture of powdered and liquid ingredients. For example, a pharmaceutical composition comprising an admixture of a composition comprising Compound 3 Amorphous Form and one or more excipients selected from: a filler, a diluent, a glidant, a surfactant, a lubricant, a disintegrant, are weighed as per the formula set herein. Next, all of the intragranular ingredients are sifted and mixed in a high shear or low shear granulator using water or water with a surfactant or water with a binder or water with a surfactant and a binder to granulate the powder blend. A fluid other than water can also be used with or without surfactant and/or binder to granulate the powder blend. Next, the wet granules can optionally be milled using a suitable mill. Next, water may optionally be removed from the admixture by drying the ingredients in any suitable manner. Next, the dried granules can optionally be milled to the required size. Next, extra granular excipients can be added by blending (for example a filler, a diluent, and a disintegrant). Next, the sized granules can be further lubricated with magnesium stearate and a disintegrant, for example, croscarmellose sodium. Next the granular composition of the invention can be sifted for sufficient time to obtain the correct size and then compressed on suitable punches into various pharmaceutical formulations in accordance with the invention. Optionally, the tablets can be coated with a film, colorant or other coating.

Each of the ingredients of this exemplary admixture is described above and in the Examples below. Furthermore, the admixture can comprise optional additives, such as, one or more colorants, one or more flavors, and/or one or more fragrances as described above and in the Examples below. In some embodiments, the relative concentrations (e.g., wt%) of each of these ingredients (and any optional additives) in the admixture are also presented above and in the Examples below. The ingredients constituting the admixture can be provided sequentially or in any combination of additions; and, the ingredients or combination of ingredients can be provided in any order. In one embodiment, the

lubricant is the last component added to the admixture.

In another embodiment, the admixture comprises a composition of Compound 3 Amorphous Form, and any one or more of the excipients; a glidant, a surfactant, a diluent, a lubricant, a disintegrant, and a filler, wherein each of these ingredients is provided in a powder form (e.g., provided as particles having a mean or average diameter, measured by light scattering, of 250 μm or less (e.g., 150 μm or less, 100 μm or less, 50 μm or less, 45 μm or less, 40 μm or less, or 35 μm or less)). For instance, the admixture comprises a composition of Compound 3 Amorphous Form, a diluent, a glidant, a surfactant, a lubricant, a disintegrant, and a filler, wherein each of these ingredients is provided in a powder form (e.g., provided as particles having a mean diameter, measured by light scattering, of 250 μm or less (e.g., 150 μm or less, 100 μm or less, 50 μm or less, 45 μm or less, 40 μm or less, or 35 μm or less)). In another example, the admixture comprises a composition of Compound 3 Amorphous Form, a diluent, a surfactant, a lubricant, a disintegrant, and a filler, wherein each of these ingredients is provided in a powder form (e.g., provided as particles having a mean diameter, measured by light scattering, of 250 μm or less (e.g., 150 μm or less, 100 μm or less, 50 μm or less, 45 μm or less, 40 μm or less, or 35 μm or less))

In another embodiment, the admixture comprises a composition of Compound 3 Amorphous Form, and any combination of: a glidant, a diluent, a surfactant, a lubricant, a disintegrant, and a filler, wherein each of these ingredients is substantially free of water. Each of the ingredients comprises less than 5 wt% (e.g., less than 2 wt%, less than 1 wt%, less than 0.75 wt%, less than 0.5 wt%, or less than 0.25 wt%) of water by weight of the ingredient. For instance, the admixture comprises a composition of Compound 3 Amorphous Form, a diluent, a glidant, a surfactant, a lubricant, a disintegrant, and a filler, wherein each of these ingredients is substantially free of water. In some embodiments, each of the ingredients comprises less than 5 wt% (e.g., less than 2 wt%, less than 1 wt%, less than 0.75 wt%, less than 0.5 wt%, or less than 0.25 wt%) of water by weight of the ingredient.

In another embodiment, compressing the admixture into a tablet is accomplished by filling a form (e.g., a mold) with the admixture and applying pressure to admixture. This can be accomplished using a die press or other similar apparatus. In some embodiments, the admixture of Compound 3 Amorphous Form and excipients can be first processed into granular form. The granules can then be sized and compressed into tablets or formulated for encapsulation according to known methods in the pharmaceutical art. It is also noted that the application of pressure to the admixture in the form can be repeated using the same pressure during each compression or using different pressures during the compressions. In another example, the admixture of powdered ingredients or granules can be compressed using a die press that applies sufficient pressure to form a tablet having a dissolution of about 50% or more at about 30 minutes (e.g., about 55% or more at about 30 minutes or about 60% or more at about 30 minutes). For instance, the admixture is compressed using a die press to produce a tablet hardness of at least about 5 kP (at least about 5.5 kP, at least about 6 kP, at least about 7 kP, at least about 10 kP, or at least 15 kP). In some instances, the admixture is compressed to produce a tablet hardness of between about 5 and 20 kP.

In some embodiments, tablets comprising a pharmaceutical composition as described herein can be coated with about 3.0 wt% of a film coating comprising a colorant by weight of the tablet. In certain instances, the colorant suspension or solution used to coat the tablets comprises about 20%w/w of solids by weight of the colorant suspension or solution. In still further instances, the coated tablets can be labeled with a logo, other image or text.

In another embodiment, the method for producing a pharmaceutical composition comprises providing an admixture of a solid forms, e.g. an admixture of powdered and/or liquid ingredients, the admixture comprising Compound 3 Amorphous Form and one or more excipients selected from: a glidant, a diluent, a surfactant, a lubricant, a disintegrant, and a filler; mixing the admixture until the admixture is substantially homogenous, and compressing or compacting the admixture into a granular form. Then the granular composition comprising Compound 3 Amorphous Form can be compressed into tablets or formulated into capsules as described above or in the Examples below. Alternatively, methods for producing a pharmaceutical composition comprises providing an admixture of Compound 3 Amorphous Form, and one or more excipients, e.g. a glidant, a diluent, a surfactant, a lubricant, a disintegrant, and a filler; mixing the admixture until the admixture is substantially homogenous, and compressing/compacting the admixture into a granular form using a roller compactor using a dry granulation composition as set forth in the Examples below or alternatively, compressed/compacted into granules using a high shear wet granule compaction process as set forth in the Examples below. Pharmaceutical formulations, for example a tablet as described herein, can be made using the granules prepared incorporating Compound 3 Amorphous Form in addition to the selected excipients described herein.

In some embodiments, the admixture is mixed by stirring, blending, shaking, or the like using hand mixing, a mixer, a blender, any combination thereof, or the like. When ingredients or combinations of ingredients are added sequentially, mixing can occur between successive additions, continuously throughout the ingredient addition, after the addition of all of the ingredients or combinations of ingredients, or any combination thereof. The admixture is mixed until it has a substantially homogenous composition.

In one embodiment, the pharmaceutical compositions of the present invention may be prepared according to the following flow chart:

**Intra-Granula Blend**

Combine Compound 3 with Glidant and screen through a 20 mesh screen.

Blend in a shaker mixer for 10 minutes.

Add Disintegrant, Filler, and Diluent and blend in the shaker mixer for an additional 15 minutes.

Pass the above blend through a cone mill.

Screen Lubricant with 2-3 times that amount of blend through 20 mesh screen.

Blend for 4 minute.

**Dry Granulation**

Slug the above blend to about 0.5 to 1.0 solid fraction. Calculate solid fraction by measuring the weight, height and using the true density of the material determined during the development.

**Mill**

Lightly break slugs into roughly ¼ inch pieces with mortar and pestle.

Pass the broken slugs through a mill.

**Extragranular Blend**

Screen extragranular Glidant with 2-3x that amount (volume) of blend through a #20 US Mesh screen.

Add the extragranular Glidant pre-blend to the main blend.

Blend in shaker mixer for 15 minutes.

Screen Lubricant through a 20 mesh screen with 2-3 times that amount (volume) of blend.

Blend in shaker mixer for 4 minutes.

**Compression**

Compress tablets to target hardness using specified tooling with gravity fed tablet press.

In another embodiment, the pharmaceutical compositions of the present invention may be prepared according to the following flow chart:

**Intra-Granula Blend**

Combine Compound 3 Amorphous Form with colloidal silica dioxide and screen through a 20 mesh screen.

Blend in a shaker mixer for 10 minutes.

Add crosscarmelose sodium, microcrystalline cellulose, and lactose monohydrate and blend in the shaker mixer for an additional 15 minutes.

Pass the above blend through a cone mill.

Screen magnesium stearate with 2-3 times that amount of blend through 20 mesh screen.

Blend for 4 minute.

↓

**Dry Granulation**

Slug the above blend to about 0.5 to 1.0 solid fraction. Calculate solid fraction by measuring the weight, height and using the true density of the material determined during the development.

↓

**Mill**

Lightly break slugs into roughly ¼ inch pieces with mortar and pestle.

Pass the broken slugs through a mill.

↓

**Extragranular Blend**

Screen extragranular colloidal silica dioxide with 2-3x that amount (volume) of blend through a #20 US Mesh screen.

Add the extragranular colloidal silica dioxide pre-blend to the main blend.

Blend in shaker mixer for 15 minutes.

Screen magnesium stearate through a 20 mesh screen with 2-3 times that amount (volume) of blend.

Blend in shaker mixer for 4 minutes.

↓

**Compression**

Compress tablets to target hardness using specified tooling with gravity fed tablet press.

In another embodiment, Compound 3 Amorphous Form is in a 50% by wgt. mixture with a polymer and surfactant, the brand of colloidal silica dioxide glidant used is Cabot M5P, the brand of crosscarmelose sodium disintegrant used is AcDiSol, the brand of microcrystalline cellulose filler used is Avicel PH101, and the brand of lactose monohydrate diluent used is Foremost 310. In another embodiment, the Compound 3 Amorphous Form polymer is a hydroxylpropylmethylcellulose (HPMC) and the surfactant is sodium lauryl sulfate. In another embodiment, the Compound 3 Amorphous Form polymer is hydroxypropylmethylcellulose acetate succinate (HPMCAS). In another embodiment, the Compound 3 Amorphous Form polymer is hydroxypropylmethylcellulose acetate succinate - high grade (HPMCAS-HG).

In various embodiments, a second therapeutic agent can be formulated together with Compound 3 Amorphous Form to form a unitary or single dose form, for example, a tablet or capsule.

Dosage forms prepared as above can be subjected to in vitro dissolution evaluations according to Test 711 "Dissolution" in United States Pharmacopoeia 29, United States Pharmacopeial Convention, Inc., Rockville, Md., 2005 ("USP"), to determine the rate at which the active substance is released from the dosage forms. The content of active substance and the impurity levels are conveniently measured by techniques such as high performance liquid chromatography (HPLC).

In some embodiments, the invention includes use of packaging materials such as containers and closures of high-density polyethylene (HDPE), low-density polyethylene (LDPE) and or polypropylene and/or glass, glassine foil, aluminum pouches, and blisters or strips composed of aluminum or high-density polyvinyl chloride (PVC), optionally including a desiccant, polyethylene (PE), polyvinylidene dichloride (PVDC), PVC/PE/PVDC, and the like. These package materials can be used to store the various pharmaceutical compositions and formulations in a sterile fashion after appropriate sterilization of the package and its contents using chemical or physical sterilization techniques commonly employed in the pharmaceutical arts.

## VII. Examples

Exemplary Oral Pharmaceutical Formulations Comprising Compound 3

A tablet is prepared with the components and amounts listed in Table 3-11 and Table 3-12.

**Table 3-11**

| Component | Function | Final Blend Composition %w/w | Tablet (mg/tablet) |
|---|---|---|---|
| 50% Compound 3 /49.5% HPMCAS-HG/0.5% SLS | Active as a spray dried dispersion (SSD) | 50.00 | 200.0 SDD (100.00 Compound 3) |
| Microcrystalline cellulose (Avicel PH101) | Filler | 22.63 | 90.5 |
| Lactose Monohydrate (Foremost 310) | Diluent | 22.63 | 90.5 |
| Crosscarmelose Sodium (AcDiSol) | Disintegrant | 3.00 | 12.0 |
| Magnesium Stearate | Lubricant | 0.25 | 1.0 |
| Colloidal Silica Dioxide (Cabot M5P) | Glidant | 1.00 | 4.0 |
| | **Intragranular content** | **99.5** | |
| ***Extragranular Blend*** | | | |
| Colloidal Silica Dioxide (Cabot M5P) | Glidant | 0.25 | 1.0 |
| Magnesium Stearate | Lubricant | 0.25 | 1.0 |
| | **Extragranular content** | **0.5** | |
| | ***Total*** | **100.00** | **400.0** |

**Table 3-12**

| Component | Function | Final Blend Composition %w/w | Tablet (mg/tablet) |
|---|---|---|---|
| 50% Compound 3 /49.5% HPMCAS-HG/0.5% SLS | Active as a spray dried dispersion (SSD) | 50.00 | 100.0 SDD (50.00 Compound 3) |
| Microcrystalline cellulose (Avicel PH101) | Filler | 22.63 | 45.25 |
| Lactose Monohydrate (Foremost 310) | Diluent | 22.63 | 45.25 |

(continued)

| Component | Function | Final Blend Composition %w/w | Tablet (mg/tablet) |
|---|---|---|---|
| Crosscarmelose Sodium (AcDiSol) | Disintegrant | 3.00 | 6.0 |
| Magnesium Stearate | Lubricant | 0.25 | 0.5 |
| Colloidal Silica Dioxide (Cabot M5P) | Glidant | 1.00 | 2.0 |
| | **Intragranular content** | **99.5** | |
| ***Extragranular Blend*** | | | |
| Colloidal Silica Dioxide (Cabot M5P) | Glidant | 0.25 | 0.5 |
| Magnesium Stearate | Lubricant | 0.25 | 0.5 |
| | **Extragranular content** | **0.5** | |
| | ***Total*** | **100.00** | **200.0** |

Tablet Formation from Roller Compaction Granule Composition

Equipment/Process

*Equipment*

| Equipment | Description/Comment |
|---|---|
| Balance(s) (mg to kg scale) | To weigh the powder and individual tablets. |
| Screening and blending equipment | |
| 2-L Turbula T2F Shaker Mixer | Delump/blend/lubrication. |
| Quadro Comill 197 hand screen: size #20 US Mesh screen | Prepare blends for dry granulation and tableting. |
| Dry Granulation equipment | |
| Tableting machine: Korsch XL100 rotary tablet press with gravity feed frame ½ inch diameter, round, flat faced tooling | Prepare slugs with 0.72-0.77 solid fraction. |
| Milling | |
| Mortar/pestle | Particle size reduction. |
| Quadro co-mill (U5/193) | |
| Fitzpatrick (Fitzmill L1A) | |
| Tablet Compression | |
| Tablet machine: Korsch XL100 rotary tablet press with gravity feed frame with 0.2839"x0.5879" modified oval tooling. | Single tooling press. Tablet manufacture. |

Other ancillary equipment for determining
Hardness
Weight sorter
Friability
Deduster
Metal Checker

*ScreeninglWeighing*

Compound 3 Amorphous Form as the solid spray dried dispersion and Cabot M5P are combined and screened

through a 20 mesh screen, and blended in the 2-L Turbula T2F Shaker Mixer for 10 minutes at 32 RPM.

*Intragranular Blending*

The AcDiSol, Avicel PH101, and Foremost 310 are added and blended for an additional 15 minutes. The blend is then passed through the Quadro Comill 197 (screen: 0.032"R; impeller: 1607; RPM: 1000 RPM). Magnesium stearate is screened with 2-3 times that amount (volume) of the above blend through 20 mesh screen by hand. The resulting mixture is blended in the Turbula mixer for 4 minutes at 32 RPM.

*Roller Compaction*

Slug the above blend in the Korsch XL100 rotary tablet press (gravity feed frame ½" diameter, round, flat-faced tooling) to about 0.72 - 0.77 solid fraction. Calculate solid fraction by measuring the weight, height and using the true density of the material determined during the development. For the rotary tablet press slug process, compression force will vary depending on fill volume of the die and final weight of the slug. Lightly break slugs into roughly ¼ inch pieces with mortar and pestle. Pass the broken slugs through the Quadro Comill 197 (screen: 0.079"G; impeller: 1607; RPM: 1000).

*Extragranular Blending*

The extragranular Cabot M5P is screened with 2-3 times that amount (volume) of the above blend through a 20 mesh screen by hand. Add this extragranular Cabot M5P pre-blend to the main blend and blend in the 2-L Turbula T2F Shaker Mixer for 15 minutes at 32 RPM. Screen the extragranular magnesium stearate through a 20 mesh screen with 2-3 times that amount (volume) of the above blend by hand. Add this extragranular magnesium stearate pre-blend to the main blend and blend in the Turbular mixer for 4 minutes at 32 RPM.

*Compression*

Tablets are compressed to target hardness of 14.5 $\pm$ 3.5 kp using a Korsch XL 100 with gravity feed frame and 0.289" x 0.5879" modified oval tooling.

*Film Coating*

Tablets may be film coated using a pan coater, such as, for example an O'Hara Labcoat.

*Pointing*

Film coated tablets may be printed with a monogram on one or both tablet faces with, for example, a Hartnett Delta printer.

**IV.B.1.c. Dosing Administration Schedule of Compound 3 Tablet Formulation**

In another aspect, the invention relates to a method of treating a CFTR mediated disease in a subject comprising administering to a subject in need thereof an effective amount of the pharmaceutical composition provided by the invention. In another embodiment, the pharmaceutical composition is administered to the subject once every two weeks. In another embodiment, the pharmaceutical composition is administered to the subject once a week. In another embodiment, the pharmaceutical composition is administered to the subject once every three days. In another embodiment, the pharmaceutical composition is administered to the subject once a day. In one embodiment, when the pharmaceutical composition is a tablet according to Table 3-11 or 3-12, dosing is once a day.

In one embodiment, 100 mg of Compound 3 may be administered to a subject in need thereof followed by co-administration of 150 mg of a pharmaceutical composition comprising Compound 1 and, optionally, Compound 2. In another embodiment, 100 mg of Compound 3 may be administered to a subject in need thereof followed by co-administration of 250 mg of a pharmaceutical composition comprising Compound 1 and, optionally, Compound 2. In these embodiments, the dosage amounts may be achieved by administration of one or more tablets of the invention. A pharmaceutical composition comprising Compound 1 and, optionally, Compound 2 may be administered as a pharmaceutical composition further comprising Compound 3 and a pharmaceutically acceptable carrier. The duration of administration may continue until amelioration of the disease is achieved or until a subject's physician advises, e.g. duration of administration may be less than a week, 1 week, 2 weeks, 3 weeks, or a month or longer. The co-administration period may

be preceded by an administration period of just Compound 3 alone. For example, there could be administration of 100 mg of Compound 3 for 2 weeks followed by co-administration of 150 mg or 250 mg of a pharmaceutical composition comprising Compound 1 and, optionally, Compound 2 for 1 additional week.

In one embodiment, 100 mg of Compound 3 may be administered once a day to a subject in need thereof followed by co-administration of 150 mg of a pharmaceutical composition comprising Compound 1 and, optionally, Compound 2 once a day. In another embodiment, 100 mg of Compound 3 may be administered once a day to a subject in need thereof followed by co-administration of 250 mg of a pharmaceutical composition comprising Compound 1 and, optionally, Compound 2 once a day. In these embodiments, the dosage amounts may be achieved by administration of one or more tablets of the invention. a pharmaceutical composition comprising Compound 1 and, optionally, Compound 2 may be administered as a pharmaceutical composition further comprising Compound 3 and a pharmaceutically acceptable carrier. The duration of administration may continue until amelioration of the disease is achieved or until a subject's physician advises, e.g. duration of administration may be less than a week, 1 week, 2 weeks, 3 weeks, or a month or longer. The co-administration period may be preceded by an administration period of just Compound 3 alone. For example, there could be administration of 100 mg of Compound 3 for 2 weeks followed by co-administration of 150 mg or 250 mg of a pharmaceutical composition comprising Compound 1 and, optionally, Compound 2 for 1 additional week.

In one embodiment, 100 mg of Compound 3 may be administered once a day to a subject in need thereof followed by co-administration of 150 mg of a pharmaceutical composition comprising Compound 1 and, optionally, Compound 2 every 12 hours. In another embodiment, 100 mg of Compound 3 may be administered once a day to a subject in need thereof followed by co-administration of 250 mg of a pharmaceutical composition comprising Compound 1 and, optionally, Compound 2 every 12 hours. In these embodiments, the dosage amounts may be achieved by administration of one or more tablets of the invention. A pharmaceutical composition comprising Compound 1 and, optionally, Compound 2 may be administered as a pharmaceutical composition further comprising Compound 3 and a pharmaceutically acceptable carrier. The duration of administration may continue until amelioration of the disease is achieved or until a subject's physician advises, e.g. duration of administration may be less than a week, 1 week, 2 weeks, 3 weeks, or a month or longer. The co-administration period may be preceded by an administration period of just Compound 3 alone. For example, there could be administration of 100 mg of Compound 3 for 2 weeks followed by co-administration of 150 mg or 250 mg of a pharmaceutical composition comprising Compound 1 and, optionally, Compound 2 for 1 additional week.

## V. Methods of Use

In yet another aspect, the present invention provides a method of treating a condition, disease, or disorder implicated by CFTR comprising a Compound of Formula I in combination with a Compound of Formula II and/or a Compound of Formula III, comprising administering the formulation to a subject, preferably a mammal, in need thereof. In one embodiment, the pharmaceutical composition comprises Compound 1 and Compound 2. In another embodiment, the pharmaceutical composition comprises Compound 1 and Compound 3. In another embodiment, the pharmaceutical composition comprises Compound 1, Compound 2 and Compound 3. In another embodiment, the pharmaceutical composition comprises components as provided in Table I.

In certain embodiments, the present invention provides a method of treating a condition, disease, or disorder implicated by a deficiency of CFTR activity, the method comprising administering the pharmaceutical composition of the invention to a subject, preferably a mammal, in need thereof.

In yet another aspect, the present invention provides a method of treating, or lessening the severity of a condition, disease, or disorder implicated by CFTR mutation. In certain embodiments, the present invention provides a method of treating a condition, disease, or disorder implicated by a deficiency of the CFTR activity, the method comprising administering the pharmaceutical composition of the invention to a subject, preferably a mammal, in need thereof.

In another aspect, the invention also provides a method of treating or lessening the severity of a disease in a patient, the method comprising administering the pharmaceutical composition of the invention to a subject, preferably a mammal, in need thereof, and said disease is selected from cystic fibrosis, asthma, smoke induced COPD, chronic bronchitis, rhinosinusitis, constipation, pancreatitis, pancreatic insufficiency, male infertility caused by congenital bilateral absence of the vas deferens (CBAVD), mild pulmonary disease, idiopathic pancreatitis, allergic bronchopulmonary aspergillosis (ABPA), liver disease, hereditary emphysema, hereditary hemochromatosis, coagulationfibrinolysis deficiencies, such as protein C deficiency, Type 1 hereditary angioedema, lipid processing deficiencies, such as familial hypercholesterolemia, Type 1 chylomicronemia, abetalipoproteinemia, lysosomal storage diseases, such as 1-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, neprogenic DI, Charcot-Marie Tooth syndrome, Perlizaeus-Merzbacher

disease, neurodegenerative diseases such as Alzheimer's disease, Parkinson's disease, amyotrophic lateral sclerosis, progressive supranuclear plasy, Pick's disease, several polyglutamine neurological disorders such as Huntington's, spinocerebullar ataxia type I, spinal and bulbar muscular atrophy, dentatorubal pallidoluysian, and myotonic dystrophy, as well as spongiform encephalopathies, such as hereditary Creutzfeldt-Jakob disease (due to prion protein processing defect), Fabry disease, Straussler-Scheinker syndrome, COPD, dry-eye disease, or Sjogren's disease, Osteoporosis, Osteopenia, bone healing and bone growth (including bone repair, bone regeneration, reducing bone resorption and increasing bone deposition), Gorham's Syndrome, chloride channelopathies such as myotonia congenita (Thomson and Becker forms), Bartter's syndrome type III, Dent's disease, hyperekplexia, epilepsy, lysosomal storage disease, Angelman syndrome, and Primary Ciliary Dyskinesia (PCD), a term for inherited disorders of the structure and/or function of cilia, including PCD with situs inversus (also known as Kartagener syndrome), PCD without situs inversus and ciliary aplasia.

In some embodiments, the method includes treating or lessening the severity of cystic fibrosis in a patient comprising administering to said patient one of the compositions as defined herein. In certain embodiments, the patient possesses mutant forms of human CFTR. In other embodiments, the patient possesses one or more of the following mutations ΔF508, R117H, and G551D of human CFTR. In one embodiment, the method includes treating or lessening the severity of cystic fibrosis in a patient possessing the ΔF508 mutation of human CFTR comprising administering to said patient one of the compositions as defined herein. In one embodiment, the method includes treating or lessening the severity of cystic fibrosis in a patient possessing the G551D mutation of human CFTR comprising administering to said patient one of the compositions as defined herein. In one embodiment, the method includes treating or lessening the severity of cystic fibrosis in a patient possessing the ΔF508 mutation of human CFTR on at least one allele comprising administering to said patient one of the compositions as defined herein. In one embodiment, the method includes treating or lessening the severity of cystic fibrosis in a patient possessing the ΔF508 mutation of human CFTR on both alleles comprising administering to said patient one of the compositions as defined herein. In one embodiment, the method includes treating or lessening the severity of cystic fibrosis in a patient possessing the G551D mutation of human CFTR on at least one allele comprising administering to said patient one of the compositions as defined herein. In one embodiment, the method includes treating or lessening the severity of cystic fibrosis in a patient possessing the G551D mutation of human CFTR on both alleles comprising administering to said patient one of the compositions as defined herein.

In some embodiments, the method includes lessening the severity of cystic fibrosis in a patient comprising administering to said patient one of the compositions as defined herein. In certain embodiments, the patient possesses mutant forms of human CFTR. In other embodiments, the patient possesses one or more of the following mutations ΔF508, R117H, and G551D of human CFTR. In one embodiment, the method includes lessening the severity of cystic fibrosis in a patient possessing the ΔF508 mutation of human CFTR comprising administering to said patient one of the compositions as defined herein. In one embodiment, the method includes lessening the severity of cystic fibrosis in a patient possessing the G551D mutation of human CFTR comprising administering to said patient one of the compositions as defined herein. In one embodiment, the method includes lessening the severity of cystic fibrosis in a patient possessing the ΔF508 mutation of human CFTR on at least one allele comprising administering to said patient one of the compositions as defined herein. In one embodiment, the method includes lessening the severity of cystic fibrosis in a patient possessing the ΔF508 mutation of human CFTR on both alleles comprising administering to said patient one of the compositions as defined herein. In one embodiment, the method includes lessening the severity of cystic fibrosis in a patient possessing the G551D mutation of human CFTR on at least one allele comprising administering to said patient one of the compositions as defined herein. In one embodiment, the method includes lessening the severity of cystic fibrosis in a patient possessing the G551D mutation of human CFTR on both alleles comprising administering to said patient one of the compositions as defined herein.

In some aspects, the invention provides a method of treating or lessening the severity of Osteoporosis in a patient comprising administering to said patient a composition as defined herein.

In certain embodiments, the method of treating or lessening the severity of Osteoporosis in a patient comprises administering to said patient a pharmaceutical composition as described herein.

In some aspects, the invention provides a method of treating or lessening the severity of Osteopenia in a patient comprising administering to said patient a composition as defined herein.

In certain embodiments, the method of treating or lessening the severity of Osteopenia in a patient comprises administering to said patient a pharmaceutical composition as described herein.

In some aspects, the invention provides a method of bone healing and/or bone repair in a patient comprising administering to said patient a composition as defined herein.

In certain embodiments, the method of bone healing and/or bone repair in a patient comprises administering to said patient a pharmaceutical composition as described herein.

In some aspects, the invention provides a method of reducing bone resorption in a patient comprising administering to said patient a composition as defined herein.

In some aspects, the invention provides a method of increasing bone deposition in a patient comprising administering to said patient a composition as defined herein.

In certain embodiments, the method of increasing bone deposition in a patient comprises administering to said patient a composition as defined herein.

In some aspects, the invention provides a method of treating or lessening the severity of COPD in a patient comprising administering to said patient a composition as defined herein.

In certain embodiments, the method of treating or lessening the severity of COPD in a patient comprises administering to said patient a composition as defined herein.

In some aspects, the invention provides a method of treating or lessening the severity of smoke induced COPD in a patient comprising administering to said patient a composition as defined herein.

In certain embodiments, the method of treating or lessening the severity of smoke induced COPD in a patient comprises administering to said patient a composition as defined herein.

In some aspects, the invention provides a method of treating or lessening the severity of chronic bronchitis in a patient comprising administering to said patient a composition as described herein.

In certain embodiments, the method of treating or lessening the severity of chronic bronchitis in a patient comprises administering to said patient a composition as defined herein.

According to an alternative embodiment, the present invention provides a method of treating cystic fibrosis comprising the step of administering to said mammal a composition as defined herein.

According to the invention an "effective amount" of the composition is that amount effective for treating or lessening the severity of one or more of the diseases, disorders or conditions as recited above.

Another aspect of the present invention provides a method of administering a pharmaceutical composition by orally administering to a patient at least once per day the composition as described herein. In one embodiment, the method comprises administering a composition to said patient a composition as defined herein once of Table I every 24 hours. In another embodiment, the method comprises administering to said patient a composition as defined herein every 12 hours. In a further embodiment, the method comprises administering a to said patient a composition as defined herein three times per day. In still a further embodiment, the method comprises administering to said patient a composition as defined herein.

The compositions, according to the method of the present invention, may be administered using any amount and any route of administration effective for treating or lessening the severity of one or more of the diseases, disorders or conditions as recited above.

In certain embodiments, the compositions of the present invention are useful for treating or lessening the severity of cystic fibrosis in patients who exhibit residual CFTR activity in the apical membrane of respiratory and non-respiratory epithelia. The presence of residual CFTR activity at the epithelial surface can be readily detected using methods known in the art, e.g., standard electrophysiological, biochemical, or histochemical techniques. Such methods identify CFTR activity using *in vivo* or *ex vivo* electrophysiological techniques, measurement of sweat or salivary $Cl^-$ concentrations, or *ex vivo* biochemical or histochemical techniques to monitor cell surface density. Using such methods, residual CFTR activity can be readily detected in patients heterozygous or homozygous for a variety of different mutations, including patients homozygous or heterozygous for the most common mutation, ΔF508.

In another embodiment, the compositions of the present invention are useful for treating or lessening the severity of cystic fibrosis in patients who have residual CFTR activity induced or augmented using pharmacological methods or gene therapy. Such methods increase the amount of CFTR present at the cell surface, thereby inducing a hitherto absent CFTR activity in a patient or augmenting the existing level of residual CFTR activity in a patient.

In one embodiment, a composition as defined herein can be useful for treating or lessening the severity of cystic fibrosis in patients within certain genotypes exhibiting residual CFTR activity, e.g., class III mutations (impaired regulation or gating), class IV mutations (altered conductance), or class V mutations (reduced synthesis) (Lee R. Choo-Kang, Pamela L., Zeitlin, Type I, II, III, IV, and V cystic fibrosis Transmembrane Conductance Regulator Defects and Opportunities of Therapy; Current Opinion in Pulmonary Medicine 6:521 - 529, 2000). Other patient genotypes that exhibit residual CFTR activity include patients homozygous for one of these classes or heterozygous with any other class of mutations, including class I mutations, class II mutations, or a mutation that lacks classification.

In one aspect, the invention includes a method of treating a class III mutation as described above, comprising administering to a patient in need thereof a composition comprising a compound of Formula I in combination with one or both of a compound of Formula II and/or a compound of Formula III. In some embodiments of this aspect, the composition includes a compound of Formula I in combination with a compound of Formula II. In some embodiments of this aspect, the composition includes a compound of Formula I in combination with a compound of Formula III. In some embodiments of this aspect, the composition includes a compound of Formula I in combination with a compound of Formula II and a compound of Formula III. In a further embodiment of this aspect, the pharmaceutical composition includes Compound 1 and Compound 2. In another embodiment, the pharmaceutical composition includes Compound 1 and Compound 3. In another embodiment, the pharmaceutical composition includes Compound 1, Compound 2 and Compound 3.

In one embodiment, a composition as defined herein can be useful for treating or lessening the severity of cystic fibrosis in patients within certain clinical phenotypes, e.g., a moderate to mild clinical phenotype that typically correlates with the amount of residual CFTR activity in the apical membrane of epithelia. Such phenotypes include patients exhibiting pancreatic insufficiency or patients diagnosed with idiopathic pancreatitis and congenital bilateral absence of the vas deferens, or mild lung disease.

The exact amount required will vary from subject to subject, depending on the species, age, and general condition of the subject, the severity of the infection, the particular agent, its mode of administration, and the like. The compositions of the invention are preferably formulated in dosage unit form for ease of administration and uniformity of dosage. The expression "dosage unit form" as used herein refers to a physically discrete unit of agent appropriate for the patient to be treated. It will be understood, however, that the total daily usage of the compositions of the present invention will be decided by the attending physician within the scope of sound medical judgment. The specific effective dose level for any particular patient or organism will depend upon a variety of factors including the disorder being treated and the severity of the disorder; the activity of the composition employed; the specific composition employed; the age, body weight, general health, sex and diet of the patient; the time of administration, route of administration, and rate of excretion of the specific composition employed; the duration of the treatment; drugs used in combination or coincidental with the specific composition employed, and like factors well known in the medical arts. The term "patient", as used herein, means an animal, preferably a mammal, and most preferably a human.

In one aspect, the present invention features a kit comprising a composition as defined herein.

## VI. Assays

### VI.A. Protocol 1

### Assays for Detecting and Measuring ΔF508-CFTR Potentiation

### Properties of Compounds

Membrane potential optical methods for assaying ΔF508-CFTR modulation properties of compounds

The assay utilizes fluorescent voltage sensing dyes to measure changes in membrane potential using a fluorescent plate reader (e.g., FLIPR III, Molecular Devices, Inc.) as a readout for increase in functional ΔF508-CFTR in NIH 3T3 cells. The driving force for the response is the creation of a chloride ion gradient in conjunction with channel activation by a single liquid addition step after the cells have previously been treated with compounds and subsequently loaded with a voltage sensing dye.

Identification of Potentiator Compounds

To identify potentiators of ΔF508-CFTR, a double-addition HTS assay format was developed. This HTS assay utilizes fluorescent voltage sensing dyes to measure changes in membrane potential on the FLIPR III as a measurement for increase in gating (conductance) of ΔF508 CFTR in temperature-corrected ΔF508 CFTR NIH 3T3 cells. The driving force for the response is a $Cl^-$ ion gradient in conjunction with channel activation with forskolin in a single liquid addition step using a fluorescent plate reader such as FLIPR III after the cells have previously been treated with potentiator compounds (or DMSO vehicle control) and subsequently loaded with a redistribution dye.

*Solutions*

Bath Solution #1: (in mM) NaCl 160, KCl 4.5, $CaCl_2$ 2, $MgCl_2$ 1, HEPES 10, pH 7.4 with NaOH.

Chloride-free bath solution: Chloride salts in Bath Solution #1 (above) are substituted with gluconate salts.

*Cell Culture*

NIH3T3 mouse fibroblasts stably expressing ΔF508-CFTR are used for optical measurements of membrane potential. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For all optical assays, the cells were seeded at -20,000/well in 384-well matrigel-coated plates and cultured for 2 hrs at 37 °C before culturing at 27 °C for 24 hrs. for the potentiator assay. For the correction assays, the cells are cultured at 27 °C or 37 °C with and without compounds for 16 - 24 hours.Electrophysiological Assays for

assaying ΔF508-CFTR modulation properties of compounds.

*Using Chamber Assay*

Using chamber experiments were performed on polarized airway epithelial cells expressing ΔF508-CFTR to further characterize the ΔF508-CFTR modulators identified in the optical assays. Non-CF and CF airway epithelia were isolated from bronchial tissue, cultured as previously described (Galietta, L.J.V., Lantero, S., Gazzolo, A., Sacco, O., Romano, L., Rossi, G.A., & Zegarra-Moran, O. (1998) In Vitro Cell. Dev. Biol. 34, 478-481), and plated onto Costar® Snapwell™ filters that were precoated with NIH3T3-conditioned media. After four days the apical media was removed and the cells were grown at an air liquid interface for >14 days prior to use. This resulted in a monolayer of fully differentiated columnar cells that were ciliated, features that are characteristic of airway epithelia. Non-CF HBE were isolated from non-smokers that did not have any known lung disease. CF-HBE were isolated from patients homozygous for ΔF508-CFTR.

HBE grown on Costar® Snapwell™ cell culture inserts were mounted in an Using chamber (Physiologic Instruments, Inc., San Diego, CA), and the transepithelial resistance and short-circuit current in the presence of a basolateral to apical $Cl^-$ gradient (Isc) were measured using a voltage-clamp system (Department of Bioengineering, University of Iowa, IA). Briefly, HBE were examined under voltage-clamp recording conditions ($V_{hold}$ = 0 mV) at 37 °C. The basolateral solution contained (in mM) 145 NaCl, 0.83 $K_2HPO_4$, 3.3 $KH_2PO_4$, 1.2 $MgCl_2$, 1.2 $CaCl_2$, 10 Glucose, 10 HEPES (pH adjusted to 7.35 with NaOH) and the apical solution contained (in mM) 145 NaGluconate, 1.2 $MgCl_2$, 1.2 $CaCl_2$, 10 glucose, 10 HEPES (pH adjusted to 7.35 with NaOH).

*Identification of Potentiator Compounds*

Typical protocol utilized a basolateral to apical membrane $Cl^-$ concentration gradient. To set up this gradient, normal ringers was used on the basolateral membrane, whereas apical NaCl was replaced by equimolar sodium gluconate (titrated to pH 7.4 with NaOH) to give a large $Cl^-$ concentration gradient across the epithelium. Forskolin (10 μM) and all test compounds were added to the apical side of the cell culture inserts. The efficacy of the putative ΔF508-CFTR potentiators was compared to that of the known potentiator, genistein.

*Patch-clamp Recordings*

Total $Cl^-$ current in ΔF508-NIH3T3 cells was monitored using the perforated-patch recording configuration as previously described (Rae, J., Cooper, K., Gates, P., & Watsky, M. (1991) J. Neurosci. Methods 37, 15-26). Voltage-clamp recordings were performed at 22 °C using an Axopatch 200B patch-clamp amplifier (Axon Instruments Inc., Foster City, CA). The pipette solution contained (in mM) 150 *N*-methyl-D-glucamine (NMDG)-Cl, 2 $MgCl_2$, 2 $CaCl_2$, 10 EGTA, 10 HEPES, and 240 μg/mL amphotericin-B (pH adjusted to 7.35 with HCl). The extracellular medium contained (in mM) 150 NMDG-Cl, 2 $MgCl_2$, 2 $CaCl_2$, 10 HEPES (pH adjusted to 7.35 with HCl). Pulse generation, data acquisition, and analysis were performed using a PC equipped with a Digidata 1320 A/D interface in conjunction with Clampex 8 (Axon Instruments Inc.). To activate ΔF508-CFTR, 10 μM forskolin and 20 μM genistein were added to the bath and the current-voltage relation was monitored every 30 sec.

*Identification of Potentiator Compounds*

The ability of ΔF508-CFTR potentiators to increase the macroscopic ΔF508-CFTR $Cl^-$ current ($I_{\Delta F508}$) in NIH3T3 cells stably expressing ΔF508-CFTR was also investigated using perforated-patch-recording techniques. The potentiators identified from the optical assays evoked a dose-dependent increase in $I_{\Delta F508}$ with similar potency and efficacy observed in the optical assays. In all cells examined, the reversal potential before and during potentiator application was around -30 mV, which is the calculated $E_{Cl}$ (-28 mV).

*Cell Culture*

NIH3T3 mouse fibroblasts stably expressing ΔF508-CFTR are used for whole-cell recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For whole-cell recordings, 2,500 - 5,000 cells were seeded on poly-L-lysine-coated glass coverslips and cultured for 24 - 48 hrs at 27 °C before use to test the activity of potentiators; and incubated with or without the corrector compound at 37 °C for measuring the activity of correctors.

*Single-channel recordings*

Gating activity of wt-CFTR and temperature-corrected ΔF508-CFTR expressed in NIH3T3 cells was observed using excised inside-out membrane patch recordings as previously described (Dalemans, W., Barbry, P., Champigny, G., Jallat, S., Dott, K., Dreyer, D., Crystal, R.G., Pavirani, A., Lecocq, J-P., Lazdunski, M. (1991) Nature 354, 526 - 528) using an Axopatch 200B patch-clamp amplifier (Axon Instruments Inc.). The pipette contained (in mM): 150 NMDG, 150 aspartic acid, 5 $CaCl_2$, 2 $MgCl_2$, and 10 HEPES (pH adjusted to 7.35 with Tris base). The bath contained (in mM): 150 NMDG-Cl, 2 $MgCl_2$, 5 EGTA, 10 TES, and 14 Tris base (pH adjusted to 7.35 with HCl). After excision, both wt- and ΔF508-CFTR were activated by adding 1 mM Mg-ATP, 75 nM of the catalytic subunit of cAMP-dependent protein kinase (PKA; Promega Corp. Madison, WI), and 10 mM NaF to inhibit protein phosphatases, which prevented current rundown. The pipette potential was maintained at 80 mV. Channel activity was analyzed from membrane patches containing ≤ 2 active channels. The maximum number of simultaneous openings determined the number of active channels during the course of an experiment. To determine the single-channel current amplitude, the data recorded from 120 sec of ΔF508-CFTR activity was filtered "off-line" at 100 Hz and then used to construct all-point amplitude histograms that were fitted with multigaussian functions using Bio-Patch Analysis software (Bio-Logic Comp. France). The total microscopic current and open probability ($P_o$) were determined from 120 sec of channel activity. The $P_o$ was determined using the Bio-Patch software or from the relationship $P_o$ = I/i(N), where I = mean current, i = single-channel current amplitude, and N = number of active channels in patch.

*Cell Culture*

NIH3T3 mouse fibroblasts stably expressing ΔF508-CFTR are used for excised-membrane patch-clamp recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For single channel recordings, 2,500 - 5,000 cells were seeded on poly-L-lysine-coated glass coverslips and cultured for 24 - 48 hrs at 27 °C before use.

*Activity of the Compound 1*

Compounds of the invention are useful as modulators of ATP binding cassette transporters. The table below illustrates the EC50 and relative efficacy of Compound 1. The following meanings apply. EC50: "+++" means <10 uM; "++" means between 10uM to 25 uM; "+" means between 25 uM to 60uM. % Efficacy: "+" means < 25%; "++" means between 25% to 100%; "+++" means > 100%.

| Cmpd # | EC50 (uM) | % Activity |
|---|---|---|
| 1 | +++ | ++ |

**VI.C. Protocol 2**

**Assays for Detecting and Measuring ΔF508-CFTR Correction Properties of Compounds**

Membrane potential optical methods for assaying ΔF508-CFTR modulation properties of compounds.

The optical membrane potential assay utilized voltage-sensitive FRET sensors described by Gonzalez and Tsien (*See* Gonzalez, J. E. and R. Y. Tsien (1995) "Voltage sensing by fluorescence resonance energy transfer in single cells" Biophys J 69(4): 1272-80, and Gonzalez, J. E. and R. Y. Tsien (1997) "Improved indicators of cell membrane potential that use fluorescence resonance energy transfer" Chem Biol 4(4): 269-77) in combination with instrumentation for measuring fluorescence changes such as the Voltage/Ion Probe Reader (VIPR) (See, Gonzalez, J. E., K. Oades, et al. (1999) "Cell-based assays and instrumentation for screening ion-channel targets" Drug Discov Today 4(9): 431-439).

These voltage sensitive assays are based on the change in fluorescence resonant energy transfer (FRET) between the membrane-soluble, voltage-sensitive dye, $DiSBAC_2(3)$, and a fluorescent phospholipid, CC2-DMPE, which is attached to the outer leaflet of the plasma membrane and acts as a FRET donor. Changes in membrane potential ($V_m$) cause the negatively charged $DiSBAC_2(3)$ to redistribute across the plasma membrane and the amount of energy transfer from CC2-DMPE changes accordingly. The changes in fluorescence emission were monitored using VIPR™ II, which is an integrated liquid handler and fluorescent detector designed to conduct cell-based screens in 96- or 384-well microtiter plates.

Identification of Corrector Compounds

To identify small molecules that correct the trafficking defect associated with ΔF508-CFTR; a single-addition HTS assay format was developed. The cells were incubated in serum-free medium for 16 hrs at 37 °C in the presence or absence (negative control) of test compound. As a positive control, cells plated in 384-well plates were incubated for 16 hrs at 27 °C to "temperature-correct" ΔF508-CFTR.The cells were subsequently rinsed 3X with Krebs Ringers solution and loaded with the voltage-sensitive dyes. To activate ΔF508-CFTR, 10 μM forskolin and the CFTR potentiator, genistein (20 μM), were added along with $Cl^-$-free medium to each well. The addition of $Cl^-$-free medium promoted $Cl^-$ efflux in response to ΔF508-CFTR activation and the resulting membrane depolarization was optically monitored using the FRET-based voltage-sensor dyes.

Identification of Potentiator Compounds

To identify potentiators of ΔF508-CFTR, a double-addition HTS assay format was developed. During the first addition, a $Cl^-$-free medium with or without test compound was added to each well. After 22 sec, a second addition of $Cl^-$-free medium containing 2 - 10 μM forskolin was added to activate ΔF508-CFTR. The extracellular $Cl^-$concentration following both additions was 28 mM, which promoted $Cl^-$ efflux in response to ΔF508-CFTR activation and the resulting membrane depolarization was optically monitored using the FRET-based voltage-sensor dyes.

Solutions

Bath Solution #1: (in mM) NaCl 160, KCl 4.5, $CaCl_2$ 2, $MgCl_2$ 1, HEPES 10, pH 7.4 with NaOH.
Chloride-free bath solution: Chloride salts in Bath Solution #1 (above) are substituted with gluconate salts.
CC2-DMPE: Prepared as a 10 mM stock solution in DMSO and stored at - 20°C.
$DiSBAC_2(3)$: Prepared as a 10 mM stock in DMSO and stored at -20°C.

Cell Culture

NIH3T3 mouse fibroblasts stably expressing ΔF508-CFTR are used for optical measurements of membrane potential. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For all optical assays, the cells were seeded at 30,000/well in 384-well matrigel-coated plates and cultured for 2 hrs at 37 °C before culturing at 27 °C for 24 hrs for the potentiator assay. For the correction assays, the cells are cultured at 27 °C or 37 °C with and without compounds for 16 - 24 hours.
Electrophysiological Assays for assaying ΔF508-CFTR modulation properties of compounds

Ussing Chamber Assay

Using chamber experiments were performed on polarized epithelial cells expressing ΔF508-CFTR to further characterize the ΔF508-CFTR modulators identified in the optical assays. $FRT^{\Delta F508\text{-}CFTR}$ epithelial cells grown on Costar Snapwell cell culture inserts were mounted in an Ussing chamber (Physiologic Instruments, Inc., San Diego, CA), and the monolayers were continuously short-circuited using a Voltage-clamp System (Department of Bioengineering, University of Iowa, IA, and, Physiologic Instruments, Inc., San Diego, CA). Transepithelial resistance was measured by applying a 2-mV pulse. Under these conditions, the FRT epithelia demonstrated resistances of 4 KΩ/ $cm^2$ or more. The solutions were maintained at 27 °C and bubbled with air. The electrode offset potential and fluid resistance were corrected using a cell-free insert. Under these conditions, the current reflects the flow of $Cl^-$ through Δ508-CFTR expressed in the apical membrane. The Isc was digitally acquired using an MP100A-CE interface and AcqKnowledge software (v3.2.6; BIOPAC Systems, Santa Barbara, CA).

Identification of Corrector Compounds

Typical protocol utilized a basolateral to apical membrane $Cl^-$ concentration gradient. To set up this gradient, normal ringer was used on the basolateral membrane, whereas apical NaCl was replaced by equimolar sodium gluconate (titrated to pH 7.4 with NaOH) to give a large $Cl^-$ concentration gradient across the epithelium. All experiments were performed with intact monolayers. To fully activate ΔF508-CFTR, forskolin (10 μM) and the PDE inhibitor, IBMX (100 μM, were applied followed by the addition of the CFTR potentiator, genistein (50 μM).
As observed in other cell types, incubation at low temperatures of FRT cells stably expressing ΔF508-CFTR increases the functional density of CFTR in the plasma membrane. To determine the activity of corrector compounds,

the cells were incubated with 10 μM of the test compound for 24 hours at 37°C and were subsequently washed 3X prior to recording. The cAMP- and genistein-mediated Isc in compound-treated cells was normalized to the 27°C and 37°C controls and expressed as percentage activity. Preincubation of the cells with the corrector compound significantly increased the cAMP-and genistein-mediated Isc compared to the 37°C controls.

Identification of Potentiator Compounds

Typical protocol utilized a basolateral to apical membrane $Cl^-$ concentration gradient. To set up this gradient, normal ringers was used on the basolateral membrane and was permeabilized with nystatin (360 μg/ml), whereas apical NaCl was replaced by equimolar sodium gluconate (titrated to pH 7.4 with NaOH) to give a large $Cl^-$ concentration gradient across the epithelium. All experiments were performed 30 min after nystatin permeabilization. Forskolin (10 μM) and all test compounds were added to both sides of the cell culture inserts. The efficacy of the putative ΔF508-CFTR potentiators was compared to that of the known potentiator, genistein.

Solutions

Basolateral solution (in mM): NaCl (135), $CaCl_2$ (1.2), $MgCl_2$ (1.2), $K_2HPO_4$(2.4), $KHPO_4$ (0.6), N-2-hydroxyethylpiperazine-N'-2-ethanesulfonic acid (HEPES) (10), and dextrose (10). The solution was titrated to pH 7.4 with NaOH.

Apical solution (in mM): Same as basolateral solution with NaCl replaced with Na Gluconate (135).

Cell Culture

Fisher rat epithelial (FRT) cells expressing ΔF508-CFTR ($FRT^{\Delta F508\text{-}CFTR}$) were used for Ussing chamber experiments for the putative ΔF508-CFTR modulators identified from our optical assays. The cells were cultured on Costar Snapwell cell culture inserts and cultured for five days at 37 °C and 5% $CO_2$ in Coon's modified Ham's F-12 medium supplemented with 5% fetal calf serum, 100 U/ml penicillin, and 100 μ/ml streptomycin. Prior to use for characterizing the potentiator activity of compounds, the cells were incubated at 27 °C for 16 - 48 hrs to correct for the ΔF508-CFTR. To determine the activity of correctors compounds, the cells were incubated at 27 °C or 37 °C with and without the compounds for 24 hours.

Whole-cell recordings

The macroscopic ΔF508-CFTR current ($I_{\Delta F508}$) in temperature- and test compound-corrected NIH3T3 cells stably expressing ΔF508-CFTR were monitored using the perforated-patch, whole-cell recording. Briefly, voltage-clamp recordings of $I_{\Delta F508}$ were performed at room temperature using an Axopatch 200B patch-clamp amplifier (Axon Instruments Inc., Foster City, CA). All recordings were acquired at a sampling frequency of 10 kHz and low-pass filtered at 1 kHz. Pipettes had a resistance of 5 - 6 MΩ when filled with the intracellular solution. Under these recording conditions, the calculated reversal potential for $Cl^-$ ($E_{Cl}$) at room temperature was -28 mV. All recordings had a seal resistance > 20 GΩ and a series resistance < 15 MΩ. Pulse generation, data acquisition, and analysis were performed using a PC equipped with a Digidata 1320 A/D interface in conjunction with Clampex 8 (Axon Instruments Inc.). The bath contained < 250 μl of saline and was continuously perifused at a rate of 2 ml/min using a gravity-driven perfusion system,

Identification of Corrector Compounds

To determine the activity of corrector compounds for increasing the density of functional ΔF508-CFTR in the plasma membrane, we used the above-described perforated-patch-recording techniques to measure the current density following 24-hr treatment with the corrector compounds. To fully activate ΔF508-CFTR, 10 μM forskolin and 20 μM genistein were added to the cells. Under our recording conditions, the current density following 24-hr incubation at 27°C was higher than that observed following 24-hr incubation at 37 °C. These results are consistent with the known effects of low-temperature incubation on the density of ΔF508-CFTR in the plasma membrane. To determine the effects of corrector compounds on CFTR current density, the cells were incubated with 10 μM of the test compound for 24 hours at 37°C and the current density was compared to the 27°C and 37°C controls (% activity). Prior to recording, the cells were washed 3X with extracellular recording medium to remove any remaining test compound. Preincubation with 10 μM of corrector compounds significantly increased the cAMP- and genistein-dependent current compared to the 37°C controls.

Identification of Potentiator Compounds

The ability of ΔF508-CFTR potentiators to increase the macroscopic ΔF508-CFTR $Cl^-$ current ($I_{\Delta F508}$) in NIH3T3 cells stably expressing ΔF508-CFTR was also investigated using perforated-patch-recording techniques. The potentiators identified from the optical assays evoked a dose-dependent increase in $I_{\Delta F508}$ with similar potency and efficacy observed in the optical assays. In all cells examined, the reversal potential before and during potentiator application was around -30 mV, which is the calculated $E_{Cl}$ (-28 mV).

Solutions

Intracellular solution (in mM): Cs-aspartate (90), CsCl (50), $MgCl_2$ (1), HEPES (10), and 240 μg/ml amphotericin-B (pH adjusted to 7.35 with CsOH).

Extracellular solution (in mM): N-methyl-D-glucamine (NMDG)-Cl (150), $MgCl_2$ (2), $CaCl_2$ (2), HEPES (10) (pH adjusted to 7.35 with HCl).

Cell Culture

NIH3T3 mouse fibroblasts stably expressing ΔF508-CFTR are used for whole-cell recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For whole-cell recordings, 2,500 - 5,000 cells were seeded on poly-L-lysine-coated glass coverslips and cultured for 24 - 48 hrs at 27 °C before use to test the activity of potentiators; and incubated with or without the corrector compound at 37 °C for measuring the activity of correctors.

Single-channel recordings

The single-channel activities of temperature-corrected ΔF508-CFTR stably expressed in NIH3T3 cells and activities of potentiator compounds were observed using excised inside-out membrane patch. Briefly, voltage-clamp recordings of single-channel activity were performed at room temperature with an Axopatch 200B patch-clamp amplifier (Axon Instruments Inc.). All recordings were acquired at a sampling frequency of 10 kHz and low-pass filtered at 400 Hz. Patch pipettes were fabricated from Corning Kovar Sealing #7052 glass (World Precision Instruments, Inc., Sarasota, FL) and had a resistance of 5 - 8 MΩ when filled with the extracellular solution. The ΔF508-CFTR was activated after excision, by adding 1 mM Mg-ATP, and 75 nM of the cAMP-dependent protein kinase, catalytic subunit (PKA; Promega Corp. Madison, WI). After channel activity stabilized, the patch was perifused using a gravity-driven microperfusion system. The inflow was placed adjacent to the patch, resulting in complete solution exchange within 1 - 2 sec. To maintain ΔF508-CFTR activity during the rapid perifusion, the nonspecific phosphatase inhibitor $F^-$ (10 mM NaF) was added to the bath solution. Under these recording conditions, channel activity remained constant throughout the duration of the patch recording (up to 60 min). Currents produced by positive charge moving from the intra- to extracellular solutions (anions moving in the opposite direction) are shown as positive currents. The pipette potential (Vp) was maintained at 80 mV.

Channel activity was analyzed from membrane patches containing ≤ 2 active channels. The maximum number of simultaneous openings determined the number of active channels during the course of an experiment. To determine the single-channel current amplitude, the data recorded from 120 sec of ΔF508-CFTR activity was filtered "off-line" at 100 Hz and then used to construct all-point amplitude histograms that were fitted with multigaussian functions using Bio-Patch Analysis software (Bio-Logic Comp. France). The total microscopic current and open probability ($P_o$) were determined from 120 sec of channel activity. The $P_o$ was determined using the Bio-Patch software or from the relationship $P_o = I/i(N)$, where I = mean current, i = single-channel current amplitude, and N = number of active channels in patch.

Solutions

Extracellular solution (in mM): NMDG (150), aspartic acid (150), $CaCl_2$ (5), $MgCl_2$ (2), and HEPES (10) (pH adjusted to 7.35 with Tris base).

Intracellular solution (in mM): NMDG-Cl (150), $MgCl_2$ (2), EGTA (5), TES (10), and Tris base (14) (pH adjusted to 7.35 with HCl).

Cell Culture

NIH3T3 mouse fibroblasts stably expressing ΔF508-CFTR are used for excised-membrane patch-clamp recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For single channel recordings, 2,500 - 5,000 cells were seeded on poly-L-lysine-coated glass coverslips and cultured for 24 - 48 hrs at 27 °C before use.

Using the procedures described above, the activity, ($EC_{50}$), of Compound 2 has been measured and is shown in Table 2-10.

**Table 2-10**

| | | |
|---|---|---|
| IC50/EC50 Bins: +++ ⇐ 2.0 < ++ <= 5.0 < + | | |
| PercentActivity Bins: + <= 25.0 < ++ <= 100.0 < +++ | | |
| Cmpd. | Binned EC50 | Binned MaxEfficacy |
| Compound 2 | +++ | +++ |

Using the procedures described above, the activity, i.e., EC50s, of Compound 3 has been measured and is shown in Table 3-13.

**Table 3-13**

| | | |
|---|---|---|
| IC50/EC50 Bins: +++ ⇐ 2.0 < ++ ⇐ 5.0 < + | | |
| PercentActivity Bins: + ⇐ 25.0 < ++ ⇐ 100.0 < +++ | | |
| Cmpd. | Binned EC50 | Binned MaxEfficacy |
| Compound 3 | +++ | +++ |

**OTHER EMBODIMENTS**

All publications and patents referred to in this disclosure are incorporated herein by reference to the same extent as if each individual publication or patent application were specifically and individually indicated to be incorporated by reference. Should the meaning of the terms in any of the patents or publications incorporated by reference conflict with the meaning of the terms used in this disclosure, the meaning of the terms in this disclosure are intended to be controlling. Furthermore, the foregoing discussion discloses and describes merely exemplary embodiments of the present invention. One skilled in the art will readily recognize from such discussion and from the accompanying drawings and claims, that various changes, modifications and variations can be made therein without departing from the spirit and scope of the invention as defined in the following claims.

The invention is further defined with reference to the following clauses.

1. A pharmaceutical composition comprising:

A. A Compound of Formula I

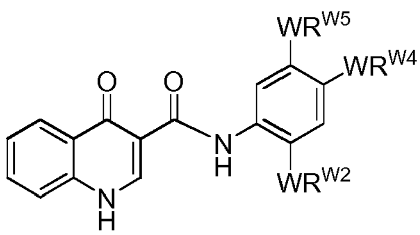

**I**

or a pharmaceutically acceptable salt thereof, wherein:

Each of $WR^{W2}$ and $WR^{W4}$ is independently selected from CN, $CF_3$, halo, $C_{2-6}$ straight or branched alkyl, $C_{3-12}$ membered cycloaliphatic, phenyl, a 5-10 membered heteroaryl or 3-7 membered heterocyclic, wherein said heteroaryl or heterocyclic has up to 3 heteroatoms selected from O, S, or N, wherein said $WR^{W2}$ and $WR^{W4}$ is

independently and optionally substituted with up to three substituents selected from -OR', $-CF_3$, $-OCF_3$, SR', S(O)R', $SO_2R'$, $-SCF_3$, halo, CN, -COOR', -COR', $-O(CH_2)_2N(R')_2$, $-O(CH_2)N(R')_2$,$-CON(R')_2$, $-(CH_2)_2OR'$, $-(CH_2)OR'$, $-CH_2CN$, optionally substituted phenyl or phenoxy, $-N(R')_2$, -NR'C(O)OR', -NR'C(O)R', $-(CH_2)_2N(R')_2$, or $-(CH_2)N(R')_2$;

$WR^{W5}$ is selected from hydrogen, $-OCF_3$ $-CF_3$ -OH, $-OCH_3$ $-NH_2$, -CN, $-CHF_2$, -NHR', $-N(R')_2$, -NHC(O)R', -NHC(O)OR', $-NHSO_2R'$, $-CH_2OH$, $-CH_2N(R')_2$, -C(O)OR', $-SO_2NHR'$, $-SO_2N(R')_2$, or $-CH_2NHC(O)OR'$; and

Each R' is independently selected from an optionally substituted group selected from a $C_{1-8}$ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;

provided that:

iii) $WR^{W2}$ and $WR^{W4}$ are not both -Cl;

$WR^{W2}$, $WR^{W4}$ and $WR^{W5}$ are not $-OCH_2CH_2Ph$, $-OCH_2CH_2$(2-trifluoromethyl-phenyl),$-OCH_2CH_2$-(6,7-dimethoxy-1,2,3,4-tetrahydroisoquinolin-2-yl), or substituted *1H*-pyrazol-3-yl;

and one or both of the following:

B. A Compound of Formula II

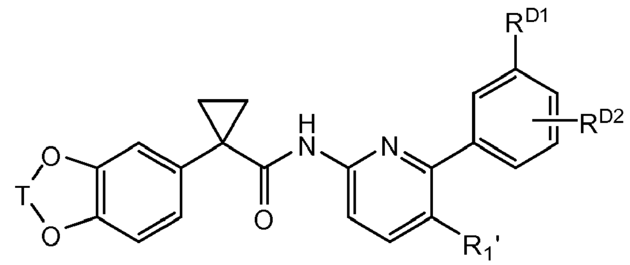

Formula II

or pharmaceutically acceptable salts thereof, wherein:

T is $-CH_2-$, $-CH_2CH_2-$, $-CF_2-$, $-C(CH_3)_2-$, or -C(O)-;

$R_1$' is H, $C_{1-6}$ aliphatic, halo, $CF_3$, $CHF_2$, $O(C_{1-6}$ aliphatic); and

$R^{D1}$ or $R^{D2}$ is $Z^DR_9$

wherein:

$Z^D$ is a bond, CONH, $SO_2NH$ $SO_2N(C_{1-6}$ alkyl), $CH_2NHSO_2$, $CH_2N(CH_3)SO_2$, $CH_2NHCO$, COO, $SO_2$, or CO; and

$R_9$ is H, $C_{1-6}$ aliphatic, or aryl; and/or

C. A Compound of Formula III

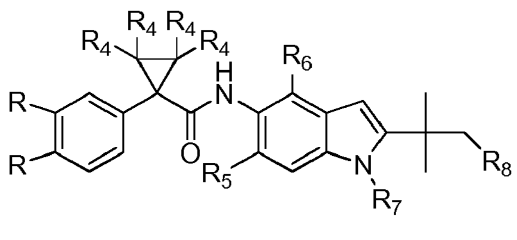

Formula III

or pharmaceutically acceptable salts thereof, wherein:

R is H, OH, $OCH_3$ or two R taken together form $-OCH_2O-$ or $-OCF_2O-$;
$R_4$ is H or alkyl;
$R_5$ is H or F;
$R_6$ is H or CN;
$R_7$ is H, $-CH_2CH(OH)CH_2OH$, $-CH_2CH_2N^+(CH_3)_3$, or $-CH_2CH_2OH$;
$R_8$ is H, OH, $-CH_2CH(OH)CH_2OH$, $-CH_2OH$, or $R_7$ and $R_8$ taken together form a five membered ring.

2. The pharmaceutical composition of clause 1, comprising a Compound of Formula I and a Compound of Formula II.
3. The pharmaceutical composition of clause 1, comprising a Compound of Formula I and a Compound of Formula III.
4. The pharmaceutical composition of clause 1, comprising a Compound of Formula I, a Compound of Formula II and a Compound of Formula III.
5. The pharmaceutical composition of any one of clauses 1-4, wherein the Compound of Formula I is Compound 1

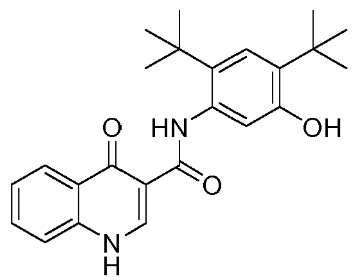

Compound 1

6. The pharmaceutical composition of any one of clauses 1-5, wherein the Compound of Formula II is Compound 2

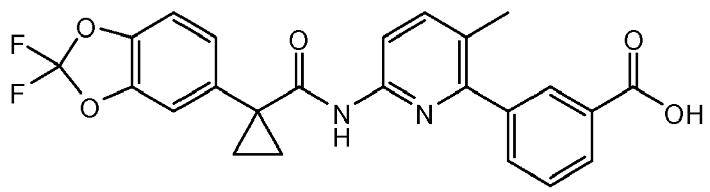

Compound 2

7. The pharmaceutical composition of any one of clauses 1-6, wherein the Compound of Formula III is Compound 3

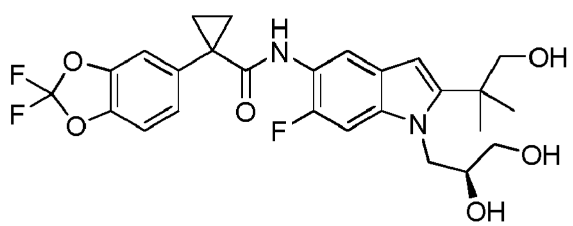

Compound 3

8. A pharmaceutical composition comprising a component selected from any embodiment described in Column A of Table I in combination with one or both of the following:
a) a component selected from any embodiment described in Column B of Table I; and/or
b) a component selected from any embodiment described in Column C of Table I.

**Table I**

| Column A Embodiments | | Column B Embodiments | | Column C Embodiments | |
|---|---|---|---|---|---|
| **Section** | **Heading** | **Section** | **Heading** | **Section** | **Heading** |
| **II.A.1.** | Compounds of Formula I | **II.B.1.** | Compounds of Formula II | **II.C.1.** | Compounds of Formula III |
| **II.A.2.** | Compound 1 | **II.B.2.** | Compound 2 | **II.C.2.** | Compound 3 |
| **III.A.1.a.** | Compound 1 Form C | **III.B.1.a.** | Compound 2 Form I | **III.C.1.a.** | Compound 3 Form A |
| **IV.A.1.a.** | Compound 1 First Formulation | **III.B.2.a.** | Compound 2 Solvate Form A | **III.C.2.a.** | Compound 3 Amorphous Form |
| **IV.A.2.a.** | Compound 1 Tablet and SDD Formulation | **III.B.3.a.** | Compound 2 HCl Salt Form A | **IV.B.1.a.** | Compound 3 Tablet Formulation |

9. A method of treating a CFTR mediated disease in a human comprising administering to the human an effective amount of a pharmaceutical composition according to any one of clauses 1-8.

10. The method of clause 9, wherein the CFTR mediated disease is selected from cystic fibrosis, asthma, smoke induced COPD, chronic bronchitis, rhinosinusitis, constipation, pancreatitis, pancreatic insufficiency, male infertility caused by congenital bilateral absence of the vas deferens (CBAVD), mild pulmonary disease, idiopathic pancreatitis, allergic bronchopulmonary aspergillosis (ABPA), liver disease, hereditary emphysema, hereditary hemochromatosis, coagulation-fibrinolysis deficiencies, such as protein C deficiency, Type 1 hereditary angioedema, lipid processing deficiencies, such as familial hypercholesterolemia, Type 1 chylomicronemia, abetalipoproteinemia, lysosomal storage diseases, such as I-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, neprogenic DI, Charcot-Marie Tooth syndrome, Perlizaeus-Merzbacher disease, neurodegenerative diseases such as Alzheimer's disease, Parkinson's disease, amyotrophic lateral sclerosis, progressive supranuclear plasy, Pick's disease, several polyglutamine neurological disorders such as Huntington's, spinocerebullar ataxia type I, spinal and bulbar muscular atrophy, dentatorubal pallidoluysian, and myotonic dystrophy, as well as spongiform encephalopathies, such as hereditary Creutzfeldt-Jakob disease (due to prion protein processing defect), Fabry disease, Straussler-Scheinker syndrome, COPD, dry-eye disease, or Sjogren's disease, Osteoporosis, Osteopenia, bone healing and bone growth (including bone repair, bone regeneration, reducing bone resorption and increasing bone deposition), Gorham's Syndrome, chloride channelopathies such as myotonia congenita (Thomson and Becker forms), Bartter's syndrome type III, Dent's disease, hyperekplexia, epilepsy, lysosomal storage disease, Angelman syndrome, and Primary Ciliary Dyskinesia (PCD), a term for inherited disorders of the structure and/or function of cilia, including PCD with situs inversus (also known as Kartagener syndrome), PCD without situs inversus and ciliary aplasia.

11. The method of any one of clauses 9-10, wherein the CFTR mediated disease is cystic fibrosis, COPD, emphysema, dry-eye disease or osteoporosis.

12. The method of any one of clauses 9-11, wherein the CFTR mediated disease is cystic fibrosis.

13. The method according to any one of clauses 9-12, wherein the patient possesses one or more of the following mutations of human CFTR: ΔF508, R117H, and G551D.

14. The method according to any one of clauses 9-13, wherein the method includes treating or lessening the severity of cystic fibrosis in a patient possessing the ΔF508 mutation of human CFTR.

15. The method according to any one of clauses 9-14, wherein the method includes treating or lessening the severity of cystic fibrosis in a patient possessing the G551D mutation of human CFTR.

16. The method according to any one of clauses 9-15, wherein the method includes treating or lessening the severity of cystic fibrosis in a patient possessing the ΔF508 mutation of human CFTR on at least one allele.

17. The method according to any one of clauses 9-16, wherein the method includes treating or lessening the severity of cystic fibrosis in a patient possessing the ΔF508 mutation of human CFTR on both alleles.

18. The method according to any one of clauses 9-17, wherein the method includes treating or lessening the severity of cystic fibrosis in a patient possessing the G551D mutation of human CFTR on at least one allele.

19. The method according to any one of clauses 9-18, wherein the method includes treating or lessening the severity of cystic fibrosis in a patient possessing the G551D mutation of human CFTR on both alleles.

20. A kit for use in measuring the activity of a CFTR or a fragment thereof in a biological sample *in vitro* or *in vivo,* comprising:

(i) a pharmaceutical composition according to any one of clauses 1-8;
(ii) instructions for:

a) contacting the composition with the biological sample;
b) measuring activity of said CFTR or a fragment thereof.

21. The kit of clause 20, further comprising instructions for

a) contacting an additional compound with the biological sample;
b) measuring the activity of said CFTR or a fragment thereof in the presence of said additional compound, and
c) comparing the activity of said CFTR or fragment thereof in the presence of said additional compound with the activity of the CFTR or fragment thereof in the presence of a composition comprising a pharmaceutical composition according to any one of clauses 1-8.

22. The kit of clause 21, wherein the step of comparing the activity of said CFTR or fragment thereof provides a measure of the density of said CFTR or fragment thereof.

## Claims

**1.** A pharmaceutical composition comprising:

A. A Compound of Formula I

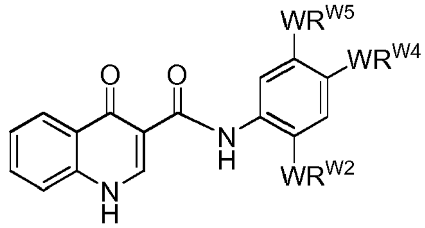

**I**

or a pharmaceutically acceptable salt thereof, wherein:

Each of $WR^{W2}$ and $WR^{W4}$ is independently selected from CN, $CF_3$, halo, $C_{2-6}$ straight or branched alkyl, $C_{3-12}$ membered cycloaliphatic, phenyl, a 5-10 membered heteroaryl or 3-7 membered heterocyclic, wherein said heteroaryl or heterocyclic has up to 3 heteroatoms selected from O, S, or N, wherein said $WR^{W2}$ and $WR^{W4}$ is independently and optionally substituted with up to three substituents selected from-OR', $-CF_3$, $-OCF_3$, SR', S(O)R', $SO_2R'$, $-SCF_3$, halo, CN, -COOR', -COR',$-O(CH_2)_2N(R')_2$, $-O(CH_2)N(R')_2$, $-CON(R')_2$, $-(CH_2)_2OR'$, $-(CH_2)OR'$, $-CH_2CN$, optionally substituted phenyl or phenoxy, $-N(R')_2$, -NR'C(O)OR', -NR'C(O)R', $-(CH_2)_2N(R')_2$, or $-(CH_2)N(R')_2$;
$WR^{W5}$ is selected from hydrogen, -OCF3 -CF3 -OH, $-OCH_3$ - $-NH_2$, -CN,$-CHF_2$, -NHR', $-N(R')_2$, -NHC(O)R', -NHC(O)OR', $-NHSO_2R'$, $-CH_2OH$, $-CH_2N(R')_2$, -C(O)OR', $-SO_2NHR'$, $-SO_2N(R')_2$, or $-CH_2NHC(O)OR'$; and
Each R' is independently selected from an optionally substituted group selected from a $C_{1-8}$ aliphatic group, a 3-8-membered saturated, partially unsaturated, or fully unsaturated monocyclic ring having 0-3 heteroatoms independently selected from nitrogen, oxygen, or sulfur, or an 8-12 membered saturated, partially unsaturated, or fully unsaturated bicyclic ring system having 0-5 heteroatoms independently selected from nitrogen, oxygen, or sulfur; or two occurrences of R' are taken together with the atom(s) to which they are bound to form an optionally substituted 3-12 membered saturated, partially unsaturated, or fully unsaturated monocyclic or bicyclic ring having 0-4 heteroatoms independently selected from nitrogen, oxygen, or sulfur;

provided that:

i) $WR^{W2}$ and $WR^{W4}$ are not both -Cl;
$WR^{W2}$, $WR^{W4}$ and $WR^{W5}$are not $-OCH_2CH_2Ph$, $-OCH_2CH_2$(2-trifluoromethyl-phenyl),$-OCH_2CH_2$-(6,7-

dimethoxy-1,2,3,4-tetrahydroisoquinolin-2-yl), or substituted 1*H* pyrazol-3-yl; and one or both of the following:

B. A Compound of Formula II

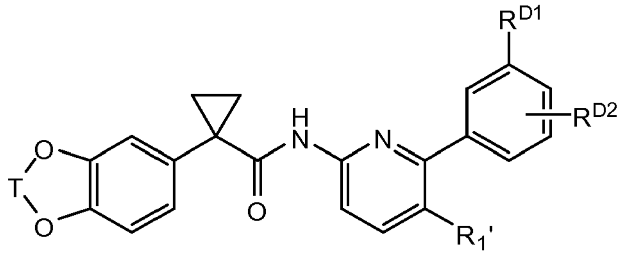

Formula II

or pharmaceutically acceptable salts thereof, wherein:

T is $-CH_2-$, $-CH_2CH_2-$, $-CF_2-$, $-C(CH_3)_2-$, or -C(O)-;
$R_1$' is H, $C_{1-6}$ aliphatic, halo, $CF_3$, $CHF_2$, O($C_{1-6}$ aliphatic); and
$R^{D1}$ or $R^{D2}$ is $Z^DR_9$
wherein:

$Z^D$ is a bond, CONH, $SO_2NH$ $SO_2N(C_{1-6}$ alkyl), $CH_2NHSO_2$, $CH_2N(CH_3)SO_2$, $CH_2NHCO$, COO, $SO_2$, or CO; and
$R_9$ is H, $C_{1-6}$ aliphatic, or aryl; and/or

C. A Compound of Formula III

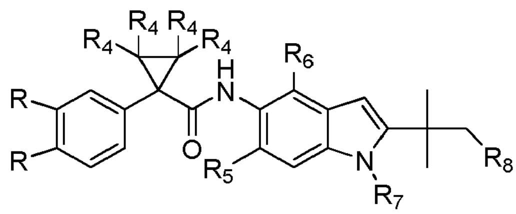

Formula III

or pharmaceutically acceptable salts thereof, wherein:

R is H, OH, $OCH_3$ or two R taken together form $-OCH_2O-$ or $-OCF_2O-$;
$R_4$ is H or alkyl;
$R_5$ is H or F;
$R_6$ is H or CN;
$R_7$ is H, $-CH_2CH(OH)CH_2OH$, $-CH_2CH_2N^+(CH_3)_3$, or $-CH_2CH_2OH$;
$R_8$ is H, OH, $-CH_2CH(OH)CH_2OH$, $-CH_2OH$, or $R_7$ and $R_8$ taken together form a five membered ring.

**2.** The pharmaceutical composition of claim 1, comprising a Compound of Formula I and a Compound of Formula II.

**3.** The pharmaceutical composition of claim 1, comprising a Compound of Formula I and a Compound of Formula III.

**4.** The pharmaceutical composition of claim 1, comprising a Compound of Formula I, a Compound of Formula II and a Compound of Formula III.

**5.** The pharmaceutical composition of any one of claims 1-4, wherein the Compound of Formula I is Compound 1

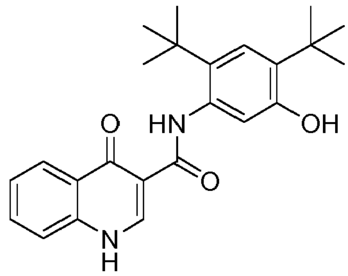

Compound 1

**6.** The pharmaceutical composition of any one of claims 1-5, wherein the Compound of Formula II is Compound 2

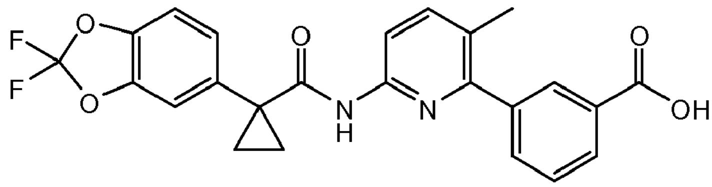

Compound 2

**7.** The pharmaceutical composition of any one of claims 1-6, wherein the Compound of Formula III is (*R*)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide (Compound 3).

**8.** A pharmaceutical composition comprising a component selected from any embodiment described in Column A of Table I in combination with one or both of the following:

a) a component selected from any embodiment described in Column B of Table I; and/or
b) a component selected from any embodiment described in Column C of Table I.

**Table I**

| Column A Embodiments | | Column B Embodiments | | Column C Embodiments | |
|---|---|---|---|---|---|
| **Section** | **Heading** | **Section** | **Heading** | **Section** | **Heading** |
| **II.A.1.** | Compounds of Formula I | **II.B.1.** | Compounds of Formula II | **II.C.1.** | Compounds of Formula III |
| **II.A.2.** | Compound 1 | **II.B.2.** | Compound 2 | **II.C.2.** | Compound 3 |
| **III.A.1.a.** | Compound 1 Form C | **III.1.a.** | Compound 2 Form I | **III.C.1.a.** | Compound 3 Form A |
| **IV.A.1.a.** | Compound 1 First Formulation | **III.B.2.a.** | Compound 2 Solvate Form A | **III.C.2.a.** | Compound 3 Amorphous Form |
| **IV.A.2.a.** | Compound 1 Tablet and SDD Formulation | **III.B.3.a.** | Compound 2 HCl Salt Form A | **IV.B.1.a.** | Compound 3 Tablet Formulation |

**9.** A pharmaceutical composition according to any one of claim 1-8 for use in a method of treating a CFTR mediated disease in a human, wherein the method comprises administering to the human an effective amount of the pharmaceutical composition according to any one of claims 1-8.

**10.** The pharmaceutical composition for use as in claim 9, wherein the CFTR mediated disease is selected from cystic

fibrosis, asthma, smoke induced COPD, chronic bronchitis, rhinosinusitis, constipation, pancreatitis, pancreatic insufficiency, male infertility caused by congenital bilateral absence of the vas deferens (CBAVD), mild pulmonary disease, idiopathic pancreatitis, allergic bronchopulmonary aspergillosis (ABPA), liver disease, hereditary emphysema, hereditary hemochromatosis, coagulation-fibrinolysis deficiencies, such as protein C deficiency, Type 1 hereditary angioedema, lipid processing deficiencies, such as familial hypercholesterolemia, Type 1 chylomicronemia, abetalipoproteinemia, lysosomal storage diseases, such as I-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, neprogenic DI, Charcot-Marie Tooth syndrome, Perlizaeus-Merzbacher disease, neurodegenerative diseases such as Alzheimer's disease, Parkinson's disease, amyotrophic lateral sclerosis, progressive supranuclear plasy, Pick's disease, several polyglutamine neurological disorders such as Huntington's, spinocerebullar ataxia type I, spinal and bulbar muscular atrophy, dentatorubal pallidoluysian, and myotonic dystrophy, as well as spongiform encephalopathies, such as hereditary Creutzfeldt-Jakob disease (due to prion protein processing defect), Fabry disease, Straussler-Scheinker syndrome, COPD, dry-eye disease, or Sjogren's disease, Osteoporosis, Osteopenia, bone healing and bone growth (including bone repair, bone regeneration, reducing bone resorption and increasing bone deposition), Gorham's Syndrome, chloride channelopathies such as myotonia congenita (Thomson and Becker forms), Bartter's syndrome type III, Dent's disease, hyperekplexia, epilepsy, lysosomal storage disease, Angelman syndrome, and Primary Ciliary Dyskinesia (PCD), a term for inherited disorders of the structure and/or function of cilia, including PCD with situs inversus (also known as Kartagener syndrome), PCD without situs inversus and ciliary aplasia.

**11.** The pharmaceutical composition for use as in any one of claims 9-10, wherein the CFTR mediated disease is cystic fibrosis, COPD, emphysema, dry-eye disease or osteoporosis,
preferably wherein the CFTR mediated disease is cystic fibrosis.

**12.** The pharmaceutical composition for use as in any one of claims 9-11, wherein the patient possesses one or more of the following mutations of human CFTR: ΔF508, R117H, and G551D,
preferably wherein the method includes treating or lessening the severity of cystic fibrosis in a patient possessing the ΔF508 mutation of human CFTR, or wherein the method includes treating or lessening the severity of cystic fibrosis in a patient possessing the G551D mutation of human CFTR.

**13.** The pharmaceutical composition for use as in any one of claims 9-12, wherein the method includes treating or lessening the severity of cystic fibrosis in a patient possessing the ΔF508 mutation of human CFTR on at least one allele, preferably wherein the method includes treating or lessening the severity of cystic fibrosis in a patient possessing the ΔF508 mutation of human CFTR on both alleles.

**14.** The pharmaceutical composition for use as in any one of claims 9-13, wherein the method includes treating or lessening the severity of cystic fibrosis in a patient possessing the G551D mutation of human CFTR on at least one allele, preferably wherein the method includes treating or lessening the severity of cystic fibrosis in a patient possessing the G551D mutation of human CFTR on both alleles.

**15.** A kit for use in measuring the activity of a CFTR or a fragment thereof in a biological sample *in vitro* or *in vivo,* comprising:

(i) a pharmaceutical composition according to any one of claims 1-8;
(ii) instructions for:

a) contacting the composition with the biological sample;
b) measuring activity of said CFTR or a fragment thereof;

preferably further comprising instructions for

a) contacting an additional compound with the biological sample;
b) measuring the activity of said CFTR or a fragment thereof in the presence of said additional compound, and
c) comparing the activity of said CFTR or fragment thereof in the presence of said additional compound with the activity of the CFTR or fragment thereof in the presence of a composition comprising a pharmaceutical composition according to any one of claims 1-8;

more preferably, wherein the step of comparing the activity of said CFTR or fragment thereof provides a measure of the density of said CFTR or fragment thereof.

Figure 1-1

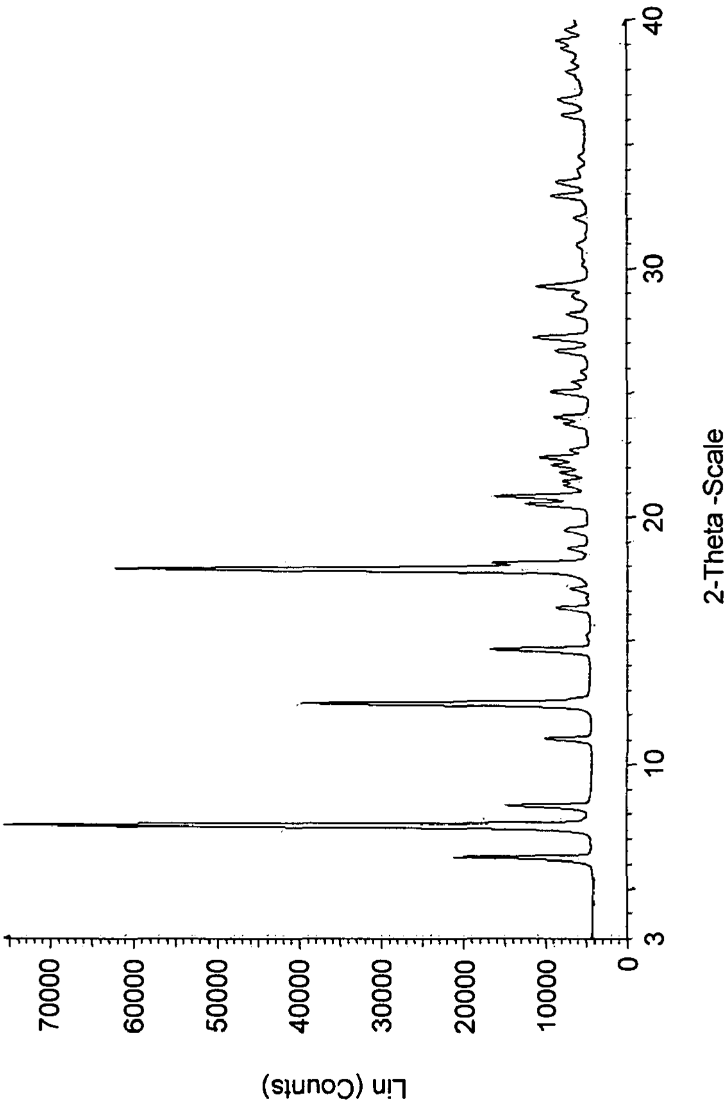
Lin (Counts)
0
10000
20000
30000
40000
50000
60000
70000
2-Theta -Scale
3
10
20
30
40

Figure 1-2

DSC

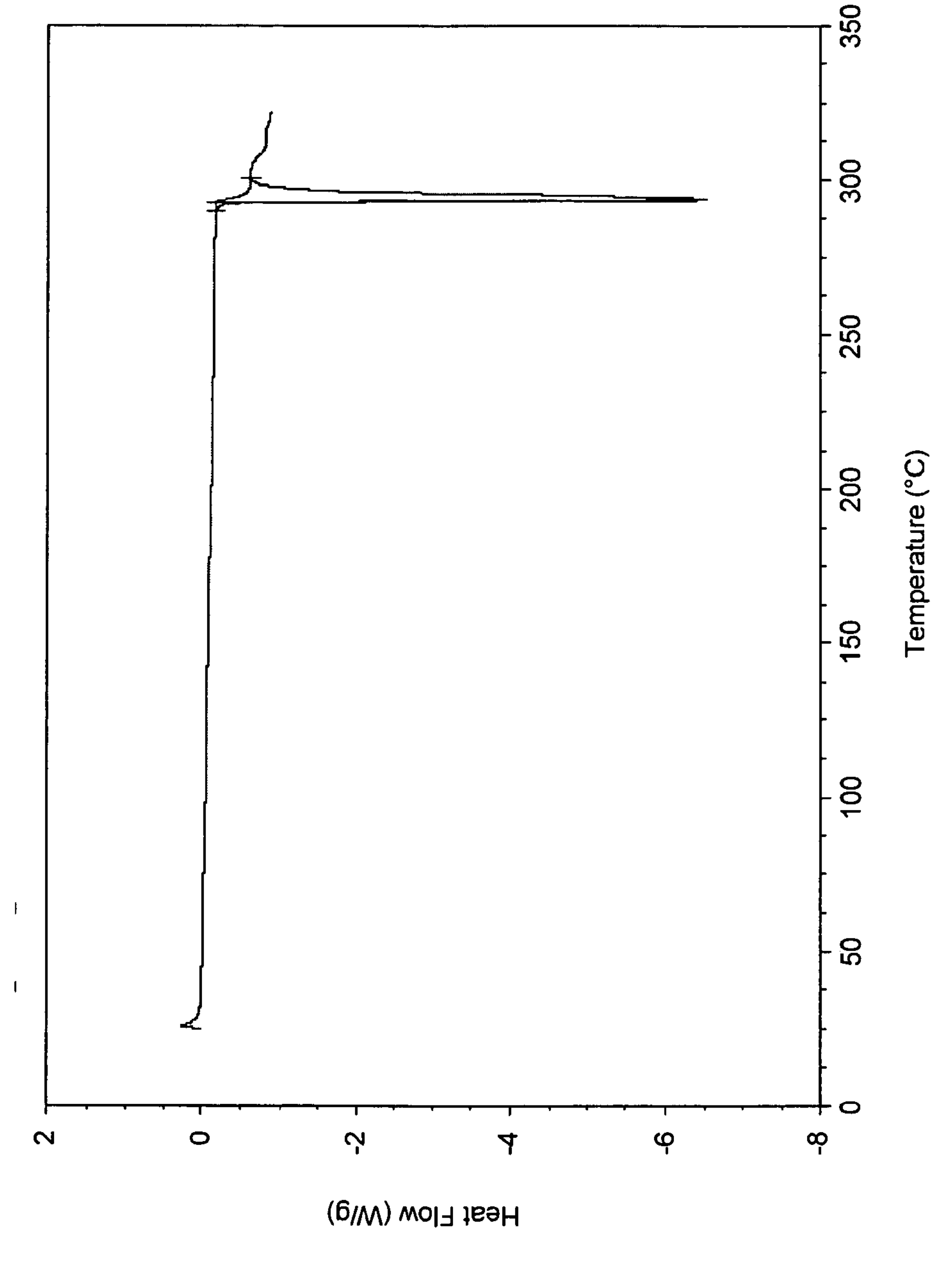

Figure 1-3

TGA

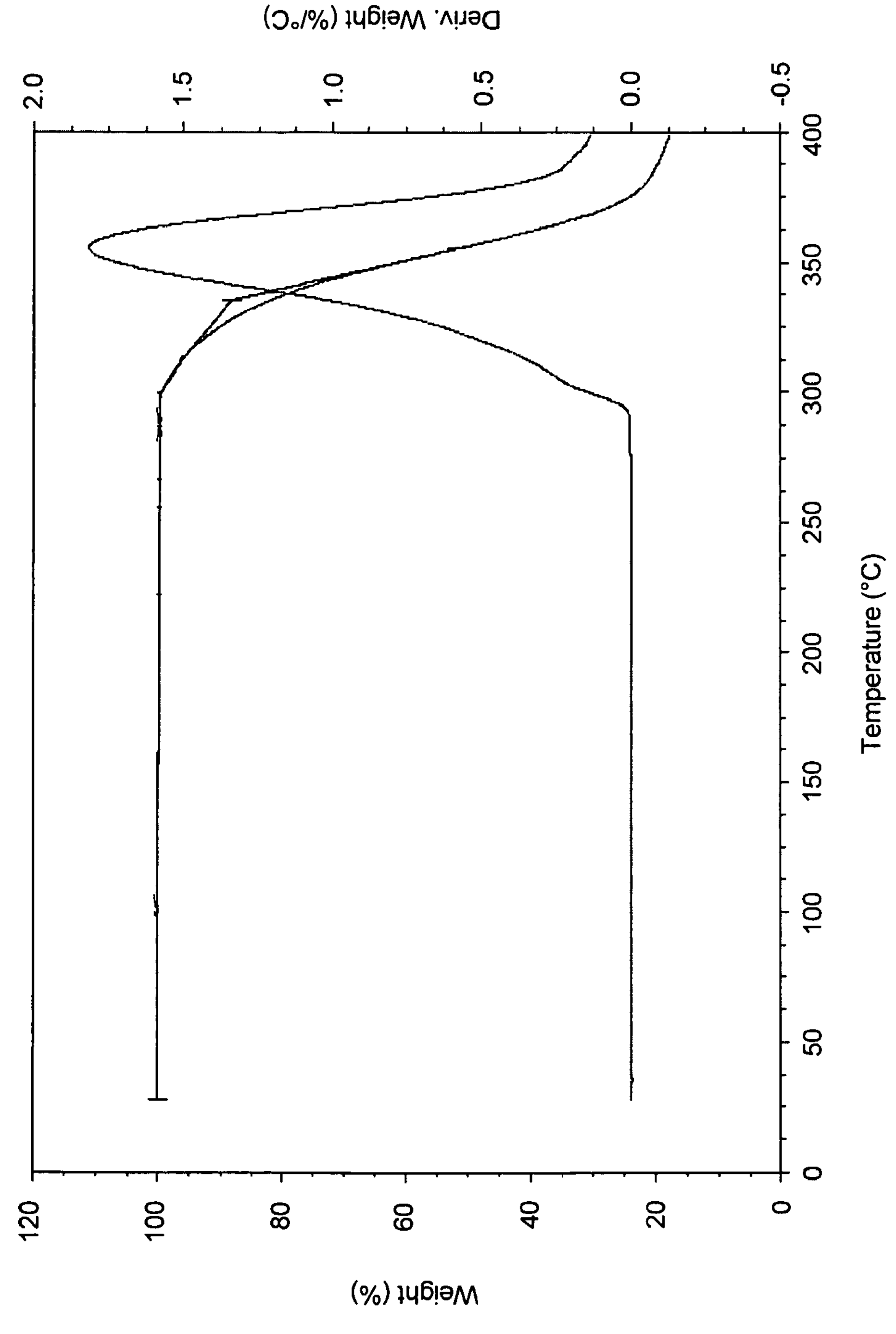

Figure 1-4

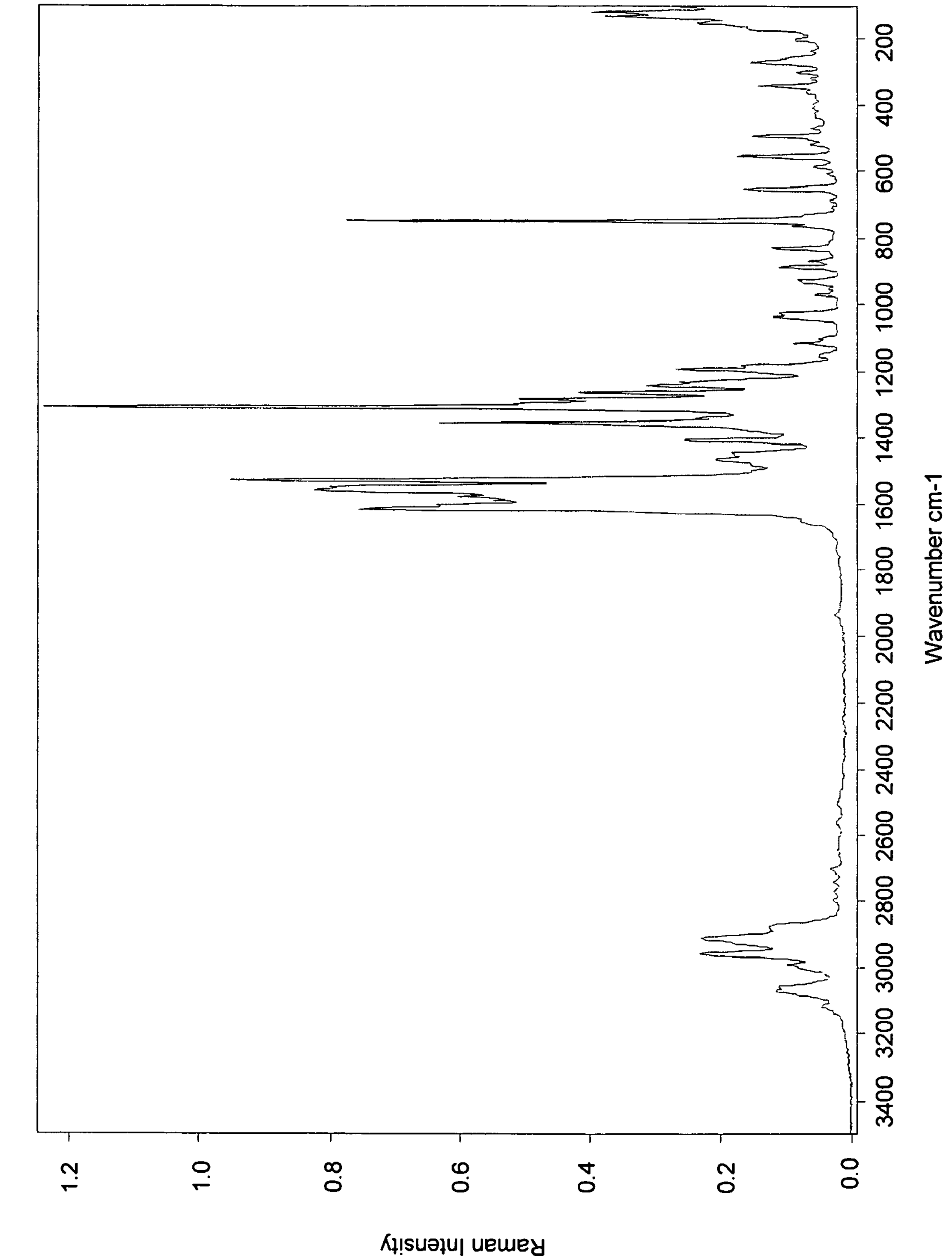
Raman Intensity
0.0
0.2
0.4
0.6
0.8
1.0
1.2
Wavenumber cm-1
3400
3200
3000
2800
2600
2400
2200
2000
1800
1600
1400
1200
1000
800
600
400
200

Figure 1-5

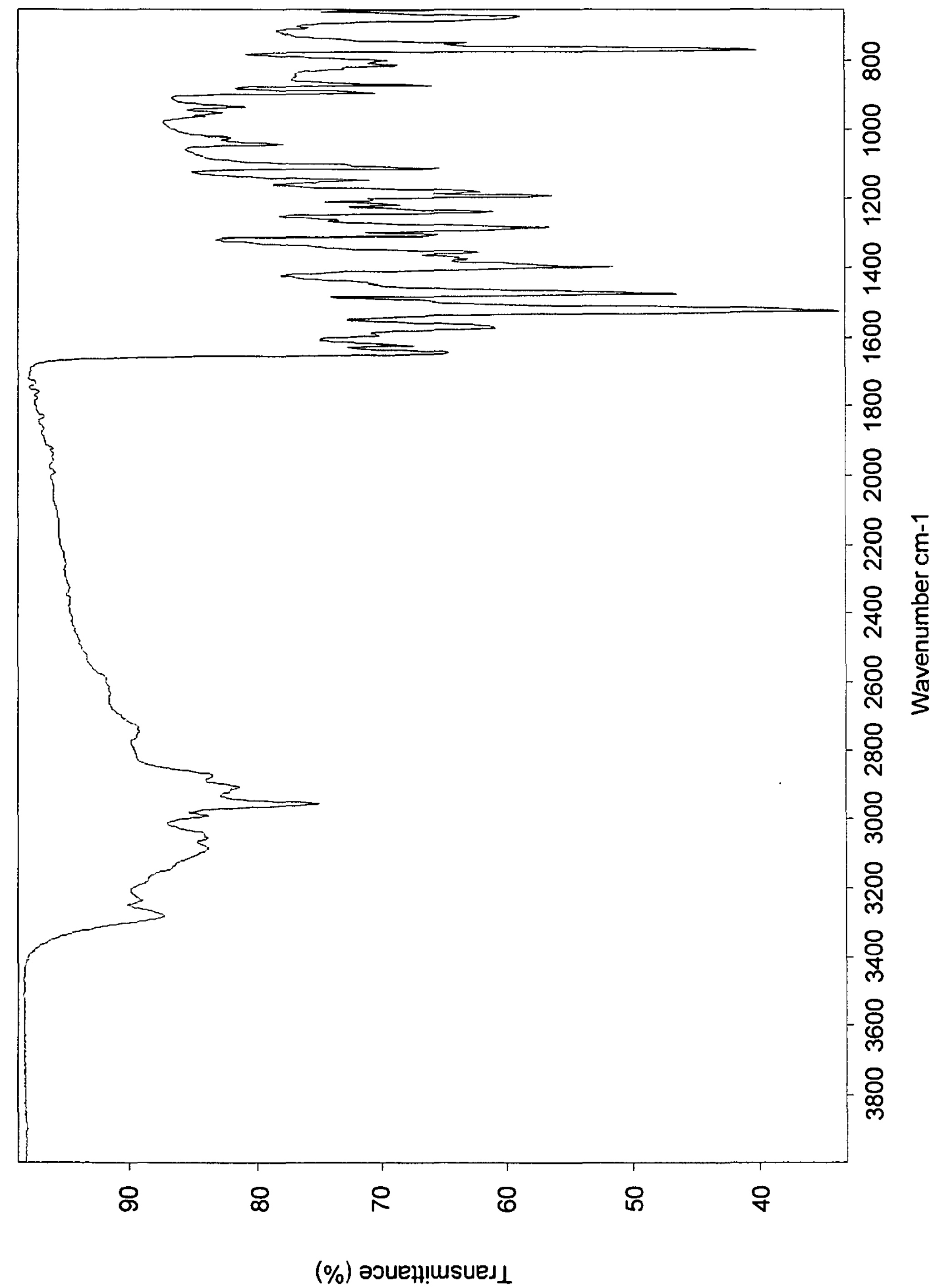
Transmittance (%)
90
80
70
60
50
40
Wavenumber cm-1
3800
3600
3400
3200
3000
2800
2600
2400
2200
2000
1800
1600
1400
1200
1000
800

Figure 1-6

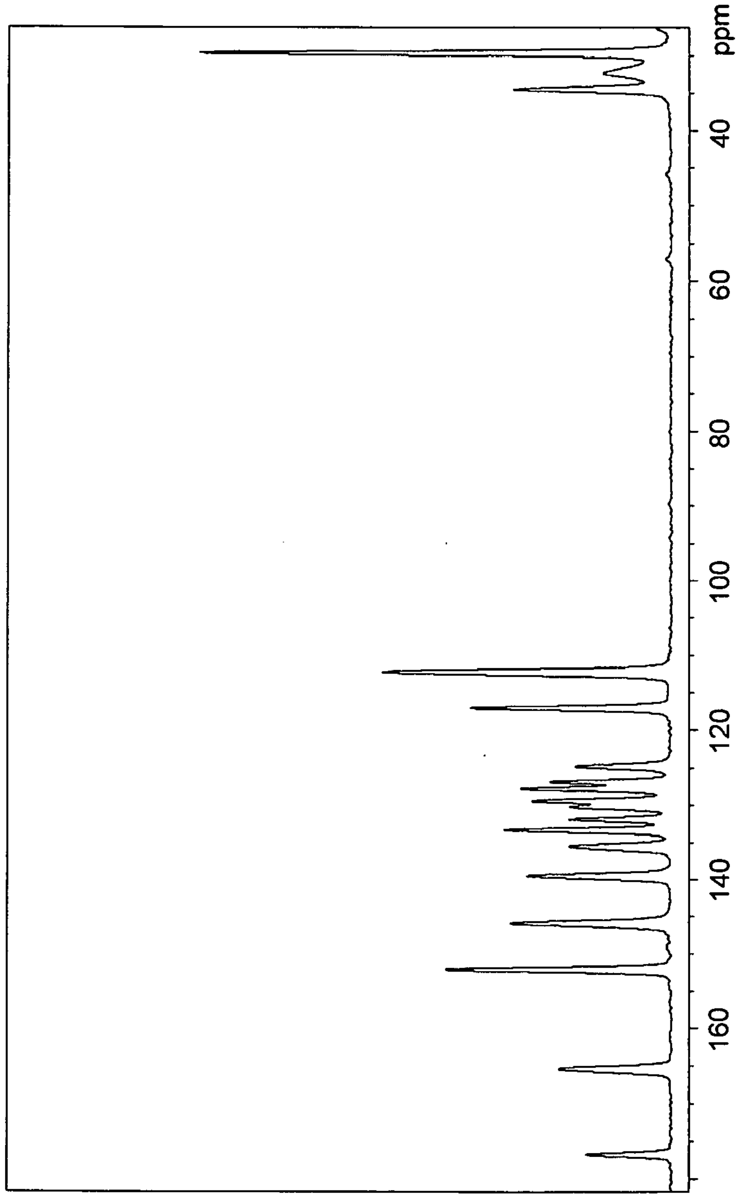

Figure 2-1

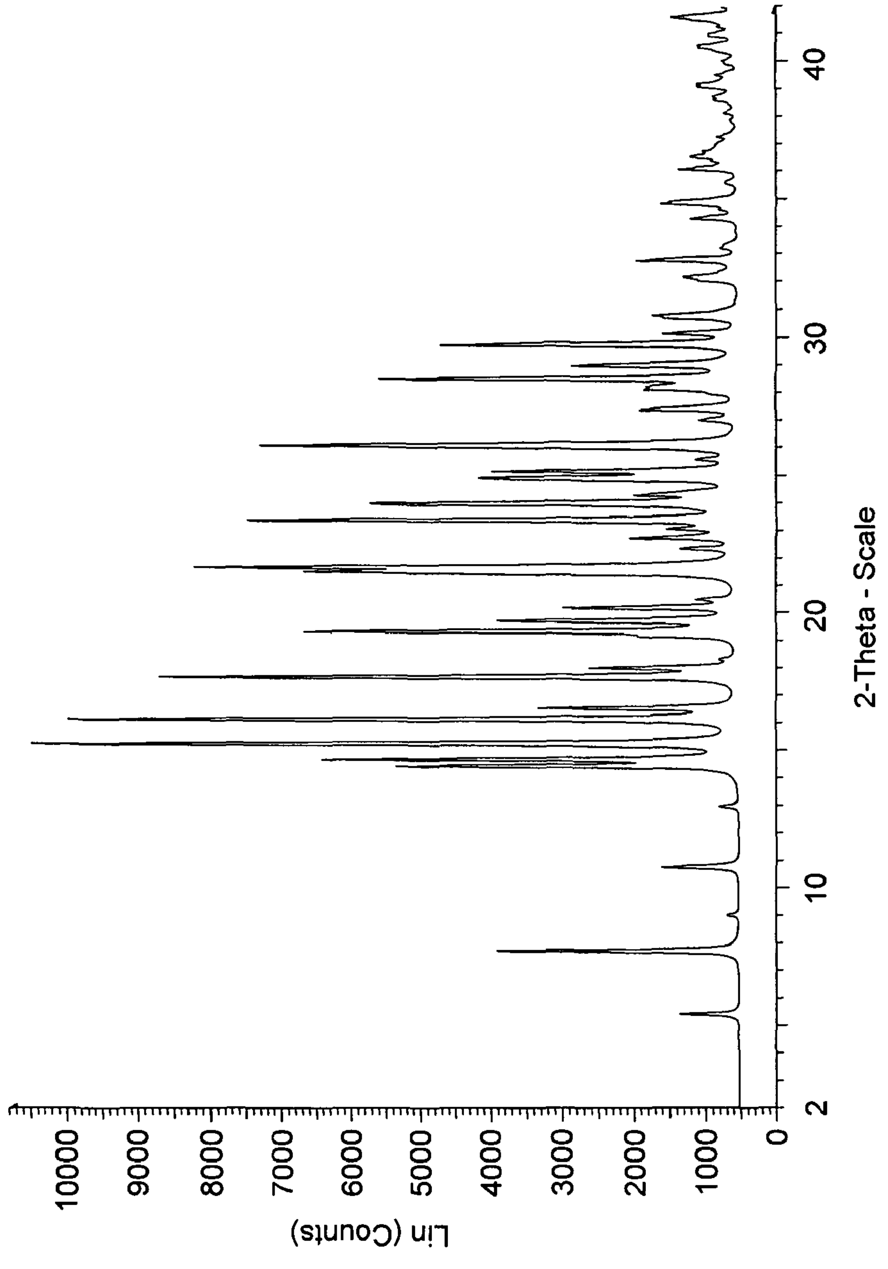

Figure 2-2

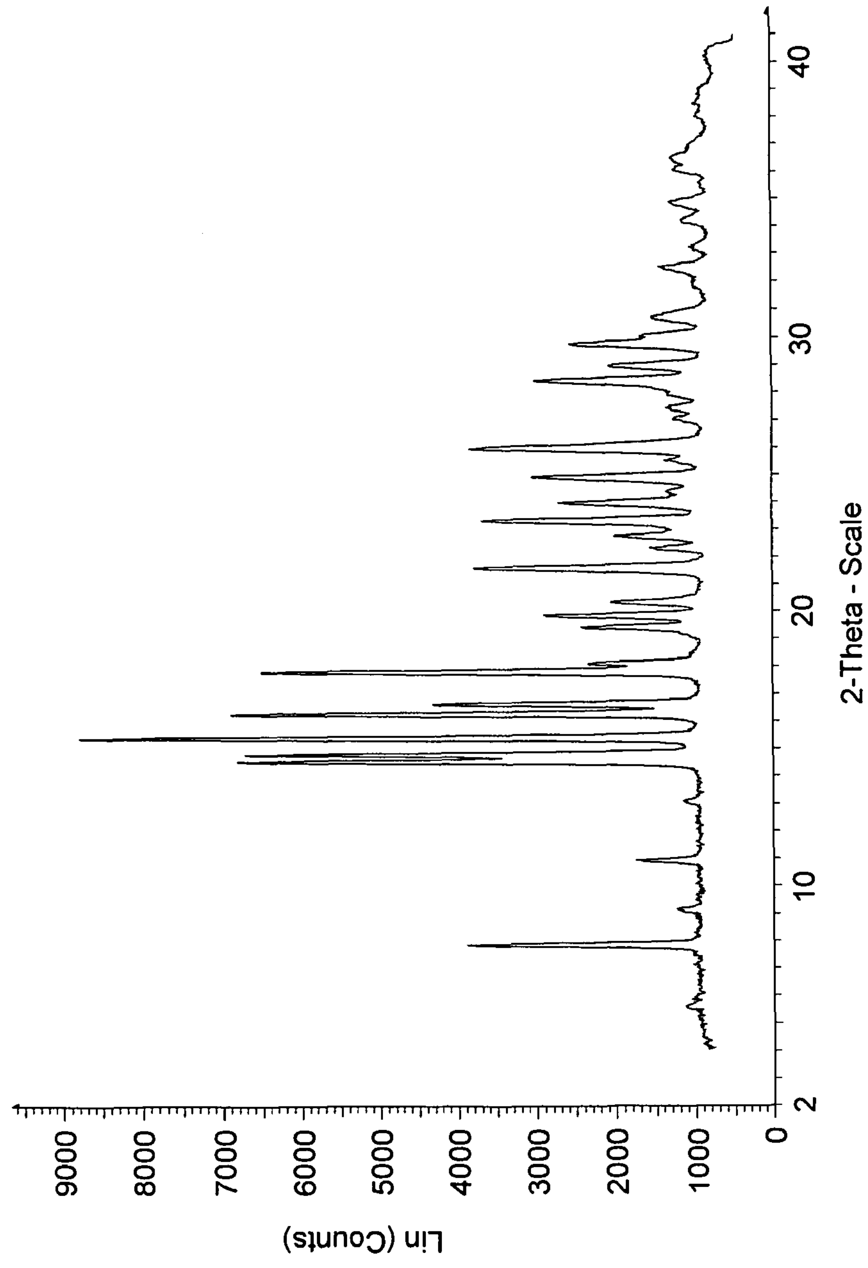

Lin (Counts)
9000
8000
7000
6000
5000
4000
3000
2000
1000
0
2
10
20
30
40
2-Theta - Scale

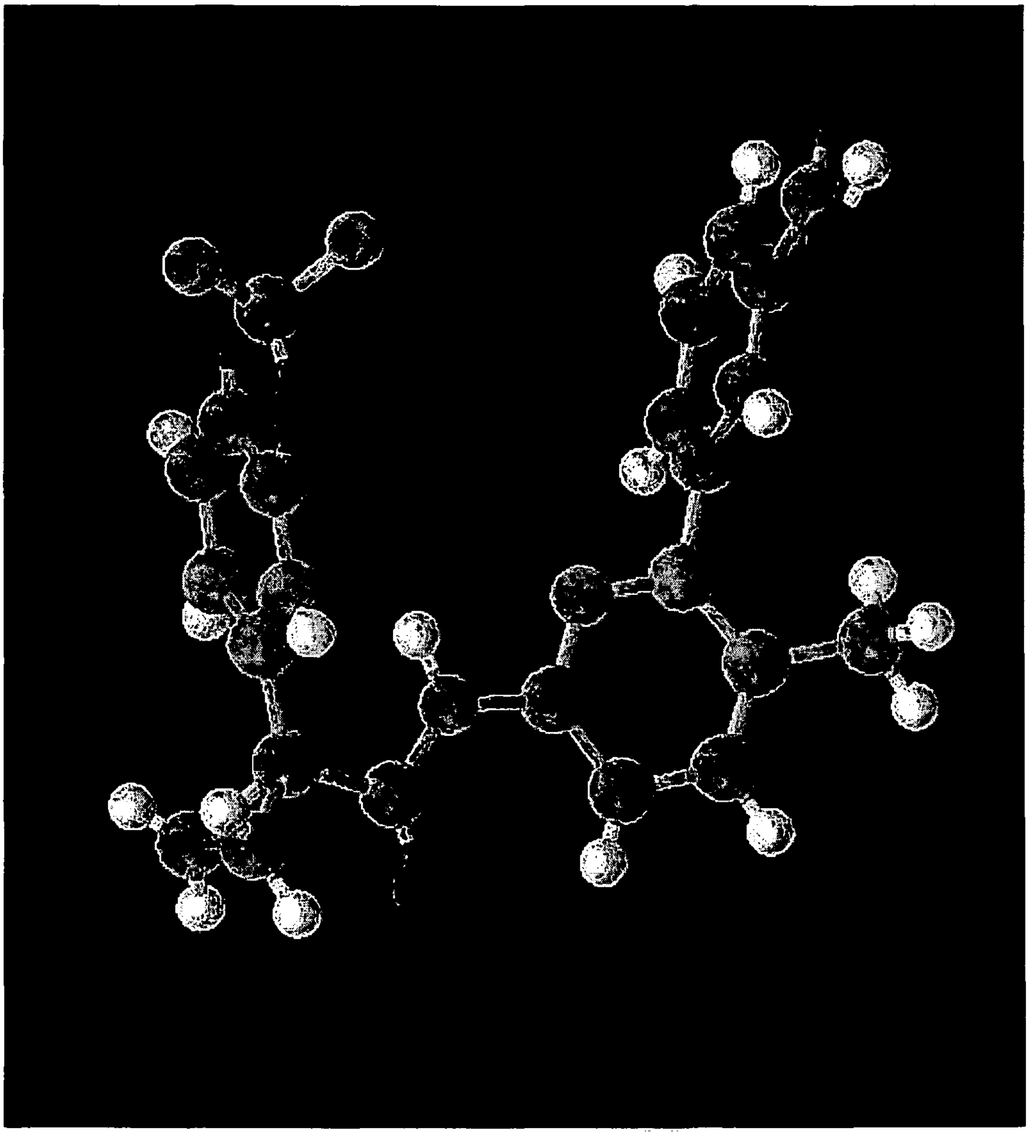

Figure 2-3

Figure 2-4

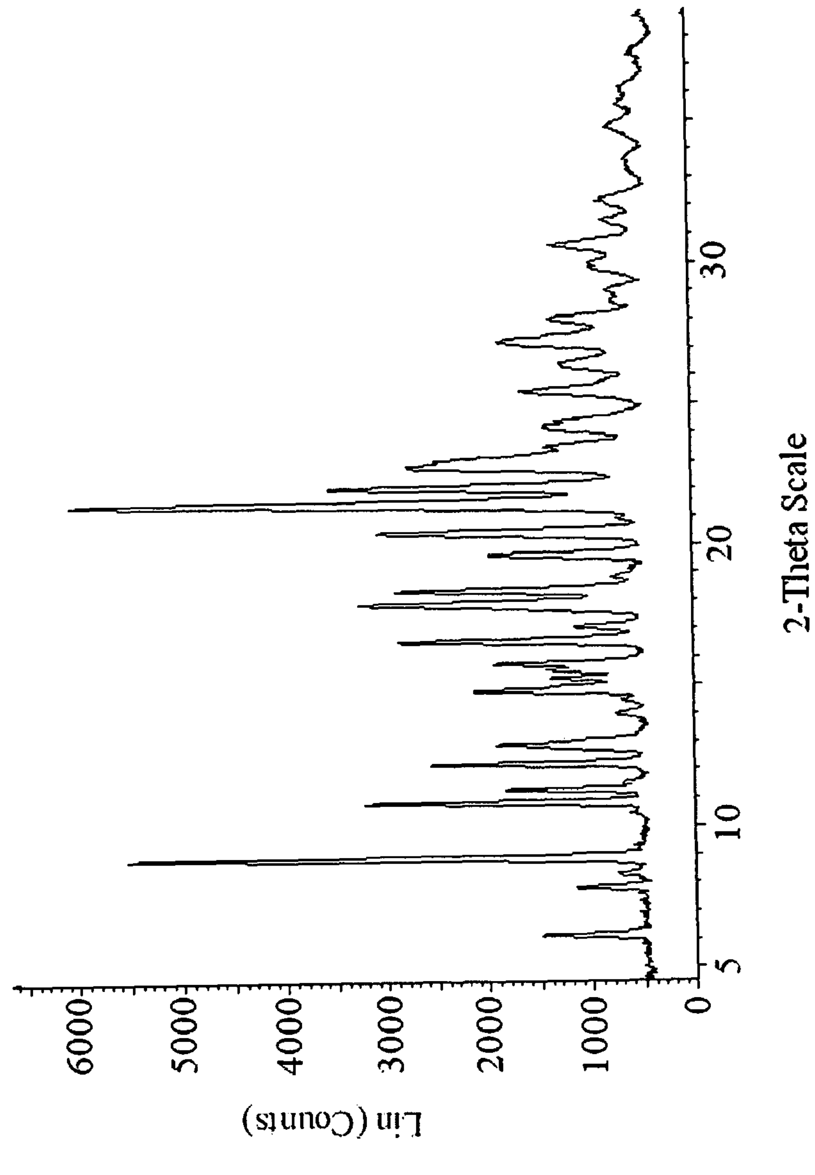

Lin (Counts)
6000
5000
4000
3000
2000
1000
0
5
10
20
30
2-Theta Scale

Figure 2-5

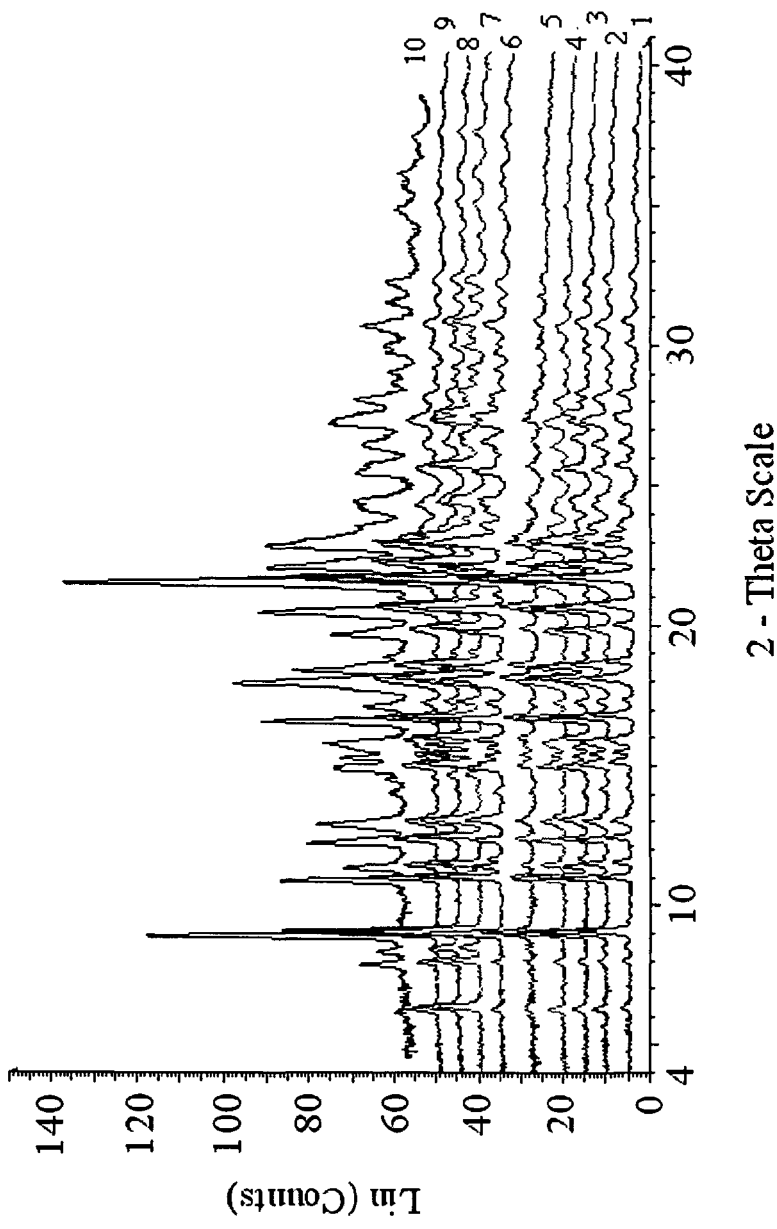
Lin (Counts)
140
120
100
80
60
40
20
0
4
10
20
30
40
2 - Theta Scale
10
9
8
7
6
5
4
3
2
1

Figure 2-6

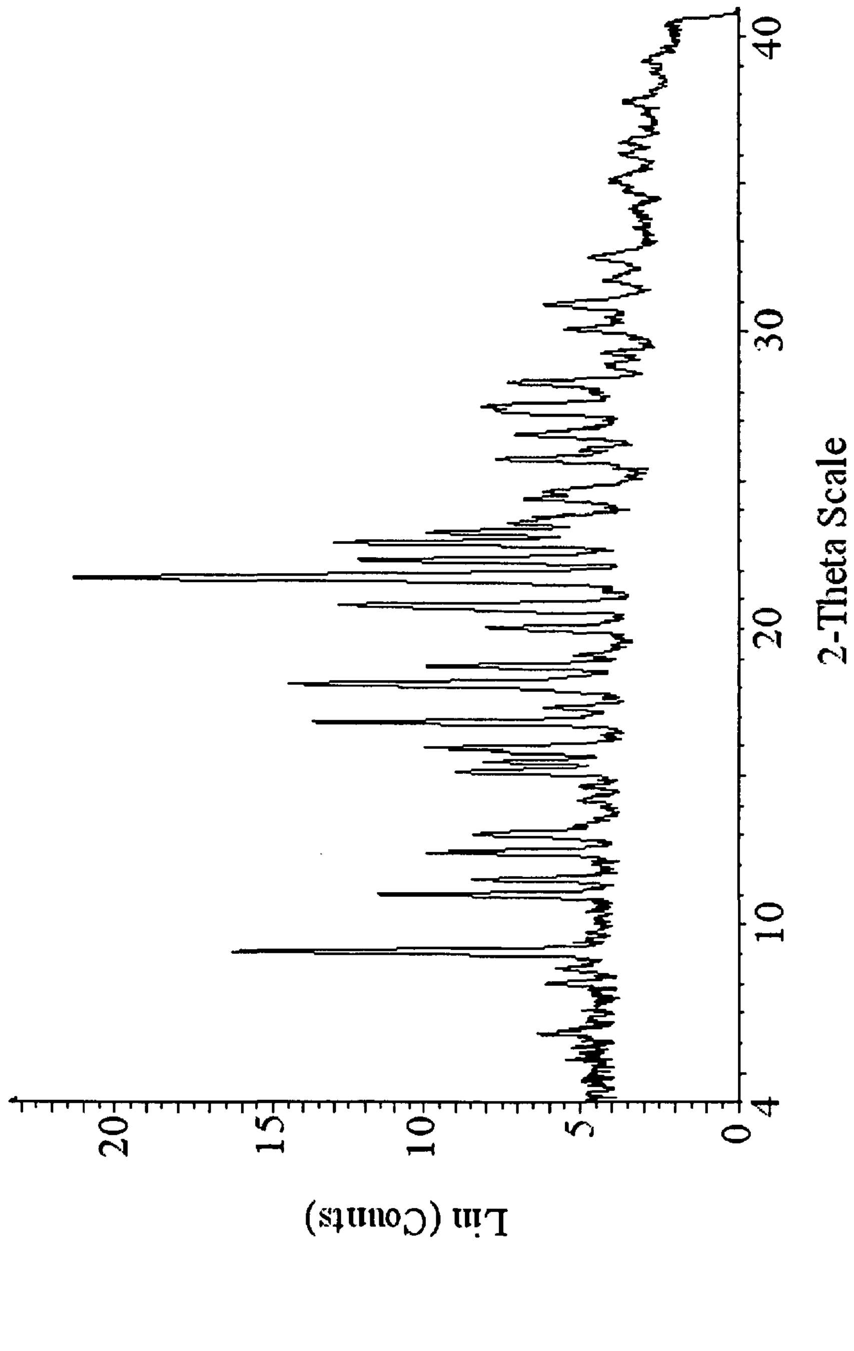

Figure 2-7

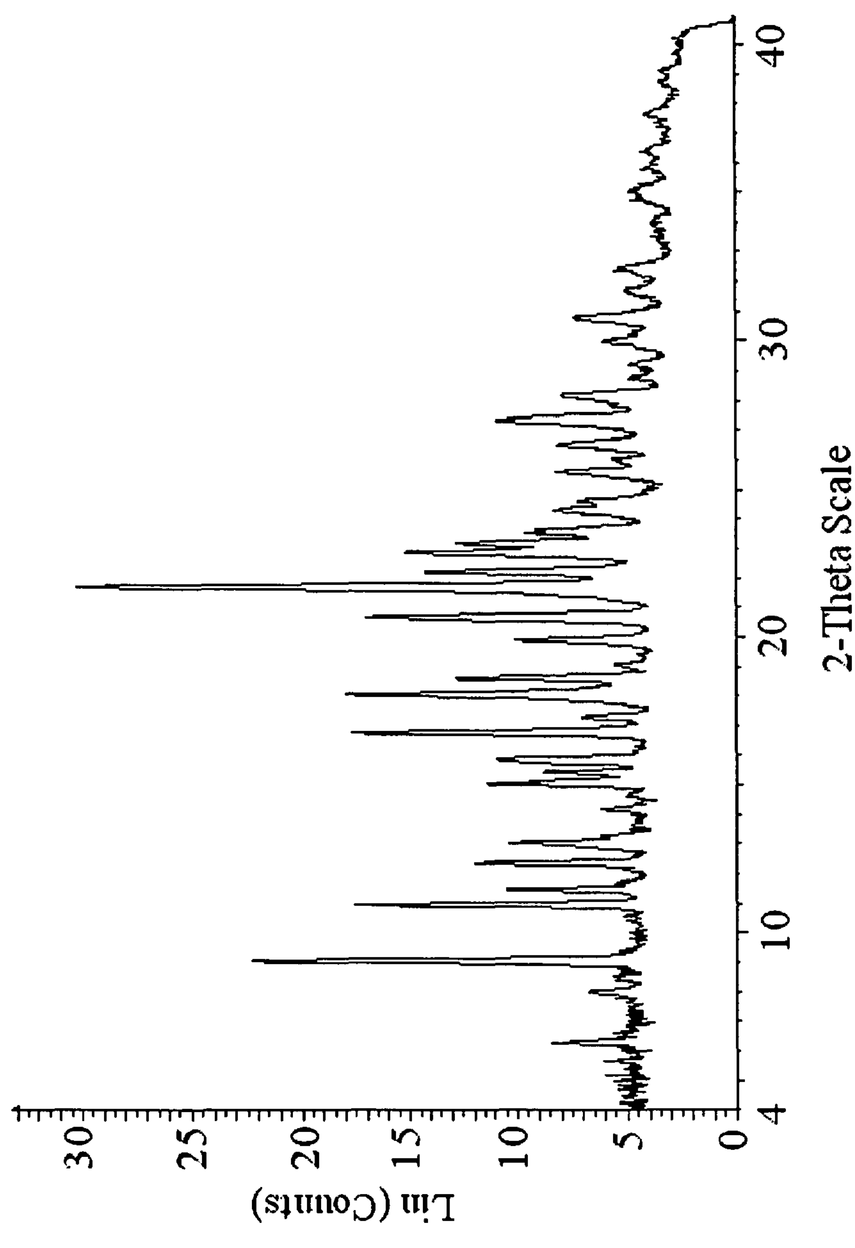

Figure 2-8

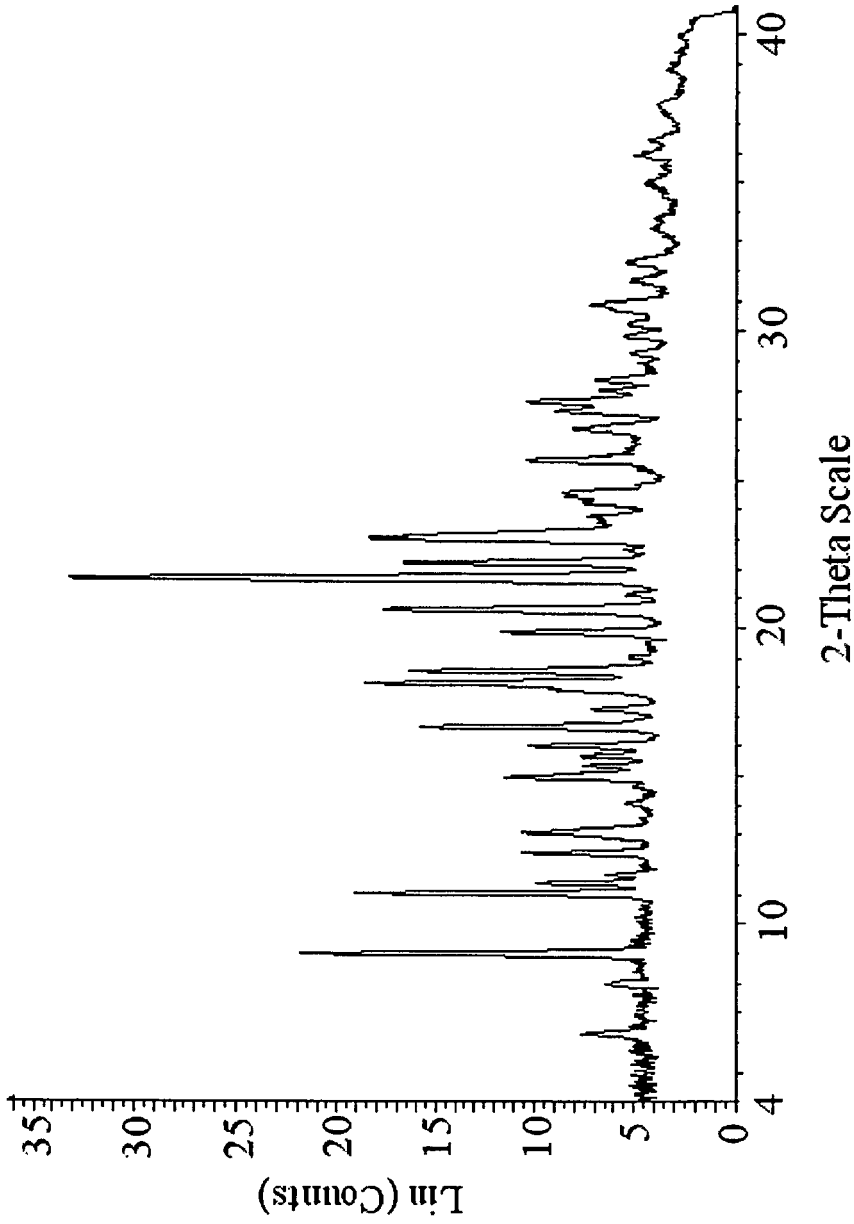

Figure 2-9

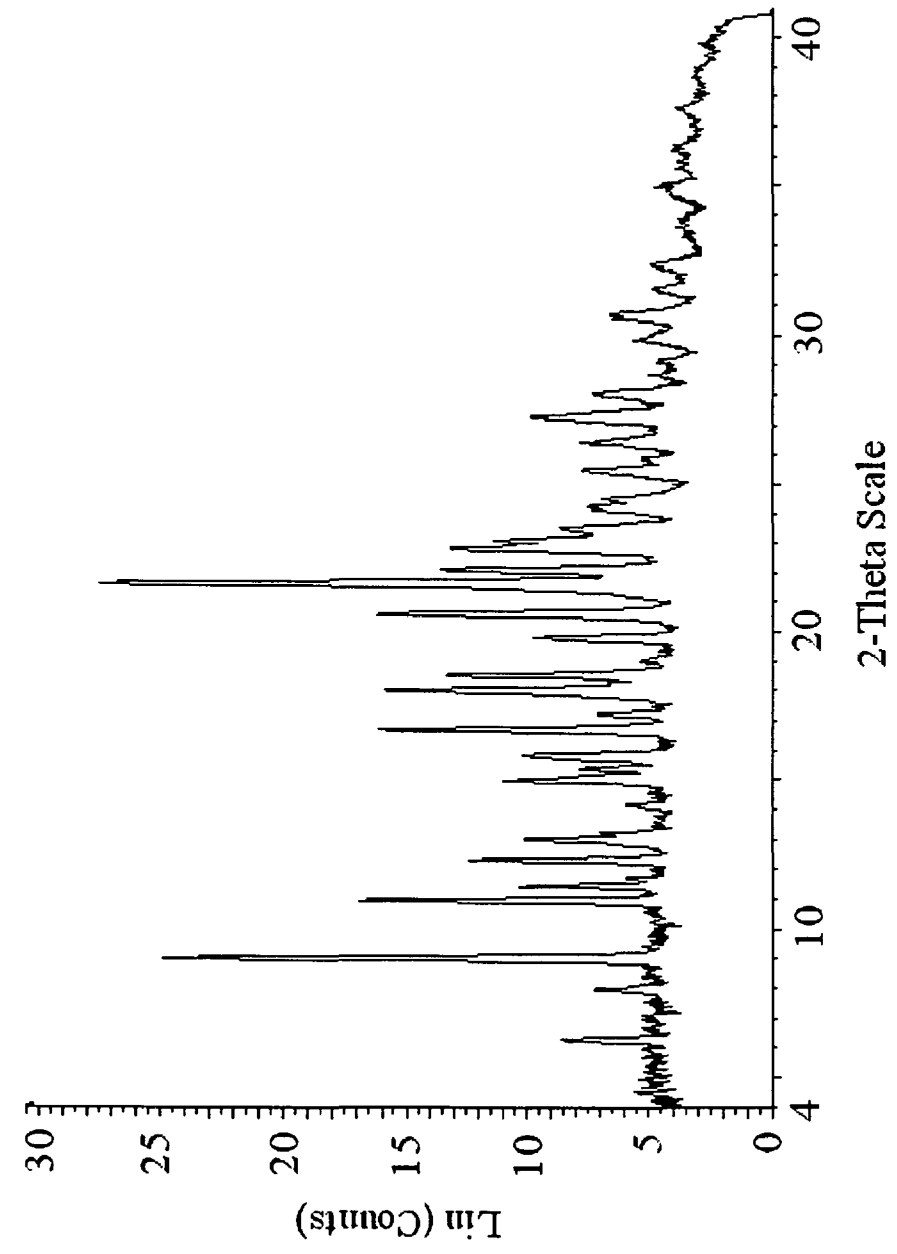

Figure 2-10

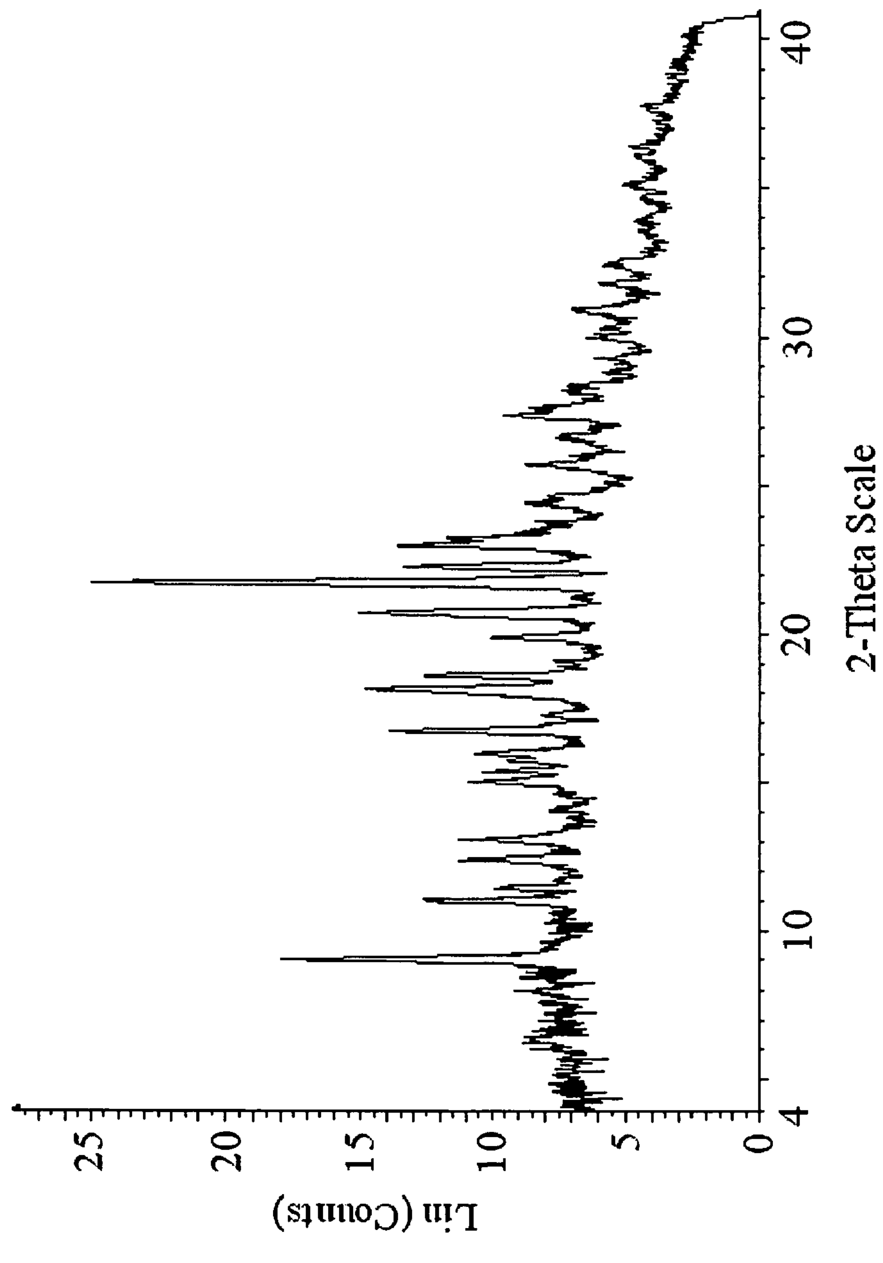

Figure 2-11

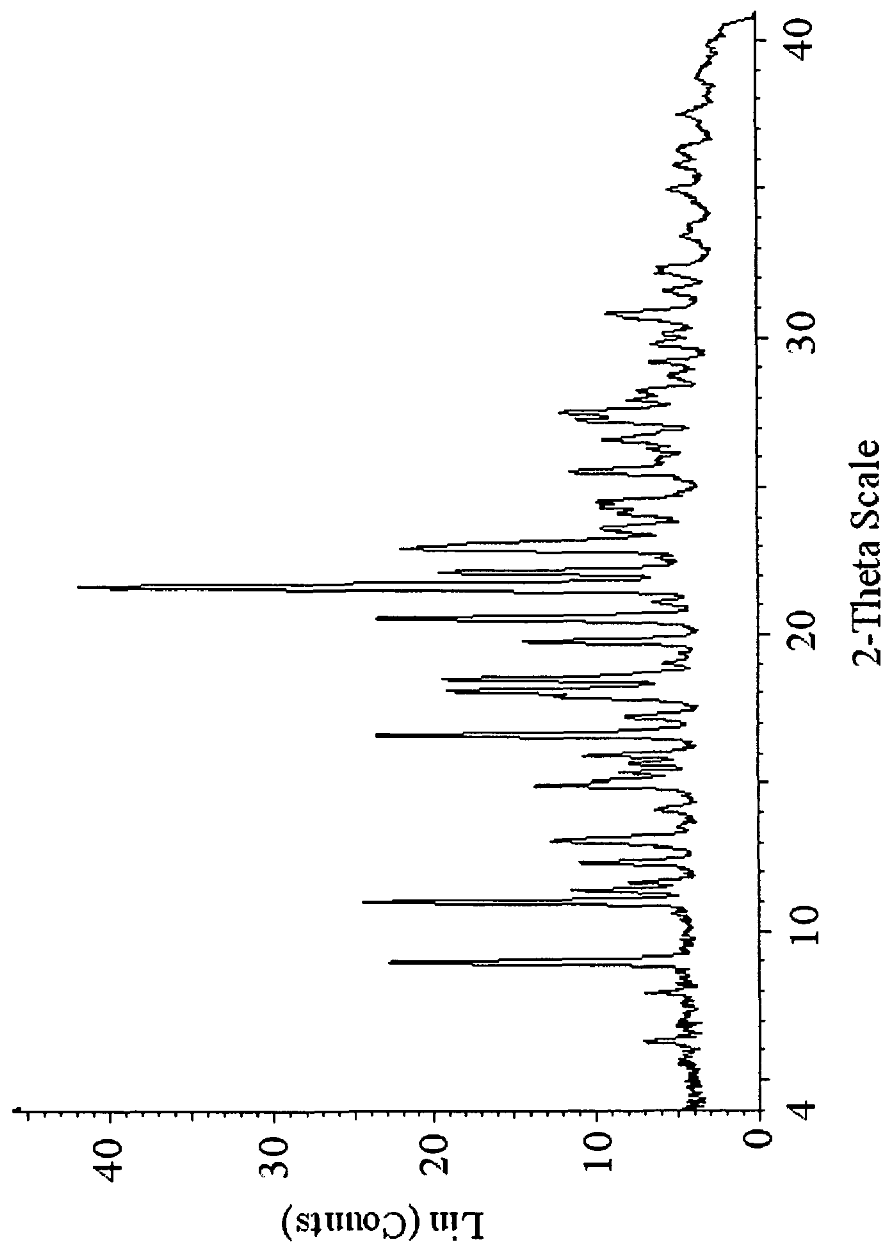

Figure 2-12

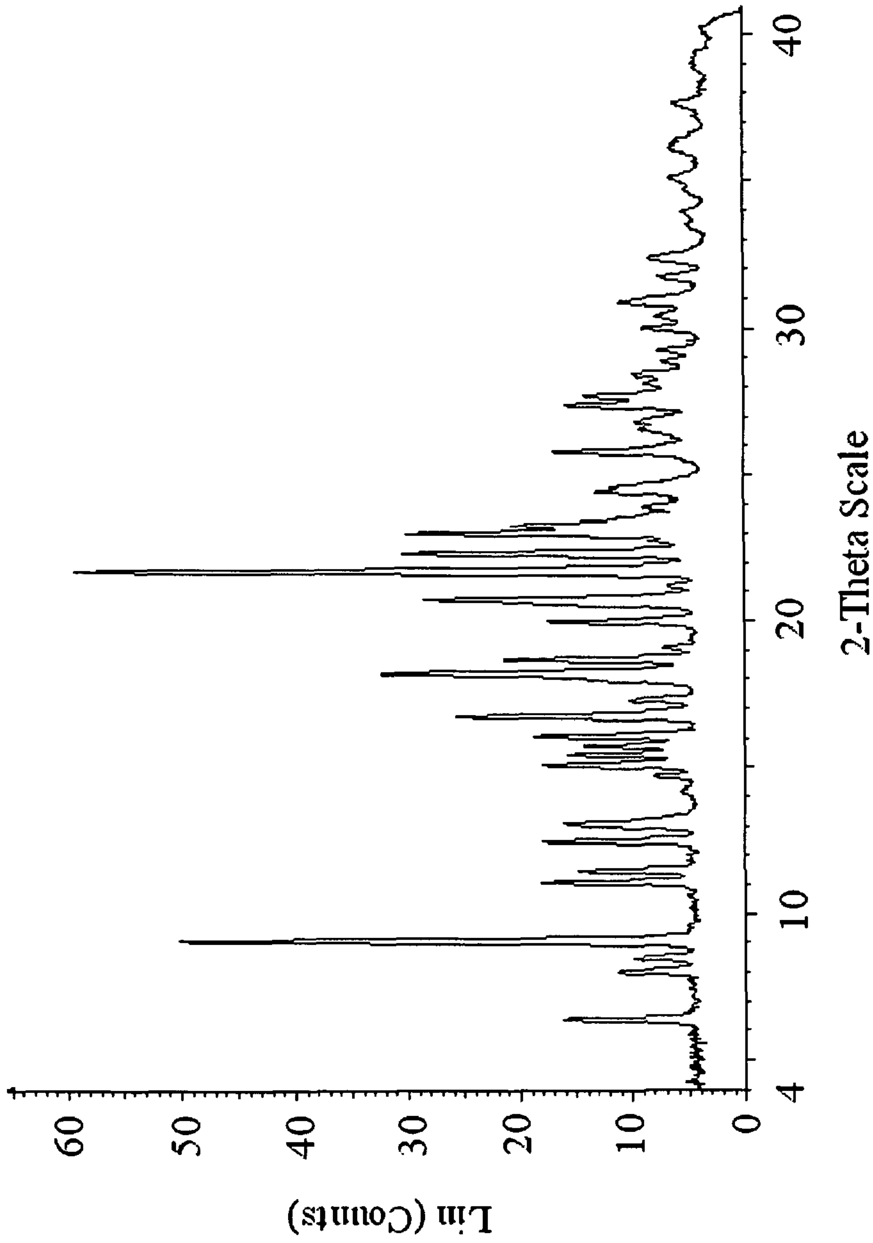

Figure 2-13

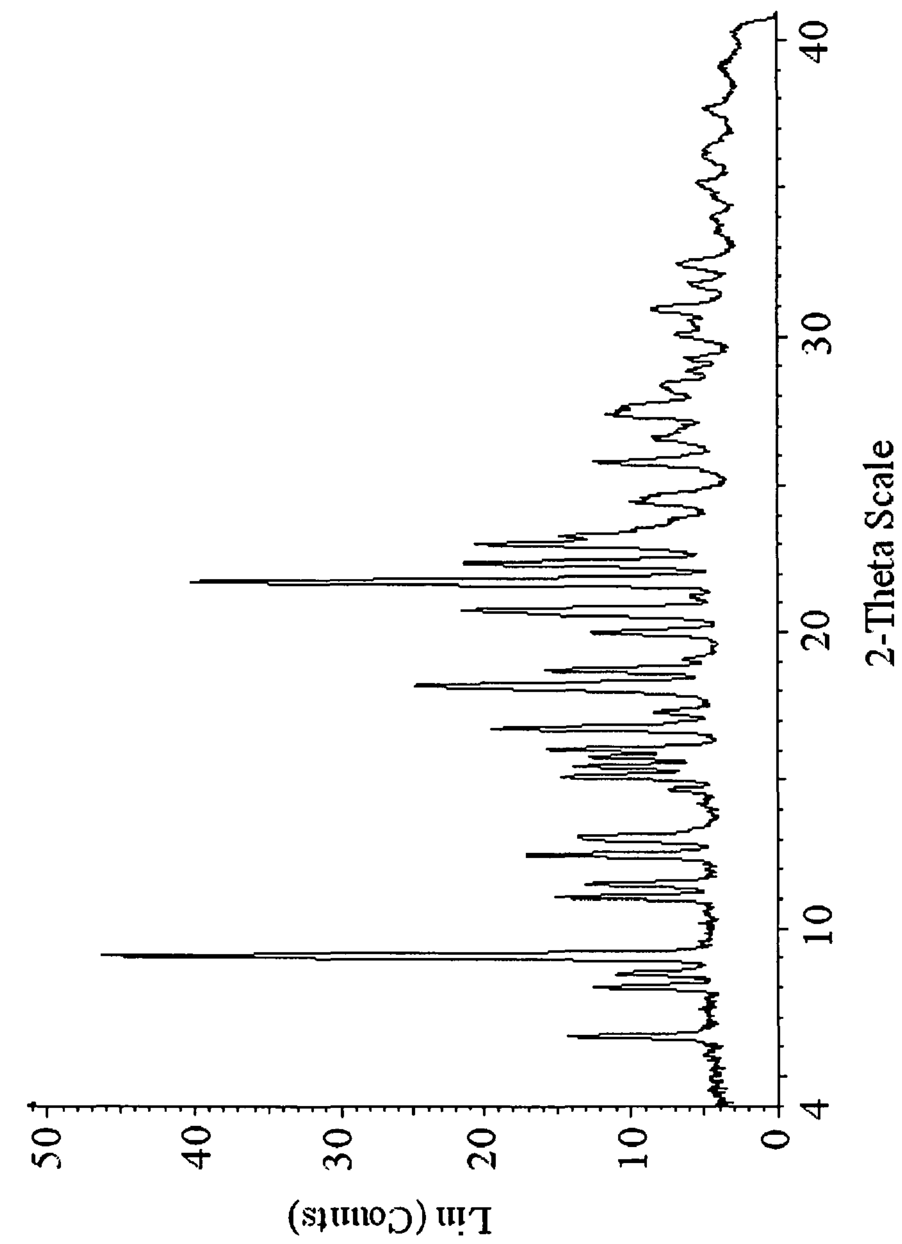

Figure 2-14

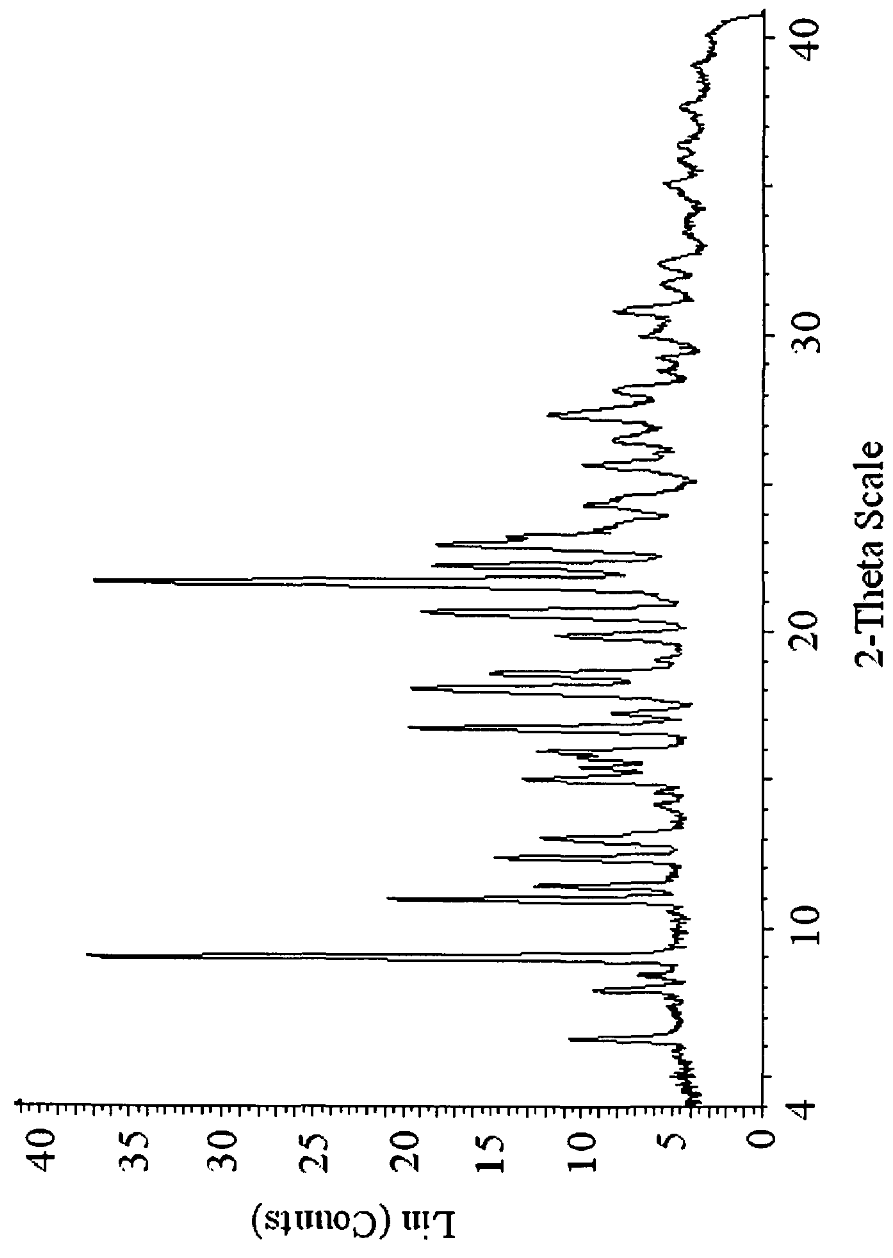

Figure 2-15

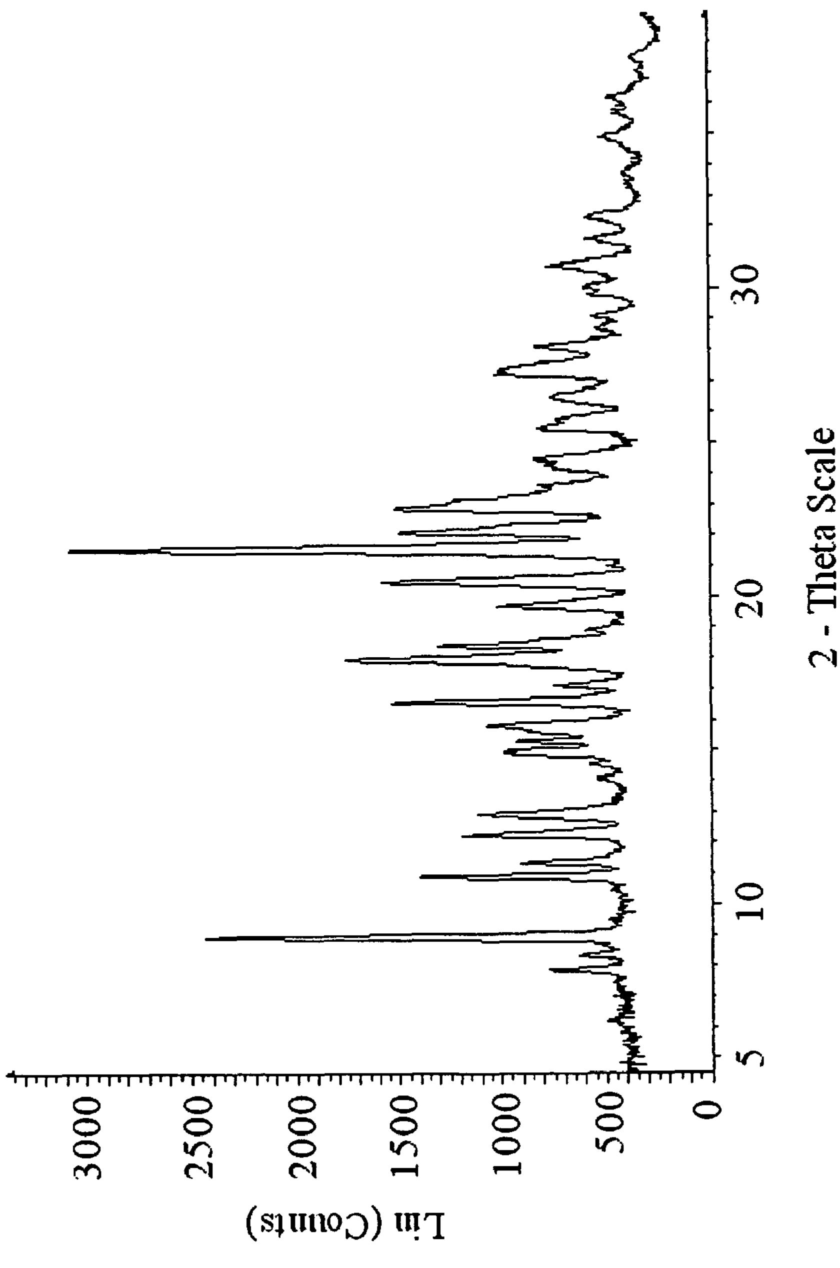
Lin (Counts)
3000
2500
2000
1500
1000
500
0
5
10
20
30
2 - Theta Scale

Figure 2-16

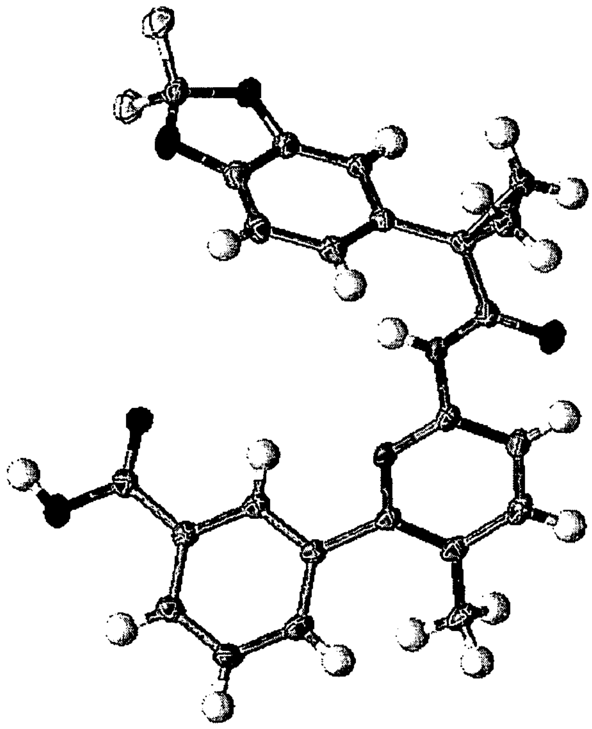

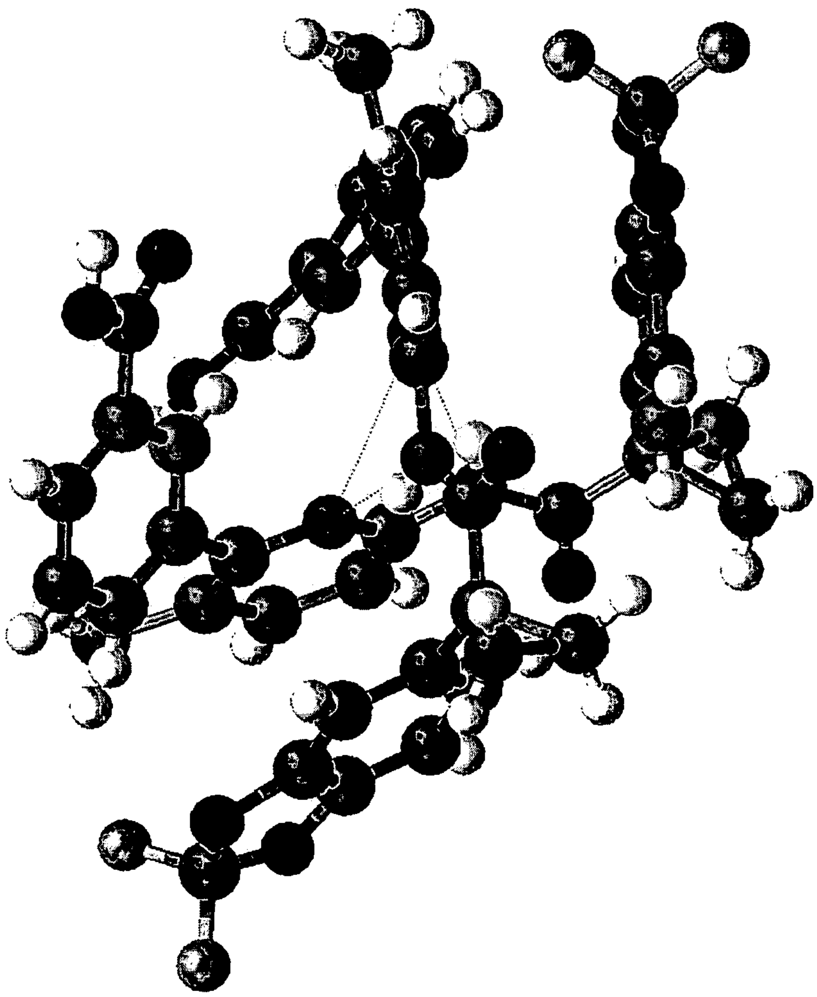

Figure 2-17

Figure 2-18

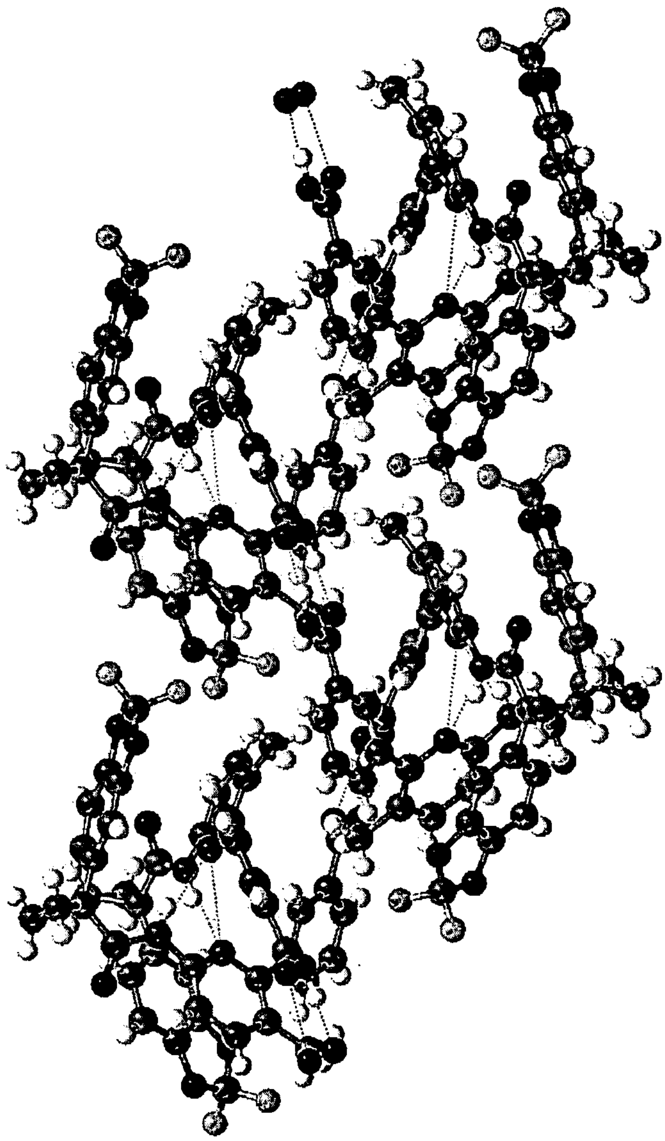

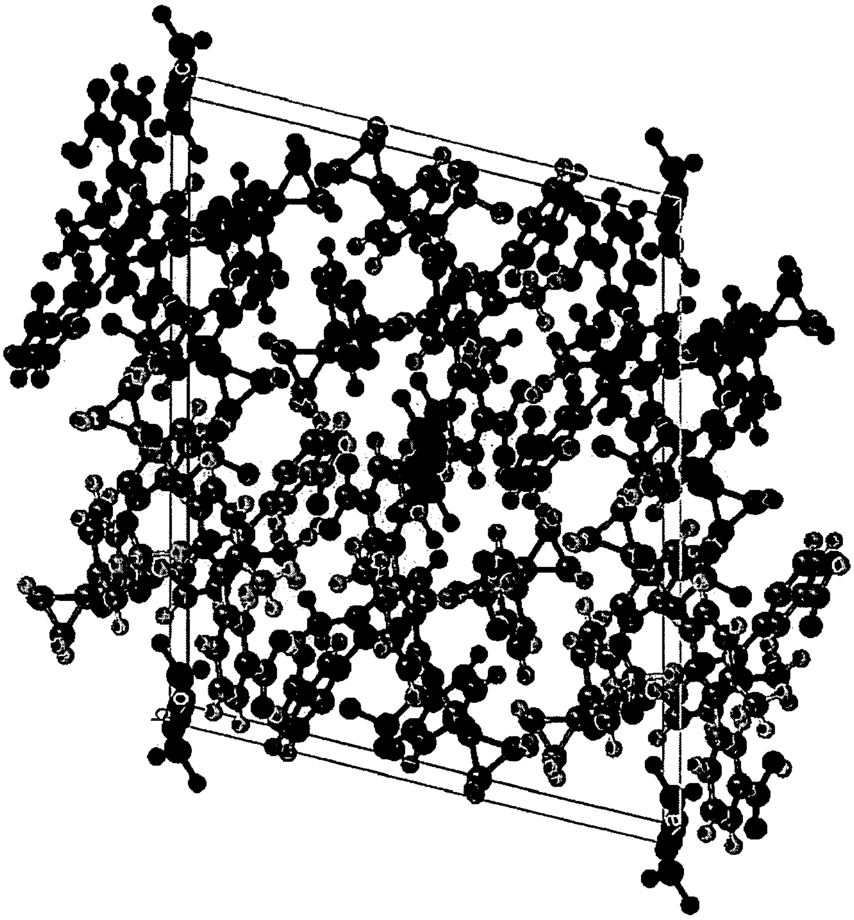

Figure 2-19

Figure 2-20

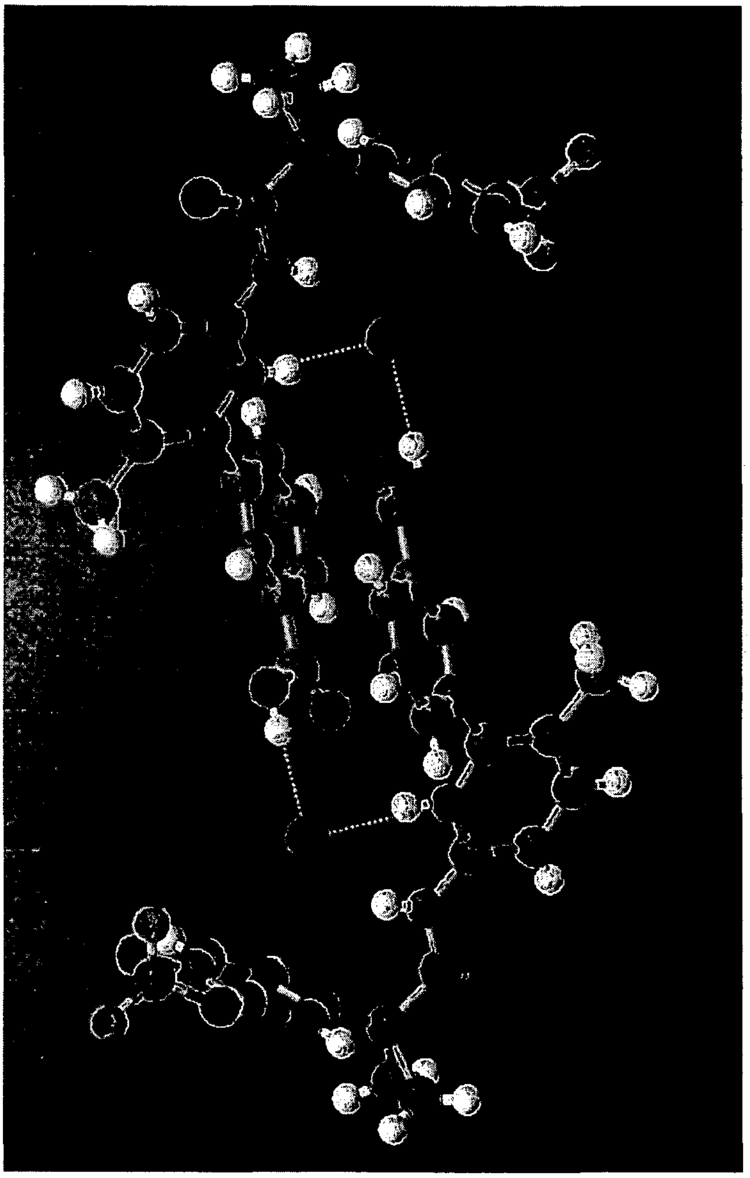

Figure 2-21

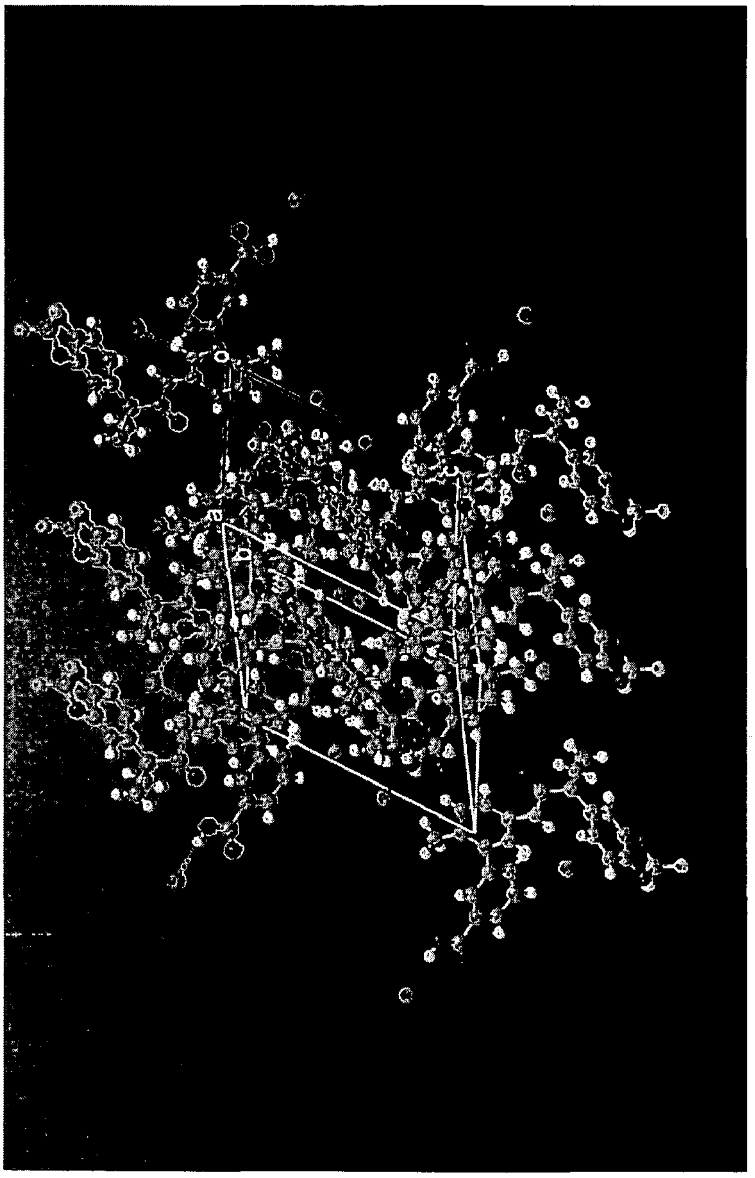

Figure 2-22

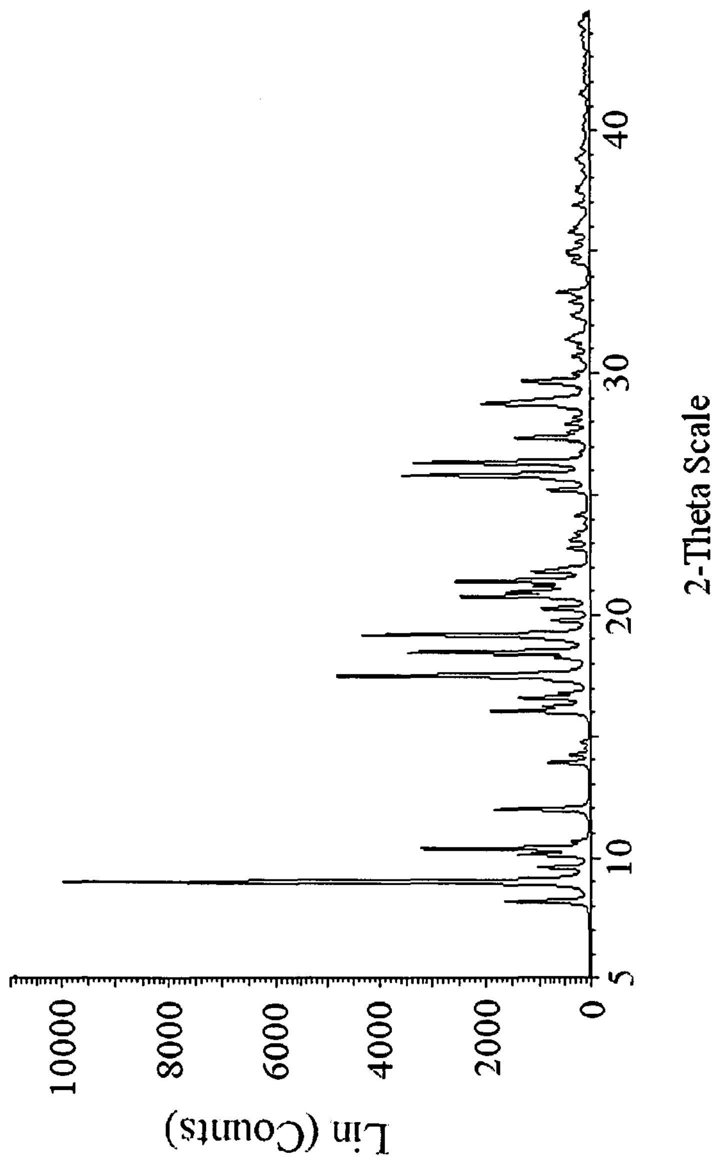
Lin (Counts)
0
2000
4000
6000
8000
10000
2-Theta Scale
5
10
20
30
40

Figure 2-23

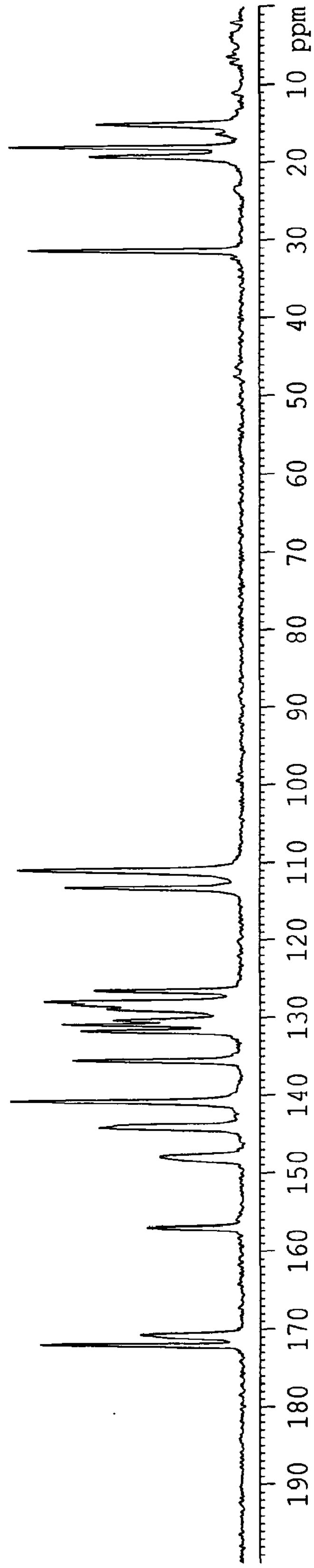
ppm
10
20
30
40
50
60
70
80
90
100
110
120
130
140
150
160
170
180
190

Figure 2-24

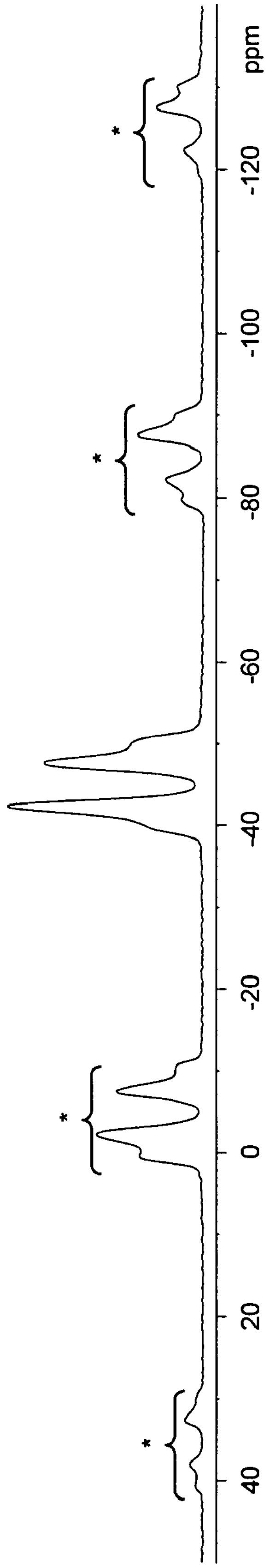

Figure 2-25

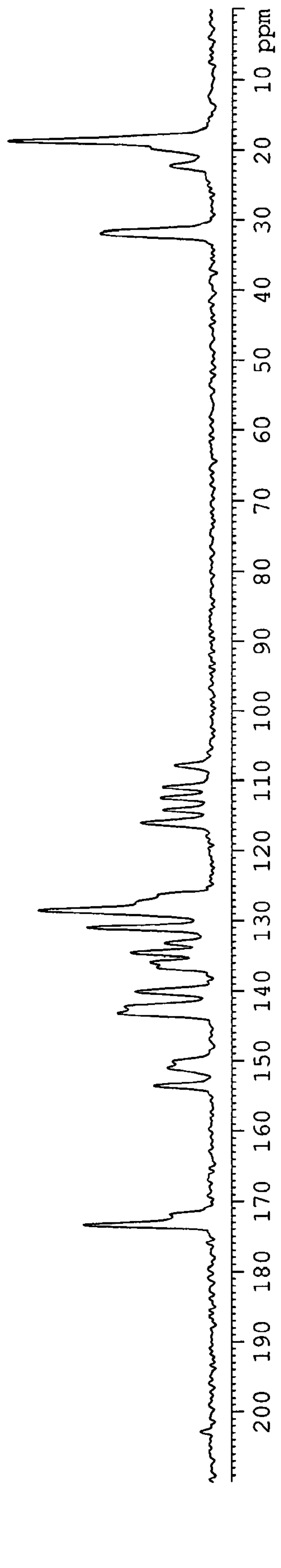
ppm
10
20
30
40
50
60
70
80
90
100
110
120
130
140
150
160
170
180
190
200

Figure 2-26

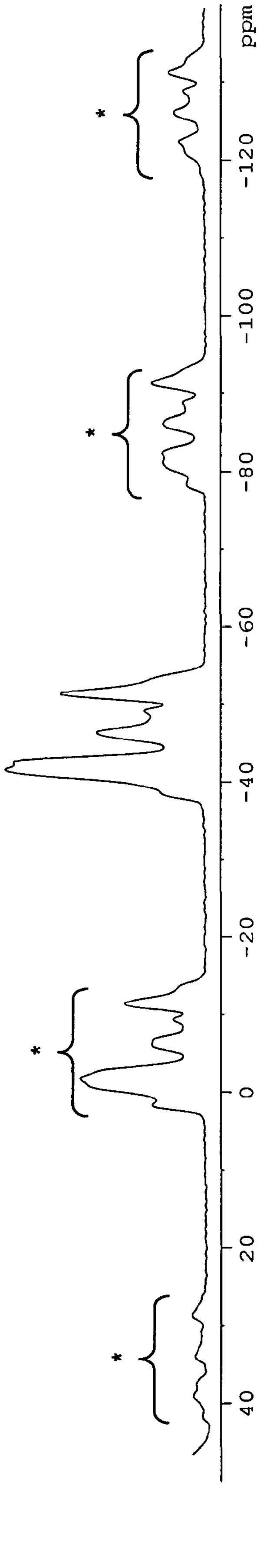

Figure 3-1

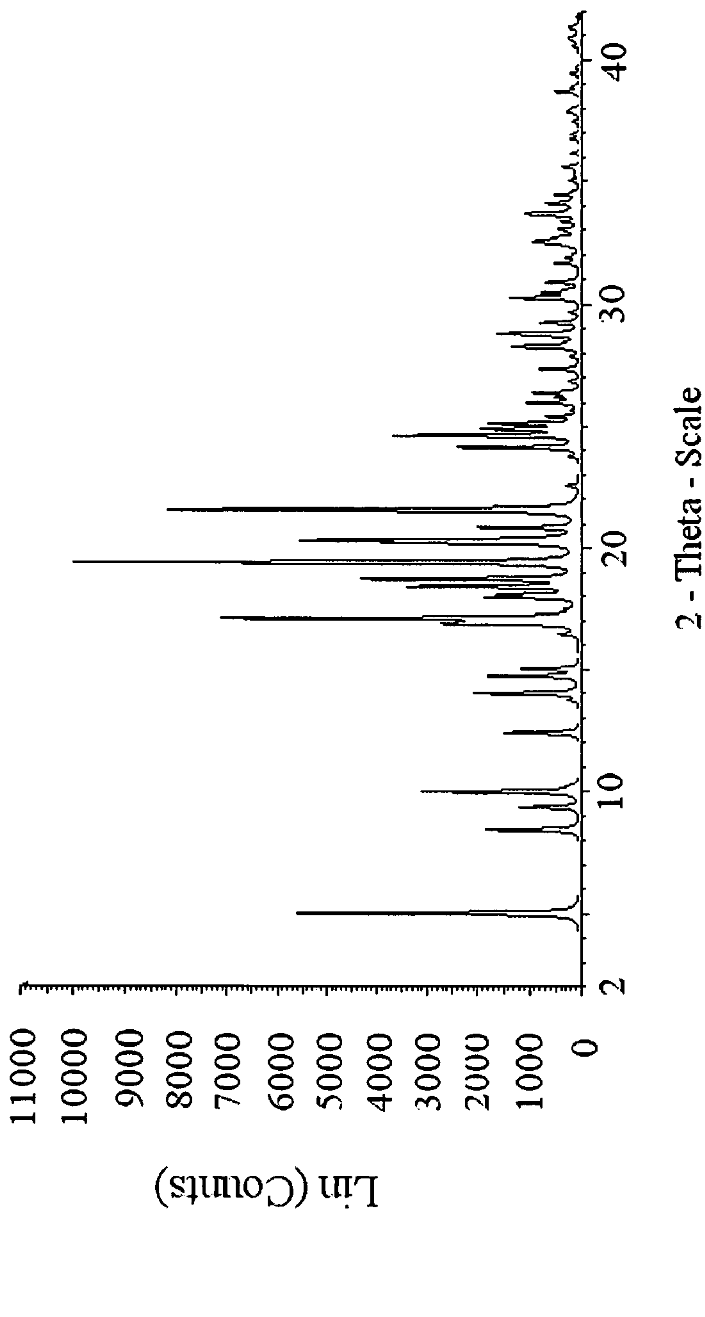
Lin (Counts)
0
1000
2000
3000
4000
5000
6000
7000
8000
9000
10000
11000
2
10
20
30
40
2 - Theta - Scale

Figure 3-2

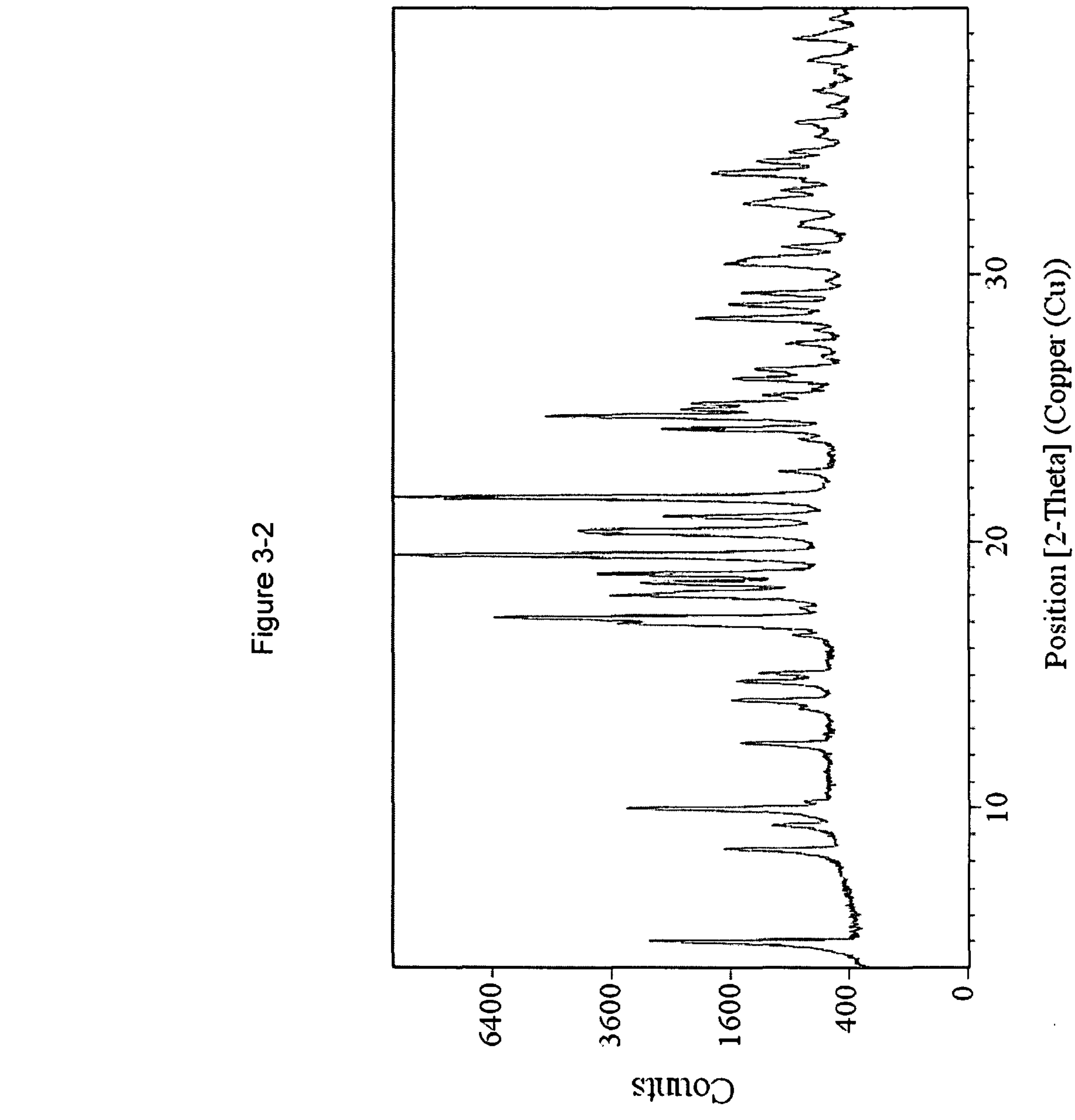
Counts
6400
3600
1600
400
0
10
20
30
Position [2-Theta] (Copper (Cu))

Figure 3-3

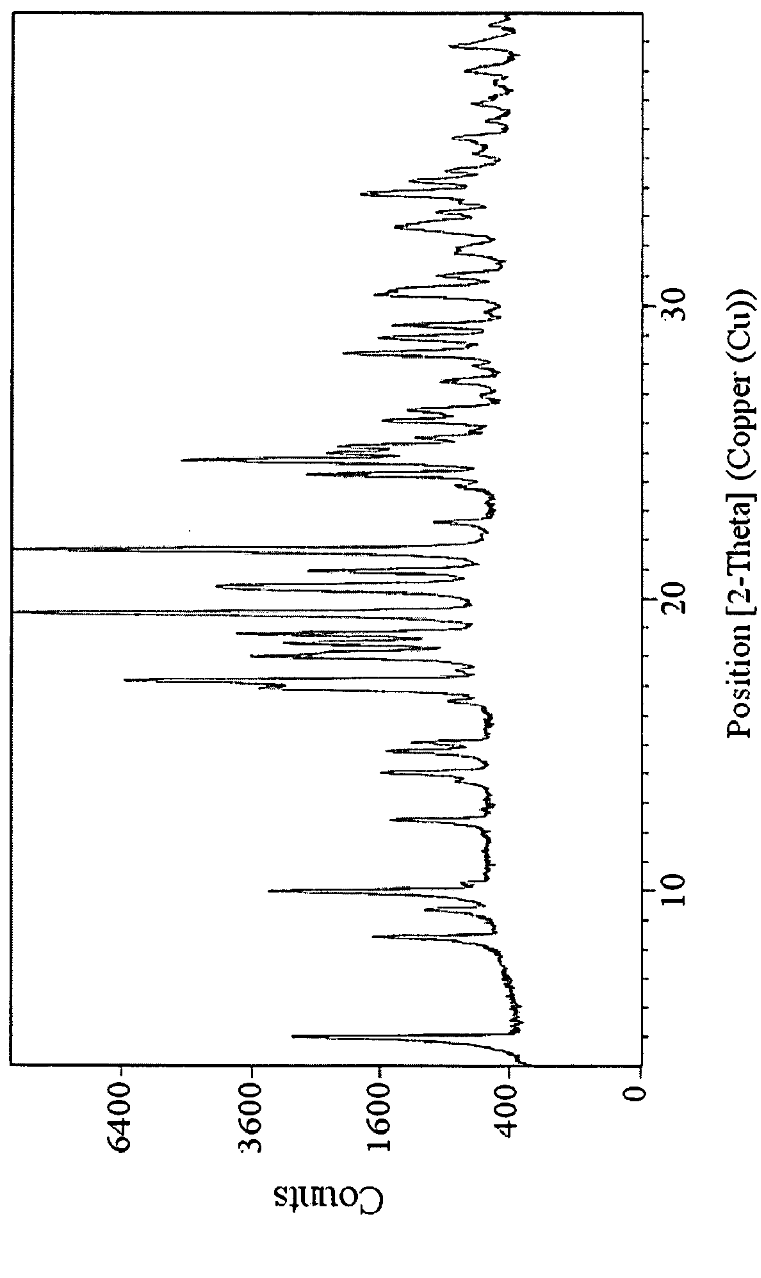
Counts
6400
3600
1600
400
0
10
20
30
Position [2-Theta] (Copper (Cu))

Figure 3-4

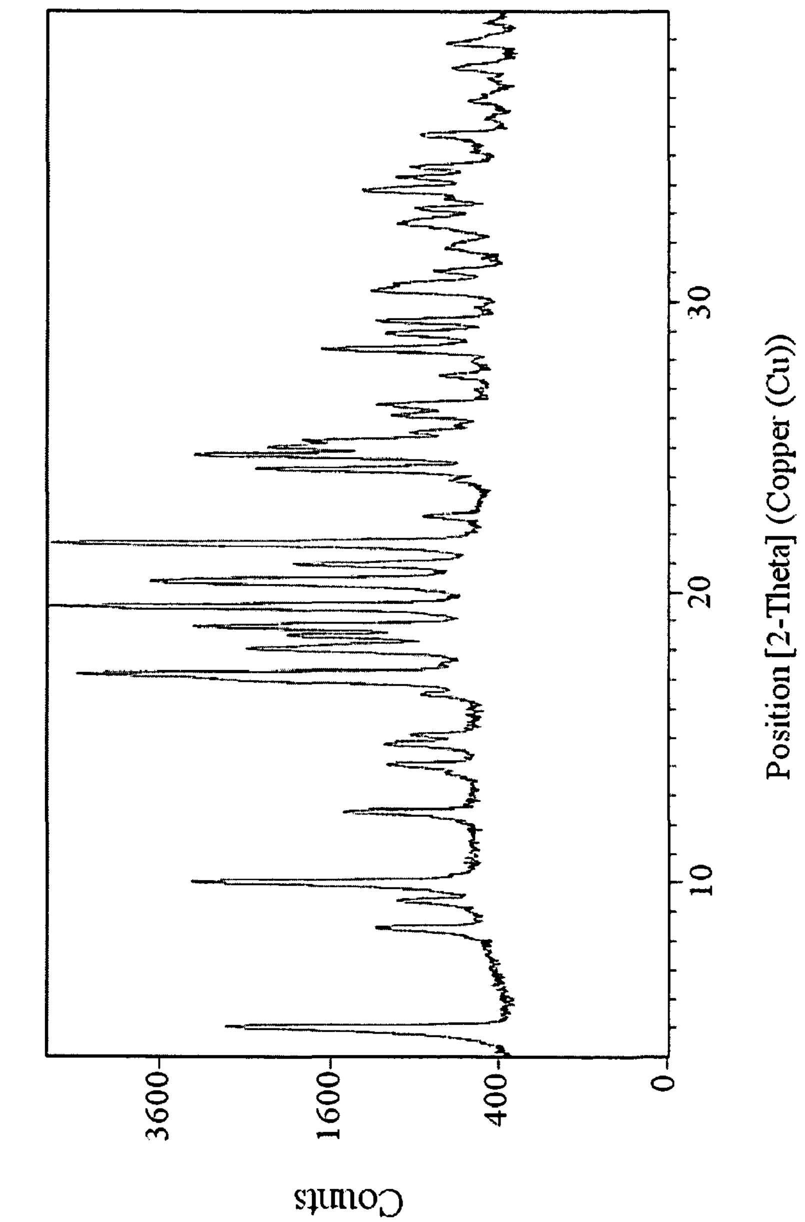

Figure 3-5

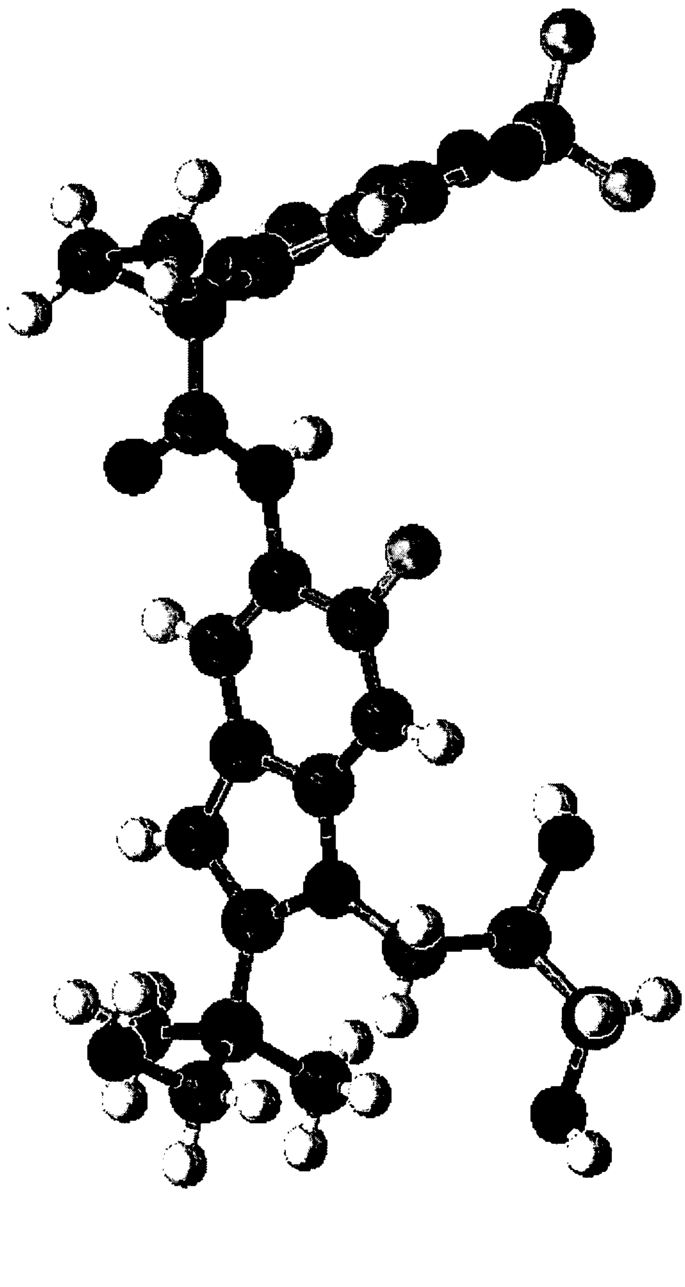

Figure 3-6

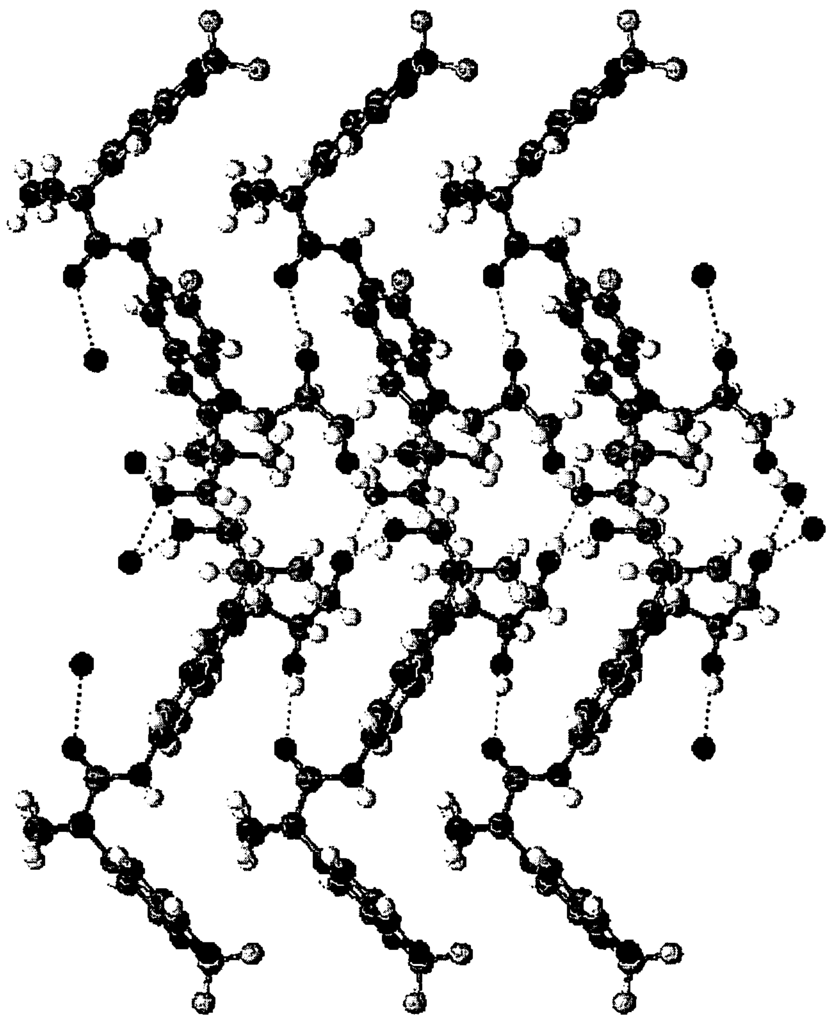

Figure 3-7

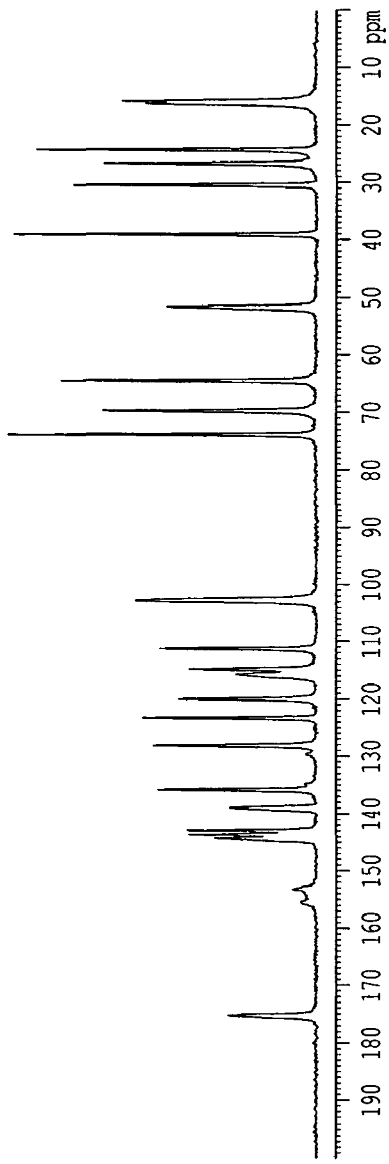
190 180 170 160 150 140 130 120 110 100 90 80 70 60 50 40 30 20 10 ppm

Figure 3-8

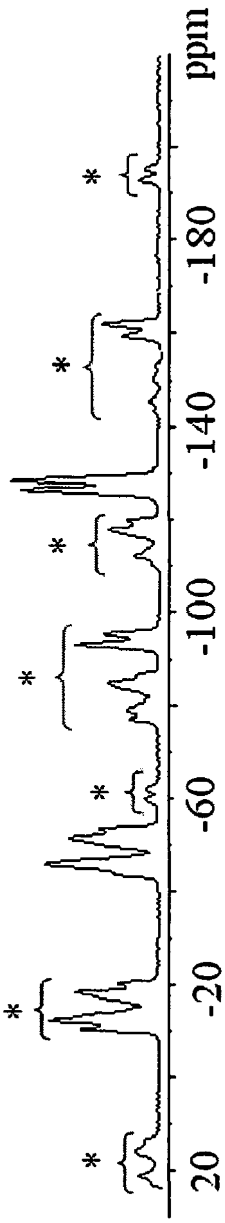

Figure 3-9

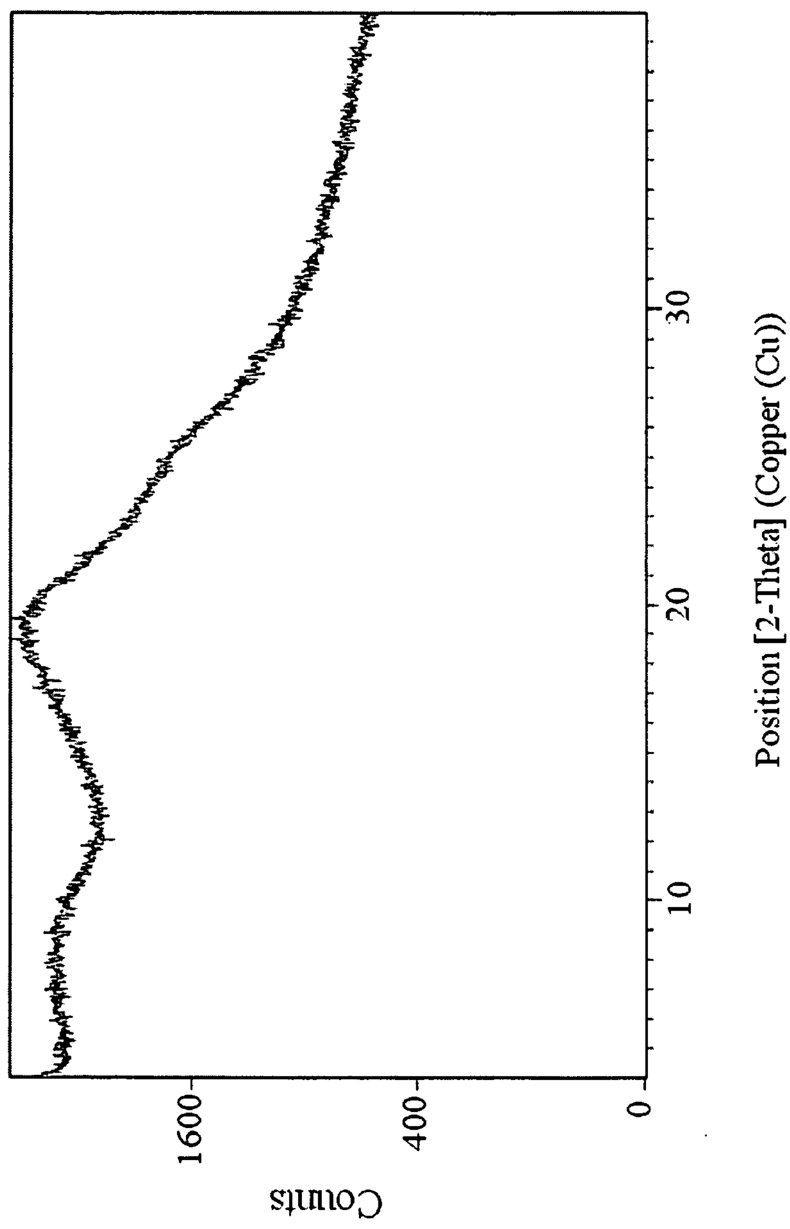

Figure 3-10

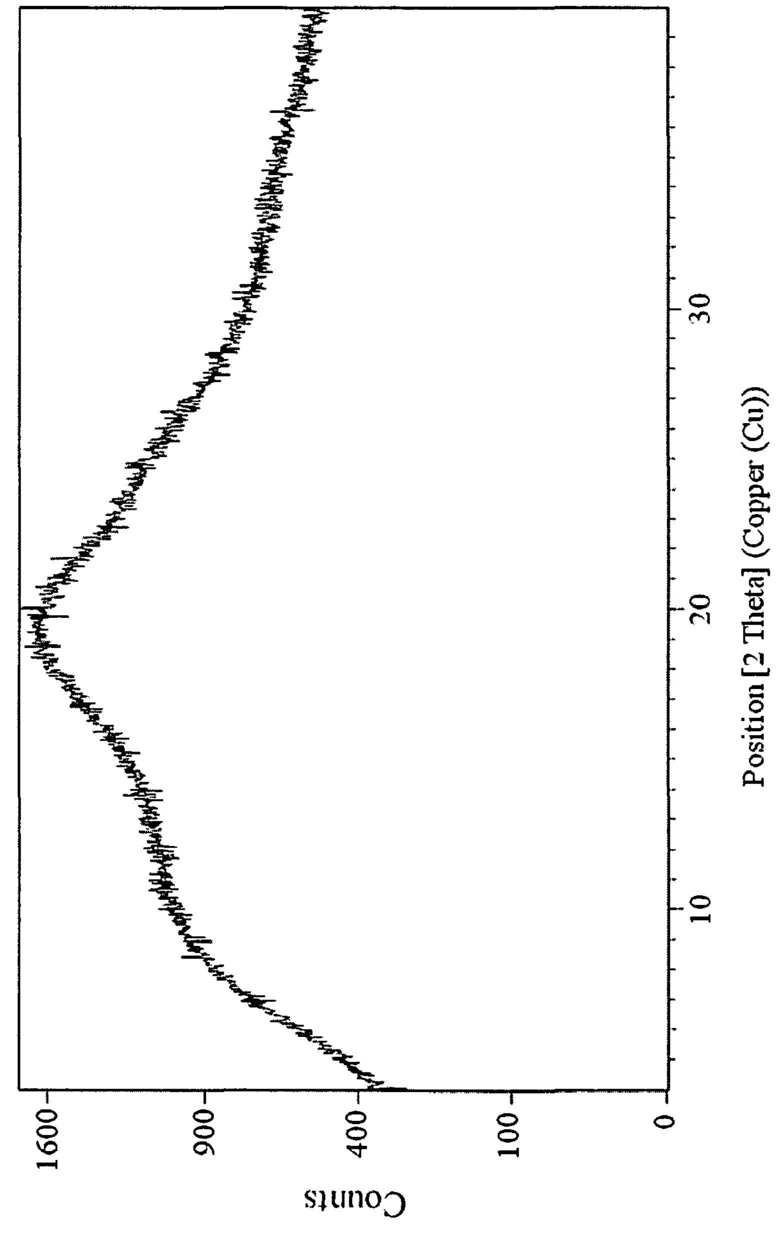

Figure 3-11

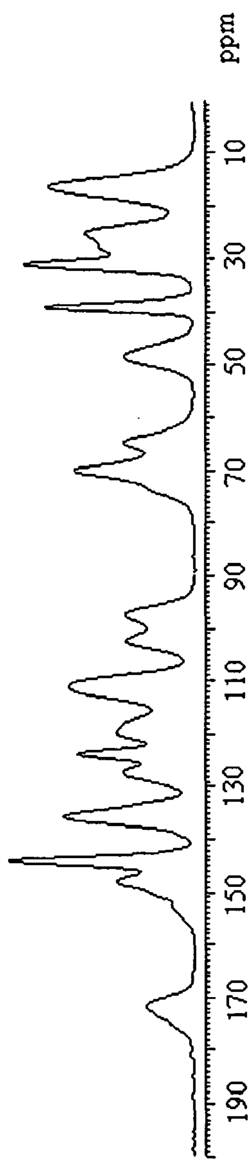

Figure 3-12

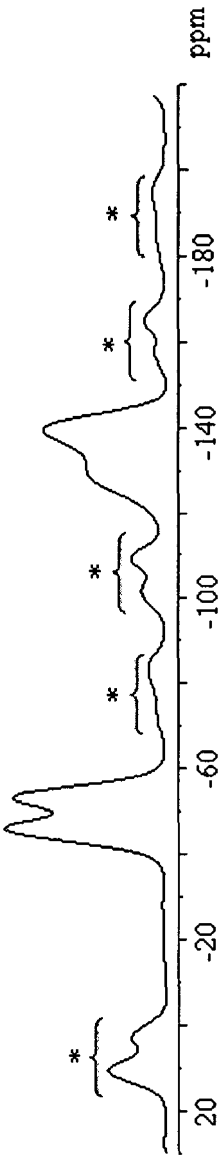

Europäisches Patentamt
European Patent Office
Office européen des brevets

# EUROPEAN SEARCH REPORT

Application Number
EP 16 19 2470

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2006/002421 A2 (VERTEX PHARMA [US]; HADIDA RUAH SARAH S [US]; HAZLEWOOD ANNA R [US]; G) 5 January 2006 (2006-01-05)<br>* page 52; example 136 *<br>* pages 25,65; example 475 *<br>* page 100, paragraph 275-276 *<br>----- | 1-15 | INV.<br>A61K31/4704<br>A61K31/404<br>A61K31/443 |
| Y | WO 2007/079139 A2 (VERTEX PHARMA [US]; HURTER PATRICIA [US] VERTEX PHARMA [US]; HURTER PA) 12 July 2007 (2007-07-12)<br>* page 43, paragraph 199; claims *<br>----- | 1-15 | |
| Y | WO 2009/038683 A2 (VERTEX PHARMA [US]; DEMATTEI JOHN [US]; FENG YUSHI [US]; HARRISON CRIS) 26 March 2009 (2009-03-26)<br>* page 44; claims *<br>----- | 1-15 | |
| Y | WO 2009/023509 A2 (VERTEX PHARMA [US]; SINGH ASHVANI [US]; WORLEY JENNINGS FRANKLIN III [])<br>19 February 2009 (2009-02-19)<br>* page 40; compound 433 *<br>* page 8, paragraph 29; claims *<br>----- | 1-15 | TECHNICAL FIELDS SEARCHED (IPC)<br>A61K |
| Y | WO 2007/056341 A1 (VERTEX PHARMA [US]; HADIDA RUAH SARA [US]; HAMILTON MATTHEW [US]; MILL) 18 May 2007 (2007-05-18)<br>* page 96; example 422 *<br>* page 107, paragraph 338 *<br>----- | 1-15 | |
| X<br>Y | WO 2009/073757 A1 (VERTEX PHARMA [US]; KESHAVARZ-SHOKRI ALI [US]; ZHANG BEILI [US]; KRAWI) 11 June 2009 (2009-06-11)<br>* page 18, paragraph 105 *<br>----- | 1,2,5,6,8-15<br>1-15 | |
| | -/-- | | |

The present search report has been drawn up for all claims

1

| Place of search | Date of completion of the search | Examiner |
|---|---|---|
| Munich | 24 January 2017 | Steendijk, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 19 2470

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br>Y | WO 2009/076141 A2 (VERTEX PHARMA [US]; KESHAVARZ-SHOKRI ALI [US]; YOUNG CHRISTOPHER [US]) 18 June 2009 (2009-06-18)<br>* page 22, paragraph 116 *<br>----- | 1,2,5,6, 8-15<br><br><br>1-15 | |
| X<br><br><br>Y | WO 2010/037066 A2 (VERTEX PHARMA [US]; YOUNG CHRISTOPHER [US]) 1 April 2010 (2010-04-01)<br>* page 22, paragraph 108 *<br>----- | 1,2,5,6, 8-15<br><br>1-15 | |
| Y | WO 2007/117715 A2 (VERTEX PHARMA [US]; RUAH SARA S HADIDA [US]; GROOTENHUIS PETER D J [US) 18 October 2007 (2007-10-18)<br>* page 62; example 302 *<br>* page 94, paragraph 257 *<br>----- | 1-15 | |
| Y | US 2009/131492 A1 (RUAH SARA S HADIDA [US] ET AL HADIDA RUAH SARA S [US] ET AL) 21 May 2009 (2009-05-21)<br>* pages 25,85,87 *<br>* page 113, paragraph 296 *<br>----- | 1-15 | TECHNICAL FIELDS SEARCHED (IPC) |
| X,P<br><br><br>Y,P | WO 2010/053471 A1 (VERTEX PHARMA [US]; RUAH SARA HADIDA S [US]; GROOTENHUIS PETER D J [US) 14 May 2010 (2010-05-14)<br>* page 27, paragraph 108 *<br>* page 27; compound 322 *<br>----- | 1,3,5, 7-15<br><br>1-15 | |
| X,P<br><br><br>Y,P | WO 2010/054138 A2 (VERTEX PHARMA [US]; RUAH SARA S HADIDA [US]; GROOTENHUIS PETER D J [US) 14 May 2010 (2010-05-14)<br>* page 72 *<br>-----<br>-/-- | 1,3,5, 7-15<br><br>1-15 | |

The present search report has been drawn up for all claims

| Place of search | Date of completion of the search | Examiner |
|---|---|---|
| Munich | 24 January 2017 | Steendijk, Martin |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 19 2470

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JONES ANDREW M ET AL: "Emerging Treatments in Cystic Fibrosis", DRUGS, ADIS INTERNATIONAL LTD, NZ, vol. 69, no. 14, 1 January 2009 (2009-01-01), pages 1903-1910, XP008134754, ISSN: 0012-6667, DOI: DOI:10.2165/11318500-000000000-00000 * page 1907, right-hand column * ----- | 1-15 | |

TECHNICAL FIELDS SEARCHED (IPC)

1

The present search report has been drawn up for all claims

| Place of search | Date of completion of the search | Examiner |
|---|---|---|
| Munich | 24 January 2017 | Steendijk, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 16 19 2470

This annex lists the patent family members relating to the patent documents cited in the above-mentioned European search report. The members are as contained in the European Patent Office EDP file on
The European Patent Office is in no way liable for these particulars which are merely given for the purpose of information.

24-01-2017

| Patent document cited in search report | Publication date | Patent family member(s) | Publication date |
|---|---|---|---|
| WO 2006002421 A2 | 05-01-2006 | AU 2005258320 A1 | 05-01-2006 |
| | | AU 2010249302 A1 | 06-01-2011 |
| | | AU 2010251787 A1 | 06-01-2011 |
| | | AU 2010251789 A1 | 06-01-2011 |
| | | BR PI0511321 A | 31-07-2007 |
| | | CA 2571949 A1 | 05-01-2006 |
| | | CA 2810655 A1 | 05-01-2006 |
| | | CA 2881078 A1 | 05-01-2006 |
| | | CN 101006076 A | 25-07-2007 |
| | | CN 101891680 A | 24-11-2010 |
| | | CN 101935301 A | 05-01-2011 |
| | | CN 103641765 A | 19-03-2014 |
| | | CN 104788328 A | 22-07-2015 |
| | | DK 1773816 T3 | 26-01-2015 |
| | | EP 1773816 A2 | 18-04-2007 |
| | | EP 2489659 A1 | 22-08-2012 |
| | | EP 2502902 A2 | 26-09-2012 |
| | | EP 2522659 A2 | 14-11-2012 |
| | | EP 2530075 A2 | 05-12-2012 |
| | | EP 2532650 A2 | 12-12-2012 |
| | | ES 2534606 T3 | 24-04-2015 |
| | | HK 1105970 A1 | 29-07-2011 |
| | | HK 1150602 A1 | 18-09-2015 |
| | | HK 1152707 A1 | 11-07-2014 |
| | | HK 1212672 A1 | 17-06-2016 |
| | | HR P20150277 T1 | 10-04-2015 |
| | | IL 180224 A | 31-10-2012 |
| | | IL 213158 A | 31-05-2016 |
| | | JP 4947658 B2 | 06-06-2012 |
| | | JP 5654979 B2 | 14-01-2015 |
| | | JP 5712117 B2 | 07-05-2015 |
| | | JP 5762940 B2 | 12-08-2015 |
| | | JP 2008504291 A | 14-02-2008 |
| | | JP 2012056962 A | 22-03-2012 |
| | | JP 2012056963 A | 22-03-2012 |
| | | JP 2012062319 A | 29-03-2012 |
| | | JP 2012107069 A | 07-06-2012 |
| | | JP 2014156468 A | 28-08-2014 |
| | | JP 2015172087 A | 01-10-2015 |
| | | LU 92761 I2 | 02-07-2016 |
| | | NL 300748 I1 | 12-01-2016 |
| | | NZ 552543 A | 30-09-2010 |
| | | NZ 587547 A | 28-09-2012 |
| | | NZ 587548 A | 25-05-2012 |
| | | NZ 587549 A | 26-10-2012 |
| | | NZ 587551 A | 12-01-2012 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 16 19 2470

This annex lists the patent family members relating to the patent documents cited in the above-mentioned European search report. The members are as contained in the European Patent Office EDP file on
The European Patent Office is in no way liable for these particulars which are merely given for the purpose of information.

24-01-2017

| Patent document cited in search report | Publication date | Patent family member(s) | Publication date |
|---|---|---|---|
| | | NZ 598393 A | 30-05-2014 |
| | | PT 1773816 E | 29-04-2015 |
| | | RU 2382779 C2 | 27-02-2010 |
| | | US 2006074075 A1 | 06-04-2006 |
| | | US 2008071095 A1 | 20-03-2008 |
| | | US 2009227797 A1 | 10-09-2009 |
| | | US 2009298876 A1 | 03-12-2009 |
| | | US 2013035327 A1 | 07-02-2013 |
| | | US 2013331567 A1 | 12-12-2013 |
| | | US 2014051724 A1 | 20-02-2014 |
| | | US 2014155431 A1 | 05-06-2014 |
| | | US 2014163011 A1 | 12-06-2014 |
| | | US 2014343315 A1 | 20-11-2014 |
| | | US 2015031722 A1 | 29-01-2015 |
| | | US 2015065487 A1 | 05-03-2015 |
| | | US 2016318931 A1 | 03-11-2016 |
| | | WO 2006002421 A2 | 05-01-2006 |
| | | ZA 200700601 B | 26-11-2008 |
| WO 2007079139 A2 | 12-07-2007 | AU 2006332726 A1 | 12-07-2007 |
| | | BR PI0620960 A2 | 29-11-2011 |
| | | CA 2635581 A1 | 12-07-2007 |
| | | CN 101384172 A | 11-03-2009 |
| | | EP 1993360 A2 | 26-11-2008 |
| | | HK 1125540 A1 | 03-06-2016 |
| | | JP 5409010 B2 | 05-02-2014 |
| | | JP 5734369 B2 | 17-06-2015 |
| | | JP 2009522278 A | 11-06-2009 |
| | | JP 2014012701 A | 23-01-2014 |
| | | JP 2015096539 A | 21-05-2015 |
| | | US 2011064811 A1 | 17-03-2011 |
| | | US 2012214841 A1 | 23-08-2012 |
| | | US 2013317060 A1 | 28-11-2013 |
| | | US 2014242172 A1 | 28-08-2014 |
| | | US 2016229806 A1 | 11-08-2016 |
| | | WO 2007079139 A2 | 12-07-2007 |
| WO 2009038683 A2 | 26-03-2009 | AU 2008301907 A1 | 26-03-2009 |
| | | BR PI0816345 A2 | 24-02-2015 |
| | | CA 2699292 A1 | 26-03-2009 |
| | | CN 101918366 A | 15-12-2010 |
| | | EP 2222304 A2 | 01-09-2010 |
| | | JP 2010540417 A | 24-12-2010 |
| | | NZ 583848 A | 27-07-2012 |
| | | NZ 600865 A | 31-01-2014 |
| | | NZ 620027 A | 29-05-2015 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 16 19 2470

This annex lists the patent family members relating to the patent documents cited in the above-mentioned European search report. The members are as contained in the European Patent Office EDP file on
The European Patent Office is in no way liable for these particulars which are merely given for the purpose of information.

24-01-2017

| Patent document cited in search report | Publication date | Patent family member(s) | Publication date |
|---|---|---|---|
| | | RU 2010114732 A | 20-10-2011 |
| | | US 2009099230 A1 | 16-04-2009 |
| | | US 2012165372 A1 | 28-06-2012 |
| | | US 2013109717 A1 | 02-05-2013 |
| | | WO 2009038683 A2 | 26-03-2009 |
| WO 2009023509 A2 | 19-02-2009 | US 2011177999 A1 | 21-07-2011 |
| | | WO 2009023509 A2 | 19-02-2009 |
| WO 2007056341 A1 | 18-05-2007 | AU 2006311650 A1 | 18-05-2007 |
| | | CA 2627358 A1 | 18-05-2007 |
| | | CN 101356170 A | 28-01-2009 |
| | | CN 102775396 A | 14-11-2012 |
| | | DK 1945632 T3 | 16-12-2013 |
| | | DK 2395002 T3 | 08-09-2014 |
| | | DK 2404919 T3 | 04-11-2013 |
| | | EP 1945632 A1 | 23-07-2008 |
| | | EP 2395002 A1 | 14-12-2011 |
| | | EP 2404919 A1 | 11-01-2012 |
| | | EP 2774925 A1 | 10-09-2014 |
| | | ES 2431388 T3 | 26-11-2013 |
| | | ES 2439736 T3 | 24-01-2014 |
| | | ES 2501594 T3 | 02-10-2014 |
| | | HK 1125366 A1 | 12-07-2013 |
| | | HK 1178892 A1 | 28-08-2015 |
| | | IL 191141 A | 28-11-2013 |
| | | IL 222784 A | 30-06-2016 |
| | | JP 5317184 B2 | 16-10-2013 |
| | | JP 5666525 B2 | 12-02-2015 |
| | | JP 5941095 B2 | 29-06-2016 |
| | | JP 2009514962 A | 09-04-2009 |
| | | JP 2012233011 A | 29-11-2012 |
| | | JP 2014139243 A | 31-07-2014 |
| | | JP 2015134835 A | 27-07-2015 |
| | | KR 20080066086 A | 15-07-2008 |
| | | KR 20130034062 A | 04-04-2013 |
| | | KR 20130042034 A | 25-04-2013 |
| | | KR 20140009566 A | 22-01-2014 |
| | | KR 20150041174 A | 15-04-2015 |
| | | KR 20160067985 A | 14-06-2016 |
| | | LU 93073 I2 | 18-07-2016 |
| | | NZ 567892 A | 24-12-2010 |
| | | PT 1945632 E | 24-12-2013 |
| | | PT 2395002 E | 16-09-2014 |
| | | PT 2404919 E | 22-10-2013 |
| | | RU 2012123372 A | 10-12-2013 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 16 19 2470

This annex lists the patent family members relating to the patent documents cited in the above-mentioned European search report. The members are as contained in the European Patent Office EDP file on
The European Patent Office is in no way liable for these particulars which are merely given for the purpose of information.

24-01-2017

| Patent document cited in search report | Publication date | Patent family member(s) | Publication date |
|---|---|---|---|
| | | SI 1945632 T1 | 31-03-2014 |
| | | SI 2395002 T1 | 30-10-2014 |
| | | SI 2404919 T1 | 31-12-2013 |
| | | US 2008019915 A1 | 24-01-2008 |
| | | US 2008113985 A1 | 15-05-2008 |
| | | US 2008306062 A1 | 11-12-2008 |
| | | US 2010087435 A1 | 08-04-2010 |
| | | US 2010210638 A1 | 19-08-2010 |
| | | US 2011172229 A1 | 14-07-2011 |
| | | US 2011312958 A1 | 22-12-2011 |
| | | US 2012322798 A1 | 20-12-2012 |
| | | US 2013237568 A1 | 12-09-2013 |
| | | US 2013237569 A1 | 12-09-2013 |
| | | US 2013245010 A1 | 19-09-2013 |
| | | US 2013245011 A1 | 19-09-2013 |
| | | US 2016143898 A1 | 26-05-2016 |
| | | WO 2007056341 A1 | 18-05-2007 |
| | | ZA 200803887 B | 30-12-2009 |
| WO 2009073757 A1 | 11-06-2009 | AU 2008333845 A1 | 11-06-2009 |
| | | BR PI0821039 A2 | 16-06-2015 |
| | | CA 2706920 A1 | 11-06-2009 |
| | | CN 101910156 A | 08-12-2010 |
| | | CN 103626744 A | 12-03-2014 |
| | | EA 201070698 A1 | 28-02-2011 |
| | | EP 2225230 A1 | 08-09-2010 |
| | | HK 1146819 A1 | 05-09-2014 |
| | | IL 206203 A | 31-07-2016 |
| | | JP 2011506330 A | 03-03-2011 |
| | | JP 2013253111 A | 19-12-2013 |
| | | JP 2014088447 A | 15-05-2014 |
| | | JP 2015145426 A | 13-08-2015 |
| | | KR 20100101130 A | 16-09-2010 |
| | | KR 20150063170 A | 08-06-2015 |
| | | KR 20160040745 A | 14-04-2016 |
| | | NZ 585880 A | 31-08-2012 |
| | | NZ 599889 A | 27-09-2013 |
| | | NZ 614151 A | 24-04-2015 |
| | | NZ 702159 A | 31-03-2016 |
| | | SG 186638 A1 | 30-01-2013 |
| | | UA 102534 C2 | 25-07-2013 |
| | | US 2009170905 A1 | 02-07-2009 |
| | | US 2012277268 A1 | 01-11-2012 |
| | | US 2014011846 A1 | 09-01-2014 |
| | | US 2014371275 A1 | 18-12-2014 |
| | | US 2016052916 A1 | 25-02-2016 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 16 19 2470

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2017

| Patent document cited in search report | Publication date | Patent family member(s) | Publication date |
|---|---|---|---|
| | | US 2016221995 A1 | 04-08-2016 |
| | | WO 2009073757 A1 | 11-06-2009 |
| | | ZA 201003623 B | 31-08-2011 |
| WO 2009076141 A2 | 18-06-2009 | AU 2008335439 A1 | 18-06-2009 |
| | | CA 2708146 A1 | 18-06-2009 |
| | | CN 101998854 A | 30-03-2011 |
| | | EA 201070699 A1 | 28-02-2011 |
| | | EP 2237768 A2 | 13-10-2010 |
| | | JP 2011506331 A | 03-03-2011 |
| | | KR 20100098545 A | 07-09-2010 |
| | | NZ 586272 A | 25-05-2012 |
| | | UA 95199 C2 | 11-07-2011 |
| | | US 2009176839 A1 | 09-07-2009 |
| | | WO 2009076141 A2 | 18-06-2009 |
| | | ZA 201004124 B | 31-08-2011 |
| WO 2010037066 A2 | 01-04-2010 | AR 073709 A1 | 24-11-2010 |
| | | AU 2009296271 A1 | 01-04-2010 |
| | | CA 2736545 A1 | 01-04-2010 |
| | | CN 102164587 A | 24-08-2011 |
| | | EP 2349223 A2 | 03-08-2011 |
| | | JP 2012504143 A | 16-02-2012 |
| | | KR 20110063578 A | 10-06-2011 |
| | | RU 2011117177 A | 10-11-2012 |
| | | US 2010087490 A1 | 08-04-2010 |
| | | US 2014206720 A1 | 24-07-2014 |
| | | US 2016039800 A1 | 11-02-2016 |
| | | WO 2010037066 A2 | 01-04-2010 |
| WO 2007117715 A2 | 18-10-2007 | AU 2007235260 A1 | 18-10-2007 |
| | | BR PI0710965 A2 | 31-01-2012 |
| | | CA 2648719 A1 | 18-10-2007 |
| | | CA 2869945 A1 | 18-10-2007 |
| | | CN 101460489 A | 17-06-2009 |
| | | CN 103254177 A | 21-08-2013 |
| | | DK 2007756 T3 | 05-10-2015 |
| | | DK 2674428 T3 | 18-04-2016 |
| | | EP 2007756 A2 | 31-12-2008 |
| | | EP 2674428 A1 | 18-12-2013 |
| | | EP 3091011 A1 | 09-11-2016 |
| | | ES 2554353 T3 | 18-12-2015 |
| | | ES 2580803 T3 | 26-08-2016 |
| | | IL 194576 A | 29-12-2016 |
| | | JP 5420395 B2 | 19-02-2014 |
| | | JP 5827297 B2 | 02-12-2015 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 16 19 2470

This annex lists the patent family members relating to the patent documents cited in the above-mentioned European search report. The members are as contained in the European Patent Office EDP file on
The European Patent Office is in no way liable for these particulars which are merely given for the purpose of information.

24-01-2017

| Patent document cited in search report | Publication date | Patent family member(s) | Publication date |
|---|---|---|---|
| | | JP 2009533351 A | 17-09-2009 |
| | | JP 2014031384 A | 20-02-2014 |
| | | JP 2015120762 A | 02-07-2015 |
| | | NZ 571803 A | 22-12-2011 |
| | | NZ 596889 A | 28-06-2013 |
| | | NZ 611485 A | 26-09-2014 |
| | | PT 2007756 E | 02-11-2015 |
| | | RU 2008144124 A | 20-05-2010 |
| | | SI 2007756 T1 | 30-11-2015 |
| | | SI 2674428 T1 | 29-07-2016 |
| | | US 2007244159 A1 | 18-10-2007 |
| | | US 2010331344 A1 | 30-12-2010 |
| | | US 2014057906 A1 | 27-02-2014 |
| | | US 2015119441 A1 | 30-04-2015 |
| | | US 2016332997 A1 | 17-11-2016 |
| | | WO 2007117715 A2 | 18-10-2007 |
| | | ZA 200809290 B | 28-10-2009 |
| US 2009131492 A1 | 21-05-2009 | US 2009131492 A1 | 21-05-2009 |
| | | US 2010113555 A1 | 06-05-2010 |
| | | US 2011060024 A1 | 10-03-2011 |
| | | US 2011071206 A1 | 24-03-2011 |
| | | US 2013178471 A1 | 11-07-2013 |
| | | US 2014080826 A1 | 20-03-2014 |
| | | US 2015094304 A1 | 02-04-2015 |
| | | US 2015174098 A1 | 25-06-2015 |
| WO 2010053471 A1 | 14-05-2010 | AU 2008363828 A1 | 14-05-2010 |
| | | BR PI0823228 A2 | 11-10-2016 |
| | | CA 2742821 A1 | 14-05-2010 |
| | | CN 102272127 A | 07-12-2011 |
| | | DK 2365972 T3 | 19-01-2015 |
| | | EP 2365972 A1 | 21-09-2011 |
| | | EP 2940016 A1 | 04-11-2015 |
| | | ES 2532753 T3 | 31-03-2015 |
| | | HK 1162477 A1 | 26-06-2015 |
| | | HK 1216526 A1 | 18-11-2016 |
| | | HR P20150288 T1 | 24-04-2015 |
| | | IL 212737 A | 31-08-2014 |
| | | IL 233837 A | 30-07-2015 |
| | | JP 5600322 B2 | 01-10-2014 |
| | | JP 5728611 B2 | 03-06-2015 |
| | | JP 5905620 B2 | 20-04-2016 |
| | | JP 2012507580 A | 29-03-2012 |
| | | JP 2014208719 A | 06-11-2014 |
| | | JP 2015127345 A | 09-07-2015 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 16 19 2470

This annex lists the patent family members relating to the patent documents cited in the above-mentioned European search report. The members are as contained in the European Patent Office EDP file on
The European Patent Office is in no way liable for these particulars which are merely given for the purpose of information.

24-01-2017

| Patent document cited in search report | Publication date | Patent family member(s) | Publication date |
|---|---|---|---|
| | | JP 2016147887 A | 18-08-2016 |
| | | KR 20110082188 A | 18-07-2011 |
| | | KR 20150036819 A | 07-04-2015 |
| | | KR 20160013251 A | 03-02-2016 |
| | | NZ 592694 A | 31-05-2013 |
| | | NZ 609962 A | 28-11-2014 |
| | | PT 2365972 E | 31-03-2015 |
| | | RU 2011122461 A | 20-12-2012 |
| | | RU 2013157877 A | 27-06-2015 |
| | | SI 2365972 T1 | 30-04-2015 |
| | | WO 2010053471 A1 | 14-05-2010 |
| | | ZA 201103883 B | 29-02-2012 |
| WO 2010054138 A2 | 14-05-2010 | AU 2009313409 A1 | 14-05-2010 |
| | | BR PI0921234 A2 | 23-02-2016 |
| | | CA 2742980 A1 | 14-05-2010 |
| | | CA 2948104 A1 | 14-05-2010 |
| | | CN 102272128 A | 07-12-2011 |
| | | CN 105693701 A | 22-06-2016 |
| | | EP 2362874 A2 | 07-09-2011 |
| | | IL 212727 A | 31-08-2014 |
| | | IL 233996 A | 24-09-2015 |
| | | JP 2012508246 A | 05-04-2012 |
| | | JP 2015061860 A | 02-04-2015 |
| | | KR 20110089170 A | 04-08-2011 |
| | | NZ 592693 A | 31-05-2013 |
| | | NZ 610972 A | 28-11-2014 |
| | | RU 2011122646 A | 20-12-2012 |
| | | RU 2014139599 A | 20-04-2016 |
| | | SG 10201501168R A | 29-04-2015 |
| | | UA 104876 C2 | 25-03-2014 |
| | | UA 110192 C2 | 10-12-2015 |
| | | WO 2010054138 A2 | 14-05-2010 |
| | | ZA 201103856 B | 29-02-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61327078 B
- US 61327091 B
- US 61329510 B
- WO 2006002421 A
- WO 2005075435 A
- US 20110065928 A1
- US 20100184739 A
- US 20100267768 A
- US 20110064811 A
- US 20090105272 A
- US 20090246820 A
- US 20090099230 A
- US 7776905 B
- US 7645789 B
- US 7495103 B
- US 7553855 B
- US 20100074949 A
- US 20100256184 A
- US 7741321 B
- US 7659268 B
- US 20080306062 A1
- US 20090170905 A1
- US 20090176839 A
- US 20100087490 A
- US 20040006237 A
- US 20090131492 A
- US 20040105820 A
- US 20030144257 A
- US 6099562 A
- US 5886026 A
- US 5304121 A


### Non-patent literature cited in the description


- **GREGORY, R. J. et al.** *Nature,* 1990, vol. 347, 382-386
- **RICH, D. P. et al.** *Nature,* 1990, vol. 347, 358-362
- **RIORDAN, J. R. et al.** *Science,* 1989, vol. 245, 1066-1073
- **CUTTING, G. R. et al.** *Nature,* 1990, vol. 346, 366-369
- **DEAN, M. et al.** *Cell,* 1990, vol. 61 (863), 870
- **KEREM, B-S. et al.** *Science,* 1989, vol. 245, 1073-1080
- **KEREM, B-S et al.** *Proc. Natl. Acad. Sci. USA,* 1990, vol. 87, 8447-8451
- **QUINTON, P. M.** *FASEB J.,* 1990, vol. 4, 2709-2727
- **DALEMANS et al.** *Nature Lond.,* 1991, vol. 354, 526-528
- **PASYK ; FOSKETT.** *J. Cell. Biochem.,* 1995, vol. 270, 12347-50
- **HWANG, T. C. et al.** *J. Gen. Physiol.,* 1998, vol. 111 (3), 477-90
- **GREENE ; WUTS.** Protective Groups in Organic Synthesis. John Wiley & Sons, 1999
- **P.J.KOCIENSKI.** Protecting Groups. Thieme, 1994
- **SHELDRICK, G.M.** *Acta Cryst.,* 2008, vol. A64, 112-122
- **SHELDRICK,G.M.** *Acta Cryst.,* 2008, vol. A64, 112-122
- **PERRY.** Chemical Engineering Handbook. McGraw-Hill book co, 1984
- **MARSHALL.** Atomization and Spray-Drying. *Chem. Eng. Prog. Monogr.,* 1954, vol. 50
- *J. Pharmaceutical Sciences,* 1977, vol. 66, 1-19
- **E. W. MARTIN.** Remington's Pharmaceutical Sciences. Mack Publishing Co, 1980
- **REMINGTON.** The Science and Practice of Pharmacy. Lippincott Williams & Wilkins, 2003
- **ANSEL et al.** Pharmaceutical Dosage Forms And Drug Delivery Systems. Lippincott Williams & Wilkins, 1999
- The Handbook of Pharmaceutical Excipients. American Pharmaceuticals Association, 2003
- **GIBSON.** Pharmaceutical Preformulation And Formulation. CRC Press, 2001
- Dissolution. United States Pharmacopoeia. United States Pharmacopeial Convention, Inc, 2005, vol. 29
- **R. CHOO-KANG ; PAMELA L. ; ZEITLIN.** Type I, II, III, IV, and V cystic fibrosis Transmembrane Conductance Regulator Defects and Opportunities of Therapy. *Current Opinion in Pulmonary Medicine,* 2000, vol. 6, 521-529
- **GALIETTA, L.J.V. ; LANTERO, S. ; GAZZOLO, A. ; SACCO, O. ; ROMANO, L. ; ROSSI, G.A. ; ZEGARRA-MORAN, O.** *In Vitro Cell. Dev. Biol.,* 1998, vol. 34, 478-481

- **RAE, J. ; COOPER, K. ; GATES, P. ; WATSKY, M.** *J. Neurosci. Methods,* 1991, vol. 37, 15-26
- **DALEMANS, W. ; BARBRY, P. ; CHAMPIGNY, G. ; JALLAT, S. ; DOTT, K. ; DREYER, D. ; CRYSTAL, R.G. ; PAVIRANI, A. ; LECOCQ, J-P. ; LAZDUNSKI, M.** *Nature,* 1991, vol. 354, 526-528
- **GONZALEZ, J. E. ; R. Y. TSIEN.** Voltage sensing by fluorescence resonance energy transfer in single cells. *Biophys J,* 1995, vol. 69 (4), 1272-80
- **GONZALEZ, J. E. ; R. Y. TSIEN.** Improved indicators of cell membrane potential that use fluorescence resonance energy transfer. *Chem Biol,* 1997, vol. 4 (4), 269-77
- **GONZALEZ, J. E. ; K. OADES et al.** Cell-based assays and instrumentation for screening ion-channel targets. *Drug Discov Today,* 1999, vol. 4 (9), 431-439